(19) Europäisches Patentamt
European Patent Office
Office européen des brevets

(11) EP 3 433 280 B1

(12) **EUROPEAN PATENT SPECIFICATION**

(45) Date of publication and mention of the grant of the patent:
**19.04.2023 Bulletin 2023/16**

(21) Application number: **17711223.2**

(22) Date of filing: **20.03.2017**

(51) International Patent Classification (IPC):
***C07K 16/46*** *(2006.01)* ***C07K 16/42*** *(2006.01)*
***C07K 16/28*** *(2006.01)* ***C07K 16/30*** *(2006.01)*
***C07K 16/32*** *(2006.01)*

(52) Cooperative Patent Classification (CPC):
**C07K 16/2809; A61P 35/00; C07K 16/28; C07K 16/2863; C07K 16/30; C07K 16/32; C07K 16/4258; C07K 16/468;** C07K 2317/31; C07K 2317/55; C07K 2317/622; C07K 2317/64; C07K 2317/73; C07K 2317/76; C07K 2319/50

(86) International application number:
**PCT/EP2017/056556**

(87) International publication number:
**WO 2017/162587 (28.09.2017 Gazette 2017/39)**

(54) **PROTEASE-ACTIVATED T CELL BISPECIFIC MOLECULES**

PROTEASE-AKTIVIERTE T-ZELL-BISPEZIFISCHE MOLEKÜLE

MOLÉCULES BISPÉCIFIQUES DE CELLULES T ACTIVÉES PAR UNE PROTÉASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **22.03.2016 EP 16161740**
**13.12.2016 US 201662433327 P**

(43) Date of publication of application:
**30.01.2019 Bulletin 2019/05**

(73) Proprietor: **F. Hoffmann-La Roche AG**
**4070 Basel (CH)**

(72) Inventors:
- **BRUENKER, Peter**
**8335 Hittnau (CH)**
- **CROASDALE-WOOD, Rebecca**
**Preston**
**Lancashire PR3 3AX (GB)**
- **KLEIN, Christian**
**8906 Bonstetten (CH)**
- **SCHANZER, Juergen Michael**
**81667 München (DE)**
- **STUBENRAUCH, Kay-Gunnar**
**82377 Penzberg (DE)**
- **UMANA, Pablo**
**8832 Wollerau (CH)**
- **GEIGER, Martina**
**8912 Obfelden (CH)**
- **SULLIVAN, Eric**
**Pleasanton**
**California 94588 (US)**
- **PATEL, Jigar**
**Pleasanton**
**California 94588 (US)**

(74) Representative: **Brodbeck, Michel et al**
**F. Hoffmann-La Roche AG**
**Patent Department**
**Grenzacherstrasse 124**
**4070 Basel (CH)**

(56) References cited:
**EP-A1- 2 982 694**
**WO-A1-2014/151910**
**WO-A1-2015/001085**
**WO-A1-2015/013671**
**WO-A1-2015/048272**
**WO-A1-2016/079081**
**WO-A1-2016/146894**
**WO-A2-2006/099141**
**WO-A2-2014/004549**
**WO-A2-2014/144722**
**WO-A2-2016/014974**
**WO-A2-2016/077505**
**US-A1- 2014 308 285**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CARRENO M ET AL: "First step toward the murine idiotypic network generated by OKT3", HUMAN IMMUNOLOGY, NEW YORK, NY, US, vol. 32, 11 October 1991 (1991-10-11), page 12, XP023715580, ISSN: 0198-8859, DOI: 10.1016/0198-8859(91)90133-T [retrieved on 1991-10-11]**


Remarks:

The complete document including Reference Table(s) and the Sequence Listing(s) can be downloaded from the EPO website

## Description

### Field of the Invention

The present invention generally relates to novel protease-activatable antigen-binding molecules that comprise an anti-idiotype-binding moiety which reversibly masks antigen binding of the molecule. Specifically, the invention relates to T cell binding molecules having an anti-idiotype-binding moiety that masks the CD3-binding moiety until cleaved by a protease. This allows the CD3-binding moiety to be inaccessible or "masked" until it is in proximity to a target tissue, such as a tumor, e.g., tumor-infiltrating T cells. In addition, the present invention relates to polynucleotides encoding such protease-activated T cell binding molecules and idiotype-specific polypeptides, and vectors and host cells comprising such polynucleotides. The invention further relates to methods for producing the protease-activated T cell binding molecules of the invention, and to methods of using the same, *e.g.,* in the treatment of disease.

### Background

The selective destruction of an individual target cell or a specific target cell type is often desirable in a variety of clinical settings. For example, it is a primary goal of cancer therapy to specifically destroy tumor cells, while leaving healthy cells and tissues intact and undamaged.

An attractive way of achieving this is by inducing an immune response against the tumor, to make immune effector cells such as natural killer (NK) cells or cytotoxic T lymphocytes (CTLs) attack and destroy tumor cells. In this regard, bispecific antibodies designed to bind with one "arm" to a surface antigen on target cells, and with the second "arm" to an activating, invariant component of the T cell receptor (TCR) complex, have become of interest in recent years. The simultaneous binding of such an antibody to both of its targets will force a temporary interaction between target cell and T cell, causing activation of any cytotoxic T cell and subsequent lysis of the target cell. Hence, the immune response is re-directed to the target cells and is independent of peptide antigen presentation by the target cell or the specificity of the T cell as would be relevant for normal MHC-restricted activation of CTLs.

In this context it is crucial that CTLs are activated only when in close proximity to a target cell, i.e., the immunological synapse is mimicked. Particularly desirable are T cell activating bispecific molecules that do not require lymphocyte preconditioning or co-stimulation in order to elicit efficient lysis of target cells. Several bispecific antibody formats have been developed and their suitability for T cell mediated immunotherapy investigated. These include BiTE (bispecific T cell engager) molecules (Nagorsen and Bäuerle, Exp Cell Res 317, 1255-1260 (2011)), diabodies (Holliger et al., Prot Eng 9, 299-305 (1996)) and derivatives thereof, such as tandem diabodies (Kipriyanov et al., J Mol Biol 293, 41-66 (1999)), DART (dual affinity retargeting) molecules, (Moore et al., Blood 117, 4542-51 (2011)), and triomabs (Seimetz et al., Cancer Treat Rev 36, 458-467 (2010)). WO2016014974 describes activatable anti-CD3 antibodies and methods of using the same. Carreno et al 1991 Hum Immunol 33(4):249-58 (1992) describe cross-species reactivity of the anti-idiotype anti-OKT3 cascade.

The task of generating bispecific molecules suitable for treatment provides several technical challenges related to efficacy, toxicity, applicability and produceability that have to be met. In instances where the bispecific molecule targets an antigen on a target cell, e.g., a cancer cell, that is also expressed in non-target tissue, toxicity can occur. There is thus a need for efficacious T cell activating bispecific molecules that unleash full T cell activation in the presence of target cells but not in the presence of normal cells or tissue.

### SUMMARY OF THE INVENTION

The invention generally relates to T cell activating bispecific molecules that are activated selectively in the presence of a target cell.

In one aspect, the invention relates to a protease-activatable T cell activating bispecific molecule comprising

(a) a first antigen binding moiety capable of specific binding to CD3, wherein the first antigen binding moiety is an antibody or fragment thereof;
(b) a second antigen binding moiety capable of specific binding to a target cell antigen, wherein the second antigen binding moiety is an antibody or fragment thereof; and
(c) a masking moiety covalently attached to the T cell bispecific binding molecule through a protease-cleavable linker, wherein the masking moiety is capable of specific binding to the idiotype of the first or the second antigen binding moiety thereby reversibly concealing the first or second antigen binding moiety, wherein the masking moiety is an anti-idiotypic scFv.

In one embodiment, the masking moiety of the protease-activatable T cell activating bispecific molecule is

covalently attached to the first antigen binding moiety. In one embodiment the masking moiety is covalently attached to the heavy chain variable region of the first antigen binding moiety. In one embodiment the masking moiety is covalently attached to the light chain variable region of the first antigen binding moiety. In one embodiment the masking moiety is an anti-idiotype scFv.

In one embodiment the protease-activatable T cell activating bispecific molecule comprises a second masking moiety reversibly concealing the second antigen binding moiety.

In one embodiment the protease capable of cleaving the protease-cleavable linker is expressed by the target cell. In one embodiment the second antigen binding moiety is a crossover Fab molecule wherein either the variable or the constant regions of the Fab light chain and the Fab heavy chain are exchanged. In one embodiment the second antigen binding moiety is a crossover Fab molecule wherein the constant regions of the Fab light chain and the Fab heavy chain are exchanged. In one embodiment the first antigen binding moiety is a conventional Fab molecule. In one embodiment the protease-activatable T cell activating bispecific molecule comprises not more than one antigen binding moiety capable of specific binding to CD3. In one embodiment the protease-activatable T cell activating bispecific molecule comprises a third antigen binding moiety which is a Fab molecule capable of specific binding to a target cell antigen. In one particular embodiment the third antigen binding moiety is identical to the second antigen binding moiety. In one particular embodiment the third antigen binding moiety is not identical to the second antigen binding moiety. In one embodiment the second antigen binding moiety is capable of specific binding to FolR1 or HER1. In one embodiment the second antigen binding moiety is capable of specific binding to FolR1, HER1 or Mesothelin. In one embodiment the second antigen binding moiety is capable of specific binding to FolR1, HER1, HER2 or Mesothelin.

In one embodiment the first and the second antigen binding moiety are fused to each other, optionally via a peptide linker. In one particular embodiment the second antigen binding moiety is fused at the C-terminus of the Fab heavy chain to the N-terminus of the Fab heavy chain of the first antigen binding moiety. In one particular embodiment the first antigen binding moiety is fused at the C-terminus of the Fab heavy chain to the N-terminus of the Fab heavy chain of the second antigen binding moiety. In one particular embodiment the Fab light chain of the first antigen binding moiety and the Fab light chain of the second antigen binding moiety are fused to each other, optionally via a peptide linker.

In one embodiment the protease-activatable T cell activating bispecific molecule additionally comprises an Fc domain composed of a first and a second subunit capable of stable association.

In one embodiment the Fc domain is an IgG, specifically an $IgG_1$ or $IgG_4$, Fc domain. In one embodiment the Fc domain is a human Fc domain. In one embodiment the Fc domain exhibits reduced binding affinity to an Fc receptor and/or reduced effector function, as compared to a native $IgG_1$ Fc domain. In one embodiment the Fc domain comprises one or more amino acid substitution that reduces binding to an Fc receptor and/or effector function. In one particular embodiment the one or more amino acid substitution is at one or more position selected from the group of L234, L235, and P329 (Kabat numbering). In one particular embodiment each subunit of the Fc domain comprises three amino acid substitutions that reduce binding to an activating Fc receptor and/or effector function wherein said amino acid substitutions are L234A, L235A and P329G. In one particular embodiment the Fc receptor is an Fcy receptor. In one embodiment the effector function is antibody-dependent cell-mediated cytotoxicity (ADCC). In one embodiment, the target cell is a human cell.

In one embodiment the masking moiety comprises a heavy chain variable region comprising at least one of:

(a) a heavy chain complementarity determining region (CDR H) 1 amino acid sequence of DYSIH (SEQ ID NO:20);
(b) a CDR H2 amino acid sequence of WINTETGEPAYADDFKG (SEQ ID NO:21);
and
(c) a CDR H3 amino acid sequence of PYDYDVLDY (SEQ ID NO:22).

In one embodiment the masking moiety comprises a light chain variable region comprising at least one of:

(a) a light chain (CDR L)1 amino acid sequence of RASKSVSTSNYSYIH (SEQ ID NO:23);
(b) a CDR L2 amino acid sequence of YVSYLES (SEQ ID NO:24); and
(c) a CDR L3 amino acid sequence of QHSREFPWT (SEQ ID NO:25).

In one embodiment the masking moiety comprises a heavy chain variable region comprising:

(a) a heavy chain complementarity determining region (CDR H) 1 amino acid sequence of DYSIH (SEQ ID NO:20);
(b) a CDR H2 amino acid sequence of WINTETGEPAYADDFKG (SEQ ID NO:21);
(c) a CDR H3 amino acid sequence of PYDYDVLDY (SEQ ID NO:22); and a light chain variable region comprising:

(d) a light chain (CDR L)1 amino acid sequence of RASKSVSTSNYSYIH (SEQ ID NO:23);
(e) a CDR L2 amino acid sequence of YVSYLES (SEQ ID NO:24); and

(f) a CDR L3 amino acid sequence of QHSREFPWT (SEQ ID NO:25).

In one embodiment the masking moiety comprises a heavy chain variable region comprising at least one of:

(a) a heavy chain complementarity determining region (CDR H) 1 amino acid sequence of SYGVS (SEQ ID NO:26);
(b) a CDR H2 amino acid sequence of IIWGDGSTNYHSALIS (SEQ ID NO:27);
and
(c) a CDR H3 amino acid sequence of GITTVVDDYYAMDY (SEQ ID NO:28).

In one embodiment the masking moiety comprises a light chain variable region comprising at least one of:

(a) a light chain (CDR L)1 amino acid sequence of RASENIDSYLA (SEQ ID NO:29);
(b) a CDR L2 amino acid sequence of AATFLAD (SEQ ID NO:30); and
(c) a CDR L3 amino acid sequence of QHYYSTPYT (SEQ ID NO:31).

In one embodiment the masking moiety comprises a heavy chain variable region comprising:

(a) a heavy chain complementarity determining region (CDR H) 1 amino acid sequence of SYGVS (SEQ ID NO:26);
(b) a CDR H2 amino acid sequence of IIWGDGSTNYHSALIS (SEQ ID NO:27);
(c) a CDR H3 amino acid sequence of GITTVVDDYYAMDY (SEQ ID NO:28); and a light chain variable region comprising:

(d) a light chain (CDR L)1 amino acid sequence of RASENIDSYLA (SEQ ID NO:29);
(e) a CDR L2 amino acid sequence of AATFLAD (SEQ ID NO:30); and
(f) a CDR L3 amino acid sequence of QHYYSTPYT (SEQ ID NO:31).

In one embodiment the protease cleavable linker comprises at least one protease recognition sequence. In one embodiment the protease cleavable linker comprises a protease recognition sequence. In one embodiment the protease recognition sequence is selected from the group consisting of:

(a) RQARVVNG (SEQ ID NO:36);
(b) VHMPLGFLGPGRSRGSFP (SEQ ID NO:37);
(c) RQARVVNGXXXXXVPLSLYSG (SEQ ID NO:38); and
(d) RQARVVNGVPLSLYSG (SEQ ID NO:39)
(e) PLGLWSQ (SEQ ID NO:40), wherein X is any amino acid.

In one embodiment the protease cleavable linker comprises a protease recognition sequence. In one embodiment the protease recognition sequence is selected from the group consisting of:

(a) RQARVVNG (SEQ ID NO:36);
(b) VHMPLGFLGPGRSRGSFP (SEQ ID NO:37);
(c) RQARVVNGXXXXXVPLSLYSG (SEQ ID NO:38);
(d) RQARVVNGVPLSLYSG (SEQ ID NO:39);
(e) PLGLWSQ (SEQ ID NO:40);
(f) VHMPLGFLGPRQARVVNG (SEQ ID NO:97);
(g) FVGGTG (SEQ ID NO:98);
(h) KKAAPVNG (SEQ ID NO:99);
(i) PMAKKVNG (SEQ ID NO:100);
(j) QARAKVNG (SEQ ID NO:101);
(k) VHMPLGFLGP (SEQ ID NO:102);
(l) QARAK (SEQ ID NO:103);
(m) VHMPLGFLGPPMAKK (SEQ ID NO:104);
(n) KKAAP (SEQ ID NO:105); and
(o) PMAKK (SEQ ID NO:106), wherein X is any amino acid.

In one embodiment the protease capable of cleaving the protease-cleavable linker is selected from the group consisting of metalloproteinase, e.g., matrix metalloproteinase (MMP) 1-28 and A Disintegrin And Metalloproteinase (ADAM) 2, 7-12, 15, 17-23, 28-30 and 33, serine protease, e.g., urokinase-type plasminogen activator and Matriptase,

cysteine protease, aspartic protease, and cathepsin protease. In one specific embodiment the protease is MMP9 or MMP2. In a further specific embodiment, the protease is Matriptase. In one embodiment the protease cleavable linker comprises the protease recognition sequence RQARVVNG (SEQ ID NO:36).

In one embodiment of the protease-activatable T cell activating bispecific molecule described herein the first antigen binding moiety comprises at least one heavy chain complementarity determining region (CDR) comprising an amino acid sequence that is at least about 95%, 96%, 97%, 98%, 99% or 100% identical to an amino acid sequence selected from the group consisting of SEQ ID NO: 44, SEQ ID NO: 45 and SEQ ID NO: 46 and at least one light chain CDR selected from the group of SEQ ID NO: 17, SEQ ID NO: 18 and SEQ ID NO: 19.

In one embodiment of the protease-activatable T cell activating bispecific molecule described herein the first antigen binding moiety comprises the heavy chain complementarity determining region (CDRs) of SEQ ID NO: 44, SEQ ID NO: 45 and SEQ ID NO: 46 and the light chain CDRs of SEQ ID NO: 17, SEQ ID NO: 18 and SEQ ID NO: 19.

In one embodiment of the protease-activatable T cell activating bispecific molecule described herein the first antigen binding moiety comprises a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55.

In one embodiment of the protease-activatable T cell activating bispecific molecule described herein the first antigen binding moiety comprises a heavy chain variable region comprising the amino acid sequence of SEQ ID NO: 43 and a light chain variable region comprising the amino acid sequence of SEQ ID NO: 55.

In one embodiment of the protease-activatable T cell activating bispecific molecule described herein the second antigen binding moiety is capable of specific binding to FolR1 and comprises at least one heavy chain complementarity determining region (CDR) comprising an amino acid sequence that is at least about 95%, 96%, 97%, 98%, 99% or 100% identical to an amino acid sequence selected from the group consisting of SEQ ID NO: 14, SEQ ID NO: 15 and SEQ ID NO: 16 and at least one light chain CDR comprising an amino acid sequence that is at least about 95%, 96%, 97%, 98%, 99% or 100% identical to an amino acid sequence selected from the group consisting of SEQ ID NO: 17, SEQ ID NO: 18 and SEQ ID NO: 19.

In one embodiment of the protease-activatable T cell activating bispecific molecule described herein the second antigen binding moiety is capable of specific binding to FolR1 and comprises at least one heavy chain complementarity determining region (CDR) comprising an amino acid sequence that is at least about 95%, 96%, 97%, 98%, 99% or 100% identical to an amino acid sequence selected from the group consisting of SEQ ID NO: 151, SEQ ID NO: 152 and SEQ ID NO: 153 and at least one light chain CDR comprising an amino acid sequence that is at least about 95%, 96%, 97%, 98%, 99% or 100% identical to an amino acid sequence selected from the group consisting of SEQ ID NO: 154, SEQ ID NO: 155 and SEQ ID NO: 156.

In one embodiment of the protease-activatable T cell activating bispecific molecule described herein the second antigen binding moiety is capable of specific binding to FolR1 and comprises at least one heavy chain complementarity determining region (CDR) comprising an amino acid sequence selected from the group consisting of SEQ ID NO: 14, SEQ ID NO: 15 and SEQ ID NO: 16 and at least one light chain CDR selected from the group of SEQ ID NO: 17, SEQ ID NO: 18 and SEQ ID NO: 19.

In one embodiment of the protease-activatable T cell activating bispecific molecule described herein the second antigen binding moiety is capable of specific binding to FolR1 and comprises at least one heavy chain complementarity determining region (CDR) comprising an amino acid sequence selected from the group consisting of SEQ ID NO: 151, SEQ ID NO: 152 and SEQ ID NO: 153 and at least one light chain CDR selected from the group of SEQ ID NO: 154, SEQ ID NO: 155 and SEQ ID NO: 156.

In one embodiment of the protease-activatable T cell activating bispecific molecule described herein the second antigen binding moiety comprises a heavy chain variable region comprising an amino acid sequence that is at least about 95%, 96%, 97%, 98%, 99% or 100% identical to the amino acid sequence of SEQ ID NO: 47 and a light chain variable region comprising an amino acid sequence that is at least about 95%, 96%, 97%, 98%, 99% or 100% identical to the amino acid sequence of SEQ ID NO: 55.

In one embodiment of the protease-activatable T cell activating bispecific molecule described herein the second antigen binding moiety comprises a heavy chain variable region comprising an amino acid sequence that is at least about 95%, 96%, 97%, 98%, 99% or 100% identical to the amino acid sequence of SEQ ID NO: 157 and a light chain variable region comprising an amino acid sequence that is at least about 95%, 96%, 97%, 98%, 99% or 100% identical to the amino acid sequence of SEQ ID NO: 158.

In one embodiment of the protease-activatable T cell activating bispecific molecule described herein the second antigen binding moiety comprises a heavy chain variable region comprising the amino acid sequence of SEQ ID NO: 47 and a light chain variable region comprising the amino acid sequence of SEQ ID NO: 55.

In one embodiment of the protease-activatable T cell activating bispecific molecule described herein the second antigen binding moiety comprises a heavy chain variable region comprising the amino acid sequence of SEQ ID NO:

157 and a light chain variable region comprising the amino acid sequence of SEQ ID NO: 158.

In one embodiment of the protease-activatable T cell activating bispecific molecule described herein the second antigen binding moiety is capable of specific binding to Mesothelin and comprises at least one heavy chain complementarity determining region (CDR) comprising an amino acid sequence that is at least about 95%, 96%, 97%, 98%, 99% or 100% identical to an amino acid sequence selected from the group consisting of SEQ ID NO: 107, SEQ ID NO: 108 and SEQ ID NO: 109 and at least one light chain CDR comprising an amino acid sequence that is at least about 95%, 96%, 97%, 98%, 99% or 100% identical to an amino acid sequence selected from the group consisting of SEQ ID NO: 110, SEQ ID NO: 111 and SEQ ID NO: 112. In one embodiment of the protease-activatable T cell activating bispecific molecule described herein the second antigen binding moiety is capable of specific binding to Mesothelin and comprises at least one heavy chain complementarity determining region (CDR) comprising an amino acid sequence selected from the group consisting of SEQ ID NO: 107, SEQ ID NO: 108 and SEQ ID NO: 109 and at least one light chain CDR selected from the group of SEQ ID NO: 110, SEQ ID NO: 111 and SEQ ID NO: 112.

In one embodiment of the protease-activatable T cell activating bispecific molecule described herein the second antigen binding moiety comprises a heavy chain variable region comprising an amino acid sequence that is at least about 95%, 96%, 97%, 98%, 99% or 100% identical to the amino acid sequence of SEQ ID NO: 113 and a light chain variable region comprising an amino acid sequence that is at least about 95%, 96%, 97%, 98%, 99% or 100% identical to the amino acid sequence of SEQ ID NO: 114.

In one embodiment of the protease-activatable T cell activating bispecific molecule described herein the second antigen binding moiety comprises a heavy chain variable region comprising the amino acid sequence of SEQ ID NO: 113 and a light chain variable region comprising the amino acid sequence of SEQ ID NO: 114.

In one embodiment of the protease-activatable T cell activating bispecific molecule described herein the second antigen binding moiety is capable of specific binding to HER1 and comprises at least one heavy chain complementarity determining region (CDR) of any one of the antibodies disclosed in WO/2006/082515.

In one embodiment of the protease-activatable T cell activating bispecific molecule described herein the second antigen binding moiety is capable of specific binding to HER1 and comprises at least one heavy chain complementarity determining region (CDR) comprising an amino acid sequence that is at least about 95%, 96%, 97%, 98%, 99% or 100% identical to an amino acid sequence selected from the group consisting of SEQ ID NO: 56, SEQ ID NO: 57 and SEQ ID NO: 58 and at least one light chain CDR comprising an amino acid sequence that is at least about 95%, 96%, 97%, 98%, 99% or 100% identical to an amino acid sequence selected from the group consisting of SEQ ID NO: 59, SEQ ID NO: 60 and SEQ ID NO: 61.

In one embodiment of the protease-activatable T cell activating bispecific molecule described herein the second antigen binding moiety is capable of specific binding to HER1 and comprises at least one heavy chain complementarity determining region (CDR) selected from the group consisting of SEQ ID NO: 56, SEQ ID NO: 57 and SEQ ID NO: 58 and at least one light chain CDR selected from the group of SEQ ID NO: 59, SEQ ID NO: 60 and SEQ ID NO: 61.

In one embodiment of the protease-activatable T cell activating bispecific molecule described herein the second antigen binding moiety comprises a heavy chain comprising an amino acid sequence that is at least about 95%, 96%, 97%, 98%, 99% or 100% identical to the amino acid sequence of SEQ ID NO: 32 and a light chain comprising an amino acid sequence that is at least about 95%, 96%, 97%, 98%, 99% or 100% identical to the amino acid sequence of SEQ ID NO: 33. In one embodiment of the protease-activatable T cell activating bispecific molecule described herein the second antigen binding moiety comprises a heavy chain comprising the amino acid sequence of SEQ ID NO: 32 and a light chain comprising the amino acid sequence of SEQ ID NO: 33.

In one embodiment, the first antigen binding moiety is capable of specific binding to CD3, and comprises a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, and the second and third antigen binding moieties are capable of specific binding to HER2, wherein the second antigen binding moiety comprises a heavy chain variable region comprising an amino acid sequence that is at least about 95%, 96%, 97%, 98%, 99% or 100% identical to the amino acid sequence of SEQ ID NO: 160 and a light chain variable region comprising an amino acid sequence that is at least about 95%, 96%, 97%, 98%, 99% or 100% identical to the amino acid sequence of SEQ ID NO: 161, wherein the third antigen binding moiety comprises a heavy chain variable region comprising an amino acid sequence that is at least about 95%, 96%, 97%, 98%, 99% or 100% identical to the amino acid sequence of SEQ ID NO: 159 and a light chain variable region comprising an amino acid sequence that is at least about 95%, 96%, 97%, 98%, 99% or 100% identical to the amino acid sequence of SEQ ID NO: 161.

In one particular embodiment the protease-activatable T cell activating bispecific molecule described herein comprises

(a) a first heavy chain comprising the amino acid sequence of SEQ ID NO:2;

(b) a second heavy chain comprising the amino acid sequence of SEQ ID NO:3; and
(c) a light chain comprising the amino acid sequence of SEQ ID NO: 1.

In one particular embodiment the protease-activatable T cell activating bispecific molecule described herein comprises

(a) a first heavy chain comprising the amino acid sequence of SEQ ID NO:4;
(b) a second heavy chain comprising the amino acid sequence of SEQ ID NO:3; and
(c) a light chain comprising the amino acid sequence of SEQ ID NO:1.

In one particular embodiment the protease-activatable T cell activating bispecific molecule described herein comprises

(a) at least one heavy chain comprising the amino acid sequence of SEQ ID NO:32;
(b) at least one light chain comprising the amino acid sequence of SEQ ID NO:34.

In one particular embodiment the protease-activatable T cell activating bispecific molecule described herein comprises

(a) a first heavy chain comprising the amino acid sequence of SEQ ID NO:72;
(b) a second heavy chain comprising the amino acid sequence of SEQ ID NO:3; and
(c) a light chain comprising an amino acid sequence of SEQ ID NO:1.

In one particular embodiment the protease-activatable T cell activating bispecific molecule described herein comprises

(a) a first heavy chain comprising the amino acid sequence of SEQ ID NO:85;
(b) a second heavy chain comprising the amino acid sequence of SEQ ID NO:3; and
(c) a light chain comprising an amino acid sequence of SEQ ID NO:1.

In one particular embodiment the protease-activatable T cell activating bispecific molecule described herein comprises

(a) a first heavy chain comprising the amino acid sequence of SEQ ID NO:73;
(b) a second heavy chain comprising the amino acid sequence of SEQ ID NO:3;
(c) a first light chain comprising an amino acid sequence of SEQ ID NO: 1; and
(d) a second light chain comprising an amino acid sequence of SEQ ID NO: 74.

In one particular embodiment the protease-activatable T cell activating bispecific molecule described herein comprises

(a) a first heavy chain comprising the amino acid sequence of SEQ ID NO:77;
(b) a second heavy chain comprising the amino acid sequence of SEQ ID NO:82;
(c) a first light chain comprising an amino acid sequence of SEQ ID NO:78; and
(d) a second light chain comprising an amino acid sequence of SEQ ID NO:81.

In one particular embodiment the protease-activatable T cell activating bispecific molecule described herein comprises

(a) a first heavy chain comprising the amino acid sequence of SEQ ID NO:76;
(b) a second heavy chain comprising the amino acid sequence of SEQ ID NO:77;
(c) a first light chain comprising an amino acid sequence of SEQ ID NO:78; and
(d) a second light chain comprising an amino acid sequence of SEQ ID NO:79.

In one particular embodiment the protease-activatable T cell activating bispecific molecule described herein comprises

(a) a first heavy chain comprising the amino acid sequence of SEQ ID NO: 132;

(b) a second heavy chain comprising the amino acid sequence of SEQ ID NO: 136;
(c) a first light chain comprising an amino acid sequence of SEQ ID NO:81; and
(d) a second light chain comprising an amino acid sequence of SEQ ID NO: 133.

In one particular embodiment the protease-activatable T cell activating bispecific molecule described herein comprises

(a) a first heavy chain comprising the amino acid sequence of SEQ ID NO: 137;
(b) a second heavy chain comprising the amino acid sequence of SEQ ID NO: 139;
(c) a first light chain comprising an amino acid sequence of SEQ ID NO:81; and
(d) a second light chain comprising an amino acid sequence of SEQ ID NO: 138.

In one aspect, the invention relates to an idiotype-specific polypeptide for reversibly concealing an anti-CD3 antigen binding site of a molecule, wherein the idiotype-specific polypeptide is an anti-idiotype scFv, and wherein the idiotype-specific polypeptide is covalently attached to the molecule through a peptide linker. In one embodiment the linker is a protease-cleavable linker. In one embodiment the peptide linker comprises at least one protease recognition site. In one embodiment the protease is selected from the group consisting of metalloproteinase, e.g., matrix metalloproteinase (MMP) 1-28 and A Disintegrin And Metalloproteinase (ADAM) 2, 7-12, 15, 17-23, 28-30 and 33, serine protease, e.g., urokinase-type plasminogen activator and Matriptase, cysteine protease, aspartic protease, and cathepsin protease. In one specific embodiment the protease is MMP9 or MMP2. In a further specific embodiment, the protease is Matriptase. In one embodiment the idiotype-specific polypeptide is covalently attached to the molecule through more than one linker. In one embodiment the idiotype-specific polypeptide is covalently attached to the molecule through two linkers.

In one embodiment the molecule which comprises the anti-CD3 antigen binding site is a T-cell activating bispecific molecule. In one particular embodiment the idiotype-specific polypeptide comprises a heavy chain variable region comprising at least one of:

(a) a heavy chain complementarity determining region (CDR H) 1 amino acid sequence of DYSIH (SEQ ID NO:20);
(b) a CDR H2 amino acid sequence of WINTETGEPAYADDFKG (SEQ ID NO:21);
and
(c) a CDR H3 amino acid sequence of PYDYDVLDY (SEQ ID NO:22).

In one particular embodiment the idiotype-specific polypeptide comprises a light chain variable region comprising at least one of

(a) a light chain (CDR L)1 amino acid sequence of RASKSVSTSNYSYIH (SEQ ID NO:23);
(b) a CDR L2 amino acid sequence of YVSYLES (SEQ ID NO:24); and
(c) a CDR L3 amino acid sequence of QHSREFPWT (SEQ ID NO:25).

In one particular embodiment the idiotype-specific polypeptide comprises:

(a) a heavy chain complementarity determining region (CDR H) 1 amino acid sequence of DYSIH (SEQ ID NO:20);
(b) a CDR H2 amino acid sequence of WINTETGEPAYADDFKG (SEQ ID NO:21);
(c) a CDR H3 amino acid sequence of PYDYDVLDY (SEQ ID NO:22); and a light chain variable region comprising:
(d) a light chain (CDR L)1 amino acid sequence of RASKSVSTSNYSYIH (SEQ ID NO:23);
(e) a CDR L2 amino acid sequence of YVSYLES (SEQ ID NO:24); and
(f) a CDR L3 amino acid sequence of QHSREFPWT (SEQ ID NO:25).

In one particular embodiment the idiotype-specific polypeptide comprises a heavy chain variable region comprising at least one of:

(a) a heavy chain complementarity determining region (CDR H) 1 amino acid sequence of SYGVS (SEQ ID NO:26);
(b) a CDR H2 amino acid sequence of IIWGDGSTNYHSALIS (SEQ ID NO:27);
and
(c) a CDR H3 amino acid sequence of GITTVVDDYYAMDY (SEQ ID NO:28).

In one particular embodiment the idiotype-specific polypeptide comprises a light chain variable region comprising at least one of:

(a) a light chain (CDR L)1 amino acid sequence of RASENIDSYLA (SEQ ID NO:29);
(b) a CDR L2 amino acid sequence of AATFLAD (SEQ ID NO:30); and
(c) a CDR L3 amino acid sequence of QHYYSTPYT (SEQ ID NO:31).

In one particular embodiment the idiotype-specific polypeptide comprises a heavy chain variable region comprising:

(a) a heavy chain complementarity determining region (CDR H) 1 amino acid sequence of SYGVS (SEQ ID NO:26);
(b) a CDR H2 amino acid sequence of IIWGDGSTNYHSALIS (SEQ ID NO:27);
(c) a CDR H3 amino acid sequence of GITTVVDDYYAMDY (SEQ ID NO:28); and a light chain variable region comprising:
(d) a light chain (CDR L)1 amino acid sequence of RASENIDSYLA (SEQ ID NO:29);
(e) a CDR L2 amino acid sequence of AATFLAD (SEQ ID NO:30); and
(f) a CDR L3 amino acid sequence of QHYYSTPYT (SEQ ID NO:31).

According to another aspect of the invention, an isolated polynucleotide encoding a protease-activatable T cell activating bispecific molecule of the invention. The invention further provides an expression vector comprising the isolated polynucleotide of the invention, and a host cell comprising the isolated polynucleotide or the expression vector of the invention. In some embodiments the host cell is a eukaryotic cell, particularly a mammalian cell.

In another aspect is provided a method of producing the protease-activated T cell molecule of the invention, comprising the steps of a) culturing the host cell of the invention under conditions suitable for the expression of the protease-activated T cell molecule and b) recovering the protease-activated T cell molecule.

In another aspect there is provided for reference only a method of producing the idiotype-specific polypeptide as described herein above, comprising the steps of a) culturing the host cell of the invention under conditions suitable for the expression of the protease-activated T cell molecule and b) recovering the idiotype-specific polypeptide.

The invention further provides a pharmaceutical composition comprising the protease-activatable T cell activating bispecific molecule of the invention and a pharmaceutically acceptable carrier. Also encompassed are methods of using the protease-activated T cell molecule and pharmaceutical composition as herein before described. In one aspect the invention provides a protease-activated T cell molecule or a pharmaceutical composition of the invention for use as a medicament. In one aspect is provided a protease-activated T cell molecule or a pharmaceutical composition according to the invention for use in the treatment of a disease in an individual in need thereof. In a specific embodiment the disease is cancer.

In a specific embodiment the disease is cancer. In any of the above embodiments the individual preferably is a mammal, particularly a human.

In another aspect the invention also provides a composition comprising a protease-activatable T cell activating bispecific molecule described herein and a pharmaceutically acceptable carrier.

In another aspect there is provided for reference only a composition comprising an idiotype-specific polypeptide as described herein above and a pharmaceutically acceptable carrier.

In another aspect provided is a protease-activatable T cell activating bispecific molecule as described herein above, or the composition described herein above, for use as a medicament.

In another aspect the invention also provides a protease-activatable T cell activating bispecific molecule as described herein above for use in the treatment of a disease in an individual in need thereof. In one embodiment, the disease is a proliferative disorder, particularly cancer.

In another aspect the invention also provides an anti-idiotype CD3 antibody or antigen-binding fragment thereof specific for an idiotype of an anti-CD3 antigen-binding molecule, wherein the anti-idiotype CD3 antibody or fragment thereof when bound to the anti-CD3 antigen-binding molecule specifically blocks binding of the anti-CD3 antigen-binding molecule to CD3, wherein the anti-idiotype CD3 antibody or antigen-binding fragment thereof is reversibly associated with the anti-CD3 antigen-binding molecule through a peptide linker comprising a protease recognition site. In one embodiment, the CD3 is a mouse, monkey or human CD3.

## SHORT DESCRIPTION OF THE FIGURES

**Figures 1A-E** depict schematics of different CD3 binders with masking moieties. FIG. 1A: 7859 anti-ID CH2527 scFv 4.15.64 MK062 Matriptase site CD3 Fc. FIG. 1B: 7860 anti-ID CH2527 scFv 4.32.63 MK062 Matriptase site CD3 Fc. FIG. 1C: 7857 anti-ID CH2527 scFv 4.15.64 non-cleavable linker CD3 Fc. FIG. 1D: ID 7858 anti-ID CH2527 scFv 4.32.63 non-cleavable linker CD3 Fc. FIG. 1E: 7861 monovalent CD3 Fc.

**Figure 2** shows a table summarizing the affinities of the anti-idiotypic masking moieties to the CD3 binder (CH2527).

**Figure 3A-D** shows Capillary Electrophoresis-SDS analysis of the molecules depicted in Figures 1A and B. FIGs. 3A-B Capillary Electrophoresis-SDS analysis of the molecule depicted in FIG. 1A under non reducing (FIG. 3A) and reducing conditions (FIG. 3B). Comparison of the untreated (I) and treated molecule (III) shows complete cleavage of the anti-ID scFv after rhMatriptase/ST14 treatment for 48 h at 37°C. One sample (II) was untreated but incubated at 37°C for 48 h. FIGs. 3C-D Capillary Electrophoresis-SDS analysis of the molecule depicted in FIG. 1B under non-reducing (FIG. 3C) and reducing conditions (FIG. 3D). Comparison of the untreated (I) and treated molecule (III) shows complete cleavage of the anti-ID scFv after rhMatriptase/ST14 treatment for 48 h at 37°C. One sample (II) was untreated but incubated at 37°C for 48 h.

**Figure 4A-C** show the effect of anti-idiotypic masking of CD3 binding. FIGs. 4A and B depict results of Jurkat NFAT reporter assays to show the masking effect of anti-idiotypic CD3 scFv 4.15.64 (FIG. 4A) or anti-idiotypic CD3 scFv 4.32.63 (FIG. 4B). Monovalent CD3 IgGs were crosslinked via an anti-human Fc antibody (coated on assay plate) before Jurkat NFAT (acute lymphatic leukemia reporter cell line with a NFAT promoter, expressing human CD3ε) were added. The Jurkat-NFAT reporter cell line (Promega) is a human acute lymphatic leukemia reporter cell line with a NFAT promoter, expressing human CD3ε. If CD3 binder binds CD3ε Luciferase is expressed and this can be measured in Luminescence after addition of One-Glo substrate (Promega). FIG. 4C shows a comparison of EC50 values of CD3ε binding for masked and unmasked monovalent CD3 binder.

**Figure 5A-H** depict schematics of different T cell bispecific molecules with masking moieties. FIG. 5A: 7344 anti-ID CH2527 scFv 4.15.64 MK062 Matriptase site CD3 16D5 Fc. FIG. 5B: 7676 anti-ID CH2527 scFv 4.15.64 non-cleavable linker CD3 16D5 Fc. FIG. 5C: 7496 anti-ID CH2527 scFv 4.32.63 MK062 Matriptase site CD3 16D5 Fc. FIG. 5D: 7611 anti-ID CH2527 scFv 4.32.63 non-cleavable linker CD3 16D5 Fc. FIG. 5E: 6298 GA916-D-16D5-02 sf W(1). FolR1 16D5 classic 2+1 TCB with common light chain. FIG. 5F: 6100 GA916-D-16D5 sf W(3a). FolR1 16D5 inverted 2+1 TCB with common light chain. FIG. 5G: ID 6182 DP47GS TCB sf CHO W(9a). DP47 inverted 2+1 TCB. FIG. 5H: 7494 anti-ID CH2527 Fab 4.15.64 MK062 Matriptase site CD3 16D5 Fc.

**Figure 6** shows a first plasmid ratios used for transfection by size exclusion chromatography (1(hole): 1 (knob): 3 (CLC)).

**Figure 7** shows a second plasmid ratios used for transfection by size exclusion chromatography. (1(hole): 2 (knob): 3 (CLC)).

**Figure 8** shows CE-SDS analysis of the TCB molecule depicted in FIG. 5A (ID 7344) (final purified preparation): Lane A = non-reduced, lane B = reduced, lane C = Protein standard.

**Figure 9** shows CE-SDS analysis of the TCB molecule depicted in FIG. 5B (ID 7676) (final purified preparation): Lane A = non-reduced, lane B = reduced, lane C = Protein standard.

**Figure 10** shows CE-SDS analysis of the TCB molecule depicted in FIG. 5C (ID 7496) (final purified preparation): Lane A = non-reduced, lane B = reduced, lane C = Protein standard.

**Figure 11** shows CE-SDS analysis of the TCB molecule depicted in FIG. 5D (ID 7611) (final purified preparation): Lane A = non-reduced, lane B = reduced, lane C = Protein standard.

**Figures 12A-D** show shows Capillary Electrophoresis-SDS analysis of the molecules depicted in Figures 5A and C. FIGs. 12A and B shows Capillary Electrophoresis of the molecules depicted in Figures 5A (ID 7344) anti-ID CH2527 scFv 4.15.64 MK062 CD3 16D6 Fc under non reducing (FIGs. 12A) and reducing conditions (FIGs. 12B). Comparison of the untreated (I) and treated molecule (III) shows complete cleavage of the anti-ID scFv after rh-Matriptase/ST14 treatment for 48 h at 37°C. One sample (II) was untreated but incubated at 37°C for 48 h. Pre-stained protein Marker (IV) Mark 12 (Invitrogen) was used for estimation of correct molecule weight. FIGs. 12C and D dhows Capillary Electrophoresis of the molecule depicted in FIG. 5C (ID 7496) anti-ID CH2527 scFv 4.32.63 MK062 CD3 16D6 Fc under non reducing (FIGs. 12C) and reducing conditions (FIGs. 12D). Comparison of the untreated (I) and treated molecule (III) shows complete cleavage of the anti-ID scFv after rhMatriptase/ST14 treatment for 48 h at 37°C. One sample (II) was untreated but incubated at 37°C for 48 h. Pre-stained protein Marker (IV) Mark 12 (Invitrogen) was used for estimation of correct molecule weight.

**Figure 13** shows FolR1 expression level quantification done by Qifikit (Dako). Antibody for FolR1: #LS-C125620-100 (LifeSpan BioSciences Inc); used at 20 μg/ml; mouse IgG1 isotype: #554121 (BD).

**Figures 14A and B** show T cell activation by protease activated TCBs. FIG. 14A shows killing of Skov3 cells induced by protease-activated TCB molecules at a concentration of 10nM (TCBs with different anti-idiotypic CD3 masks, cleavable and non-cleavable linker, molecules pretreated with purified rhMatriptase/ST14) and human PBMCs after 48 h of incubation (E:T = 7:1, effectors are human PBMCs). Pre-treatment with rhMatriptase/ST14 (R&D Systems) was done for 24 h at 37°C. FIG. 14B shows T cell activation of human PBMCs induced by protease activated TCB binding of 10nM (TCBs with different anti-idiotypic CD3 masks, cleavable and non-cleavable linker, treated molecules) on Skov3 cells after 48 h of incubation (E:T = 7:1, effectors are human PBMCs). T cell activation markers CD25 (left panels) and CD69 (right panels). CD4+ and CD8+ T cells as indicated.

**Figures 15A and B** show T cell activation by protease activated TCBs. FIG. 15A shows killing of Mkn-45 cells

induced by protease activated TCB molecules at a concentration of 100nM (TCBs with different anti-idiotypic CD3 masks, cleavable and non-cleavable linker, treated molecules) and human PBMCs after 48 h of incubation (E:T = 7:1, effectors are human PBMCs). Pre-treatment with rhMatriptase/ST14 (R&D Systems) was done for 24 h at 37°C. FIG. 15B shows T cell activation of human PBMCs induced by protease activated TCB binding of 100nM (TCBs with different anti-idiotypic CD3 masks, cleavable and non-cleavable linker, treated molecules) on Mkn-45 cells after 48 h of incubation (E:T = 7:1, effectors are human PBMCs). T cell activation markers CD25 (left panels) and CD69 (right panels). CD4+ and CD8+ T cells as indicated.

**Figure 16** shows killing of HT29 cells induced by protease activated TCB molecules at a concentration of 10nM (TCBs with different anti-idiotypic CD3 masks, cleavable and non-cleavable linker, treated molecules) and human PBMCs after 48 h of incubation (E:T = 10:1, effectors are human PBMCs). Pre-treatment with rhMatriptase/ST14 (R&D Systems) was done for 24 h at 37°C. Bars from left to right are 7344: anti-ID CH2527 scFv 4.15.64 MK062 CD3 16D6Fc; 7344: anti-ID CH2527 scFv 4.15.64 MK062 CD3 16D6Fc_treated; 7676: anti-ID CH2527 scFv 4.15.64 non-cleavable CD3 16D6Fc; 7496 anti-ID CH2527 scFv 4.32.63 MK062 CD3 16D6 Fc; 7496 anti-ID CH2527 scFv 4.32.63 MK062 CD3 16D6 Fc_treated; 7611: ID anti CH2527 scFv 4.32.63 non-cleavable linker CD3 16D6 Fc; 6298 GA916-D-16D5-02 sf W(1); 6182 DP47GS TCB sf CHO W(9a).

**Figure 17** shows killing of Skov3 cells induced by protease activated TCB molecules at a concentration of 10nM (TCBs with different anti-idiotypic CD3 masks, cleavable and non-cleavable linker, treated molecules) and human PBMCs (from a different donor than PBMCs used for FIG. 14A) after 48 h of incubation (E:T = 10:1, effectors are human PBMCs). Pre-treatment with rhMatriptase/ST14 (R&D Systems) was done for 24 h at 37°C. Bars from left to right are 7344: anti-ID CH2527 scFv 4.15.64 MK062 CD3 16D6Fc; 7344: anti-ID CH2527 scFv 4.15.64 MK062 CD3 16D6Fc_treated; 7676: anti-ID CH2527 scFv 4.15.64 non-cleavable CD3 16D6Fc; 7496 anti-ID CH2527 scFv 4.32.63 MK062 CD3 16D6 Fc; 7496 anti-ID CH2527 scFv 4.32.63 MK062 CD3 16D6 Fc_treated; 7611: ID anti CH2527 scFv 4.32.63 non-cleavable linker CD3 16D6 Fc; 6298 GA916-D-16D5-02 sf W(1); 6182 DP47GS TCB sf CHO W(9a).

**Figures 18A and B** show T cell activation by protease activated TCBs. FIG. 18A shows dose-dependent killing of HeLa cells induced by protease activated TCB molecules (TCB with anti-idiotypic CD3 4.15.64 mask, cleavable and non-cleavable linker, treated molecule) and human PBMCs (isolated from buffy coat) after 48 h of incubation (E:T = 10:1, effectors are human PBMCs). Pre-treatment with rhMatriptase/ST14 (R&D Systems) was done for 24 h at 37°C.

FIG. 18B shows dose-dependent T cell activation of human PBMCs induced by protease activated TCB binding (TCB with anti-idiotypic CD3 4.15.64 mask, cleavable and non-cleavable linker, treated molecule) on HeLa cells after 48 h of incubation (E:T = 10:1, effectors are human PBMCs). T cell activation markers CD25 (left panels) and CD69 (right panels). CD4+ and CD8+ T cells as indicated.

**Figures 19A and B** show T cell activation by protease activated TCBs. FIG. 19A shows dose-dependent killing of HeLa cells induced by protease activated TCB molecules (TCB with anti-idiotypic CD3 4.32.63 mask, cleavable and non-cleavable linker, treated molecule) and human PBMCs (isolated from buffy coat) after 48 h of incubation (E:T = 10:1, effectors are human PBMCs). Pre-treatment with rhMatriptase/ST14 (R&D Systems) was done for 24 h at 37°C. Fig. 19B shows dose-dependent T cell activation of human PBMCs induced by protease activated TCB binding (TCB with anti-idiotypic CD3 4.32.63 mask, cleavable and non-cleavable linker, treated molecule) on HeLa cells after 48 h of incubation (E:T = 10:1, effectors are human PBMCs). T cell activation markers CD25 (left panels) and CD69 (right panels). CD4+ and CD8+ T cells as indicated.

**Figures 20A and** B show T cell activation by protease activated TCBs. FIG. 20A shows dose-dependent killing of Skov3 cells induced by protease activated TCB molecules (TCB with anti-idiotypic CD3 4.15.64 mask, cleavable and non-cleavable linker, treated molecule) and human PBMCs (isolated from buffy coat) after 48 h of incubation (E:T = 10:1, effectors are human PBMCs). Pre-treatment with rhMatriptase/ST14 (R&D Systems) was done for 24 h at 37°C. FIG. 20B shows dose-dependent T cell activation of human PBMCs induced by protease activated TCB binding (TCB with anti-idiotypic CD3 4.15.64 mask, cleavable and non-cleavable linker, treated molecule) on Skov3 cells after 48 h of incubation (E:T = 10:1, effectors are human PBMCs). T cell activation markers CD25 (left panels) and CD69 (right panels). CD4+ and CD8+ T cells as indicated.

**Figures 21A and B** show T cell activation by protease activated TCBs. FIG. 21A shows dose-dependent killing of Skov3 cells induced by protease activated TCB molecules (TCB with anti-idiotypic CD3 4.32.63 mask, cleavable and non-cleavable linker, treated molecule) and human PBMCs (isolated from buffy coat) after 48 h of incubation (E:T = 10:1, effectors are human PBMCs). Pre-treatment with rhMatriptase/ST14 (R&D Systems) was done for 24 h at 37°C. FIG. 21B shows dose-dependent T cell activation of human PBMCs induced by protease activated TCB binding (TCB with anti-idiotypic CD3 4.32.63 mask, cleavable and non-cleavable linker, treated molecule) on Skov3 cells after 48 h of incubation (E:T = 10:1, effectors are human PBMCs). T cell activation markers CD25 (left panels) and CD69 (right panels). CD4+ and CD8+ T cells as indicated.

**Figures 22A and B** show T cell activation by protease activated TCBs. FIG. 22A shows dose-dependent T cell

activation of human PBMCs (different donor than in experiments described above) induced by protease activated TCB binding (TCB with anti-idiotypic CD3 4.15.64 mask, cleavable and non-cleavable linker, treated molecule) on HT29 cells after 48 h of incubation (E:T = 10:1, effectors are human PBMCs). T cell activation markers CD25 (left panels) and CD69 (right panels). CD4+ and CD8+ T cells as indicated. FIG. 22B shows dose-dependent T cell activation of human PBMCs (different donor than in Figure 16) induced by protease activated TCB binding (TCB with anti-idiotypic CD3 4.32.63 mask, cleavable and non-cleavable linker, treated molecule) on HT29 cells after 48 h of incubation (E:T = 10:1, effectors are human PBMCs). T cell activation markers CD25 (left panels) and CD69 (right panels). CD4+ and CD8+ T cells as indicated.

**Figure 23** shows dose-dependent T cell activation of human PBMCs (different donor than in experiments described above) induced by protease activated TCB binding (TCB with anti-idiotypic CD3 4.15.64 mask, cleavable and non-cleavable linker, treated molecule) on HRCEpiC cells after 48 h of incubation (E:T = 10:1, effectors are human PBMCs). T cell activation markers CD25 (left panels) and CD69 (right panels). CD4+ and CD8+ T cells as indicated.

**Figure 24** shows killing of Ovcar3 cells induced by protease activated TCB molecules at a concentration of 50nM (TCBs with different anti-idiotypic CD3 masks, cleavable and non-cleavable linker, treated molecules) and human PBMCs after 48 h of incubation (E:T = 10:1, effectors are human PBMCs). Pre-treatment with rhMatriptase/ST14 (R&D Systems) was done for 10 min at 37°C (not fully cleaved).

**Figure 25** shows killing of Skov3 cells induced by 10nM of protease activated TCB molecules (TCB with anti-idiotypic CD3 4.15.64 mask, cleavable and non-cleavable linker, treated molecule) and human PBMCs (isolated from buffy coat) after 48 h of incubation (E:T = 10:1, effectors are three different Donors for human PBMCs). Pre-treatment with rhMatriptase/ST14 (R&D Systems) was done for 24 h at 37°C.

**Figure 26** shows killing of Skov3 cells induced by 10nM of protease activated TCB molecules (TCB with anti-idiotypic CD3 4.32.63 mask, cleavable and non-cleavable linker, treated molecule) and human PBMCs (isolated from buffy coat) after 48 h of incubation (E:T = 10:1, effectors are three different Donors for human PBMCs). Pre-treatment with rhMatriptase/ST14 (R&D Systems) was done for 24 h at 37°C.

**Figure 27** shows killing of HeLa cells induced by 100 pM of protease activated TCB molecules (TCB with anti-idiotypic CD3 4.32.63 mask, cleavable and non-cleavable linker, treated molecule) and human PBMCs (isolated from buffy coat) after 48 h of incubation (E:T = 10:1, effectors are three different Donors for human PBMCs). Pre-treatment with rhMatriptase/ST14 (R&D Systems) was done for 24 h at 37°C.

**Figure 28** depicts a schematic of anti-ID GA201 scFv Matrix Metalloprotease site GA201 Fc (GA201-anti-GA201-scFv).

**Figure 29** depicts a schematics of the anti HER1 antibody GA201.

**Figures 30A and B** show capillary Electrophoresis-SDS analysis of the molecule depicted in FIG. 28 under non-reducing (FIG. 30A) and reducing conditions (FIG. 30B). The molecule depicted in FIG. 28 was purified to homogeneity by Protein A and Size Exclusion chromatography and subjected to Capillary electrophoresis-SDS analysis.

**Figure 31** shows FACS analysis of GA201-anti-GA201-scFv and GA201 binding to HER1 expressed on H322M cells to confirm masking effect of anti-idiotypic GA201 scFv. GA201-anti-GA201-scFv was incubated overnight with the Matrix Metalloprotease MMP-2 and binding to H322M cells was compared to uncleaved GA201-anti-GA201-scFv, GA201 and an isotype IgG1 control antibody. Binding to HER1 on H322M cells was detected with a F(ab')2-goat anti-human IgG Fc secondary antibody FITC conjugate and analyzed by FACS using the BD FACS Canto II. The median fluorescence intensity (MFI) was used for analysis.

**Figure 32** shows surface plasmon resonance analysis of HER1 binding of masked and unmasked GA201, before and after MMP2 cleavage.

**Figures 33A-J** depict schematics of different T cell bispecific molecules with masking moieties. FIG. 33A: ID 8364. 16D5 TCB, classic format, anti ID CH2527 scFv 4.32.63 MMP9-MK062 Matriptase site N-terminally fused to CD3. FIG. 33B: ID 8363. 16D5 TCB, classic format, anti ID CH2527 scFv 4.32.63 Cathepsin S/B site N-terminally fused to CD3. FIG. 33C: ID 8365. 16D5 TCB, inverted format, anti ID CH2527 scFv 4.32.63 MK062 Matriptase site N-terminally fused to common light chain. FIG. 33D: ID 8366. 16D5 TCB, inverted format, anti ID CH2527 scFv 4.32.63 non-cleavable linker N-terminally fused to common light chain. FIG. 33E: ID 8672. aMesothelin RG7787 charged residues TCB, classic format, anti ID CH2527 scFv 4.32.63 MMP9-MK062 Matriptase site N-terminally fused to CD3 X Fab. FIG. 33F: ID 8673. aMesothelin RG7787 charged residues TCB, classic format, anti ID CH2527 scFv 4.32.63 non-cleavable linker N-terminally fused to CD3 X Fab. FIG. 33G: ID 8674. aMesothelin RG7787 charged residues TCB, inverted format, anti ID CH2527 scFv 4.32.63 MMP9-MK062 Matriptase site N-terminally fused to CD3 XFab. FIG. 33H: 8675. aMesothelin RG7787 charged residues TCB, inverted format, anti ID CH2527 scFv 4.32.63 non-cleavable linker N-terminally fused to CD3 XFab. FIG. 33I: ID 8505. aMesothelin RG7787 charged residues CD3 XFab TCB, inverted format. FIG. 33J: ID 8676. CD3 XFab aMesothelin RG7787 charged residues TCB, classic format.

**Figure 34** depicts CE-SDS analysis of the TCB ID 8365 and TCB ID 8366 (final purified preparation): Lane A = Protein standard, lane B = protein stored at 4 °C, lane C = protein pretreated with rhMatriptase/ST14 (R&D Systems),

lane D = protein incubated for 72 h at 37 °C and lane E = molecule 3.

**Figures 35A and B.** depicts CE-SDS analysis of the TCB depicted in FIG. 33A (ID 8364) and the TCB depicted in FIG. 33B (ID 8363). FIG. 35A: CE-SDS analysis of the TCB 8364 (final purified preparation): Lane A = Protein standard, lane B = protein stored at 4 °C, lane C = protein pretreated with rhMatriptase/ST14 (R&D Systems), lane D = protein incubated for 72 h at 37 °C and lane E = non-cleavable linker construct. FIG. 35B: CE-SDS analysis of the TCB 8363 (final purified preparation): Lane A = Protein standard, lane B = protein stored at 4 °C, lane C = protein pretreated with rhCathepsin B (R&D Systems), lane D = protein pretreated with rhCathepsin S (R&D Systems), lane E = protein incubated for 72 h at 37 °C and lane F = non-cleavable linker construct.

**Figures 36A and B.** depicts Jurkat NFAT activation assay using HeLa and Skov-3 cells as target cells. Each point represents the mean value of triplicates. Standard deviation is indicated by error bars. Jurkat-NFAT reporter cell line (Promega) is a human acute lymphatic leukemia reporter cell line with a NFAT promoter, expressing human CD3ε. If the CD3 binder of the TCB binds the tumor target and the CD3 (cross-linkage is necessary) binds CD3ε the Luciferase expression can be measured in Luminescence after addition of One-Glo substrate (Promega). The FolR1 TCB (black triangles pointing down) and the pretreated protease activated TCB (8364, grey filled squares) with N-terminally fused anti ID CD3 4.32.63 scFv and MMP9-Matriptase MK062 site were compared. The molecule was treated with rhMatriptase/ST14 (R&D Systems) for about 20 h at 37 °C. The masked TCB (containing a GS non-cleavable linker, grey triangles pointing up) and the non-targeted TCB control (empty triangle pointing down) are shown as well. The dotted line shows the Luminescence of target cells and effector cells without any TCB. FIG. 36A shows a Jurkat NFAT activation assay using HeLa cells as target cells. FIG. 36B shows a Jurkat NFAT activation assay using Skov-3 cells as target cells.

**Figures 37A-D** depicts tumor cell cytotoxicity mediated by FolR1 TCBs and human PBMCs (Effector : Target = 10 : 1). Each point represents the mean value of triplicates. Standard deviation is indicated by error bars. FIG. 37A: HeLa target cell cytotoxicity. Comparison of two different formats of the Protease activated TCBs both containing an anti idiotypic CD3 scFv linked with a MK062 Matriptase linker. FIG. 37B: Skov-3 target cell cytotoxicity. Comparison of two different formats of the Protease activated TCBs both containing an anti idiotypic CD3 scFv linked with a MK062 Matriptase linker. FIG. 37C: HeLa target cell cytotoxicity. Comparison of classic Protease activated TCB containing an anti idiotypic CD3 scFv and GS linkers with different protease sites. Protease activated TCB containing the MMP9-Matriptase MK062 linker (8364, grey squares), FolR1 TCB (light grey triangles pointing down), protease activated TCB containing only Matriptase MK062 (light grey rhomb)/ Cathepsin site (grey circles) or non-cleavable linker (black triangles pointing down). FIG 37D: Skov-3 target cell cytotoxicity. Comparison of classic Protease activated TCB containing an anti idiotypic CD3 scFv and GS linkers with different protease sites. Protease activated TCB containing the MMP9-Matriptase MK062 linker (8364, grey squares), FolR1 TCB (light grey triangles pointing down), protease activated TCB containing only Matriptase MK062 (light grey rhomb)/ Cathepsin site (grey circles) or non-cleavable linker (black triangles pointing down).

**Figures 38A and B** depicts quantification of CD69 of CD8 positive cells after co-incubation of primary human renal epithelial cortical cells (FIG. 38A) or human bronchial epithelial cells (FIG. 38B) with 200 nM of the different TCBs and three different donors of human PBMCs. T cells were stained after 48 h of incubation. (E:T = 10:1, effectors are human PBMCs). Median fluorescence intensity of T cell activation marker CD69 for $CD8^+$ T cells is shown. Each point represents the mean value of triplicates of three different human PBMC donors. Standard deviation is indicated in error bars. Unpaired t test was used for statistical analysis.

**Figures 39A and B** depicts tumor cell cytotoxicity mediated by MSLN TCBs and human PBMCs (Effector : Target = 10 : 1). Maximal lysis of the target cells (= 100%) was achieved by incubation of target cells with 1% Triton X-100 20 h before LDH readout. Minimal lysis (= 0%) refers to target cells co-incubated with effector cells without any TCB. Each point represents the mean value of triplicates. Standard deviation is indicated by error bars. FIG.39A: NCI H596 target cell cytotoxicity. Protease activated MSLN TCB containing an anti idiotypic CD3 scFv linked with a MMP9-MK062 Matriptase linker. The protease activated TCB (8672, light grey circles), the MSLN TCB (dark grey triangles pointing down) and the protease activated TCB containing a non-cleavable linker (8673, grey triangles pointing up) are compared. FIG. 39B: AsPC-1 target cell cytotoxicity. Protease activated MSLN TCB containing an anti idiotypic CD3 scFv linked with a MMP9-MK062 Matriptase linker. The protease activated TCB (8672, light grey circles), the MSLN TCB (dark grey triangles pointing down) and the protease activated TCB containing a non-cleavable linker (8673, grey triangles pointing up) are compared.

**Figure 40** depicts a Jurkat-NFAT activation assay with primary tumor samples and Protease activated FolR1 TCBs. Jurkat NFAT reporter cells are activated after co-incubation with FolR1 TCB (6298) and Protease activated FolR1 TCB containing MMP9-Matriptase cleavage site (8364). Protease activated FolR1 TCBs (8363, 8408) and control TCBs (8409, 7235) do not induce Luciferase expression. The dotted line indicates the baseline Luminescence for Jurkat NFAT cells co-incubated with tumor.

**Figures 41A-C:** Capillary electrophoresis of protease activated TCBs after incubation in human serum. Molecules were incubated for 0 or 14 days in human IgG depleted serum at 37 °C in a humidified incubator (5 % $CO_2$). All

molecules were purified by affinity chromatography (ProteinA) and then analyzed by Capillary electrophoresis. FIG. 41A: CE-SDS analysis of serum, FolR1 TCB (6298) in serum at day 0 and day 14. FIG. 41B: CE-SDS analysis of serum, Protease activated FolR1 TCB with MMP9-Matriptase linker (8364) in serum at day 0 and day 14. FIG. 41C: CE-SDS analysis of serum, Protease activated FolR1 TCB with Matriptase linker (8408) in serum at day 0 and day 14 and the precleaved molecule in serum.

**Figures 42A-F** depict schematics of different T cell bispecific molecules with masking moieties. FIG. 42A: ID 8955. Herceptarg TCB, classic format, anti ID CH2527 scFv 4.32.63 MK062 MMP9 linker N-terminally fused to VH. FIG. 42B: ID 8957. Herceptarg TCB, classic format, anti ID CH2527 scFv 4.32.63 non cleavable linker N-terminally fused to VH. FIG. 42C: ID 8959. Herceptarg TCB, classic format. FIG. 42D: ID 8997. FolR1 36F2 TCB, classic format, anti ID CH2527 scFv 4.32.63 MK062 MMP9 linker N-terminally fused to VH. FIG. 42E: ID 8998. FolR1 36F2 TCB, classic format, anti ID CH2527 scFv 4.32.63 non cleavable linker N-terminally fused to VH. FIG. 42F: ID 8996. FolR1 36F2 TCB, classic format.

**Figure 43** depicts Human Bronchial Epithelial Cell toxicity mediated by human PBMCs and 100 nM or 10 nM of TCBs. Maximal lysis of the target cells (= 100%) was achieved by incubation of target cells with 1% Triton X-100 20 h before LDH readout. Minimal lysis (= 0%) refers to target cells co-incubated with effector cells without any TCB. Each point represents the mean value of triplicates. Standard deviation is indicated by error bars.

**Figure 44** depicts FolR1 negative target cell (Mkn-45) cytotoxicity mediated by 100 nM of FolR1 TCBs and human PBMCs. Maximal lysis of the target cells (= 100%) was achieved by incubation of target cells with 1% Triton X-100 20 h before LDH readout. Minimal lysis (= 0%) refers to target cells co-incubated with effector cells without any TCB. Each point represents the mean value of triplicates. Standard deviation is indicated by error bars.

## DETAILED DESCRIPTION

### Definitions

Terms are used herein as generally used in the art, unless otherwise defined in the following.

As used herein, the term "antigen binding molecule" refers in its broadest sense to a molecule that specifically binds an antigenic determinant. Examples of antigen binding molecules are immunoglobulins and derivatives, e.g., fragments, thereof.

The term "bispecific" means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

As used herein, the term "antigen binding moiety" refers to a polypeptide molecule that specifically binds to an antigenic determinant. In one embodiment, an antigen binding moiety is able to direct the entity to which it is attached (e.g., a second antigen binding moiety) to a target site, for example to a specific type of tumor cell or tumor stroma bearing the antigenic determinant. In another embodiment an antigen binding moiety is able to activate signaling through its target antigen, for example a T cell receptor complex antigen. Antigen binding moieties include antibodies and fragments thereof as further defined herein. Particular antigen binding moieties include an antigen binding domain of an antibody, comprising an antibody heavy chain variable region and an antibody light chain variable region. In certain embodiments, the antigen binding moieties may comprise antibody constant regions as further defined herein and known in the art. Useful heavy chain constant regions include any of the five isotypes: $\alpha$, $\delta$, $\varepsilon$, $\gamma$, or $\mu$. Useful light chain constant regions include any of the two isotypes: $\kappa$ and $\lambda$.

As used herein, the term "antigenic determinant" is synonymous with "antigen" and "epitope," and refers to a site (e.g., a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein (*e.g.*, FolR1, HER1, HER2, CD3, Mesothelin) can be any native form of the proteins from any vertebrate source, including mammals such

as primates (*e.g*., humans) and rodents (e.g., mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g., splice variants or allelic variants. Exemplary human proteins useful as antigens include, but are not limited to: FolR1, HER1 and CD3, particularly the epsilon subunit of CD3 (see UniProt no. P07766 (version 130), NCBI RefSeq no. NP_000724.1, SEQ ID NO: 54 for the human sequence; or UniProt no. Q95LI5 (version 49), NCBI GenBank no. BAB71849.1 for the cynomolgus [Macaca fascicularis] sequence). In certain embodiments the protease-activatable T cell activating bispecific molecule of the invention binds to an epitope of CD3 or a target cell antigen that is conserved among the CD3 or target antigen from different species. In certain embodiments the protease-activatable T cell activating bispecific molecule of the invention binds to CD3 and FolR1, but does not bind to FolR2 or FolR3. In certain embodiments the protease-activatable T cell activating bispecific molecule of the invention binds to CD3 and HER1. In certain embodiments the protease-activatable T cell activating bispecific molecule of the invention binds to CD3 and Mesothelin. In certain embodiments the protease-activatable T cell activating bispecific molecule of the invention binds to CD3 and HER2. By "specific binding" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g., surface plasmon resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antigen binding molecule comprising that antigen binding moiety, has a dissociation constant ($K_D$) of $\leq 1$ μM, $\leq 100$ nM, $\leq 10$ nM, $\leq 1$ nM, $\leq 0.1$ nM, $\leq 0.01$ nM, or $\leq 0.001$ nM (e.g., $10^{-8}$ M or less, e.g., from $10^{-8}$ M to $10^{-13}$ M, e.g., from $10^{-9}$ M to $10^{-13}$ M).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant ($K_D$), which is the ratio of dissociation and association rate constants ($k_{off}$ and $k_{on}$, respectively). Thus, equivalent affinities may comprise different rate constants, as long as the ratio of the rate constants remains the same. Affinity can be measured by well-established methods known in the art, including those described herein. A particular method for measuring affinity is Surface Plasmon Resonance (SPR).

"Reduced binding", for example reduced binding to an Fc receptor, refers to a decrease in affinity for the respective interaction, as measured for example by SPR. For clarity the term includes also reduction of the affinity to zero (or below the detection limit of the analytic method), i.e. complete abolishment of the interaction. Conversely, "increased binding" refers to an increase in binding affinity for the respective interaction.

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. The protease-activatable T cell activating bispecific molecules of the invention are capable of inducing T cell activation. Suitable assays to measure T cell activation are known in the art described herein.

A "target cell antigen" as used herein refers to an antigenic determinant presented on the surface of a target cell, for example a cell in a tumor such as a cancer cell or a cell of the tumor stroma.

As used herein, the terms "first" and "second" with respect to antigen binding moieties etc., are used for convenience of distinguishing when there is more than one of each type of moiety. Use of these terms is not intended to confer a specific order or orientation of the protease-activatable T cell activating bispecific molecule unless explicitly so stated.

A "Fab molecule" refers to a protein consisting of the VH and CH1 domain of the heavy chain (the "Fab heavy chain") and the VL and CL domain of the light chain (the "Fab light chain") of an immunoglobulin.

By "fused" is meant that the components (e.g., a Fab molecule and an Fc domain subunit) are linked by peptide bonds, either directly or via one or more peptide linkers.

As used herein, the term "single-chain" refers to a molecule comprising amino acid monomers linearly linked by peptide bonds. In certain embodiments, one of the antigen binding moieties is a single-chain Fab molecule, i.e. a Fab molecule wherein the Fab light chain and the Fab heavy chain are connected by a peptide linker to form a single peptide chain. In a particular such embodiment, the C-terminus of the Fab light chain is connected to the N-terminus of the Fab heavy chain in the single-chain Fab molecule.

By a "crossover" Fab molecule (also termed "Crossfab") is meant a Fab molecule wherein either the variable regions or the constant regions of the Fab heavy and light chain are exchanged, i.e. the crossover Fab molecule comprises a peptide chain composed of the light chain variable region and the heavy chain constant region, and a peptide chain

composed of the heavy chain variable region and the light chain constant region. For clarity, in a crossover Fab molecule wherein the variable regions of the Fab light chain and the Fab heavy chain are exchanged, the peptide chain comprising the heavy chain constant region is referred to herein as the "heavy chain" of the crossover Fab molecule. Conversely, in a crossover Fab molecule wherein the constant regions of the Fab light chain and the Fab heavy chain are exchanged, the peptide chain comprising the heavy chain variable region is referred to herein as the "heavy chain" of the crossover Fab molecule.

In contrast thereto, by a "conventional" Fab molecule is meant a Fab molecule in its natural format, i.e. comprising a heavy chain composed of the heavy chain variable and constant regions (VH-CH1), and a light chain composed of the light chain variable and constant regions (VL-CL).

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five types, called $\alpha$ (IgA), $\delta$ (IgD), $\varepsilon$ (IgE), $\gamma$ (IgG), or $\mu$ (IgM), some of which may be further divided into subtypes, e.g., $\gamma_1$ ($IgG_1$), $\gamma_2$ ($IgG_2$), $\gamma_3$ ($IgG_3$), $\gamma_4$ ($IgG_4$), $\alpha$1 ($IgA_1$) and $\alpha_2$ ($IgA_2$). The light chain of an immunoglobulin may be assigned to one of two types, called kappa ($\kappa$) and lambda ($\lambda$), based on the amino acid sequence of its constant domain. An immunoglobulin essentially consists of two Fab molecules and an Fc domain, linked via the immunoglobulin hinge region. The term "antibody" herein is used in the broadest sense and encompasses various antibody structures, including but not limited to monoclonal antibodies, polyclonal antibodies, and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$, diabodies, linear antibodies, single-chain antibody molecules (e.g., scFv), and single-domain antibodies. For a review of certain antibody fragments, see Hudson et al., Nat Med 9, 129-134 (2003). For a review of scFv fragments, see e.g., Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and $F(ab')_2$ fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g., U.S. Patent No. 6,248,516 B1). Antibody fragments can be made by various techniques, including but not limited to proteolytic digestion of an intact antibody as well as production by recombinant host cells (e.g., E. coli or phage), as described herein.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable region (VL) and an antibody heavy chain variable region (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "hypervariable region" or "HVR", as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the complementarity determining regions (CDRs), the latter being of highest sequence variability and/or involved in antigen recognition. With the exception of CDR1 in VH, CDRs generally comprise the amino acid residues that form the hypervariable loops. Hypervariable regions (HVRs) are also referred to as "complementarity determining regions" (CDRs), and these terms are used herein interchangeably in reference to portions of the variable region that form the antigen binding regions. This particular region has been described by Kabat et al., U.S. Dept. of Health and Human Services, Sequences of Proteins of Immunological Interest (1983) and by Chothia et al., J Mol Biol 196:901-917 (1987), where the definitions include overlapping

or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table 1 as a comparison. The exact residue numbers which encompass a particular CDR will vary depending on the sequence and size of the CDR. Those skilled in the art can routinely determine which residues comprise a particular CDR given the variable region amino acid sequence of the antibody.

TABLE 1. CDR Definitions[1]

| CDR | Kabat | Chothia | AbM[2] |
|---|---|---|---|
| $V_H$ CDR1 | 31-35 | 26-32 | 26-35 |
| $V_H$ CDR2 | 50-65 | 52-58 | 50-58 |
| $V_H$ CDR3 | 95-102 | 95-102 | 95-102 |
| $V_L$ CDR1 | 24-34 | 26-32 | 24-34 |
| $V_L$ CDR2 | 50-56 | 50-52 | 50-56 |
| $V_L$ CDR3 | 89-97 | 91-96 | 89-97 |

[1]Numbering of all CDR definitions in Table 1 is according to the numbering conventions set forth by Kabat et al. (see below).
[2] "AbM" with a lowercase "b" as used in Table 1 refers to the CDRs as defined by Oxford Molecular's "AbM" antibody modeling software.

Kabat et al. also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

The polypeptide sequences of the sequence listing are not numbered according to the Kabat numbering system. However, it is well within the ordinary skill of one in the art to convert the numbering of the sequences of the Sequence Listing to Kabat numbering.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their

association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g., antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

The term "effector functions" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g., B cell receptor), and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino- and/or carboxy-terminal deletions and insertions of amino acids. Particular amino acid mutations are amino acid substitutions. For the purpose of altering e.g., the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g., 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, $G_{329}$, P329G, or Pro329Gly.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the invention, as are native or recombinant polypeptides which have been separated, fractionated, or partially or substantially purified by any suitable technique.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any

algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "polynucleotide" refers to an isolated nucleic acid molecule or construct, e.g., messenger RNA (mRNA), virally-derived RNA, or plasmid DNA (pDNA). A polynucleotide may comprise a conventional phosphodiester bond or a non-conventional bond (e.g., an amide bond, such as found in peptide nucleic acids (PNA). The term "nucleic acid molecule" refers to any one or more nucleic acid segments, e.g., DNA or RNA fragments, present in a polynucleotide.

By "isolated" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include *in vivo* or *in vitro* RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g., ALIGN-2).

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term "vector" or "expression vector" is synonymous with "expression construct" and refers to a DNA molecule that is used to introduce and direct the expression of a specific gene to which it is operably associated in a target cell.

The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, *e.g.*, mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "activating Fc receptor" is an Fc receptor that following engagement by an Fc domain of an antibody elicits signaling events that stimulate the receptor-bearing cell to perform effector functions. Human activating Fc receptors include FcγRIIIa (CD16a), FcγRI (CD64), FcγRIIa (CD32), and FcαRI (CD89).

Antibody-dependent cell-mediated cytotoxicity (ADCC) is an immune mechanism leading to the lysis of antibody-coated target cells by immune effector cells. The target cells are cells to which antibodies or derivatives thereof comprising an Fc region specifically bind, generally via the protein part that is N-terminal to the Fc region. As used herein, the term "reduced ADCC" is defined as either a reduction in the number of target cells that are lysed in a given time, at a given concentration of antibody in the medium surrounding the target cells, by the mechanism of ADCC defined above, and/or an increase in the concentration of antibody in the medium surrounding the target cells, required to achieve the lysis of a given number of target cells in a given time, by the mechanism of ADCC. The reduction in ADCC is relative to the ADCC mediated by the same antibody produced by the same type of host cells, using the same standard production, purification, formulation and storage methods (which are known to those skilled in the art), but that has not been engineered. For example the reduction in ADCC mediated by an antibody comprising in its Fc domain an amino acid substitution that reduces ADCC, is relative to the ADCC mediated by the same antibody without this amino acid substitution in the Fc domain. Suitable assays to measure ADCC are well known in the art (see e.g., PCT publication no. WO 2006/082515 or PCT publication no. WO 2012/130831).

An "effective amount" of an agent refers to the amount that is necessary to result in a physiological change in the cell or tissue to which it is administered.

A "therapeutically effective amount" of an agent, e.g., a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Particularly, the individual or subject is a human.

The term "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, protease-activatable T cell activating bispecific molecules of the invention are used to delay development of a disease or to slow the progression of a disease. The term "package insert" is used to refer to instructions customarily included in commercial packages of therapeutic products, that contain information about the indications, usage, dosage, administration, combination therapy, contraindications and/or warnings concerning the use of such therapeutic products.

An "idiotype-specific polypeptide" as used herein refers to a polypeptide that recognizes the idiotype of an antigen-binding moiety, *e.g.,* an antigen-binding moiety specific for CD3. The idiotype-specific polypeptide is capable of specifically binding to the variable region of the antigen-binding moiety and thereby reducing or preventing specific binding of the antigen-binding moiety to its cognate antigen. When associated with a molecule that comprises the antigen-binding moiety, the idiotype-specific polypeptide can function as a masking moiety of the molecule. Specifically disclosed herein are anti-idiotype antibodies or anti-idiotype-binding antibody fragments specific for the idiotype of anti-CD3 binding molecules.

"Protease" or "proteolytic enzyme" as used herein refers to any proteolytic enzyme that cleaves the linker at a recognition site and that is expressed by a target cell. Such proteases might be secreted by the target cell or remain associated with the target cell, e.g., on the target cell surface. Examples of proteases include but are not limited to metalloproteinases, e.g., matrix metalloproteinase 1-28 and A Disintegrin And Metalloproteinase (ADAM) 2, 7-12, 15, 17-23, 28-30 and 33, serine proteases, e.g., urokinase-type plasminogen activator and Matriptase, cysteine protease, aspartic proteases, and members of the cathepsin family.

"Protease activatable" as used herein, with respect to the T cell activating bispecific molecule, refers to a T cell activating bispecific molecule having reduced or abrogated ability to activate T cells due to a masking moiety that reduces or abrogates the T cell activating bispecific molecule's ability to bind to CD3. Upon dissociation of the masking moiety by proteolytic cleavage, e.g., by proteolytic cleavage of a linker connecting the masking moiety to the T cell activating bispecific molecule, binding to CD3 is restored and the T cell activating bispecific molecule is thereby activated.

"Reversibly concealing" as used herein refers to the binding of a masking moiety or idiotype-specific polypeptide to an antigen-binding moiety or molecule such as to prevent the antigen-binding moiety or molecule from its antigen, e.g., CD3. This concealing is reversible in that the idiotype-specific polypeptide can be released from the antigen-binding moiety or molecule, e.g., by protease cleavage, and thereby freeing the antigen-binding moiety or molecule to bind to its antigen.

**Detailed Description**

In one aspect, the invention relates to a protease-activatable T cell activating bispecific molecule comprising

(a) a first antigen binding moiety capable of specific binding to CD3, wherein the first antigen binding moiety is an antibody or fragment thereof;
(b) a second antigen binding moiety capable of specific binding to a target cell antigen, wherein the second antigen binding moiety is an antibody or fragment thereof; and
(c) a masking moiety covalently attached to the T cell bispecific binding molecule through a protease-cleavable linker, wherein the masking moiety is capable of specific binding to the idiotype of the first or the second antigen binding moiety thereby reversibly concealing the first or second antigen binding moiety, wherein the masking moiety is an anti-idiotypic scFv.

The first antigen binding moiety capable of specific binding to CD3 comprises an idiotype. In one embodiment, the masking moiety of the protease-activatable T cell activating bispecific molecule is covalently attached to the first antigen binding moiety. In one embodiment the masking moiety is covalently attached to the heavy chain variable region of the first antigen binding moiety. In one embodiment the masking moiety is covalently attached to the light chain variable region of the first antigen binding moiety. This covalent bond is separate from the specific binding, which is preferably non-covalent, of the masking moiety to the idiotype first antigen binding site. The idiotype of the first antigen binding moiety comprises its variable region. In one embodiment the masking moiety binds to amino acid residues that make contact with CD3 when the first antigen biding moiety is bound to CD3. In a preferred embodiment, the masking moiety is not the cognate antigen or fragments thereof of the first antigen binding moiety, i.e., the masking moiety is not a CD3 or fragments thereof. In one embodiment the masking moiety is an anti-idiotypic antibody or fragment thereof. In one embodiment, the masking moiety is an anti-idiotypic scFv. Exemplary embodiments of masking moieties which are anti-idiotypic scFv, and protease activatable T cell activating molecules comprising such masking moieties, are described in detail in the examples.

In one embodiment the protease-activatable T cell activating bispecific molecule comprises a second masking moiety reversibly concealing the second antigen binding moiety.

In one embodiment the protease-activatable T cell activating bispecific molecule comprises

(i) a first antigen binding moiety which is a Fab molecule capable of specific binding to CD3, and which comprises at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19;

(ii) a second antigen binding moiety which is a Fab molecule capable of specific binding to a target cell antigen.

In one embodiment the first antigen binding moiety comprises a heavy chain variable region comprising an amino acid sequence that is at least about 95%, 96%, 97%, 98%, 99% or 100% identical to an amino acid sequence of SEQ ID NO: 43 and a light chain variable region comprising an amino acid sequence that is at least about 95%, 96%, 97%, 98%, 99% or 100% identical to an amino acid sequence of SEQ ID NO: 55.

In one embodiment the first antigen binding moiety comprises the heavy chain variable region comprising an amino acid sequence of SEQ ID NO: 43 and the light chain variable region comprising an amino acid sequence of SEQ ID NO: 55.

In a specific embodiment the second antigen binding moiety is capable of specific binding to FolR1 and comprises at least one heavy chain complementarity determining region (CDR) selected from the group consisting of SEQ ID NO: 14, SEQ ID NO: 15 and SEQ ID NO: 16 and at least one light chain CDR selected from the group of SEQ ID NO: 17, SEQ ID NO: 18 and SEQ ID NO: 19.

In another specific embodiment, the second antigen binding moiety is capable of specific binding to FolR1 and comprises a heavy chain variable region comprising an amino acid sequence that is at least about 95%, 96%, 97%, 98%, 99% or 100% identical to the amino acid sequence of SEQ ID NO: 47 and a light chain variable region comprising an amino acid sequence that is at least about 95%, 96%, 97%, 98%, 99% or 100% identical to the amino acid sequence of SEQ ID NO: 55.

In a specific embodiment the second antigen binding moiety is capable of specific binding to FolR1 and comprises at least one heavy chain complementarity determining region (CDR) selected from the group consisting of SEQ ID NO: 151, SEQ ID NO: 152 and SEQ ID NO: 153 and at least one light chain CDR selected from the group of SEQ ID NO: 154, SEQ ID NO: 155 and SEQ ID NO: 156.

In another specific embodiment, the second antigen binding moiety is capable of specific binding to FolR1 and comprises a heavy chain variable region comprising an amino acid sequence that is at least about 95%, 96%, 97%, 98%, 99% or 100% identical to the amino acid sequence of SEQ ID NO: 157 and a light chain variable region comprising an amino acid sequence that is at least about 95%, 96%, 97%, 98%, 99% or 100% identical to the amino acid sequence of SEQ ID NO: 158.

In another specific embodiment, the second antigen binding moiety is capable of specific binding to HER1 and comprises at least one heavy chain complementarity determining region (CDR) selected from the group consisting of SEQ ID NO: 56, SEQ ID NO: 57 and SEQ ID NO: 58 and at least one light chain CDR selected from the group of SEQ ID NO: 59, SEQ ID NO: 60 and SEQ ID NO: 61.

In another specific embodiment, the second antigen binding moiety is capable of specific binding to HER1 and comprises a heavy chain comprising an amino acid sequence that is at least about 95%, 96%, 97%, 98%, 99% or 100% identical to an amino acid sequence of SEQ ID NO: 32, and a light chain comprising an amino acid sequence that is at least about 95%, 96%, 97%, 98%, 99% or 100% identical to an amino acid sequence of SEQ ID NO: 33.

In another specific embodiment, the second antigen binding moiety is capable of specific binding to HER1 and comprises a heavy chain variable region comprising an amino acid sequence that is at least about 95%, 96%, 97%, 98%, 99% or 100% identical to the amino acid sequence of SEQ ID NO: 115 and a light chain variable region comprising an amino acid sequence that is at least about 95%, 96%, 97%, 98%, 99% or 100% identical to the amino acid sequence of SEQ ID NO: 116.

In a specific embodiment the second antigen binding moiety is capable of specific binding to Mesothelin and comprises at least one heavy chain complementarity determining region (CDR) selected from the group consisting of SEQ ID NO: 107, SEQ ID NO: 108 and SEQ ID NO: 109 and at least one light chain CDR selected from the group of SEQ ID NO: 110, SEQ ID NO: 111 and SEQ ID NO: 112.

In another specific embodiment, the second antigen binding moiety is capable of specific binding Mesothelin and comprises a heavy chain variable region comprising an amino acid sequence that is at least about 95%, 96%, 97%, 98%, 99% or 100% identical to the amino acid sequence of SEQ ID NO: 113 and a light chain variable region comprising an amino acid sequence that is at least about 95%, 96%, 97%, 98%, 99% or 100% identical to the amino acid sequence of SEQ ID NO: 114.

In one embodiment the present invention provides a protease-activatable T cell activating bispecific molecule comprising

(i) a first antigen binding moiety which is a Fab molecule capable of specific binding to CD3, comprising at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19;

(ii) a second antigen binding moiety which is a Fab molecule capable of specific binding to FolR1 comprising at least one heavy chain complementarity determining region (CDR) selected from the group consisting of SEQ ID

NO: 14, SEQ ID NO: 15 and SEQ ID NO: 16 and at least one light chain CDR selected from the group of SEQ ID NO: 17, SEQ ID NO: 18 and SEQ ID NO: 19.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55,
(ii) a second antigen binding moiety which is a Fab molecule capable of specific binding to FolR1 comprising heavy chain variable region comprising an amino acid sequence that is at least about 95%, 96%, 97%, 98%, 99% or 100% identical to the amino acid sequence of SEQ ID NO: 47 and a light chain variable region comprising an amino acid sequence that is at least about 95%, 96%, 97%, 98%, 99% or 100% identical to the amino acid sequence of SEQ ID NO: 55.

In one embodiment the present invention provides a protease-activatable T cell activating bispecific molecule comprising

(i) a first antigen binding moiety which is a Fab molecule capable of specific binding to CD3, comprising at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19;
(ii) a second antigen binding moiety which is a Fab molecule capable of specific binding to FolR1 comprising at least one heavy chain complementarity determining region (CDR) selected from the group consisting of SEQ ID NO: 151, SEQ ID NO: 152 and SEQ ID NO: 153 and at least one light chain CDR selected from the group of SEQ ID NO: 154, SEQ ID NO: 155 and SEQ ID NO: 156.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55,
(ii) a second antigen binding moiety which is a Fab molecule capable of specific binding to FolR1 comprising heavy chain variable region comprising an amino acid sequence that is at least about 95%, 96%, 97%, 98%, 99% or 100% identical to the amino acid sequence of SEQ ID NO: 157 and a light chain variable region comprising an amino acid sequence that is at least about 95%, 96%, 97%, 98%, 99% or 100% identical to the amino acid sequence of SEQ ID NO: 158. In one embodiment the present invention provides a protease-activatable T cell activating bispecific molecule comprising

(i) a first antigen binding moiety which is a Fab molecule capable of specific binding to CD3, comprising at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19;
(ii) a second antigen binding moiety which is a Fab molecule capable of specific binding to HER1 comprising at least one heavy chain complementarity determining region (CDR) selected from the group consisting of SEQ ID NO: 56, SEQ ID NO: 57 and SEQ ID NO: 58 and at least one light chain CDR selected from the group of SEQ ID NO: 59, SEQ ID NO: 60 and SEQ ID NO: 61.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100%

identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55.

(ii) a second antigen binding moiety which is a Fab molecule capable of specific binding to HER1 comprising a heavy chain variable region comprising an amino acid sequence that is at least about 95%, 96%, 97%, 98%, 99% or 100% identical to the amino acid sequence of SEQ ID NO: 115 and a light chain variable region comprising an amino acid sequence that is at least about 95%, 96%, 97%, 98%, 99% or 100% identical to the amino acid sequence of SEQ ID NO: 116.

In a particular embodiment, the first antigen binding moiety is a crossover Fab molecule wherein either the variable or the constant regions of the Fab light chain and the Fab heavy chain are exchanged.

In one embodiment the present invention provides a protease-activatable T cell activating bispecific molecule comprising

(i) a first antigen binding moiety which is a Fab molecule capable of specific binding to CD3, comprising at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19;

(ii) a second antigen binding moiety which is a Fab molecule capable of specific binding to Mesothelin comprising at least one heavy chain complementarity determining region (CDR) selected from the group consisting of SEQ ID NO: 107, SEQ ID NO: 108 and SEQ ID NO: 109 and at least one light chain CDR selected from the group of SEQ ID NO: 110, SEQ ID NO: 111 and SEQ ID NO: 112.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55,

(ii) a second antigen binding moiety which is a Fab molecule capable of specific binding to Mesothelin comprising heavy chain variable region comprising an amino acid sequence that is at least about 95%, 96%, 97%, 98%, 99% or 100% identical to the amino acid sequence of SEQ ID NO: 113 and a light chain variable region comprising an amino acid sequence that is at least about 95%, 96%, 97%, 98%, 99% or 100% identical to the amino acid sequence of SEQ ID NO: 114.

In one embodiment, the second antigen binding moiety is a conventional Fab molecule.

In a particular embodiment, the first antigen binding moiety is a crossover Fab molecule wherein the constant regions of the Fab light chain and the Fab heavy chain are exchanged, and the second antigen binding moiety is a conventional Fab molecule. In a further particular embodiment, the first and the second antigen binding moiety are fused to each other, optionally through a peptide linker.

In particular embodiments, the protease-activatable T cell activating bispecific molecule further comprises an Fc domain composed of a first and a second subunit capable of stable association.

In a further particular embodiment, not more than one antigen binding moiety capable of specific binding to CD3 is present in the protease-activatable T cell activating bispecific molecule (i.e. the protease-activatable T cell activating bispecific molecule provides monovalent binding to CD3).

**Protease-activatable T cell activating bispecific molecule formats**

The components of the protease-activatable T cell activating bispecific molecule can be fused to each other in a variety of configurations. Exemplary configurations are depicted in Figures 1A-E and 5A-H. Further exemplary configurations are depicted in Figures 33A-K.

In particular embodiments, the protease-activatable T cell activating bispecific molecule comprises an Fc domain composed of a first and a second subunit capable of stable association. In some embodiments, the second antigen binding moiety is fused at the C-terminus of the Fab heavy chain to the N-terminus of the first or the second subunit of the Fc domain.

In one such embodiment, the first antigen binding moiety is fused at the C-terminus of the Fab heavy chain to

the N-terminus of the Fab heavy chain of the second antigen binding moiety. In a specific such embodiment, the protease-activatable T cell activating bispecific molecule essentially consists of a first and a second antigen binding moiety, an Fc domain composed of a first and a second subunit, and optionally one or more peptide linkers, wherein the first antigen binding moiety is fused at the C-terminus of the Fab heavy chain to the N-terminus of the Fab heavy chain of the second antigen binding moiety, and the second antigen binding moiety is fused at the C-terminus of the Fab heavy chain to the N-terminus of the first or the second subunit of the Fc domain. Optionally, the Fab light chain of the first antigen binding moiety and the Fab light chain of the second antigen binding moiety may additionally be fused to each other. In another such embodiment, the first antigen binding moiety is fused at the C-terminus of the Fab heavy chain to the N-terminus of the first or second subunit of the Fc domain. In a specific such embodiment, the protease-activatable T cell activating bispecific molecule essentially consists of a first and a second antigen binding moiety, an Fc domain composed of a first and a second subunit, and optionally one or more peptide linkers, wherein the first and the second antigen binding moiety are each fused at the C-terminus of the Fab heavy chain to the N-terminus of one of the subunits of the Fc domain.

In other embodiments, the first antigen binding moiety is fused at the C-terminus of the Fab heavy chain to the N-terminus of the first or second subunit of the Fc domain.

In a particular such embodiment, the second antigen binding moiety is fused at the C-terminus of the Fab heavy chain to the N-terminus of the Fab heavy chain of the first antigen binding moiety. In a specific such embodiment, the protease-activatable T cell activating bispecific molecule essentially consists of a first and a second antigen binding moiety, an Fc domain composed of a first and a second subunit, and optionally one or more peptide linkers, wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first or the second subunit of the Fc domain. Optionally, the Fab light chain of the first antigen binding moiety and the Fab light chain of the second antigen binding moiety may additionally be fused to each other. The antigen binding moieties may be fused to the Fc domain or to each other directly or through a peptide linker, comprising one or more amino acids, typically about 2-20 amino acids. Peptide linkers are known in the art and are described herein. Suitable, non-immunogenic peptide linkers include, for example, $(G_4S)_n$, $(SG_4)_n$, $(G_4S)_n$ or $G_4(SG_4)_n$ peptide linkers. "n" is generally a number between 1 and 10, typically between 2 and 4. A particularly suitable peptide linker for fusing the Fab light chains of the first and the second antigen binding moiety to each other is $(G_4S)_2$. An exemplary peptide linker suitable for connecting the Fab heavy chains of the first and the second antigen binding moiety is EPKSC(D)-$(G_4S)_2$ (SEQ ID NOs 105 and 106). Additionally, linkers may comprise (a portion of) an immunoglobulin hinge region. Particularly where an antigen binding moiety is fused to the N-terminus of an Fc domain subunit, it may be fused via an immunoglobulin hinge region or a portion thereof, with or without an additional peptide linker.

A protease-activatable T cell activating bispecific molecule with a single antigen binding moiety capable of specific binding to a target cell antigen is useful, particularly in cases where internalization of the target cell antigen is to be expected following binding of a high affinity antigen binding moiety. In such cases, the presence of more than one antigen binding moiety specific for the target cell antigen may enhance internalization of the target cell antigen, thereby reducing its availability.

In many other cases, however, it will be advantageous to have a protease-activatable T cell activating bispecific molecule comprising two or more antigen binding moieties specific for a target cell antigen (see examples in shown in Figure 5A-H), for example to optimize targeting to the target site or to allow crosslinking of target cell antigens.

Accordingly, in certain embodiments, the protease-activatable T cell activating bispecific molecule of the invention further comprises a third antigen binding moiety which is a Fab molecule capable of specific binding to a target cell antigen. In one embodiment, the third antigen binding moiety is a conventional Fab molecule. In one embodiment, the third antigen binding moiety is capable of specific binding to the same target cell antigen as the second antigen binding moiety. In a particular embodiment, the first antigen binding moiety is capable of specific binding to CD3, and the second and third antigen binding moieties are capable of specific binding to a target cell antigen. In a particular embodiment, the second and the third antigen binding moiety are identical (i.e. they comprise the same amino acid sequences).

In a particular embodiment, the first antigen binding moiety is capable of specific binding to CD3, and the second and third antigen binding moieties are capable of specific binding to FolR1, wherein the second and third antigen binding moieties comprise at least one heavy chain complementarity determining region (CDR) selected from the group consisting of SEQ ID NO: 14, SEQ ID NO: 15 and SEQ ID NO: 16 and at least one light chain CDR selected from the group of SEQ ID NO: 17, SEQ ID NO: 18 and SEQ ID NO: 19.

In a particular embodiment, the first antigen binding moiety is capable of specific binding to CD3, and comprises at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19; and the second and third antigen binding moieties are capable of specific binding to FolR1, wherein the second and third antigen binding moieties comprise at least one heavy chain complementarity determining region (CDR) selected from the group consisting of SEQ ID NO: 14, SEQ ID NO: 15 and SEQ ID NO: 16

and at least one light chain CDR selected from the group of SEQ ID NO: 17, SEQ ID NO: 18 and SEQ ID NO: 19.

In a particular embodiment, the first antigen binding moiety is capable of specific binding to CD3, and comprises at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19; and the second and third antigen binding moieties are capable of specific binding to FolR1, wherein the second and third antigen binding moieties comprise at least one heavy chain complementarity determining region (CDR) selected from the group consisting of SEQ ID NO: 14, SEQ ID NO: 15 and SEQ ID NO: 16 and at least one light chain CDR selected from the group of SEQ ID NO: 17, SEQ ID NO: 18 and SEQ ID NO: 19.

In a particular embodiment, the first antigen binding moiety is capable of specific binding to CD3, and comprises a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, and the second and third antigen binding moieties are capable of specific binding to FolR1, wherein the second and third antigen binding moieties comprise a heavy chain variable region comprising an amino acid sequence that is at least about 95%, 96%, 97%, 98%, 99% or 100% identical to the amino acid sequence of SEQ ID NO: 47 and a light chain variable region comprising an amino acid sequence that is at least about 95%, 96%, 97%, 98%, 99% or 100% identical to the amino acid sequence of SEQ ID NO: 55.

In one embodiment, the first antigen binding moiety is capable of specific binding to CD3, and the second and third antigen binding moieties are capable of specific binding to HER1, wherein the second and third antigen binding moieties comprise at least one heavy chain complementarity determining region (CDR) selected from the group consisting of SEQ ID NO: 56, SEQ ID NO: 57 and SEQ ID NO: 58 and at least one light chain CDR selected from the group of SEQ ID NO: 59, SEQ ID NO: 60 and SEQ ID NO: 61.

In one embodiment, the first antigen binding moiety is capable of specific binding to CD3, and comprises at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19; and the second and third antigen binding moieties are capable of specific binding to HER1, wherein the second and third antigen binding moieties comprise at least one heavy chain complementarity determining region (CDR) selected from the group consisting of SEQ ID NO: 56, SEQ ID NO: 57 and SEQ ID NO: 58 and at least one light chain CDR selected from the group of SEQ ID NO: 59, SEQ ID NO: 60 and SEQ ID NO: 61.

In one embodiment, the first antigen binding moiety is capable of specific binding to CD3, and comprises at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18 and SEQ ID NO: 19; and the second and third antigen binding moieties are capable of specific binding to HER1, wherein the second and third antigen binding moieties comprise at least one heavy chain complementarity determining region (CDR) selected from the group consisting of SEQ ID NO: 56, SEQ ID NO: 57 and SEQ ID NO: 58 and at least one light chain CDR selected from the group of SEQ ID NO: 59, SEQ ID NO: 60 and SEQ ID NO: 61.

In one embodiment, the first antigen binding moiety is capable of specific binding to CD3, and comprises a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, and the second and third antigen binding moieties are capable of specific binding to HER1, wherein the second and third antigen binding moieties comprise a heavy chain variable region comprising an amino acid sequence that is at least about 95%, 96%, 97%, 98%, 99% or 100% identical to the amino acid sequence of SEQ ID NO: 115 and a light chain variable region comprising an amino acid sequence that is at least about 95%, 96%, 97%, 98%, 99% or 100% identical to the amino acid sequence of SEQ ID NO: 116.

In one embodiment, the first antigen binding moiety is capable of specific binding to CD3, and the second and third antigen binding moieties are capable of specific binding to HER2, wherein the second antigen binding moiety comprises at least one heavy chain complementarity determining region (CDR) selected from the group consisting of SEQ ID NO: 142, SEQ ID NO: 143 and SEQ ID NO: 144 and at least one light chain CDR selected from the group of SEQ ID NO: 148, SEQ ID NO: 149 and SEQ ID NO: 150, and wherein the third antigen binding moiety comprises at least one heavy chain complementarity determining region (CDR) selected from the group consisting of SEQ ID NO: 145, SEQ ID NO: 146 and SEQ ID NO: 147 and at least one light chain CDR selected from the group of SEQ ID NO: 148, SEQ ID NO: 149 and SEQ ID NO: 150.

In a particular embodiment, the first antigen binding moiety is capable of specific binding to CD3, and comprises at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19; and the second and third antigen binding moieties are capable of specific binding to HER2, wherein the second antigen binding moiety comprises at least one heavy chain complementarity determining

region (CDR) selected from the group consisting of SEQ ID NO: 142, SEQ ID NO: 143 and SEQ ID NO: 144 and at least one light chain CDR selected from the group of SEQ ID NO: 148, SEQ ID NO: 149 and SEQ ID NO: 150, and wherein the third antigen binding moiety comprises at least one heavy chain complementarity determining region (CDR) selected from the group consisting of SEQ ID NO: 145, SEQ ID NO: 146 and SEQ ID NO: 147 and at least one light chain CDR selected from the group of SEQ ID NO: 148, SEQ ID NO: 149 and SEQ ID NO: 150.

In one embodiment, the first antigen binding moiety is capable of specific binding to CD3, and comprises a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, and the second and third antigen binding moieties are capable of specific binding to HER2, wherein the second antigen binding moiety comprises a heavy chain variable region comprising an amino acid sequence that is at least about 95%, 96%, 97%, 98%, 99% or 100% identical to the amino acid sequence of SEQ ID NO: 160 and a light chain variable region comprising an amino acid sequence that is at least about 95%, 96%, 97%, 98%, 99% or 100% identical to the amino acid sequence of SEQ ID NO: 161, wherein the third antigen binding moiety comprises a heavy chain variable region comprising an amino acid sequence that is at least about 95%, 96%, 97%, 98%, 99% or 100% identical to the amino acid sequence of SEQ ID NO: 159 and a light chain variable region comprising an amino acid sequence that is at least about 95%, 96%, 97%, 98%, 99% or 100% identical to the amino acid sequence of SEQ ID NO: 161.

In a particular embodiment, the first antigen binding moiety is capable of specific binding to CD3, and the second and third antigen binding moieties are capable of specific binding to Mesothelin, wherein the second and third antigen binding moieties comprise at least one heavy chain complementarity determining region (CDR) selected from the group consisting of SEQ ID NO: 107, SEQ ID NO: 108 and SEQ ID NO: 109 and at least one light chain CDR selected from the group of SEQ ID NO: 110, SEQ ID NO: 111 and SEQ ID NO: 112.

In a particular embodiment, the first antigen binding moiety is capable of specific binding to CD3, and comprises at least one heavy chain complementarity determining region (CDR) selected from the group consisting of SEQ ID NO: 44, SEQ ID NO: 45 and SEQ ID NO: 46 and at least one light chain CDR selected from the group of SEQ ID NO: 17, SEQ ID NO: 18, SEQ ID NO: 19; and the second and third antigen binding moieties are capable of specific binding to Mesothelin, wherein the second and third antigen binding moieties comprise at least one heavy chain complementarity determining region (CDR) selected from the group consisting of SEQ ID NO: 107, SEQ ID NO: 108 and SEQ ID NO: 109 and at least one light chain CDR selected from the group of SEQ ID NO: 110, SEQ ID NO: 111 and SEQ ID NO: 112.

In a particular embodiment, the first antigen binding moiety is capable of specific binding to CD3, and comprises a heavy chain variable region comprising an amino acid sequence that is at least about 95%, 96%, 97%, 98%, 99% or 100% identical to the amino acid sequence of SEQ ID NO: 43, and a light chain variable region comprising an amino acid sequence that is at least about 95%, 96%, 97%, 98%, 99% or 100% identical to the amino acid sequence of SEQ ID NO: 55, and the second and third antigen binding moieties are capable of specific binding to Mesothelin, wherein the second and third antigen binding moieties comprise a heavy chain variable region comprising an amino acid sequence that is at least about 95%, 96%, 97%, 98%, 99% or 100% identical to the amino acid sequence of SEQ ID NO: 113 and a light chain variable region comprising an amino acid sequence that is at least about 95%, 96%, 97%, 98%, 99% or 100% identical to the amino acid sequence of SEQ ID NO: 114.

In one embodiment, the first antigen binding moiety is capable of specific binding to CD3, and the second and third antigen binding moieties are capable of specific binding to HER1, wherein the second and third antigen binding moieties comprise at least one heavy chain complementarity determining region (CDR) selected from the group consisting of SEQ ID NO: 56, SEQ ID NO: 57 and SEQ ID NO: 58 and at least one light chain CDR selected from the group consisting of SEQ ID NO: 59, SEQ ID NO: 60 and SEQ ID NO: 61.

In a particular embodiment, the first antigen binding moiety is capable of specific binding to CD3, and comprises at least one heavy chain complementarity determining region (CDR) selected from the group consisting of SEQ ID NO: 44, SEQ ID NO: 45 and SEQ ID NO: 46 and at least one light chain CDR selected from the group consisting of SEQ ID NO: 17, SEQ ID NO: 18 and SEQ ID NO: 19; and the second and third antigen binding moieties are capable of specific binding to HER1, wherein the second and third antigen binding moieties comprise at least one heavy chain complementarity determining region (CDR) selected from the group consisting of SEQ ID NO: 56, SEQ ID NO: 57 and SEQ ID NO: 58 and at least one light chain CDR selected from the group consisting of SEQ ID NO: 59, SEQ ID NO: 60 and SEQ ID NO: 61.

In one embodiment, the third antigen binding moiety is fused at the C-terminus of the Fab heavy chain to the N-terminus of the first or second subunit of the Fc domain. In a more specific embodiment, the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety. Optionally, the Fab light chain of the first antigen binding moiety and the Fab light chain of the second antigen binding moiety may additionally be fused to each other.

The second and the third antigen binding moiety may be fused to the Fc domain directly or through a peptide linker. In a particular embodiment the second and the third antigen binding moiety are each fused to the Fc domain through an immunoglobulin hinge region. In a specific embodiment, the immunoglobulin hinge region is a human $IgG_1$ hinge region. In one embodiment the second and the third antigen binding moiety and the Fc domain are part of an immunoglobulin molecule. In a particular embodiment the immunoglobulin molecule is an IgG class immunoglobulin. In an even more particular embodiment the immunoglobulin is an $IgG_1$ subclass immunoglobulin. In another embodiment the immunoglobulin is an $IgG_4$ subclass immunoglobulin. In a further particular embodiment the immunoglobulin is a human immunoglobulin. In other embodiments the immunoglobulin is a chimeric immunoglobulin or a humanized immunoglobulin. In one embodiment, the protease-activatable T cell activating bispecific molecule essentially consists of an immunoglobulin molecule capable of specific binding to a target cell antigen, and an antigen binding moiety capable of specific binding to CD3 wherein the antigen binding moiety is a Fab molecule, particularly a crossover Fab molecule, fused to the N-terminus of one of the immunoglobulin heavy chains, optionally via a peptide linker.

In a particular embodiment, the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety. In a specific such embodiment, the protease-activatable T cell activating bispecific molecule essentially consists of a first, a second and a third antigen binding moiety, an Fc domain composed of a first and a second subunit, and optionally one or more peptide linkers, wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first subunit of the Fc domain, and wherein the third antigen binding moiety is fused at the C-terminus of the Fab heavy chain to the N-terminus of the second subunit of the Fc domain. Optionally, the Fab light chain of the first antigen binding moiety and the Fab light chain of the second antigen binding moiety may additionally be fused to each other.

In one embodiment the present invention provides a protease-activatable T cell activating bispecific molecule comprising

(i) a first antigen binding moiety which is a Fab molecule capable of specific binding to CD3, comprising the heavy chain complementarity determining region (CDR) 1 of SEQ ID NO: 44, the heavy chain CDR 2 of SEQ ID NO: 45, the heavy chain CDR 3 of SEQ ID NO: 46, the light chain CDR 1 of SEQ ID NO: 17, the light chain CDR 2 of SEQ ID NO: 18 and the light chain CDR 3 of SEQ ID NO: 19, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to FolR1 comprising the heavy chain CDR 1 of SEQ ID NO: 14, the heavy chain CDR 2 of SEQ ID NO: 15, the heavy chain CDR 3 of SEQ ID NO: 16, the light chain CDR 1 of SEQ ID NO: 17, the light chain CDR 2 of SEQ ID NO: 18 and the light chain CDR3 of SEQ ID NO: 19.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to FolR1 comprising heavy chain variable region comprising an amino acid sequence that is at least about 95%, 96%, 97%, 98%, 99% or 100% identical to the amino acid sequence of SEQ ID NO: 47 and a light chain variable region comprising an amino acid sequence that is at least about 95%, 96%, 97%, 98%, 99% or 100% identical to the amino acid sequence of SEQ ID NO: 55.

In one embodiment the present invention provides a protease-activatable T cell activating bispecific molecule comprising

(i) a first antigen binding moiety which is a Fab molecule capable of specific binding to CD3, comprising the heavy chain complementarity determining region (CDR) 1 of SEQ ID NO: 44, the heavy chain CDR 2 of SEQ ID NO: 45, the heavy chain CDR 3 of SEQ ID NO: 46, the light chain CDR 1 of SEQ ID NO: 17, the light chain CDR 2 of SEQ

ID NO: 18 and the light chain CDR 3 of SEQ ID NO: 19, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to FolR1 comprising the heavy chain CDR 1 of SEQ ID NO: 151, the heavy chain CDR 2 of SEQ ID NO: 152, the heavy chain CDR 3 of SEQ ID NO: 153, the light chain CDR 1 of SEQ ID NO: 154, the light chain CDR 2 of SEQ ID NO: 155 and the light chain CDR3 of SEQ ID NO: 156.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to FolR1 comprising heavy chain variable region comprising an amino acid sequence that is at least about 95%, 96%, 97%, 98%, 99% or 100% identical to the amino acid sequence of SEQ ID NO: 157 and a light chain variable region comprising an amino acid sequence that is at least about 95%, 96%, 97%, 98%, 99% or 100% identical to the amino acid sequence of SEQ ID NO: 158.

In one embodiment the present invention provides a protease-activatable T cell activating bispecific molecule comprising

(i) a first antigen binding moiety which is a Fab molecule capable of specific binding to CD3, comprising the heavy chain complementarity determining region (CDR) 1 of SEQ ID NO: 44, the heavy chain CDR 2 of SEQ ID NO: 45, the heavy chain CDR 3 of SEQ ID NO: 46, the light chain CDR 1 of SEQ ID NO: 17, the light chain CDR 2 of SEQ ID NO: 18 and the light chain CDR 3 of SEQ ID NO: 19, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to HER1 comprising the heavy chain CDR 1 of SEQ ID NO: 56, the heavy chain CDR 2 of SEQ ID NO: 57, the heavy chain CDR 3 of SEQ ID NO: 58, the light chain CDR 1 of SEQ ID NO: 59, the light chain CDR 2 of SEQ ID NO: 60 and the light chain CDR3 of SEQ ID NO: 61.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to HER1 comprising a heavy chain variable region comprising an amino acid sequence that is at least about 95%, 96%, 97%, 98%, 99% or 100% identical to the amino acid sequence of SEQ ID NO: 115 and a light chain variable region comprising an amino acid sequence that is at least about 95%, 96%, 97%, 98%, 99% or 100% identical to the amino acid sequence of SEQ ID NO: 116.

In one embodiment the present invention provides a protease-activatable T cell activating bispecific molecule comprising

(i) a first antigen binding moiety which is a Fab molecule capable of specific binding to CD3, comprising the heavy chain complementarity determining region (CDR) 1 of SEQ ID NO: 44, the heavy chain CDR 2 of SEQ ID NO: 45, the heavy chain CDR 3 of SEQ ID NO: 46, the light chain CDR 1 of SEQ ID NO: 17, the light chain CDR 2 of SEQ

ID NO: 18 and the light chain CDR 3 of SEQ ID NO: 19, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to HER2, wherein the second antigen binding moiety comprises the heavy chain CDR 1 of SEQ ID NO: 142, the heavy chain CDR 2 of SEQ ID NO: 143, the heavy chain CDR 3 of SEQ ID NO: 144, the light chain CDR 1 of SEQ ID NO: 148, the light chain CDR 2 of SEQ ID NO: 149 and the light chain CDR3 of SEQ ID NO: 150, and wherein the third antigen binding moiety comprises the heavy chain CDR 1 of SEQ ID NO: 145, the heavy chain CDR 2 of SEQ ID NO: 146, the heavy chain CDR 3 of SEQ ID NO: 148, the light chain CDR 1 of SEQ ID NO: 148, the light chain CDR 2 of SEQ ID NO: 149 and the light chain CDR3 of SEQ ID NO: 150.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to HER2, wherein the second antigen binding moiety comprises a heavy chain variable region comprising an amino acid sequence that is at least about 95%, 96%, 97%, 98%, 99% or 100% identical to the amino acid sequence of SEQ ID NO: 160 and a light chain variable region comprising an amino acid sequence that is at least about 95%, 96%, 97%, 98%, 99% or 100% identical to the amino acid sequence of SEQ ID NO: 161, and wherein the third antigen binding moiety comprises a heavy chain variable region comprising an amino acid sequence that is at least about 95%, 96%, 97%, 98%, 99% or 100% identical to the amino acid sequence of SEQ ID NO: 159 and a light chain variable region comprising an amino acid sequence that is at least about 95%, 96%, 97%, 98%, 99% or 100% identical to the amino acid sequence of SEQ ID NO: 161.

In one embodiment the present invention provides a protease-activatable T cell activating bispecific molecule comprising

(i) a first antigen binding moiety which is a Fab molecule capable of specific binding to CD3, comprising the heavy chain complementarity determining region (CDR) 1 of SEQ ID NO: 44, the heavy chain CDR 2 of SEQ ID NO: 45, the heavy chain CDR 3 of SEQ ID NO: 46, the light chain CDR 1 of SEQ ID NO: 17, the light chain CDR 2 of SEQ ID NO: 18 and the light chain CDR 3 of SEQ ID NO: 19, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to Mesothelin comprising the heavy chain CDR 1 of SEQ ID NO: 107, the heavy chain CDR 2 of SEQ ID NO: 108, the heavy chain CDR 3 of SEQ ID NO: 109, the light chain CDR 1 of SEQ ID NO: 110, the light chain CDR 2 of SEQ ID NO: 111 and the light chain CDR3 of SEQ ID NO: 112.

In one embodiment the present invention provides a protease-activatable T cell activating bispecific molecule comprising

(i) a first antigen binding moiety which is a Fab molecule capable of specific binding to CD3 comprising a heavy chain variable region comprising an amino acid sequence that is at least about 95%, 96%, 97%, 98%, 99% or 100% identical to the amino acid sequence of SEQ ID NO: 43 and a light chain variable region comprising an amino acid sequence that is at least about 95%, 96%, 97%, 98%, 99% or 100% identical to the amino acid sequence of SEQ ID NO: 55, wherein the first antigen binding moiety is a crossover Fab molecule wherein either the variable or the constant regions, particularly the constant regions, of the Fab light chain and the Fab heavy chain are exchanged;
(ii) a second and a third antigen binding moiety each of which is a Fab molecule capable of specific binding to Mesothelin comprising heavy chain variable region comprising an amino acid sequence that is at least about 95%, 96%, 97%, 98%, 99% or 100% identical to the amino acid sequence of SEQ ID NO: 113 and a light chain variable region comprising an amino acid sequence that is at least about 95%, 96%, 97%, 98%, 99% or 100% identical to the amino acid sequence of SEQ ID NO: 114.

The protease-activatable T cell activating bispecific molecule according to any of the ten above embodiments may further comprise (iii) an Fc domain composed of a first and a second subunit capable of stable association, wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first subunit of the Fc domain, and wherein the third antigen binding moiety is fused at the C-terminus of the Fab heavy chain to the N-terminus of the second subunit of the Fc domain.

In some of the protease-activatable T cell activating bispecific molecule of the invention, the Fab light chain of the first antigen binding moiety and the Fab light chain of the second antigen binding moiety are fused to each other, optionally via a linker peptide. Depending on the configuration of the first and the second antigen binding moiety, the Fab light chain of the first antigen binding moiety may be fused at its C-terminus to the N-terminus of the Fab light chain of the second antigen binding moiety, or the Fab light chain of the second antigen binding moiety may be fused at its C-terminus to the N-terminus of the Fab light chain of the first antigen binding moiety. Fusion of the Fab light chains of the first and the second antigen binding moiety further reduces mispairing of unmatched Fab heavy and light chains, and also reduces the number of plasmids needed for expression of some of the protease-activatable T cell activating bispecific molecule of the invention.

In certain embodiments the protease-activatable T cell activating bispecific molecule comprises a polypeptide wherein the Fab light chain variable region of the first antigen binding moiety shares a carboxy-terminal peptide bond with the Fab heavy chain constant region of the first antigen binding moiety (i.e. a the first antigen binding moiety comprises a crossover Fab heavy chain, wherein the heavy chain variable region is replaced by a light chain variable region), which in turn shares a carboxy-terminal peptide bond with an Fc domain subunit ($VL_{(1)}$-$CH1_{(1)}$-CH2-CH3(-CH4)), and a polypeptide wherein a the Fab heavy chain of the second antigen binding moiety shares a carboxy-terminal peptide bond with an Fc domain subunit ($VH_{(2)}$-$CH1_{(2)}$-CH2-CH3(-CH4)). In some embodiments the protease-activatable T cell activating bispecific molecule further comprises a polypeptide wherein the Fab heavy chain variable region of the first antigen binding moiety shares a carboxy-terminal peptide bond with the Fab light chain constant region of the first antigen binding moiety ($VH_{(1)}$-$CL_{(1)}$) and the Fab light chain polypeptide of the second antigen binding moiety ($VL_{(2)}$-$CL_{(2)}$). In certain embodiments the polypeptides are covalently linked, e.g., by a disulfide bond.

In alternative embodiments the protease-activatable T cell activating bispecific molecule comprises a polypeptide wherein the Fab heavy chain variable region of the first antigen binding moiety shares a carboxy-terminal peptide bond with the Fab light chain constant region of the first antigen binding moiety (i.e. the first antigen binding moiety comprises a crossover Fab heavy chain, wherein the heavy chain constant region is replaced by a light chain constant region), which in turn shares a carboxy-terminal peptide bond with an Fc domain subunit (VH(i)-CL(i)-CH2-CH3(-CH4)), and a polypeptide wherein the Fab heavy chain of the second antigen binding moiety shares a carboxy-terminal peptide bond with an Fc domain subunit ($VH_{(2)}$-$CH1_{(2)}$-CH2-CH3(-CH4)). In some embodiments the protease-activatable T cell activating bispecific molecule further comprises a polypeptide wherein the Fab light chain variable region of the first antigen binding moiety shares a carboxy-terminal peptide bond with the Fab heavy chain constant region of the first antigen binding moiety ($VL_{(1)}$-$CH1_{(1)}$) and the Fab light chain polypeptide of the second antigen binding moiety ($VL_{(2)}$-$CL_{(2)}$). In certain embodiments the polypeptides are covalently linked, e.g., by a disulfide bond.

In some embodiments, the protease-activatable T cell activating bispecific molecule comprises a polypeptide wherein the Fab light chain variable region of the first antigen binding moiety shares a carboxy-terminal peptide bond with the Fab heavy chain constant region of the first antigen binding moiety (i.e. the first antigen binding moiety comprises a crossover Fab heavy chain, wherein the heavy chain variable region is replaced by a light chain variable region), which in turn shares a carboxy-terminal peptide bond with the Fab heavy chain of the second antigen binding moiety, which in turn shares a carboxy-terminal peptide bond with an Fc domain subunit ($VL_{(1)}$-$CH1_{(1)}$-$VH_{(2)}$-$CH1_{(2)}$-CH2-CH3(-CH4)). In other embodiments, the protease-activatable T cell activating bispecific molecule comprises a polypeptide wherein the Fab heavy chain variable region of the first antigen binding moiety shares a carboxy-terminal peptide bond with the Fab light chain constant region of the first antigen binding moiety (i.e. the first antigen binding moiety comprises a crossover Fab heavy chain, wherein the heavy chain constant region is replaced by a light chain constant region), which in turn shares a carboxy-terminal peptide bond with the Fab heavy chain of the second antigen binding moiety, which in turn shares a carboxy-terminal peptide bond with an Fc domain subunit ($VH_{(1)}$-$CL_{(1)}$-$VH_{(2)}$-$CH1_{(2)}$-CH2-CH3(-CH4)). In still other embodiments, the protease-activatable T cell activating bispecific molecule comprises a polypeptide wherein the Fab heavy chain of the second antigen binding moiety shares a carboxy-terminal peptide bond with the Fab light chain variable region of the first antigen binding moiety which in turn shares a carboxy-terminal peptide bond with the Fab heavy chain constant region of the first antigen binding moiety (i.e. the first antigen binding moiety comprises a crossover Fab heavy chain, wherein the heavy chain variable region is replaced by a light chain variable region), which in turn shares a carboxy-terminal peptide bond with an Fc domain subunit ($VH_{(2)}$-$CH1_{(2)}$-$VL_{(1)}$-$CH1_{(1)}$-CH2-CH3(-CH4)). In other embodiments, the protease-activatable T cell activating bispecific molecule comprises a polypeptide wherein the Fab heavy chain of the second antigen binding moiety shares a carboxy-terminal peptide bond with the Fab heavy chain variable region of the first antigen binding moiety which in turn shares a carboxy-terminal peptide bond with the

Fab light chain constant region of the first antigen binding moiety (i.e. the first antigen binding moiety comprises a crossover Fab heavy chain, wherein the heavy chain constant region is replaced by a light chain constant region), which in turn shares a carboxy-terminal peptide bond with an Fc domain subunit ($VH_{(2)}$-$CH1_{(2)}$-$VH_{(1)}$-$CL_{(1)}$-CH2-CH3(-CH4)).

In some of these embodiments the protease-activatable T cell activating bispecific molecule further comprises a crossover Fab light chain polypeptide of the first antigen binding moiety, wherein the Fab heavy chain variable region of the first antigen binding moiety shares a carboxy-terminal peptide bond with the Fab light chain constant region of the first antigen binding moiety (VH(i)-CL(i)), and the Fab light chain polypeptide of the second antigen binding moiety ($VL_{(2)}$-$CL_{(2)}$). In others of these embodiments the protease-activatable T cell activating bispecific molecule further comprises a crossover Fab light chain polypeptide, wherein the Fab light chain variable region of the first antigen binding moiety shares a carboxy-terminal peptide bond with the Fab heavy chain constant region of the first antigen binding moiety ($VL_{(1)}$-$CH1_{(1)}$), and the Fab light chain polypeptide of the second antigen binding moiety ($VL_{(2)}$-$CL_{(2)}$). In still others of these embodiments the protease-activatable T cell activating bispecific molecule further comprises a polypeptide wherein the Fab light chain variable region of the first antigen binding moiety shares a carboxy-terminal peptide bond with the Fab heavy chain constant region of the first antigen binding moiety which in turn shares a carboxy-terminal peptide bond with the Fab light chain polypeptide of the second antigen binding moiety ($VL_{(1)}$-$CH1_{(1)}$-$VL_{(2)}$-$CL_{(2)}$), a polypeptide wherein the Fab heavy chain variable region of the first antigen binding moiety shares a carboxy-terminal peptide bond with the Fab light chain constant region of the first antigen binding moiety which in turn shares a carboxy-terminal peptide bond with the Fab light chain polypeptide of the second antigen binding moiety ($VH_{(1)}$-$CL_{(1)}$-$VL_{(2)}$-$CL_{(2)}$), a polypeptide wherein the Fab light chain polypeptide of the second antigen binding moiety shares a carboxy-terminal peptide bond with the Fab light chain variable region of the first antigen binding moiety which in turn shares a carboxy-terminal peptide bond with the Fab heavy chain constant region of the first antigen binding moiety ($VL_{(2)}$-$CL_{(2)}$-$VL_{(1)}$-$CH1_{(1)}$), or a polypeptide wherein the Fab light chain polypeptide of the second antigen binding moiety shares a carboxy-terminal peptide bond with the Fab heavy chain variable region of the first antigen binding moiety which in turn shares a carboxy-terminal peptide bond with the Fab light chain constant region of the first antigen binding moiety ($VL_{(2)}$-$CL_{(2)}$-$VH_{(1)}$-$CL_{(1)}$).

The protease-activatable T cell activating bispecific molecule according to these embodiments may further comprise (i) an Fc domain subunit polypeptide (CH2-CH3(-CH4)), or (ii) a polypeptide wherein the Fab heavy chain of a third antigen binding moiety shares a carboxy-terminal peptide bond with an Fc domain subunit ($VH_{(3)}$-$CH1_{(3)}$-CH2-CH3(-CH4)) and the Fab light chain polypeptide of a third antigen binding moiety ($VL_{(3)}$-$CL_{(3)}$). In certain embodiments the polypeptides are covalently linked, e.g., by a disulfide bond.

According to any of the above embodiments, components of the protease-activatable T cell activating bispecific molecule (e.g., antigen binding moiety, Fc domain) may be fused directly or through various linkers, particularly peptide linkers comprising one or more amino acids, typically about 2-20 amino acids, that are described herein or are known in the art. Suitable, non-immunogenic peptide linkers include, for example, $(G_4S)_n$, $(SG_4)_n$, $(G_4S)_n$ or $G_4(SG_4)_n$ peptide linkers, wherein n is generally a number between 1 and 10, typically between 2 and 4.

**Fc domain**

The Fc domain of the protease-activatable T cell activating bispecific molecule consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. In one embodiment the protease-activatable T cell activating bispecific molecule of the invention comprises not more than one Fc domain.

In one embodiment according the invention the Fc domain of the protease-activatable T cell activating bispecific molecule is an IgG Fc domain. In a particular embodiment the Fc domain is an $IgG_1$ Fc domain. In another embodiment the Fc domain is an $IgG_4$ Fc domain. In a more specific embodiment, the Fc domain is an $IgG_4$ Fc domain comprising an amino acid substitution at position S228 (Kabat numbering), particularly the amino acid substitution S228P. This amino acid substitution reduces in vivo Fab arm exchange of $IgG_4$ antibodies (see Stubenrauch et al., Drug Metabolism and Disposition 38, 84-91 (2010)). In a further particular embodiment the Fc domain is human.

*Fc domain modifications promoting heterodimerization*

Protease-activatable T cell activating bispecific molecules according to the invention comprise different antigen binding moieties, fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of protease-activatable T cell activating bispecific molecules in recombinant production, it will thus be advantageous to introduce in the Fc domain of the protease-activatable T cell activating bispecific molecule a modification promoting the

association of the desired polypeptides. Accordingly, in particular embodiments the Fc domain of the protease-activatable T cell activating bispecific molecule according to the invention comprises a modification promoting the association of the first and the second subunit of the Fc domain. The site of most extensive protein-protein interaction between the two subunits of a human IgG Fc domain is in the CH3 domain of the Fc domain. Thus, in one embodiment said modification is in the CH3 domain of the Fc domain.

In a specific embodiment said modification is a so-called "knob-into-hole" modification, comprising a "knob" modification in one of the two subunits of the Fc domain and a "hole" modification in the other one of the two subunits of the Fc domain.

The knob-into-hole technology is described e.g., in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g., tyrosine or tryptophan). Compensatory cavities of identical or similar size to the protuberances are created in the interface of the second polypeptide by replacing large amino acid side chains with smaller ones (e.g., alanine or threonine).

Accordingly, in a particular embodiment, in the CH3 domain of the first subunit of the Fc domain of the protease-activatable T cell activating bispecific molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g., by site-specific mutagenesis, or by peptide synthesis.

In a specific embodiment, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A).

In yet a further embodiment, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter, J Immunol Methods 248, 7-15 (2001)).

In a particular embodiment the antigen binding moiety capable of binding to CD3 is fused (optionally via the antigen binding moiety capable of binding to a target cell antigen) to the first subunit of the Fc domain (comprising the "knob" modification). Without wishing to be bound by theory, fusion of the antigen binding moiety capable of binding to CD3 to the knob-containing subunit of the Fc domain will (further) minimize the generation of antigen binding molecules comprising two antigen binding moieties capable of binding to CD3 (steric clash of two knob-containing polypeptides).

In an alternative embodiment a modification promoting association of the first and the second subunit of the Fc domain comprises a modification mediating electrostatic steering effects, e.g., as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

*Fc domain modifications reducing Fc receptor binding and/or effector function*

The Fc domain confers to the protease-activatable T cell activating bispecific molecule favorable pharmacokinetic properties, including a long serum half-life which contributes to good accumulation in the target tissue and a favorable tissue-blood distribution ratio. At the same time it may, however, lead to undesirable targeting of the protease-activatable T cell activating bispecific molecule to cells expressing Fc receptors rather than to the preferred antigen-bearing cells. Moreover, the co-activation of Fc receptor signaling pathways may lead to cytokine release which, in combination with the T cell activating properties and the long half-life of the antigen binding molecule, results in excessive activation of cytokine receptors and severe side effects upon systemic administration. Activation of (Fc receptor-bearing) immune cells other than T cells may even reduce efficacy of the protease-activatable T cell activating bispecific molecule due to the potential destruction of T cells e.g., by NK cells.

Accordingly, in particular embodiments the Fc domain of the protease-activatable T cell activating bispecific

molecules according to the invention exhibits reduced binding affinity to an Fc receptor and/or reduced effector function, as compared to a native $IgG_1$ Fc domain. In one such embodiment the Fc domain (or the protease-activatable T cell activating bispecific molecule comprising said Fc domain) exhibits less than 50%, preferably less than 20%, more preferably less than 10% and most preferably less than 5% of the binding affinity to an Fc receptor, as compared to a native $IgG_1$ Fc domain (or a protease-activatable T cell activating bispecific molecule comprising a native $IgG_1$ Fc domain), and/or less than 50%, preferably less than 20%, more preferably less than 10% and most preferably less than 5% of the effector function, as compared to a native $IgG_1$ Fc domain domain (or a protease-activatable T cell activating bispecific molecule comprising a native $IgG_1$ Fc domain). In one embodiment, the Fc domain domain (or the protease-activatable T cell activating bispecific molecule comprising said Fc domain) does not substantially bind to an Fc receptor and/or induce effector function. In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcyRIIIa, FcyRI or FcyRIIa, most specifically human FcyRIIIa. In one embodiment the effector function is one or more selected from the group of CDC, ADCC, ADCP, and cytokine secretion. In a particular embodiment the effector function is ADCC. In one embodiment the Fc domain domain exhibits substantially similar binding affinity to neonatal Fc receptor (FcRn), as compared to a native $IgG_1$ Fc domain domain. Substantially similar binding to FcRn is achieved when the Fc domain (or the protease-activatable T cell activating bispecific molecule comprising said Fc domain) exhibits greater than about 70%, particularly greater than about 80%, more particularly greater than about 90% of the binding affinity of a native $IgG_1$ Fc domain (or the protease-activatable T cell activating bispecific molecule comprising a native $IgG_1$ Fc domain) to FcRn.

In certain embodiments the Fc domain is engineered to have reduced binding affinity to an Fc receptor and/or reduced effector function, as compared to a non-engineered Fc domain. In particular embodiments, the Fc domain of the protease-activatable T cell activating bispecific molecule comprises one or more amino acid mutation that reduces the binding affinity of the Fc domain to an Fc receptor and/or effector function. Typically, the same one or more amino acid mutation is present in each of the two subunits of the Fc domain. In one embodiment the amino acid mutation reduces the binding affinity of the Fc domain to an Fc receptor. In one embodiment the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment the protease-activatable T cell activating bispecific molecule comprising an engineered Fc domain exhibits less than 20%, particularly less than 10%, more particularly less than 5% of the binding affinity to an Fc receptor as compared to a protease-activatable T cell activating bispecific molecule comprising a non-engineered Fc domain. In a particular embodiment the Fc receptor is an Fcγ receptor. In some embodiments the Fc receptor is a human Fc receptor. In some embodiments the Fc receptor is an activating Fc receptor. In a specific embodiment the Fc receptor is an activating human Fcγ receptor, more specifically human FcyRIIIa, FcyRI or FcyRIIa, most specifically human FcyRIIIa. Preferably, binding to each of these receptors is reduced. In some embodiments binding affinity to a complement component, specifically binding affinity to C1q, is also reduced. In one embodiment binding affinity to neonatal Fc receptor (FcRn) is not reduced. Substantially similar binding to FcRn, i.e. preservation of the binding affinity of the Fc domain to said receptor, is achieved when the Fc domain (or the protease-activatable T cell activating bispecific molecule comprising said Fc domain) exhibits greater than about 70% of the binding affinity of a non-engineered form of the Fc domain (or the protease-activatable T cell activating bispecific molecule comprising said non-engineered form of the Fc domain) to FcRn. The Fc domain, or protease-activatable T cell activating bispecific molecules of the invention comprising said Fc domain, may exhibit greater than about 80% and even greater than about 90% of such affinity. In certain embodiments the Fc domain of the protease-activatable T cell activating bispecific molecule is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one embodiment the reduced effector function is one or more selected from the group of reduced CDC, reduced ADCC, reduced ADCP, and reduced cytokine secretion. In a particular embodiment the reduced effector function is reduced ADCC. In one embodiment the reduced ADCC is less than 20% of the ADCC induced by a non-engineered Fc domain (or a protease-activatable T cell activating bispecific molecule comprising a non-engineered Fc domain).

In one embodiment the amino acid mutation that reduces the binding affinity of the Fc domain to an Fc receptor and/or effector function is an amino acid substitution. In one embodiment the Fc domain comprises an amino acid substitution at a position selected from the group of E233, L234, L235, N297, P331 and P329. In a more specific embodiment the Fc domain comprises an amino acid substitution at a position selected from the group of L234, L235

and P329. In some embodiments the Fc domain comprises the amino acid substitutions L234A and L235A. In one such embodiment, the Fc domain is an $IgG_1$ Fc domain, particularly a human $IgG_1$ Fc domain. In one embodiment the Fc domain comprises an amino acid substitution at position P329. In a more specific embodiment the amino acid substitution is P329A or P329G, particularly P329G. In one embodiment the Fc domain comprises an amino acid substitution at position P329 and a further amino acid substitution at a position selected from E233, L234, L235, N297 and P331. In a more specific embodiment the further amino acid substitution is E233P, L234A, L235A, L235E, N297A, N297D or P331S. In particular embodiments the Fc domain comprises amino acid substitutions at positions P329, L234 and L235. In more particular embodiments the Fc domain comprises the amino acid mutations L234A, L235A and P329G ("P329G LALA"). In one such embodiment, the Fc domain is an $IgG_1$ Fc domain, particularly a human $IgG_1$ Fc domain. The "P329G LALA" combination of amino acid substitutions almost completely abolishes Fcγ receptor (as well as complement) binding of a human $IgG_1$ Fc domain, as described in PCT publication no. WO 2012/130831. WO 2012/130831 also describes methods of preparing such mutant Fc domains and methods for determining its properties such as Fc receptor binding or effector functions.

$IgG_4$ antibodies exhibit reduced binding affinity to Fc receptors and reduced effector functions as compared to $IgG_1$ antibodies. Hence, in some embodiments the Fc domain of the protease-activatable T cell activating bispecific molecules of the invention is an $IgG_4$ Fc domain, particularly a human $IgG_4$ Fc domain. In one embodiment the $IgG_4$ Fc domain comprises amino acid substitutions at position S228, specifically the amino acid substitution S228P. To further reduce its binding affinity to an Fc receptor and/or its effector function, in one embodiment the $IgG_4$ Fc domain comprises an amino acid substitution at position L235, specifically the amino acid substitution L235E. In another embodiment, the $IgG_4$ Fc domain comprises an amino acid substitution at position P329, specifically the amino acid substitution P329G. In a particular embodiment, the $IgG_4$ Fc domain comprises amino acid substitutions at positions S228, L235 and P329, specifically amino acid substitutions S228P, L235E and P329G. Such $IgG_4$ Fc domain mutants and their Fcγ receptor binding properties are described in PCT publication no. WO 2012/130831.

In a particular embodiment the Fc domain exhibiting reduced binding affinity to an Fc receptor and/or reduced effector function, as compared to a native $IgG_1$ Fc domain, is a human $IgG_1$ Fc domain comprising the amino acid substitutions L234A, L235A and optionally P329G, or a human $IgG_4$ Fc domain comprising the amino acid substitutions S228P, L235E and optionally P329G.

In certain embodiments N-glycosylation of the Fc domain has been eliminated. In one such embodiment the Fc domain comprises an amino acid mutation at position N297, particularly an amino acid substitution replacing asparagine by alanine (N297A) or aspartic acid (N297D).

In addition to the Fc domains described hereinabove and in PCT publication no. WO 2012/130831, Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g., by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or a protease-activatable T cell activating bispecific molecule comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo*, e.g., in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the protease-activatable T cell activating bispecific molecule is able to bind C1q and hence has CDC activity. See e.g., C1q and C3c

binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

**Antigen Binding Moieties**

The antigen binding molecule of the invention is bispecific, i.e. it comprises at least two antigen binding moieties capable of specific binding to two distinct antigenic determinants. According to the invention, the antigen binding moieties are Fab molecules (i.e. antigen binding domains composed of a heavy and a light chain, each comprising a variable and a constant region). In one embodiment said Fab molecules are human. In another embodiment said Fab molecules are humanized. In yet another embodiment said Fab molecules comprise human heavy and light chain constant regions.

At least one of the antigen binding moieties is a crossover Fab molecule. Such modification prevent mispairing of heavy and light chains from different Fab molecules, thereby improving the yield and purity of the protease-activatable T cell activating bispecific molecule of the invention in recombinant production. In a particular crossover Fab molecule useful for the protease-activatable T cell activating bispecific molecule of the invention, the constant regions of the Fab light chain and the Fab heavy chain are exchanged. In another crossover Fab molecule useful for the protease-activatable T cell activating bispecific molecule of the invention, the variable regions of the Fab light chain and the Fab heavy chain are exchanged.

In a particular embodiment according to the invention, the protease-activatable T cell activating bispecific molecule is capable of simultaneous binding to a target cell antigen, particularly a tumor cell antigen, and CD3. In one embodiment, the protease-activatable T cell activating bispecific molecule is capable of crosslinking a T cell and a target cell by simultaneous binding to a target cell antigen and CD3. In an even more particular embodiment, such simultaneous binding results in lysis of the target cell, particularly a tumor cell. In one embodiment, such simultaneous binding results in activation of the T cell. In other embodiments, such simultaneous binding results in a cellular response of a T lymphocyte, particularly a cytotoxic T lymphocyte, selected from the group of proliferation, differentiation, cytokine secretion, cytotoxic effector molecule release, cytotoxic activity, and expression of activation markers. In one embodiment, binding of the protease-activatable T cell activating bispecific molecule to CD3 without simultaneous binding to the target cell antigen does not result in T cell activation.

In one embodiment, the protease-activatable T cell activating bispecific molecule is capable of re-directing cytotoxic activity of a T cell to a target cell. In a particular embodiment, said redirection is independent of MHC-mediated peptide antigen presentation by the target cell and and/or specificity of the T cell.

Particularly, a T cell according to any of the embodiments of the invention is a cytotoxic T cell. In some embodiments the T cell is a $CD4^+$ or a $CD8^+$ T cell, particularly a $CD8^+$ T cell.

*CD3 binding moiety*

The protease-activatable T cell activating bispecific molecule of the invention comprises at least one antigen binding moiety capable of binding to CD3 (also referred to herein as an "CD3 antigen binding moiety" or "first antigen binding moiety"). In a particular embodiment, the protease-activatable T cell activating bispecific molecule comprises not more than one antigen binding moiety capable of specific binding to CD3. In one embodiment the protease-activatable T cell activating bispecific molecule provides monovalent binding to CD3. The CD3 antigen binding is a crossover Fab molecule, i.e. a Fab molecule wherein either the variable or the constant regions of the Fab heavy and light chains are exchanged. In embodiments where there is more than one antigen binding moiety capable of specific binding to a target cell antigen comprised in the protease-activatable T cell activating bispecific molecule, the antigen binding moiety capable of specific binding to CD3 preferably is a crossover Fab molecule and the antigen binding moieties capable of specific binding to a target cell antigen are conventional Fab molecules.

In a particular embodiment CD3 is human CD3 or cynomolgus CD3, most particularly human CD3. In a particular embodiment the CD3 antigen binding moiety is cross-reactive for (i.e. specifically binds to) human and cynomolgus CD3. In some embodiments, the first antigen binding moiety is capable of specific binding to the epsilon subunit of CD3.

The CD3 antigen binding moiety comprises at least one heavy chain complementarity determining region (CDR) selected from the group consisting of SEQ ID NO: 11, SEQ ID NO: 12 and SEQ ID NO: 13 and at least one light chain CDR selected from the group of SEQ ID NO: 17, SEQ ID NO: 18, SEQ ID NO: 19.

In one embodiment the CD3 antigen binding moiety comprises the heavy chain CDR1 of SEQ ID NO: 11, the heavy chain CDR2 of SEQ ID NO: 12, the heavy chain CDR3 of SEQ ID NO: 13, the light chain CDR1 of SEQ ID NO: 17, the light chain CDR2 of SEQ ID NO: 18, and the light chain CDR3 of SEQ ID NO: 19.

In one embodiment the CD3 antigen binding moiety comprises the heavy chain CDR1 of SEQ ID NO: 44, the heavy chain CDR2 of SEQ ID NO: 45, the heavy chain CDR3 of SEQ ID NO: 46, the light chain CDR1 of SEQ ID NO: 17, the light chain CDR2 of SEQ ID NO: 18, and the light chain CDR3 of SEQ ID NO: 19.

In one embodiment the CD3 antigen binding moiety comprises a heavy chain variable region sequence that is at least about 95%, 96%, 97%, 98%, 99% or 100% identical to the amino acid sequence of SEQ ID NO: 43, and a light chain variable region sequence that is at least about 95%, 96%, 97%, 98%, 99% or 100% identical to the amino acid sequence of SEQ ID NO: 55.

In one embodiment the CD3 antigen binding moiety comprises a heavy chain variable region comprising the amino acid sequence of SEQ ID NO: 43 and a light chain variable region comprising the amino acid sequence of SEQ ID NO: 55.

In one embodiment the CD3 antigen binding moiety comprises the heavy chain variable region sequence of SEQ ID NO: 43 and the light chain variable region sequence of SEQ ID NO: 55.

*Target cell antigen binding moiety*

The protease-activatable T cell activating bispecific molecule of the invention comprises at least one antigen binding moiety capable of binding to a target cell antigen (also referred to herein as an "target cell antigen binding moiety" or "second" or "third" antigen binding moiety). In certain embodiments, the protease-activatable T cell activating bispecific molecule comprises two antigen binding moieties capable of binding to a target cell antigen. In a particular such embodiment, each of these antigen binding moieties specifically binds to the same antigenic determinant. In an even more particular embodiment, all of these antigen binding moieties are identical. In one embodiment, the protease-activatable T cell activating bispecific molecule comprises an immunoglobulin molecule capable of specific binding to a target cell antigen. In one embodiment the protease-activatable T cell activating bispecific molecule comprises not more than two antigen binding moieties capable of binding to a target cell antigen.

In a preferred embodiment, the target cell antigen binding moiety is a Fab molecule, particularly a conventional Fab molecule that binds to a specific antigenic determinant and is able to direct the Protease-activatable T cell activating bispecific molecule to a target site, for example to a specific type of tumor cell that bears the antigenic determinant.

In certain embodiments the target cell antigen binding moiety specifically binds to a cell surface antigen. In a particular embodiment the target cell antigen binding moiety specifically binds to a Folate Receptor 1 (FolR1) on the surface of a target cell. In another specific such embodiment the target cell antigen binding moiety specifically binds to an epidermal growth factor receptor (EGFR), specifically, a human EGFR, e.g., HER1. In another specific such embodiment the target cell antigen binding moiety specifically binds to HER2. In another specific such embodiment the target cell antigen binding moiety specifically binds to Mesothelin, specifically, to human Mesothelin.

In certain embodiments the target cell antigen binding moiety is directed to an antigen associated with a pathological condition, such as an antigen presented on a tumor cell or on a virus-infected cell. Suitable antigens are cell surface antigens, for example, but not limited to, cell surface receptors. In particular embodiments the antigen is a human antigen. In a specific embodiment the target cell antigen is selected from Folate Receptor 1 (FolR1) and epidermal growth factor receptor (EGFR), specifically, a human EGFR, e.g., HER1. In a further specific embodiment the target cell antigen is HER2. In a further specific embodiment the target cell antigen is Mesothelin.

In some embodiments the protease-activatable T cell activating bispecific molecule comprises at least one antigen binding moiety that is specific for HER1. In one embodiment, the antigen binding moiety that is specific for HER1 comprises at least one heavy chain complementarity determining region (CDR) of selected from the group consisting of SEQ ID NO: 56, SEQ ID NO: 57 and SEQ ID NO: 58 and at least one light chain CDR selected from the group of SEQ ID NO: 59, SEQ ID NO: 60, and SEQ ID NO: 61.

In one embodiment, the antigen binding moiety that is specific for HER1 comprises the heavy chain CDR1 of SEQ ID NO: 56, the heavy chain CDR2 of SEQ ID NO: 57, the heavy chain CDR3 of SEQ ID NO: 58, the light chain CDR1 of SEQ ID NO: 59, the light chain CDR2 of SEQ ID NO: 60, and the light chain CDR3 of SEQ ID NO: 61.

In one embodiment, the antigen binding moiety that is specific for HER1 comprises the heavy chain and light chain CDR sequences of an anti-HER1 antibody disclosed in PCT Application Publication Number WO2006/082515.

In one embodiment the protease-activatable T cell activating bispecific molecule comprising at least one antigen binding moiety that is specific for HER1 comprises at least one of a polypeptide sequence that is at least about 95%, 96%, 97%, 98%, 99% or 100% identical to SEQ ID NO: 32, a polypeptide sequence that is at least about 95%, 96%, 97%, 98%, 99% or 100% identical to SEQ ID NO: 33, a polypeptide sequence that is at least about 95%, 96%, 97%, 98%, 99% or 100% identical to SEQ ID NO: 34. In one embodiment the protease-activatable T cell activating bispecific molecule comprising at least one antigen binding moiety that is specific for HER1 comprises the polypeptide sequence of SEQ ID NO: 32, the polypeptide sequence of SEQ ID NO: 33, and the polypeptide sequence of SEQ ID NO: 34.

In one embodiments the protease-activatable T cell activating bispecific molecule comprising at least one antigen binding moiety that is specific for HER1 further comprises an anti-idiotypic CD3 scFv comprising at least one of the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25. In one embodiment, the anti-idiotypic scFv comprises the heavy chain CDR1 of SEQ ID NO: 20, the

heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25.

In one embodiments the protease-activatable T cell activating bispecific molecule comprising at least one antigen binding moiety that is specific for HER1 further comprises an anti-idiotypic CD3 scFv comprising at least one of the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31. In one embodiment, the anti-idiotypic scFv comprises the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31.

In one embodiments the protease-activatable T cell activating bispecific molecule comprising at least one antigen binding moiety that is specific for HER1 further comprises an anti-idiotypic CD3 scFv comprising a polypeptide sequence that is at least about 95%, 96%, 97%, 98%, 99% or 100% identical to SEQ ID NO: 41 or 42. In one embodiment, the anti-idiotypic scFv comprises the polypeptide sequence of SEQ ID NO: 41 or 42.

In one embodiments the protease-activatable T cell activating bispecific molecule comprising at least one antigen binding moiety that is specific for HER1 further comprises an anti-idiotypic HER1 scFv comprising at least one of the heavy chain CDR1 of SEQ ID NO: 48, the heavy chain CDR2 of SEQ ID NO: 49, the heavy chain CDR3 of SEQ ID NO: 50, the light chain CDR1 of SEQ ID NO: 51, the light chain CDR2 of SEQ ID NO: 52, and the light chain CDR3 of SEQ ID NO: 53. In one embodiment, the anti-idiotypic scFv comprises the heavy chain CDR1 of SEQ ID NO: 48, the heavy chain CDR2 of SEQ ID NO: 49, the heavy chain CDR3 of SEQ ID NO: 50, the light chain CDR1 of SEQ ID NO: 51, the light chain CDR2 of SEQ ID NO: 52, and the light chain CDR3 of SEQ ID NO: 53.

In one embodiments the protease-activatable T cell activating bispecific molecule that comprises at least one antigen binding moiety that is specific for HER1 further comprises a linker comprising a polypeptide sequence that is at least about 95%, 96%, 97%, 98%, 99% or 100% identical to SEQ ID NO: 35.

In one embodiment the protease-activatable T cell activating bispecific molecule comprising at least one antigen binding moiety that is specific for HER1 further comprises a linker having a protease recognition site comprising a polypeptide sequence that is at least about 95%, 96%, 97%, 98%, 99% or 100% identical to SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36. In one embodiment, the protease recognition site comprises the polypeptide sequence of SEQ ID NO: 97. In one embodiments the protease-activatable T cell activating bispecific molecule comprising at least one antigen binding moiety that is specific for HER1 further comprises a linker comprising a polypeptide sequence that is at least about 95%, 96%, 97%, 98%, 99% or 100% identical to SEQ ID NO: 7, 8, 9, 10, 86, 87, 88, 89, 90, 91, 92, 93, 94, 95 or 96. In one embodiment, the linker comprises the polypeptide sequence of SEQ ID NO: 7, 8, 9, 10, 86, 87, 88, 89, 90, 91, 92, 93, 94, 95 or 96. In one embodiment, the linker comprises the polypeptide sequence of SEQ ID NO: 7. In one embodiment, the linker comprises the polypeptide sequence of SEQ ID NO: 86.

In some embodiments the protease-activatable T cell activating bispecific molecule comprises at least one antigen binding moiety that is specific for HER2. In one embodiment, the antigen binding moiety that is specific for HER2 comprises at least one heavy chain complementarity determining region (CDR) of selected from the group consisting of SEQ ID NO: 142, SEQ ID NO: 143 and SEQ ID NO: 144 and at least one light chain CDR selected from the group of SEQ ID NO: 148, SEQ ID NO: 149, and SEQ ID NO: 150. In a further one embodiment, the antigen binding moiety that is specific for HER2 comprises at least one heavy chain complementarity determining region (CDR) of selected from the group consisting of SEQ ID NO: 145, SEQ ID NO: 146 and SEQ ID NO: 147 and at least one light chain CDR selected from the group of SEQ ID NO: 148, SEQ ID NO: 149, and SEQ ID NO: 150.

In one embodiment, the antigen binding moiety that is specific for HER2 comprises the heavy chain CDR1 of SEQ ID NO: 142, the heavy chain CDR2 of SEQ ID NO: 143, the heavy chain CDR3 of SEQ ID NO: 144, the light chain CDR1 of SEQ ID NO: 148, the light chain CDR2 of SEQ ID NO: 149, and the light chain CDR3 of SEQ ID NO: 150. In a further embodiment, the antigen binding moiety that is specific for HER2 comprises the heavy chain CDR1 of SEQ ID NO: 145, the heavy chain CDR2 of SEQ ID NO: 146, the heavy chain CDR3 of SEQ ID NO: 147, the light chain CDR1 of SEQ ID NO: 148, the light chain CDR2 of SEQ ID NO: 149, and the light chain CDR3 of SEQ ID NO: 150.

In one embodiments the protease-activatable T cell activating bispecific molecule comprising at least one antigen binding moiety that is specific for HER2 further comprises an anti-idiotypic CD3 scFv comprising at least one of the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25. In one embodiment, the anti-idiotypic scFv comprises the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25.

In one embodiments the protease-activatable T cell activating bispecific molecule comprising at least one

antigen binding moiety that is specific for HER2 further comprises an anti-idiotypic CD3 scFv comprising at least one of the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31. In one embodiment, the anti-idiotypic scFv comprises the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31.

In one embodiments the protease-activatable T cell activating bispecific molecule comprising at least one antigen binding moiety that is specific for HER2 further comprises an anti-idiotypic CD3 scFv comprising a polypeptide sequence that is at least about 95%, 96%, 97%, 98%, 99% or 100% identical to SEQ ID NO: 41 or 42. In one embodiment, the anti-idiotypic scFv comprises the polypeptide sequence of SEQ ID NO: 41 or 42.

In one embodiments the protease-activatable T cell activating bispecific molecule comprising at least one antigen binding moiety that is specific for HER2 further comprises an anti-idiotypic HER2 scFv comprising at least one of the heavy chain CDR1 of SEQ ID NO: 48, the heavy chain CDR2 of SEQ ID NO: 49, the heavy chain CDR3 of SEQ ID NO: 50, the light chain CDR1 of SEQ ID NO: 51, the light chain CDR2 of SEQ ID NO: 52, and the light chain CDR3 of SEQ ID NO: 53. In one embodiment, the anti-idiotypic scFv comprises the heavy chain CDR1 of SEQ ID NO: 48, the heavy chain CDR2 of SEQ ID NO: 49, the heavy chain CDR3 of SEQ ID NO: 50, the light chain CDR1 of SEQ ID NO: 51, the light chain CDR2 of SEQ ID NO: 52, and the light chain CDR3 of SEQ ID NO: 53.

In one embodiments the protease-activatable T cell activating bispecific molecule that comprises at least one antigen binding moiety that is specific for HER2 further comprises a linker comprising a polypeptide sequence that is at least about 95%, 96%, 97%, 98%, 99% or 100% identical to SEQ ID NO: 35.

In one embodiment the protease-activatable T cell activating bispecific molecule comprising at least one antigen binding moiety that is specific for HER2 further comprises a linker having a protease recognition site comprising a polypeptide sequence that is at least about 95%, 96%, 97%, 98%, 99% or 100% identical to SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36. In one embodiment, the protease recognition site comprises the polypeptide sequence of SEQ ID NO: 97. In one embodiments the protease-activatable T cell activating bispecific molecule comprising at least one antigen binding moiety that is specific for HER2 further comprises a linker comprising a polypeptide sequence that is at least about 95%, 96%, 97%, 98%, 99% or 100% identical to SEQ ID NO: 7, 8, 9, 10, 86, 87, 88, 89, 90, 91, 92, 93, 94, 95 or 96. In one embodiment, the linker comprises the polypeptide sequence of SEQ ID NO: 7, 8, 9, 10, 86, 87, 88, 89, 90, 91, 92, 93, 94, 95 or 96. In one embodiment, the linker comprises the polypeptide sequence of SEQ ID NO: 7. In one embodiment, the linker comprises the polypeptide sequence of SEQ ID NO: 86.

In one embodiment the protease-activatable T cell activating bispecific molecule comprises a polypeptide sequence that is at least about 95%, 96%, 97%, 98%, 99% or 100% identical to SEQ ID NO: 132, a polypeptide sequence that is at least about 95%, 96%, 97%, 98%, 99% or 100% identical to SEQ ID NO: 136, a polypeptide sequence that is at least about 95%, 96%, 97%, 98%, 99% or 100% identical to SEQ ID NO: 81 and a polypeptide sequence that is at least about 95%, 96%, 97%, 98%, 99% or 100% identical to SEQ ID NO: 133.

In one embodiment the protease-activatable T cell activating bispecific molecule comprises the polypeptide sequence of SEQ ID NO: 132, the polypeptide sequence of SEQ ID NO: 136, the polypeptide sequence of SEQ ID NO: 81 and the polypeptide sequence of SEQ ID NO: 133.

In particular embodiments the protease-activatable T cell activating bispecific molecule comprises at least one antigen binding moiety that is specific for FolR1. In one embodiment the FolR1 is a human FolR1. In one embodiment, the protease-activatable T cell activating bispecific molecule comprises at least one antigen binding moiety that is specific for human FolR1 and does not bind to human FolR2 or human FolR3. In one embodiment, the antigen binding moiety that is specific for FolR1 comprises at least one heavy chain complementarity determining region (CDR) selected from the group consisting of SEQ ID NO: 14, SEQ ID NO: 15 and SEQ ID NO: 16 and at least one light chain CDR selected from the group of SEQ ID NO: 17, SEQ ID NO: 18 and SEQ ID NO: 19.

In one embodiment, the antigen binding moiety that is specific for FolR1 comprises the heavy chain CDR1 of SEQ ID NO: 14, the heavy chain CDR2 of SEQ ID NO: 15, the heavy chain CDR3 of SEQ ID NO: 16, the light chain CDR1 of SEQ ID NO: 17, the light chain CDR2 of SEQ ID NO: 18, and the light chain CDR3 of SEQ ID NO: 19.

In a further embodiment, the antigen binding moiety that is specific for FolR1 comprises a heavy chain variable region sequence that is at least about 95%, 96%, 97%, 98%, 99% or 100% identical to SEQ ID NO: 47 and a light chain variable region sequence that is at least about 95%, 96%, 97%, 98%, 99% or 100% identical to SEQ ID NO: 55, or variants thereof that retain functionality.

In one embodiment, the antigen binding moiety that is specific for FolR1 comprises the heavy chain variable region comprising an amino acid sequence of SEQ ID NO: 47 and the light chain variable region comprising an amino acid sequence of SEQ ID NO: 55.

In one embodiment, the antigen binding moiety that is specific for FolR1 comprises at least one heavy chain complementarity determining region (CDR) selected from the group consisting of SEQ ID NO: 151, SEQ ID NO: 152 and SEQ ID NO: 153 and at least one light chain CDR selected from the group of SEQ ID NO: 154, SEQ ID NO: 155 and SEQ ID NO: 156.

In one embodiment, the antigen binding moiety that is specific for FolR1 comprises the heavy chain CDR1 of SEQ ID NO: 151, the heavy chain CDR2 of SEQ ID NO: 152, the heavy chain CDR3 of SEQ ID NO: 153, the light chain CDR1 of SEQ ID NO: 154, the light chain CDR2 of SEQ ID NO: 155, and the light chain CDR3 of SEQ ID NO: 156.

In a further embodiment, the antigen binding moiety that is specific for FolR1 comprises a heavy chain variable region sequence that is at least about 95%, 96%, 97%, 98%, 99% or 100% identical to SEQ ID NO: 157 and a light chain variable region sequence that is at least about 95%, 96%, 97%, 98%, 99% or 100% identical to SEQ ID NO: 158, or variants thereof that retain functionality.

In one embodiment, the antigen binding moiety that is specific for FolR1 comprises the heavy chain variable region comprising an amino acid sequence of SEQ ID NO: 157 and the light chain variable region comprising an amino acid sequence of SEQ ID NO: 158.

In one embodiment the protease-activatable T cell activating bispecific molecule comprises a polypeptide sequence that is at least about 95%, 96%, 97%, 98%, 99% or 100% identical to SEQ ID NO: 2, and a polypeptide sequence that is at least about 95%, 96%, 97%, 98%, 99% or 100% identical to SEQ ID NO: 1.

In one embodiment the protease-activatable T cell activating bispecific molecule comprises the polypeptide sequence of SEQ ID NO: 2, and the polypeptide sequence of SEQ ID NO: 1.

In one embodiments the protease-activatable T cell activating bispecific molecule comprising at least one antigen binding moiety that is specific for FolR1 further comprises an anti-idiotypic CD3 scFv comprising at least one of the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25. In one embodiment, the anti-idiotypic scFv comprises the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25.

In one embodiments the protease-activatable T cell activating bispecific molecule comprising at least one antigen binding moiety that is specific for FolR1 further comprises an anti-idiotypic CD3 scFv comprising at least one of the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31. In one embodiment, the anti-idiotypic scFv comprises the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31.

In one embodiments the protease-activatable T cell activating bispecific molecule comprising at least one antigen binding moiety that is specific for FolR1 further comprises an anti-idiotypic CD3 scFv comprising a polypeptide sequence that is at least about 95%, 96%, 97%, 98%, 99% or 100% identical to SEQ ID NO: 41 or 42. In one embodiment, the anti-idiotypic scFv comprises the polypeptide sequence of SEQ ID NO: 41 or 42.

In one embodiments the protease-activatable T cell activating bispecific molecule comprising at least one antigen binding moiety that is specific for FolR1 further comprises a linker having a protease recognition site comprising a polypeptide sequence that is at least about 95%, 96%, 97%, 98%, 99% or 100% identical to SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36. In one embodiment, the protease recognition site comprises the polypeptide sequence of SEQ ID NO: 97. In one embodiments the protease-activatable T cell activating bispecific molecule comprising at least one antigen binding moiety that is specific for ForR1 further comprises a linker comprising a polypeptide sequence that is at least about 95%, 96%, 97%, 98%, 99% or 100% identical to SEQ ID NO: 7, 8, 9, 10, 86, 87, 88, 89, 90, 91, 92, 93, 94, 95 or 96. In one embodiment, the linker comprises the polypeptide sequence of SEQ ID NO: 7, 8, 9, 10, 86, 87, 88, 89, 90, 91, 92, 93, 94, 95 or 96. In one embodiment, the linker comprises the polypeptide sequence of SEQ ID NO: 7. In one embodiment, the linker comprises the polypeptide sequence of SEQ ID NO: 86.

In one embodiment the protease-activatable T cell activating bispecific molecule comprises a polypeptide sequence that is at least about 95%, 96%, 97%, 98%, 99% or 100% identical to SEQ ID NO: 1, a polypeptide sequence that is at least about 95%, 96%, 97%, 98%, 99% or 100% identical to SEQ ID NO: 3 and a polypeptide sequence that is at least about 95%, 96%, 97%, 98%, 99% or 100% identical to SEQ ID NO: 72.

In one embodiment the protease-activatable T cell activating bispecific molecule comprises a polypeptide sequence that is at least about 95%, 96%, 97%, 98%, 99% or 100% identical to SEQ ID NO: 1, a polypeptide sequence that is at least about 95%, 96%, 97%, 98%, 99% or 100% identical to SEQ ID NO: 3 and a polypeptide sequence that is at least about 95%, 96%, 97%, 98%, 99% or 100% identical to SEQ ID NO: 72.

In one embodiment the protease-activatable T cell activating bispecific molecule comprises a polypeptide sequence that is at least about 95%, 96%, 97%, 98%, 99% or 100% identical to SEQ ID NO: 1, a polypeptide sequence that is at least about 95%, 96%, 97%, 98%, 99% or 100% identical to SEQ ID NO: 3 and a polypeptide sequence that is at least about 95%, 96%, 97%, 98%, 99% or 100% identical to SEQ ID NO: 85.

In one embodiment the protease-activatable T cell activating bispecific molecule comprises a polypeptide sequence that is at least about 95%, 96%, 97%, 98%, 99% or 100% identical to SEQ ID NO: 1, a polypeptide sequence that is at least about 95%, 96%, 97%, 98%, 99% or 100% identical to SEQ ID NO: 3, a polypeptide sequence that is at least about 95%, 96%, 97%, 98%, 99% or 100% identical to SEQ ID NO: 73 and a polypeptide sequence that is at least about 95%, 96%, 97%, 98%, 99% or 100% identical to SEQ ID NO: 74.

In one embodiment the protease-activatable T cell activating bispecific molecule comprises the polypeptide sequence of SEQ ID NO: 1, the polypeptide sequence of SEQ ID NO: 3 and the polypeptide sequence of SEQ ID NO: 72.

In one embodiment the protease-activatable T cell activating bispecific molecule comprises the polypeptide sequence of SEQ ID NO: 1, the polypeptide sequence of SEQ ID NO: 3 and the polypeptide sequence of SEQ ID NO: 85.

In one embodiment the protease-activatable T cell activating bispecific molecule comprises the polypeptide sequence of SEQ ID NO: 1, the polypeptide sequence of SEQ ID NO: 3, the polypeptide sequence of SEQ ID NO: 73 and the polypeptide sequence of SEQ ID NO: 74.

In one embodiment the protease-activatable T cell activating bispecific molecule comprises a polypeptide sequence that is at least about 95%, 96%, 97%, 98%, 99% or 100% identical to SEQ ID NO: 137, a polypeptide sequence that is at least about 95%, 96%, 97%, 98%, 99% or 100% identical to SEQ ID NO: 139, a polypeptide sequence that is at least about 95%, 96%, 97%, 98%, 99% or 100% identical to SEQ ID NO: 81 and a polypeptide sequence that is at least about 95%, 96%, 97%, 98%, 99% or 100% identical to SEQ ID NO: 138.

In one embodiment the protease-activatable T cell activating bispecific molecule comprises the polypeptide sequence of SEQ ID NO: 137, the polypeptide sequence of SEQ ID NO: 139, the polypeptide sequence of SEQ ID NO: 81 and the polypeptide sequence of SEQ ID NO: 138.

In particular embodiments the protease-activatable T cell activating bispecific molecule comprises at least one antigen binding moiety that is specific for Mesothelin. In one embodiment the Mesothelin is human Mesothelin. In one embodiment, the protease-activatable T cell activating bispecific molecule comprises at least one antigen binding moiety that is specific for human Mesothelin. In one embodiment, the antigen binding moiety that is specific for Mesothelin comprises at least one heavy chain complementarity determining region (CDR) selected from the group consisting of SEQ ID NO: 107, SEQ ID NO: 108 and SEQ ID NO: 109 and at least one light chain CDR selected from the group of SEQ ID NO: 110, SEQ ID NO: 111 and SEQ ID NO: 112.

In one embodiment, the antigen binding moiety that is specific for Mesothelin comprises the heavy chain CDR1 of SEQ ID NO: 107, the heavy chain CDR2 of SEQ ID NO: 108, the heavy chain CDR3 of SEQ ID NO: 109, the light chain CDR1 of SEQ ID NO: 110, the light chain CDR2 of SEQ ID NO: 111, and the light chain CDR3 of SEQ ID NO: 112.

In a further embodiment, the antigen binding moiety that is specific for Mesothelin comprises a heavy chain variable region sequence that is at least about 95%, 96%, 97%, 98%, 99% or 100% identical to SEQ ID NO: 113 and a light chain variable region sequence that is at least about 95%, 96%, 97%, 98%, 99% or 100% identical to SEQ ID NO: 114, or variants thereof that retain functionality.

In one embodiment, the antigen binding moiety that is specific for Mesothelin comprises the heavy chain variable region comprising the amino acid sequence of SEQ ID NO: 113 and the light chain variable region comprising the amino acid sequence of SEQ ID NO: 114.

In one embodiments the protease-activatable T cell activating bispecific molecule comprising at least one antigen binding moiety that is specific for Mesothelin further comprises an anti-idiotypic CD3 scFv comprising at least one of the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25. In one embodiment, the anti-idiotypic scFv comprises the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25.

In one embodiments the protease-activatable T cell activating bispecific molecule comprising at least one antigen binding moiety that is specific for Mesothelin further comprises an anti-idiotypic CD3 scFv comprising at least one of the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31. In one embodiment, the anti-idiotypic scFv comprises the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31.

In one embodiments the protease-activatable T cell activating bispecific molecule comprising at least one antigen binding moiety that is specific for Mesothelin further comprises an anti-idiotypic CD3 scFv comprising a polypeptide sequence that is at least about 95%, 96%, 97%, 98%, 99% or 100% identical to SEQ ID NO: 41 or 42. In one

embodiment, the anti-idiotypic scFv comprises the polypeptide sequence of SEQ ID NO: 41 or 42.

In one embodiments the protease-activatable T cell activating bispecific molecule comprising at least one antigen binding moiety that is specific for Mesothelin further comprises a linker having a protease recognition site comprising a polypeptide sequence that is at least about 95%, 96%, 97%, 98%, 99% or 100% identical to SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36, 37, 38, 39, 40, 97, 98, 99, 100, 101, 102, 103, 104, 105 or 106. In one embodiment, the protease recognition site comprises the polypeptide sequence of SEQ ID NO: 36. In one embodiment, the protease recognition site comprises the polypeptide sequence of SEQ ID NO: 97. In one embodiments the protease-activatable T cell activating bispecific molecule comprising at least one antigen binding moiety that is specific for Mesothelin further comprises a linker comprising a polypeptide sequence that is at least about 95%, 96%, 97%, 98%, 99% or 100% identical to SEQ ID NO: 7, 8, 9, 10, 86, 87, 88, 89, 90, 91, 92, 93, 94, 95 or 96. In one embodiment, the linker comprises the polypeptide sequence of SEQ ID NO: 7, 8, 9, 10, 86, 87, 88, 89, 90, 91, 92, 93, 94, 95 or 96. In one embodiment, the linker comprises the polypeptide sequence of SEQ ID NO: 7. In one embodiment, the linker comprises the polypeptide sequence of SEQ ID NO: 86.

In one embodiment the protease-activatable T cell activating bispecific molecule comprises a polypeptide sequence that is at least about 95%, 96%, 97%, 98%, 99% or 100% identical to SEQ ID NO: 77, a polypeptide sequence that is at least about 95%, 96%, 97%, 98%, 99% or 100% identical to SEQ ID NO: 78, a polypeptide sequence that is at least about 95%, 96%, 97%, 98%, 99% or 100% identical to SEQ ID NO: 81 and a polypeptide sequence that is at least about 95%, 96%, 97%, 98%, 99% or 100% identical to SEQ ID NO: 82.

In one embodiment the protease-activatable T cell activating bispecific molecule comprises a polypeptide sequence that is at least about 95%, 96%, 97%, 98%, 99% or 100% identical to SEQ ID NO: 76, a polypeptide sequence that is at least about 95%, 96%, 97%, 98%, 99% or 100% identical to SEQ ID NO: 77, a polypeptide sequence that is at least about 95%, 96%, 97%, 98%, 99% or 100% identical to SEQ ID NO: 78 and a polypeptide sequence that is at least about 95%, 96%, 97%, 98%, 99% or 100% identical to SEQ ID NO: 79.

In one embodiment the protease-activatable T cell activating bispecific molecule comprises the polypeptide sequence of SEQ ID NO: 77, the polypeptide sequence of SEQ ID NO: 78, the polypeptide sequence of SEQ ID NO: 81 and the polypeptide sequence of SEQ ID NO: 82.

In one embodiment the protease-activatable T cell activating bispecific molecule comprises the polypeptide sequence of SEQ ID NO: 76, the polypeptide sequence of SEQ ID NO: 77, the polypeptide sequence of SEQ ID NO: 78 and the polypeptide sequence of SEQ ID NO: 79.

In one embodiment, provided is a T cell activating bispecific molecule comprises a polypeptide sequence that is at least about 95%, 96%, 97%, 98%, 99% or 100% identical to SEQ ID NO: 76, a polypeptide sequence that is at least about 95%, 96%, 97%, 98%, 99% or 100% identical to SEQ ID NO: 77, a polypeptide sequence that is at least about 95%, 96%, 97%, 98%, 99% or 100% identical to SEQ ID NO: 78 and a polypeptide sequence that is at least about 95%, 96%, 97%, 98%, 99% or 100% identical to SEQ ID NO: 81.

In one embodiment the T cell activating bispecific molecule comprises a polypeptide sequence that is at least about 95%, 96%, 97%, 98%, 99% or 100% identical to SEQ ID NO: 77, a polypeptide sequence that is at least about 95%, 96%, 97%, 98%, 99% or 100% identical to SEQ ID NO: 78, a polypeptide sequence that is at least about 95%, 96%, 97%, 98%, 99% or 100% identical to SEQ ID NO: 81 and a polypeptide sequence that is at least about 95%, 96%, 97%, 98%, 99% or 100% identical to SEQ ID NO: 84.

In one embodiment the T cell activating bispecific molecule comprises the polypeptide sequence of SEQ ID NO: 76, the polypeptide sequence of SEQ ID NO: 77, the polypeptide sequence of SEQ ID NO: 78 and the polypeptide sequence of SEQ ID NO: 81.

In one embodiment the T cell activating bispecific molecule comprises the polypeptide sequence of SEQ ID NO: 77, the polypeptide sequence of SEQ ID NO: 78, the polypeptide sequence of SEQ ID NO: 81 and the polypeptide sequence of SEQ ID NO: 84.

*Masking moiety*

The protease-activatable T cell activating bispecific molecule of the invention comprises at least one masking moiety. Others have tried to mask binding of an antibody by capping the binding moiety with a fragment of the antigen recognized by the binding moiety (e.g., WO2013128194). This approach has several limitations. For example, using the antigen allows for less flexibility in reducing the affinity of the binding moiety. This is so because the affinity has to be high enough to be reliably masked by the antigen mask. Also, dissociated antigen could potentially bind to and interact with its cognate receptor(s) in vivo and cause undesirable signals to the cell expressing such receptor. In contrast, the approach described herein uses an anti-idiotype antibody or fragment thereof as a mask. Two countervailing considerations for designing an effective masking moiety are 1. effectiveness of the masking and 2. reversibility of the masking. If the affinity is too low, masking would be inefficient. However, if the affinity is too high, the masking process might not

be readily reversible. It was not predictable whether a high affinity anti-idiotype mask or a low affinity anti-idiotype mask would work better. As described herein, higher affinity masking moieties performed overall better in masking the antigen binding side and, at the same time, could be effectively removed for activation of the molecule. In one embodiment, the anti-idiotype mask has a KD of 1-8 nM. In one embodiment, anti-idiotype mask has a KD of 2 nM at 37°C. In one specific embodiment, the masking moiety recognizes the idiotype of the first antigen binding moiety capable of specific binding to a CD3, e.g., a human CD3. In one specific embodiment, the masking moiety recognizes the idiotype of the second antigen binding moiety capable of binding to a target cell antigen.

In one embodiment, the masking moiety masks a CD3-binding moiety and comprises at least one of the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25. In one embodiment, the masking moiety comprises the heavy chain CDR1 of SEQ ID NO: 20, the heavy chain CDR2 of SEQ ID NO: 21, the heavy chain CDR3 of SEQ ID NO: 22, the light chain CDR1 of SEQ ID NO: 23, the light chain CDR2 of SEQ ID NO: 24, and the light chain CDR3 of SEQ ID NO: 25. In one embodiment, the masking moiety masks a CD3-binding moiety and comprises a polypeptide sequence that is at least about 95%, 96%, 97%, 98%, 99% or 100% identical to SEQ ID NO: 41. In one embodiment, the masking moiety masks a CD3-binding moiety and comprises the polypeptide sequence of SEQ ID NO: 41.

In one preferred embodiment, the masking moiety masks a CD3-binding moiety and comprises at least one of the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31. In one embodiment, the masking moiety comprises the heavy chain CDR1 of SEQ ID NO: 26, the heavy chain CDR2 of SEQ ID NO: 27, the heavy chain CDR3 of SEQ ID NO: 28, the light chain CDR1 of SEQ ID NO: 29, the light chain CDR2 of SEQ ID NO: 30, and the light chain CDR3 of SEQ ID NO: 31. In one embodiment, the masking moiety masks a CD3-binding moiety and comprises a polypeptide sequence that is at least about 95%, 96%, 97%, 98%, 99% or 100% identical to SEQ ID NO: 42. In one preferred embodiment, the masking moiety masks a CD3-binding moiety and comprises the polypeptide sequence of SEQ ID NO: 42.

In one embodiment, the masking moiety masks a HER1-binding moiety and comprises at least one of the heavy chain CDR1 of SEQ ID NO: 48, the heavy chain CDR2 of SEQ ID NO: 49, the heavy chain CDR3 of SEQ ID NO: 50, the light chain CDR1 of SEQ ID NO: 51, the light chain CDR2 of SEQ ID NO: 52, and the light chain CDR3 of SEQ ID NO: 53. In one embodiment, the anti-idiotypic scFv comprises the heavy chain CDR1 of SEQ ID NO: 48, the heavy chain CDR2 of SEQ ID NO: 49, the heavy chain CDR3 of SEQ ID NO: 50, the light chain CDR1 of SEQ ID NO: 51, the light chain CDR2 of SEQ ID NO: 52, and the light chain CDR3 of SEQ ID NO: 53.

In one aspect for reference only, the disclosure relates to an idiotype-specific polypeptide for reversibly concealing antigen binding of an antigen-binding of a molecule. In one embodiment, the invention relates to an idiotype-specific polypeptide for reversibly concealing an anti-CD3 antigen binding site of a molecule, wherein the idiotype-specific polypeptide is an anti-idiotype scFv, wherein the idiotype-specific polypeptide is covalently attached to the molecule through a peptide linker. In one embodiment the linker is a protease-cleavable linker. In one embodiment, the linker comprises the sequence of SEQ ID NO: 7, 8, 9, or 10. In one embodiment, the linker comprises the sequence of SEQ ID NO: 7, 8, 9, 10, 86, 87, 88, 89, 90, 91, 92, 93, 94, 95 or 96. In one embodiment, the linker comprises the polypeptide sequence of SEQ ID NO: 7. In one embodiment, the linker comprises the polypeptide sequence of SEQ ID NO: 86. In one embodiment the peptide linker comprises at least one protease recognition site. In one embodiment, the protease recognition site comprises the polypeptide sequence of SEQ ID NO: 36, 37, 38, 39, 40, 97, 98, 99, 100, 101, 102, 103, 104, 105 or 106. In one preferred embodiment, the protease recognition site comprises the protease recognition sequence RQARVVNG (SEQ ID NO: 36). In further embodiment, the linker comprises more than one protease recognition site. In one preferred embodiment, the protease recognition site comprises the protease recognition sequence VHMPLGFLGPRQARVVNG (SEQ ID NO:97). In one embodiment the protease is selected from the group consisting of metalloproteinase, e.g., matrix metalloproteinase (MMP) 1-28 and A Disintegrin And Metalloproteinase (ADAM) 2, 7-12, 15, 17-23, 28-30 and 33, serine protease, e.g., urokinase-type plasminogen activator and Matriptase, cysteine protease, aspartic protease, and cathepsin protease. In one specific embodiment the protease is MMP9 or MMP2. In a further specific embodiment, the protease is Matriptase.

In one embodiment the molecule which comprises the anti-CD3 antigen binding site is a T-cell activating bispecific molecule. In one particular embodiment the idiotype-specific polypeptide comprises a heavy chain variable region comprising at least one of a heavy chain complementarity determining region (CDR H) 1 amino acid sequence of DYSIH (SEQ ID NO:20); CDR H2 amino acid sequence of WINTETGEPAYADDFKG (SEQ ID NO:21); and a CDR H3 amino acid sequence of PYDYDVLDY (SEQ ID NO:22). In one particular embodiment the idiotype-specific polypeptide comprises a light chain variable region comprising at least one of: a light chain (CDR L)1 amino acid sequence of RASKSVSTSNYSYIH (SEQ ID NO:23); a CDR L2 amino acid sequence of YVSYLES (SEQ ID NO:24); and a CDR L3 amino acid sequence of QHSREFPWT (SEQ ID NO:25). In one particular embodiment the idiotype-specific polypeptide comprises: a heavy chain complementarity determining region (CDR H) 1 amino acid sequence of DYSIH (SEQ ID

NO:20); a CDR H2 amino acid sequence of WINTETGEPAYADDFKG (SEQ ID NO:21); a CDR H3 amino acid sequence of PYDYDVLDY (SEQ ID NO:22); and a light chain variable region comprising: a light chain (CDR L)1 amino acid sequence of RASKSVSTSNYSYIH (SEQ ID NO:23); a CDR L2 amino acid sequence of YVSYLES (SEQ ID NO:24); and a CDR L3 amino acid sequence of QHSREFPWT (SEQ ID NO:25). In one particular embodiment the idiotype-specific polypeptide comprises a heavy chain variable region comprising at least one of: a heavy chain complementarity determining region (CDR H) 1 amino acid sequence of SYGVS (SEQ ID NO:26); a CDR H2 amino acid sequence of IIWGDGSTNYHSALIS (SEQ ID NO:27); and a CDR H3 amino acid sequence of GITTVVDDYYAMDY (SEQ ID NO:28). In one particular embodiment the idiotype-specific polypeptide comprises a light chain variable region comprising at least one of: a light chain (CDR L)1 amino acid sequence of RASENIDSYLA (SEQ ID NO:29); a CDR L2 amino acid sequence of AATFLAD (SEQ ID NO:30); and a CDR L3 amino acid sequence of QHYYSTPYT (SEQ ID NO:31). In one particular embodiment the idiotype-specific polypeptide comprises a heavy chain variable region comprising: a heavy chain complementarity determining region (CDR H) 1 amino acid sequence of SYGVS (SEQ ID NO:26); a CDR H2 amino acid sequence of IIWGDGSTNYHSALIS (SEQ ID NO:27); a CDR H3 amino acid sequence of GITTVVDDYYAMDY (SEQ ID NO:28); and a light chain variable region comprising: a light chain (CDR L)1 amino acid sequence of RASENIDSYLA (SEQ ID NO:29); a CDR L2 amino acid sequence of AATFLAD (SEQ ID NO:30); and a CDR L3 amino acid sequence of QHYYSTPYT (SEQ ID NO:31). In one embodiment, the idiotype-specific polypeptide comprises a heavy chain variable region comprising at least one of: a heavy chain complementarity determining region (CDR H) 1 amino acid sequence of SEQ ID NO:48; a CDR H2 amino acid sequence of SEQ ID NO:49; and a CDR H3 amino acid sequence of SEQ ID NO:50. In one embodiment, the idiotype-specific polypeptide comprises a light chain variable region comprising at least one of: a light chain complementarity determining region (CDR L) 1 amino acid sequence of SEQ ID NO:51; a CDR L2 amino acid sequence of SEQ ID NO:52; and a CDR L3 amino acid sequence of SEQ ID NO:53. In one embodiment, the idiotype-specific polypeptide comprises a heavy chain variable region comprising a heavy chain complementarity determining region (CDR H) 1 amino acid sequence of SEQ ID NO:48; a CDR H2 amino acid sequence of SEQ ID NO:49; and a CDR H3 amino acid sequence of SEQ ID NO:50, and a light chain variable region comprising a light chain complementarity determining region (CDR L) 1 amino acid sequence of SEQ ID NO:51; a CDR L2 amino acid sequence of SEQ ID NO:52; and a CDR L3 amino acid sequence of SEQ ID NO:53.

**Polynucleotides**

The invention further provides isolated polynucleotides encoding a protease-activatable T cell activating bispecific molecule as described herein

Polynucleotides of the invention include those that are at least about 80%, 85%, 90%, 95%, 96%, 97%, 98%, 99%, or 100% identical to the sequences set forth in SEQ ID NOs 62-71 or SEQ ID NOs. Polynucleotides of the invention further include those that are at least about 80%, 85%, 90%, 95%, 96%, 97%, 98%, 99%, or 100% identical to the sequences set forth in SEQ ID NOs 117-131. Polynucleotides of the invention further include those that are at least about 80%, 85%, 90%, 95%, 96%, 97%, 98%, 99%, or 100% identical to the sequences set forth in SEQ ID NOs 162-170.

The polynucleotides encoding protease-activatable T cell activating bispecific molecules of the invention may be expressed as a single polynucleotide that encodes the entire protease-activatable T cell activating bispecific molecule or as multiple (e.g., two or more) polynucleotides that are co-expressed. Polypeptides encoded by polynucleotides that are co-expressed may associate through, e.g., disulfide bonds or other means to form a functional protease-activatable T cell activating bispecific molecule. For example, the light chain portion of an antigen binding moiety may be encoded by a separate polynucleotide from the portion of the protease-activatable T cell activating bispecific molecule comprising the heavy chain portion of the antigen binding moiety, an Fc domain subunit and optionally (part of) another antigen binding moiety. When co-expressed, the heavy chain polypeptides will associate with the light chain polypeptides to form the antigen binding moiety. In another example, the portion of the protease-activatable T cell activating bispecific molecule comprising one of the two Fc domain subunits and optionally (part of) one or more antigen binding moieties could be encoded by a separate polynucleotide from the portion of the protease-activatable T cell activating bispecific molecule comprising the the other of the two Fc domain subunits and optionally (part of) an antigen binding moiety. When co-expressed, the Fc domain subunits will associate to form the Fc domain.

In some embodiments, the isolated polynucleotide encodes the entire protease-activatable T cell activating bispecific molecule according to the invention as described herein.

In another embodiment, the present invention is directed to an isolated polynucleotide encoding a protease-activatable T cell activating bispecific molecule of the invention, wherein the polynucleotide comprises a sequence that encodes a variable region sequence. In another embodiment, the present invention is directed to an isolated polynucleotide encoding a protease-activatable T cell activating bispecific molecule, wherein the polynucleotide comprises a sequence that encodes a polypeptide sequence as shown in SEQ ID NOs 1, 2, 3, 4, 5, 6, 7, 8, 9, 10, 32, 33, 34, 35, 36, 37, 38, 39, 40, 41, 42, 43, 47, or 55. In another embodiment, the present invention is directed to an isolated polynucleotide encoding a protease-activatable T cell activating bispecific molecule, wherein the polynucleotide comprises a sequence

that encodes a polypeptide sequence as shown in SEQ ID NOs 1, 2, 3, 4, 5, 6, 7, 8, 9, 10, 32, 33, 34, 35, 36, 37, 38, 39, 40, 41, 42, 43, 47, 55, 72, 73, 74, 75, 76, 77, 78, 79, 80, 81, 82, 83, 84, 85. In another embodiment, the present invention is directed to an isolated polynucleotide encoding a protease-activatable T cell activating bispecific molecule, wherein the polynucleotide comprises a sequence that encodes a polypeptide sequence as shown in SEQ ID NOs 1, 2, 3, 4, 5, 6, 7, 8, 9, 10, 32, 33, 34, 35, 36, 37, 38, 39, 40, 41, 42, 43, 47, 55, 72, 73, 74, 75, 76, 77, 78, 79, 80, 81, 82, 83, 84, 85, 132, 133, 134, 135, 136, 137, 138, 139, 140 or 141. In another embodiment, the invention is further directed to an isolated polynucleotide encoding a protease-activatable T cell activating bispecific molecule of the invention, wherein the polynucleotide comprises a sequence that is at least about 80%, 85%, 90%, 95%, 96%, 97%, 98%, or 99% identical to the nucleotide sequence shown in SEQ ID NOs 62, 63, 64, 65, 66, 67, 68, 69, 70, or 71. In another embodiment, the invention is further directed to an isolated polynucleotide encoding a protease-activatable T cell activating bispecific molecule of the invention, wherein the polynucleotide comprises a sequence that is at least about 80%, 85%, 90%, 95%, 96%, 97%, 98%, or 99% identical to the nucleotide sequence shown in SEQ ID NOs 62, 63, 64, 65, 66, 67, 68, 69, 70, 71, 117, 118, 119, 120, 121, 122, 123, 124, 125, 126, 127, 128, 129, 130 or 131. In another embodiment, the invention is further directed to an isolated polynucleotide encoding a protease-activatable T cell activating bispecific molecule of the invention, wherein the polynucleotide comprises a sequence that is at least about 80%, 85%, 90%, 95%, 96%, 97%, 98%, or 99% identical to the nucleotide sequence shown in SEQ ID NOs 62, 63, 64, 65, 66, 67, 68, 69, 70, 71, 117, 118, 119, 120, 121, 122, 123, 124, 125, 126, 127, 128, 129, 130, 131, 162, 163, 164, 165, 166, 167, 168, 169 or 170. In another embodiment, the invention is directed to an isolated polynucleotide encoding a protease-activatable T cell activating bispecific molecule of the invention, wherein the polynucleotide comprises the nucleic acid sequence shown in SEQ ID NOs 62, 63, 64, 65, 66, 67, 68, 69, 70, or 71. In another embodiment, the invention is directed to an isolated polynucleotide encoding a protease-activatable T cell activating bispecific molecule of the invention, wherein the polynucleotide comprises the nucleic acid sequence shown in SEQ ID NOs 62, 63, 64, 65, 66, 67, 68, 69, 70, 71, 117, 118, 119, 120, 121, 122, 123, 124, 125, 126, 127, 128, 129, 130 or 131. In another embodiment, the invention is directed to an isolated polynucleotide encoding a protease-activatable T cell activating bispecific molecule of the invention, wherein the polynucleotide comprises the nucleic acid sequence shown in SEQ ID NOs 62, 63, 64, 65, 66, 67, 68, 69, 70, 71, 117, 118, 119, 120, 121, 122, 123, 124, 125, 126, 127, 128, 129, 130, 131, 162, 163, 164, 165, 166, 167, 168, 169 or 170. In another embodiment, the invention is directed to an isolated polynucleotide encoding a protease-activatable T cell activating bispecific molecule of the invention, wherein the polynucleotide comprises a sequence that encodes a variable region sequence that is at least about 80%, 85%, 90%, 95%, 96%, 97%, 98%, or 99% identical to the amino acid sequence in SEQ ID NOs 43, 47, or 55. The invention encompasses an isolated polynucleotide encoding a protease-activatable T cell activating bispecific molecule of the invention, wherein the polynucleotide comprises a sequence that encodes the variable region sequence of SEQ ID NOs SEQ ID NOs 43, 47, or 55 with conservative amino acid substitutions.

In another embodiment, the invention is directed to an isolated polynucleotide encoding a protease-activatable T cell activating bispecific molecule of the invention, wherein the polynucleotide comprises a sequence that encodes a variable region sequence that is at least about 80%, 85%, 90%, 95%, 96%, 97%, 98%, or 99% identical to the amino acid sequence in SEQ ID NOs 43, 47, 55, 113, 114, 115 or 116. The invention encompasses an isolated polynucleotide encoding a protease-activatable T cell activating bispecific molecule of the invention, wherein the polynucleotide comprises a sequence that encodes the variable region sequence of SEQ ID NOs SEQ ID NOs 43, 47, 55, 113, 114, 115 or 116 with conservative amino acid substitutions.

In another embodiment, the invention is directed to an isolated polynucleotide encoding a protease-activatable T cell activating bispecific molecule of the invention, wherein the polynucleotide comprises a sequence that encodes a variable region sequence that is at least about 80%, 85%, 90%, 95%, 96%, 97%, 98%, or 99% identical to the amino acid sequence in SEQ ID NOs 43, 47, 55, 113, 114, 115, 116, 157, 158, 159, 160 or 161. The invention encompasses an isolated polynucleotide encoding a protease-activatable T cell activating bispecific molecule of the invention, wherein the polynucleotide comprises a sequence that encodes the variable region sequence of SEQ ID NOs SEQ ID NOs 43, 47, 55, 113, 114, 115, 116, 157, 158, 159, 160 or 161 with conservative amino acid substitutions.

In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

The disclosure provides for reference only isolated polynucleotides encoding an idiotype-specific polypeptide as described herein. In some aspects, said fragment is an idiotype binding, i.e., anti-idiotype specific antibody or fragment thereof. In one aspect the idiotype-specific polypeptide is an anti-idiotypic scFv.

The disclosure provides for reference only an isolated polynucleotide encoding an idiotype-specific polypeptide wherein the polynucleotide comprises a sequence that encodes the polypeptide sequence of one or more of SEQ ID NOs 20, 21, 22, 23, 24, 25, 26, 27, 28, 29, 30, 31, 48, 49, 50, 51, 52, and 53. The disclosure provides for reference only an isolated polynucleotide encoding an idiotype-specific polypeptide wherein the polynucleotide comprises a sequence that encodes the polypeptide sequence of one or more of SEQ ID NOs 20, 21, 22, 23, 24, 25, 26, 27, 28, 29, 30, 31,

48, 49, 50, 51, 52, and 53 with conservative amino acid substitutions.

The polynucleotides encoding idiotype-specific polypeptides useful in the invention may be expressed as a single polynucleotide that encodes the entire idiotype-specific polypeptide or as multiple (e.g., two or more) polynucleotides that are co-expressed. Polypeptides encoded by polynucleotides that are co-expressed may associate through, e.g., disulfide bonds or other means to form a functional idiotype-specific polypeptide, e.g., a masking moiety. For example, in one embodiment the idiotype-specific polypeptide is an anti-idiotypic scFv (single chain variable fragment) wherein the light chain variable portion of the anti-idiotypic scFv may be encoded by a separate polynucleotide from the portion of the anti-idiotypic scFv comprising the heavy chain variable portion of the anti-idiotypic scFv. When co-expressed, the heavy chain polypeptides will associate with the light chain polypeptides to form the anti-idiotypic scFv. In some embodiments, the isolated polynucleotide encodes the idiotype-specific polypeptide that is useful in the invention as described herein.

In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

**Recombinant Methods**

Protease-activatable T cell activating bispecific molecules of the invention may be obtained, for example, by solid-state peptide synthesis (e.g., Merrifield solid phase synthesis) or recombinant production. For recombinant production one or more polynucleotide encoding the protease-activatable T cell activating bispecific molecule (fragment), e.g., as described above, is isolated and inserted into one or more vectors for further cloning and/or expression in a host cell. Such polynucleotide may be readily isolated and sequenced using conventional procedures. In one embodiment a vector, preferably an expression vector, comprising one or more of the polynucleotides of the invention is provided. Methods which are well known to those skilled in the art can be used to construct expression vectors containing the coding sequence of a protease-activatable T cell activating bispecific molecule (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the protease-activatable T cell activating bispecific molecule (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g., on a single vector, or in separate polynucleotide constructs, e.g., on separate (different) vectors. Furthermore, any vector may contain a single coding region, or may comprise two or more coding regions, e.g., a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the protease-activatable T cell activating bispecific molecule (fragment) of the invention, or variant or derivative thereof. Heterologous coding regions include without limitation specialized elements or motifs, such as a secretory signal peptide or a heterologous functional domain. An operable association is when a coding region for a gene product, e.g., a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g., the immediate early promoter, in conjunction with intron-A), simian virus 40

(e.g., the early promoter), and retroviruses (such as, e.g., Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g., promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the protease-activatable T cell activating bispecific molecule is desired, DNA encoding a signal sequence may be placed upstream of the nucleic acid encoding a protease-activatable T cell activating bispecific molecule of the invention or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, *e.g.,* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g., a histidine tag) or assist in labeling the protease-activatable T cell activating bispecific molecule may be included within or at the ends of the protease-activatable T cell activating bispecific molecule (fragment) encoding polynucleotide.

In a further embodiment, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one such embodiment a host cell comprises (e.g., has been transformed or transfected with) a vector comprising a polynucleotide that encodes a protease-activatable T cell activating bispecific molecule of the invention. As used herein, the term "host cell" refers to any kind of cellular system which can be engineered to generate the protease-activatable T cell activating bispecific molecules of the invention or fragments thereof. Host cells suitable for replicating and for supporting expression of protease-activatable T cell activating bispecific molecules are well known in the art. Such cells may be transfected or transduced as appropriate with the particular expression vector and large quantities of vector containing cells can be grown for seeding large scale fermenters to obtain sufficient quantities of the protease-activatable T cell activating bispecific molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g., US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells,

including dhfr⁻ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain.

In one embodiment, a method of producing a protease-activatable T cell activating bispecific molecule according to the invention is provided, wherein the method comprises culturing a host cell comprising a polynucleotide encoding the protease-activatable T cell activating bispecific molecule, as provided herein, under conditions suitable for expression of the protease-activatable T cell activating bispecific molecule, and recovering the protease-activatable T cell activating bispecific molecule from the host cell (or host cell culture medium).

The components of the protease-activatable T cell activating bispecific molecule are genetically fused to each other. Protease-activatable T cell activating bispecific molecules can be designed such that its components are fused directly to each other or indirectly through a linker sequence. The composition and length of the linker may be determined in accordance with methods well known in the art and may be tested for efficacy. Examples of linker sequences between different components of protease-activatable T cell activating bispecific molecules are found in the sequences provided herein. Additional sequences may also be included to incorporate a cleavage site to separate the individual components of the fusion if desired, for example an endopeptidase recognition sequence.

In certain embodiments the one or more antigen binding moieties of the protease-activatable T cell activating bispecific molecules comprise at least an antibody variable region capable of binding an antigenic determinant. Variable regions can form part of and be derived from naturally or non-naturally occurring antibodies and fragments thereof. Methods to produce polyclonal antibodies and monoclonal antibodies are well known in the art (see e.g., Harlow and Lane, "Antibodies, a laboratory manual", Cold Spring Harbor Laboratory, 1988). Non-naturally occurring antibodies can be constructed using solid phase-peptide synthesis, can be produced recombinantly (e.g., as described in U.S. patent No. 4,186,567) or can be obtained, for example, by screening combinatorial libraries comprising variable heavy chains and variable light chains (see e.g., U.S. Patent. No. 5,969,108 to McCafferty).

Any animal species of antibody, antibody fragment, antigen binding domain or variable region can be used in the protease-activatable T cell activating bispecific molecules of the invention. Non-limiting antibodies, antibody fragments, antigen binding domains or variable regions useful in the present invention can be of murine, primate, or human origin. If the protease-activatable T cell activating bispecific molecule is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g., recipient antibody) framework and constant regions with or without retention of critical framework residues (e.g., those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de

Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g., Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable

regions in response to antigenic challenge (see e.g., Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain embodiments, the antigen binding moieties useful in the present invention are engineered to have enhanced binding affinity according to, for example, the methods disclosed in U.S. Pat. Appl. Publ. No. 2004/0132066. The ability of the protease-activatable T cell activating bispecific molecule of the invention to bind to a specific antigenic determinant can be measured either through an enzyme-linked immunosorbent assay (ELISA) or other techniques familiar to one of skill in the art, e.g., surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen, e.g., an antibody that competes with the V9 antibody for binding to CD3. In certain embodiments, such a competing antibody binds to the same epitope (e.g., a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen (e.g., CD3) is incubated in a solution comprising a first labeled antibody that binds to the antigen (e.g., V9 antibody, described in US 6,054,297) and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

Protease-activatable T cell activating bispecific molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the protease-activatable T cell activating bispecific molecule binds. For example, for affinity chromatography purification of protease-activatable T cell activating bispecific molecules of the invention, a matrix with protein A or protein G may be used. Sequential Protein A or G affinity chromatography and size exclusion chromatography can be used to isolate a protease-activatable T cell activating bispecific molecule essentially as described in the Examples. The purity of the protease-activatable T cell activating bispecific molecule can be determined by any of a variety of well-known analytical methods including gel electrophoresis, high pressure liquid chromatography, and the like. For example, the heavy chain fusion proteins expressed as described in the Examples were shown to be intact and properly assembled as demonstrated by reducing SDS-PAGE (see, e.g., FIGs. 8-12). Three bands were resolved at approximately Mr 25,000, Mr 50,000 and Mr 75,000, corresponding to the predicted molecular weights of the protease-activatable T cell activating bispecific molecule light chain, heavy chain and heavy chain/light chain fusion protein.

Assays

protease-activatable T cell activating bispecific molecules provided herein may be identified, screened for, or characterized for their physical/chemical properties and/or biological activities by various assays known in the art.

*Affinity assays*

The affinity of the protease-activatable T cell activating bispecific molecule for an Fc receptor or a target antigen can be determined in accordance with the methods set forth in the Examples by surface plasmon resonance (SPR), using standard instrumentation such as a BIAcore instrument (GE Healthcare), and receptors or target proteins such as may be obtained by recombinant expression. Alternatively, binding of protease-activatable T cell activating bispecific molecules for different receptors or target antigens may be evaluated using cell lines expressing the particular receptor or target antigen, for example by flow cytometry (FACS). A specific illustrative and exemplary embodiment for measuring binding affinity is described in the following and in the Examples below.

According to one embodiment, $K_D$ is measured by surface plasmon resonance using a BIACORE® T100 machine (GE Healthcare) at 25 °C.

To analyze the interaction between the Fc-portion and Fc receptors, His-tagged recombinant Fc-receptor is captured by an anti-Penta His antibody (Qiagen) immobilized on CM5 chips and the bispecific constructs are used as analytes. Briefly, carboxymethylated dextran biosensor chips (CM5, GE Healthcare) are activated with N-ethyl-N'-(3-dimethylaminopropyl)-carbodiimide hydrochloride (EDC) and N-hydroxysuccinimide (NHS) according to the supplier's instructions. Anti Penta-His antibody is diluted with 10 mM sodium acetate, pH 5.0, to 40 μg/ml before injection at a flow rate of 5 μl/min to achieve approximately 6500 response units (RU) of coupled protein. Following the injection of the ligand, 1 M ethanolamine is injected to block unreacted groups. Subsequently the Fc-receptor is captured for 60 s at 4 or 10 nM. For kinetic measurements, four-fold serial dilutions of the bispecific construct (range between 500 nM and 4000 nM) are injected in HBS-EP (GE Healthcare, 10 mM HEPES, 150 mM NaCl, 3 mM EDTA, 0.05 % Surfactant P20, pH 7.4) at 25 °C at a flow rate of 30 μl/min for 120 s.

To determine the affinity to the target antigen, bispecific constructs are captured by an antihuman Fab specific antibody (GE Healthcare) that is immobilized on an activated CM5-sensor chip surface as described for the anti Penta-His antibody. The final amount of coupled protein is is approximately 12000 RU. The bispecific constructs are captured for 90 s at 300 nM. The target antigens are passed through the flow cells for 180 s at a concentration range from 250 to 1000 nM with a flowrate of 30 μl/min. The dissociation is monitored for 180 s.

Bulk refractive index differences are corrected for by subtracting the response obtained on reference flow cell. The steady state response was used to derive the dissociation constant $K_D$ by non-linear curve fitting of the Langmuir binding isotherm. Association rates ($k_{on}$) and dissociation rates ($k_{off}$) are calculated using a simple one-to-one Langmuir binding model (BIACORE® T100 Evaluation Software version 1.1.1) by simultaneously fitting the association and dissociation sensorgrams. The equilibrium dissociation constant ($K_D$) is calculated as the ratio $k_{off}/k_{on}$. See, e.g., Chen et al., J Mol Biol 293, 865-881 (1999).

*Activity assays*

Biological activity of the protease-activatable T cell activating bispecific molecules of the invention can be measured by various assays as described in the Examples. Biological activities may for example include the induction of proliferation of T cells, the induction of signaling in T cells, the induction of expression of activation markers in T cells, the induction of cytokine secretion by T cells, the induction of lysis of target cells such as tumor cells, and the induction of tumor regression and/or the improvement of survival.

**Compositions, Formulations, and Routes of Administration**

In a further aspect, the invention provides pharmaceutical compositions comprising any of the protease-activatable T cell activating bispecific molecules provided herein, e.g., for use in any of the below therapeutic methods. In one embodiment, a pharmaceutical composition comprises any of the protease-activatable T cell activating bispecific molecules provided herein and a pharmaceutically acceptable carrier. In another embodiment, a pharmaceutical composition comprises any of the protease-activatable T cell activating bispecific molecules provided herein and at least one additional therapeutic agent, e.g., as described below.

Further provided is a method of producing a protease-activatable T cell activating bispecific molecule of the invention in a form suitable for administration in vivo, the method comprising (a) obtaining a protease-activatable T cell activating bispecific molecule according to the invention, and (b) formulating the protease-activatable T cell activating bispecific molecule with at least one pharmaceutically acceptable carrier, whereby a preparation of protease-activatable T cell activating bispecific molecule is formulated for administration in vivo.

Pharmaceutical compositions of the present invention comprise a therapeutically effective amount of one or more protease-activatable T cell activating bispecific molecule dissolved or dispersed in a pharmaceutically acceptable carrier. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one protease-activatable T cell activating bispecific molecule and optionally an additional active ingredient will be known to those of skill in the art in light of the present disclosure, as exemplified by Remington's Pharmaceutical Sciences, 18th Ed. Mack Printing Company, 1990. Moreover, for animal (e.g., human) administration, it will be understood that preparations should meet sterility, pyrogenicity, general safety and purity standards as required by FDA Office of Biological Standards or corresponding authorities in other countries. Preferred compositions are lyophilized formulations or aqueous solutions. As used herein, "pharmaceutically acceptable carrier" includes any and all solvents, buffers, dispersion media, coatings, surfactants, antioxidants, preservatives (e.g., antibacterial agents, antifungal agents), isotonic agents, absorption delaying agents, salts, preservatives, antioxidants, proteins, drugs, drug stabilizers, polymers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The composition may comprise different types of carriers depending on whether it is to be administered in solid, liquid or aerosol form, and whether it need to be sterile for such routes of administration as injection. Protease-activatable T cell activating bispecific molecules of the present invention (and any additional therapeutic agent) can be administered intravenously, intradermally, intraarterially, intraperitoneally, intralesionally, intracranially, intraarticularly, intraprostatically, intrasplenically, intrarenally, intrapleurally, intratracheally, intranasally, intravitreally, intravaginally, intrarectally, intratumorally, intramuscularly, intraperitoneally, subcutaneously, subconjunctivally, intravesicularlly, mucosally, intrapericardially, intraumbilically, intraocularally, orally, topically, locally, by inhalation (e.g., aerosol inhalation), injection, infusion, continuous infusion, localized perfusion bathing target cells directly, via a catheter, via a lavage, in cremes, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990). Parenteral administration, in particular intravenous injection, is most commonly used for administering polypeptide molecules such as the protease-activatable T cell activating bispecific molecules of the invention.

Parenteral compositions include those designed for administration by injection, e.g., subcutaneous, intradermal, intralesional, intravenous, intraarterial intramuscular, intrathecal or intraperitoneal injection. For injection, the protease-activatable T cell activating bispecific molecules of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the protease-activatable T cell activating bispecific molecules may be in powder form for constitution with a suitable vehicle, e.g., sterile pyrogen-free water, before use. Sterile injectable solutions are prepared by incorporating the protease-activatable T cell activating bispecific molecules of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g., films, or microcapsules. In particular embodiments, prolonged absorption of an injectable composition can be brought about by the use in the compositions of agents delaying absorption, such as, for example, aluminum monostearate, gelatin or combinations thereof.

In addition to the compositions described previously, the protease-activatable T cell activating bispecific molecules may also be formulated as a depot preparation. Such long acting formulations may be administered by implantation (for example subcutaneously or intramuscularly) or by intramuscular injection. Thus, for example, the protease-activatable T cell activating bispecific molecules may be formulated with suitable polymeric or hydrophobic materials (for example as an emulsion in an acceptable oil) or ion exchange resins, or as sparingly soluble derivatives, for example, as a

sparingly soluble salt.

Pharmaceutical compositions comprising the protease-activatable T cell activating bispecific molecules of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The protease-activatable T cell activating bispecific molecules may be formulated into a composition in a free acid or base, neutral or salt form. Pharmaceutically acceptable salts are salts that substantially retain the biological activity of the free acid or base. These include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

**Therapeutic Methods and Compositions**

Any of the protease-activatable T cell activating bispecific molecules provided herein may be used in therapeutic methods. Protease-activatable T cell activating bispecific molecules of the invention can be used as immunotherapeutic agents, for example in the treatment of cancers.

For use in therapeutic methods, protease-activatable T cell activating bispecific molecules of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, protease-activatable T cell activating bispecific molecules of the invention for use as a medicament are provided. In further aspects, protease-activatable T cell activating bispecific molecules of the invention for use in treating a disease are provided. In certain embodiments, protease-activatable T cell activating bispecific molecules of the invention for use in a method of treatment are provided. In one embodiment, the invention provides a protease-activatable T cell activating bispecific molecule as described herein for use in the treatment of a disease in an individual in need thereof. In certain embodiments, the invention provides a protease-activatable T cell activating bispecific molecule for use in a method of treating an individual having a disease comprising administering to the individual a therapeutically effective amount of the protease-activatable T cell activating bispecific molecule. In certain embodiments the disease to be treated is a proliferative disorder. In a particular embodiment the disease is cancer. In certain embodiments the method further comprises administering to the individual a therapeutically effective amount of at least one additional therapeutic agent, e.g., an anti-cancer agent if the disease to be treated is cancer. In further embodiments, the invention provides a protease-activatable T cell activating bispecific molecule as described herein for use in inducing lysis of a target cell, particularly a tumor cell. In certain embodiments, the invention provides a protease-activatable T cell activating bispecific molecule for use in a method of inducing lysis of a target cell, particularly a tumor cell, in an individual comprising administering to the individual an effective amount of the protease-activatable T cell activating bispecific molecule to induce lysis of a target cell. An "individual" according to any of the above embodiments is a mammal, preferably a human.

In a further aspect, the invention provides for the use of a protease-activatable T cell activating bispecific molecule of the invention in the manufacture or preparation of a medicament. In one embodiment the medicament is for the treatment of a disease in an individual in need thereof. In a further embodiment, the medicament is for use in a method of treating a disease comprising administering to an individual having the disease a therapeutically effective amount of the medicament. In certain embodiments the disease to be treated is a proliferative disorder. In a particular embodiment the disease is cancer. In one embodiment, the method further comprises administering to the individual a therapeutically effective amount of at least one additional therapeutic agent, e.g., an anti-cancer agent if the disease to be treated is cancer. In a further embodiment, the medicament is for inducing lysis of a target cell, particularly a tumor cell. An "individual" according to any of the above embodiments may be a mammal, preferably a human. In a further aspect, the invention provides a method for treating a disease. In one embodiment, the method comprises administering to an individual having such disease a therapeutically effective amount of a protease-activatable T cell activating bispecific molecule of the invention. In one embodiment a composition is administered to said invididual, comprising the protease-activatable T cell activating bispecific molecule of the invention in a pharmaceutically acceptable form. In certain embodiments the disease to be treated is a proliferative disorder. In a particular embodiment the disease is cancer. In certain embodiments the method further comprises administering to the individual a therapeutically effective amount of at least one additional therapeutic agent, e.g., an anti-cancer agent if the disease to be treated is cancer. An "individual" according to any of the above embodiments may be a mammal, preferably a human. In one embodiment, an "individual"

is a human.

In certain embodiments the disease to be treated is a proliferative disorder, particularly cancer. Non-limiting examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using a protease-activatable T cell activating bispecific molecule of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. A skilled artisan readily recognizes that in many cases the protease-activatable T cell activating bispecific molecule may not provide a cure but may only provide partial benefit. In some embodiments, a physiological change having some benefit is also considered therapeutically beneficial. Thus, in some embodiments, an amount of protease-activatable T cell activating bispecific molecule that provides a physiological change is considered an "effective amount" or a "therapeutically effective amount". The subject, patient, or individual in need of treatment is typically a mammal, more specifically a human.

In some embodiments, an effective amount of a protease-activatable T cell activating bispecific molecule of the invention is administered to a cell. In other embodiments, a therapeutically effective amount of a protease-activatable T cell activating bispecific molecule of the invention is administered to an individual for the treatment of disease.

For the prevention or treatment of disease, the appropriate dosage of a protease-activatable T cell activating bispecific molecule of the invention (when used alone or in combination with one or more other additional therapeutic agents) will depend on the type of disease to be treated, the route of administration, the body weight of the patient, the type of T cell activating bispecific antigen binding molecule, the severity and course of the disease, whether the T cell activating bispecific antigen binding molecule is administered for preventive or therapeutic purposes, previous or concurrent therapeutic interventions, the patient's clinical history and response to the protease-activatable T cell activating bispecific molecule, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The protease-activatable T cell activating bispecific molecule is suitably administered to the patient at one time or over a series of treatments. Depending on the type and severity of the disease, about 1 μg/kg to 15 mg/kg (e.g., 0.1 mg/kg - 10 mg/kg) of protease-activatable T cell activating bispecific molecule can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the T cell activating bispecific antigen binding molecule would be in the range from about 0.005 mg/kg to about 10 mg/kg. In other non-limiting examples, a dose may also comprise from about 1 microgram/kg body weight, about 5 microgram/kg body weight, about 10 microgram/kg body weight, about 50 microgram/kg body weight, about 100 microgram/kg body weight, about 200 microgram/kg body weight, about 350 microgram/kg body weight, about 500 microgram/kg body weight, about 1 milligram/kg body weight, about 5 milligram/kg body weight, about 10 milligram/kg body weight, about 50 milligram/kg body weight, about 100 milligram/kg body weight, about 200 milligram/kg body weight, about 350 milligram/kg body weight, about 500 milligram/kg body weight, to about 1000 mg/kg body weight or more per administration, and any range derivable therein. In non-limiting examples of a derivable range from the numbers listed herein, a range of about 5 mg/kg body weight to about 100 mg/kg body weight, about 5 microgram/kg body weight to about 500 milligram/kg body weight, etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g., every week or every three weeks (e.g., such that the patient receives from about two to about twenty, or e.g., about six doses of the protease-activatable T cell activating bispecific molecule). An initial higher loading dose, followed by one or more lower doses may be administered. However, other dosage regimens may be useful. The progress of this therapy is easily monitored by conventional techniques and assays.

The protease-activatable T cell activating bispecific molecule of the invention will generally be used in an amount effective to achieve the intended purpose. For use to treat or prevent a disease condition, the protease-activatable T cell activating bispecific molecules of the invention, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from *in vitro* assays, such

as cell culture assays. A dose can then be formulated in animal models to achieve a circulating concentration range that includes the $IC_{50}$ as determined in cell culture. Such information can be used to more accurately determine useful doses in humans.

Initial dosages can also be estimated from *in vivo* data, *e.g.,* animal models, using techniques that are well known in the art. One having ordinary skill in the art could readily optimize administration to humans based on animal data.

Dosage amount and interval may be adjusted individually to provide plasma levels of the protease-activatable T cell activating bispecific molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the protease-activatable T cell activating bispecific molecules may not be related to plasma concentration. One having skill in the art will be able to optimize therapeutically effective local dosages without undue experimentation.

A therapeutically effective dose of the protease-activatable T cell activating bispecific molecules described herein will generally provide therapeutic benefit without causing substantial toxicity. Toxicity and therapeutic efficacy of a protease-activatable T cell activating bispecific molecule can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. Protease-activatable T cell activating bispecific molecule that exhibit large therapeutic indices are preferred. In one embodiment, the protease-activatable T cell activating bispecific molecule according to the present invention exhibits a high therapeutic index. The data obtained from cell culture assays and animal studies can be used in formulating a range of dosages suitable for use in humans. The dosage lies preferably within a range of circulating concentrations that include the $ED_{50}$ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1).

The attending physician for patients treated with protease-activatable T cell activating bispecific molecules of the invention would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

**Other Agents and Treatments**

The protease-activatable T cell activating bispecific molecules of the invention may be administered in combination with one or more other agents in therapy. For instance, a protease-activatable T cell activating bispecific molecule of the invention may be co-administered with at least one additional therapeutic agent. The term "therapeutic agent" encompasses any agent administered to treat a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is an immunomodulatory agent, a cytostatic agent, an inhibitor of cell adhesion, a cytotoxic agent, an activator of cell apoptosis, or an agent that increases the sensitivity of cells to apoptotic inducers. In a particular embodiment, the additional therapeutic agent is an anti-cancer agent, for example a microtubule disruptor, an antimetabolite, a topoisomerase inhibitor, a DNA intercalator, an alkylating agent, a hormonal therapy, a kinase inhibitor, a receptor antagonist, an activator of tumor cell apoptosis, or an antiangiogenic agent.

Such other agents are suitably present in combination in amounts that are effective for the purpose intended. The effective amount of such other agents depends on the amount of protease-activatable T cell activating bispecific molecule used, the type of disorder or treatment, and other factors discussed above. The protease-activatable T cell activating bispecific molecule are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeuic agents are included in the same or separate compositions), and separate administration, in which case, administration of the protease-activatable T cell activating bispecific molecule of the invention can occur prior to, simultaneously, and/or

following, administration of the additional therapeutic agent and/or adjuvant. Protease-activatable T cell activating bispecific molecules of the invention can also be used in combination with radiation therapy.

**Articles of Manufacture**

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a protease-activatable T cell activating bispecific molecule of the invention. The label or package insert indicates that the composition is used for treating the condition of choice. Moreover, the article of manufacture may comprise (a) a first container with a composition contained therein, wherein the composition comprises a protease-activatable T cell activating bispecific molecule of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Examples**

The following are examples of methods and compositions of the invention. It is understood that various other embodiments may be practiced, given the general description provided above.

**Example 1**

**Synthesis of monovalent anti-CD3 IgG molecules with anti-idiotypic scFv.**

Described herein are CD3 binders that are masked with an N-terminally linked anti-idiotypic CD3 scFv. These constructs include a protease recognition site which is recognized by a tumor specific protease. In the presence of protease-expressing tumor cells, the linker connecting the masking moiety will be cleaved and, thereby, CD3 binding by the CD3 binder is recovered. Several monovalent anti-CD3 IgG molecules with various anti-idiotypic scFv were produced and are schematically depicted in FIGs.1A-E with their respective ID number. The following molecules were prepared:

Identification No.7859: monovalent CD3 IgG, (anti-idiotypic scFv 4.15.64 - MK062 Matriptase site - CD3 - N-terminal fused to CD3 Fab - inert Fc) with N-terminal fused anti CD3 scFv 4.15.64 and protease-cleavable linker.
Identification No.7860: monovalent CD3 IgG, (anti-idiotypic scFv 4.32.63 - MK062 Matriptase site - CD3 - N-terminal fused to CD3 Fab - inert Fc) with N-terminal fused anti CD3 scFv 4.32.63 and protease-cleavable linker.
Identification No.7857: monovalent CD3 IgG, (anti-idiotypic scFv 4.15.64 - non-cleavable linker - CD3 - N-terminal fused to CD3 Fab - inert Fc) with N-terminal fused anti CD3 scFv 4.15.64 and protease-cleavable linker.
Identification No.7858: monovalent CD3 IgG, (anti-idiotypic scFv 4.32.63 - non-cleavable linker - CD3 - N-terminal fused to CD3 Fab - inert Fc) with N-terminal fused anti CD3 scFv 4.32.63 and protease-cleavable linker.
Identification No.7861: monovalent CD3 IgG, (CD3 Fab- inert Fc) with N-terminal fused anti CD3 scFv 4.15.64 / 4.32.63 and protease linker.

Anti-idiotypic (ID) binder sequences were obtained by RACE-PCR (rapid amplification of cDNA ends) from RNA of Hybridoma cells. Hybridoma cells were obtained by immunization of mice with CH2527 (VL_7-46(13)/VH_3-23(12)) Fab-fragment. Single chain Fv (ScFv) sequence synthesis was ordered from Invitrogen including the necessary restriction sites for cloning. Six different anti-idiotypic CH2527 binders were compared for their affinities (FIG.2, result Biacore-Analytics (AG M. Schräml) at 25°C / 37°C (Analyt: MAK<CEA/CD3>rH)) and two of them were cloned as N-terminal fusions at the heavy chain of CD3 Fab - Fc.

The anti-ID single chain Fv DNA sequences were subcloned in frame with the CD3 VH chain pre-inserted into the respective recipient mammalian expression vector. Protein expression is driven by an MPSV promoter and a synthetic

polyA signal sequence is present at the 3' end of the CDS. In addition each vector contains an EBV OriP sequence.

The molecules were produced by co-transfecting HEK293-EBNA cells growing in suspension with the mammalian expression vectors using polyethylenimine (PEI). The cells were transfected with the corresponding expression vectors in a 1:1:2 ratio ("Fc hole (CH2-CH3)": "common light chain (CLC)": "vector heavy chain knob (scFv-VH-CH1-CH2-CH3)").

For transfection, HEK293 EBNA cells were cultivated in serum free ExCell culture medium containing 6 mM L-glutamine and 250 mg/l G418. For the production in 600 ml tubespin flasks (max. working volume 400 mL) 800 million HEK293 EBNA cells were seeded 24 hours before transfection without G418. For transfection 800 mio cells were centrifuged for 5 min at 210 × g and supernatant was replaced by 40 ml pre-warmed CD CHO medium containing 6mM L-Glutamine. Expression vectors were mixed with 40 ml CD CHO medium containing 6mM L-Glutamine to a total amount of 400 μg DNA. After addition of 1080 μl PEI solution (2.7 μg/ml) the mixture was vortexed for 15 s and subsequently incubated for 10 min at room temperature. Afterwards cells were mixed with the DNA/PEI solution, transferred to a 600 ml tubespin flask and incubated for 3 hours at 37°C in an incubator with a 5% $CO_2$ atmosphere. After incubation, 320 ml ExCell + 6mM L-glutamine + 5g/L Pepsoy + 1.0mM VPA + 3 g/l glucose medium was added and cells were cultivated for 24 hours prior to feeding with 7% Feed 7. After 6-7 days cultivation supernatant was collected for purification by centrifugation for 20 - 30 min at 210 × g (Sigma 8K centrifuge). The solution was sterile filtered (0.22 μm filter) and sodium azide in a final concentration of 0.01% w/v was added. The solution was kept at 4°C until purification. The secreted protein was purified from cell culture supernatants by affinity chromatography using ProteinA affinity chromatography, followed by one to two size exclusion chromatographic steps.

For affinity chromatography supernatant was loaded on a HiTrap Protein A FF column (CV = 5 mL, GE Healthcare) equilibrated with 25 ml 20 mM sodium phosphate, 20 mM sodium citrate, 0.5M sodium chloride, 0.01% Tween-20 pH 7.5. Unbound protein was removed by washing with at least 10 column volumes 20 mM sodium phosphate, 20 mM sodium citrate, 0.5M sodium chloride, 0.01% Tween-20 pH 7.5 and target protein was eluted in 20 column volumes (gradient from 0 % - 100 %) 20 mM sodium citrate, 0.5M sodium chloride, 0.01% Tween-20 pH 2.5. Protein solution was neutralized by adding 1/10 of 2 M Tris pH 10.5. Target protein was concentrated with Amicon®Ultra-15 Ultracel 30K (Merck Millipore Ltd.) to a volume of 4 ml maximum prior loading on a HiLoad Superdex 200 column (GE Healthcare) equilibrated with 20 mM histidine, 140 mM sodium chloride, pH 6.0, 0.01% Tween20.

For analytics after size exclusion chromatography the purity and molecular weight of the molecules in the single fractions were analyzed by SDS-PAGE in the absence of a reducing agent and staining with Coomassie (InstantBlue™, Expedeon). The NuPAGE® Pre-Cast gel system (4-12% Bis-Tris, Invitrogen or 3-8%Tris-Acetate, Invitrogen) was used according to the manufacturer's instruction.

The protein concentration of purified protein samples was determined by measuring the optical density (OD) at 280 nm divided by the molar extinction coefficient calculated on the basis of the amino acid sequence.

Purity and molecular weight of the molecules after the final purification step were analyzed by CE-SDS analyses in the presence and absence of a reducing agent. The Caliper LabChip GXII system (Caliper Lifescience) was used according to the manufacturer's instruction. The aggregate content of the molecules was analyzed using a TSKgel G3000 SW XL analytical size-exclusion column (Tosoh) in 25 mM K2HPO4, 125 mM NaCl, 200 mM L-arginine monohydrocloride, 0.02% (w/v) NaN3, pH 6.7 running buffer at 25°C. The final quality of all molecules was good, with ≥ 92 % monomer content.

TABLE 2. Summary of production and purification of protease activated monovalent CD3 IgG molecules.

| Molecule | Titer [mg/l] | Yield [mg/l] | Analytical SEC (HMW/Monomer/LMW) [%] |
|---|---|---|---|
| 1 | 12 | 3.38 | 2.21/95.5/2.29 |
| 2 | 9 | 1.75 | 4.86/95.14/0 |
| 3 | 15 | 4.8 | 6.93/93.07/0 |
| 4 | 4.5 | 0.26 | 4.88/95.12/0 |
| 5 | 105.3 | 26.3 | 0/100/0 |

**Example 2**

**Cleavage and stability of Protease activated IgGs.**

Capillary Electrophoresis of protease activated IgG molecules. Comparison of untreated sample and treated sample showed that the anti-ID scFv was completely cleaved off after treatment with rhMatriptase/ST14 (R&D Systems)

indicated by the size shift in the SDS page analysis (FIG. 3). Analysis of samples incubated for 48 h at 37°C confirmed stability of the molecules in formulation buffer (FIG. 3A-D).

**Example 3**

**Masking effect of anti-idiotypic scFv for CD3 IgG.**

The efficiency of masking the CD3 binder by N-terminal fusion of an anti-idiotypic CD3 scFv was shown by a Jurkat-NFAT reporter assay. Jurkat-NFAT reporter cells (a human acute lymphatic leukemia reporter cell line with a NFAT promoter-regulated luciferase expression, GloResponse Jurkat NFAT-RE-luc2P, Promega #CS176501) express active firefly luciferase if the NFAT promoter is activated by binding of CD3ε. The intensity of the luminescence signal upon addition of luciferase substrate is proportional to the intensity of CD3 activation and signaling. Completely unmasked monovalent CD3 molecules served as a positive control. The treatment was done with rhMatriptase/ST14 (R&D Systems) for 48h at 37°C. In parallel 8ug/ml Anti human Fc Antibody (BioLegends) were coated in 0.025ul/well PBS for 48h at 4°C in white-walled, clear bottom 96-well (flat)-plate (Greiner Bio-One). PBS was removed by pipetting before monovalent IgGs were added at the indicated concentration range of 200 nM - 2.56pM. Plates were incubated for about 30 min at 4°C. Subsequently, Jurkat-NFAT reporter cells were harvested and viability assessed using ViCell. Cells were resuspended in Jurkat medium (RPMI1640, 2g/l Glucose, 2 g/l NaHCO3, 10 % FCS, 25 mM HEPES, 2 mM L-Glutamin, 1 × NEAA, 1 × Sodium-pyruvate) without Hygromycine and 100 μl per well (25.000 cells / well) were added to the crosslinked monovalent CD3 IgGs. Cells were incubated for 3 h at 37°C in a humidified incubator. Plates were taken out of the incubator for about 10 min to adapt to room temperature prior to Luminescence read out. 100 μl/well of ONE-Glo solution (1:1 ONE-Glo and assay medium volume per well) were added to wells and incubated for 10 min at room temperature in the dark. Luminescence was detected using WALLAC Victor3 ELISA reader (PerkinElmer2030), 1 sec/well as detection time. 7857 (4.15.64 mask with non-cleavable linker) and 7859 (untreated) show significantly reduced CD3ε binding compared to unmasked (7861) and pretreated molecule (7859 treated) (FIG. 4A). 7760 was included as a control to show that N-terminal linkage does not block CD3 binding itself. 7858 (4.32.63 mask with non-cleavable linker) and 7860 (untreated) show significantly reduced CD3ε binding compared to unmasked (7861) and pretreated molecule (7860 treated) (FIG. 4B). In line with the affinities of the anti-idiotypic CD3 binders the 4.32.63 mask is much more efficient than the 4.15.64. In terms of EC50 values (FIG. 4C) the 4.32.63 masked CD3 binder binds 54 fold less than the unmasked CD3 binder 7861. For the 4.15.64 mask it is only 16 fold less binding than for 7861. Depending on the tumor target and the target binder the best mask can be evaluated.

**Example 4**

**Preparation of anti FolR1/anti-CD3 T cell bispecific (TCB) molecules with anti CD3 scFv.**

Several T cell bispecific (TCB) molecules with various anti-idiotypic scFv were produced and are schematically depicted in FIGs.5A-H with their respective ID number. The following molecules were prepared:

ID7344: FolR1 16D5 2+1 IgG, classic format (anti-idiotypic scFv 4.15.64 - MK062 Matriptase site - CD3 - N-terminal fused to FolR1 VH - inert Fc) with N-terminal fused anti CD3 scFv 4.15.64 and protease linker (FIG. 5A, SEQ ID NOs 1, 2 and 3).

ID7496: FolR1 16D5 2+1 IgG, classic format (anti-idiotypic scFv 4.32.63 - MK062 Matriptase site - CD3 - N-terminal fused to FolR1 VH - inert Fc) with N-terminal fused anti CD3 scFv 4.32.63 and protease linker (FIG. 5C, SEQ ID NOs 1, 3 and 4).

ID7676: FolR1 16D5 2+1 IgG, classic format (anti-idiotypic scFv 4.15.64 - non-cleavable GS linker - CD3 - N-terminal fused to FolR1 VH - inert Fc) with N-terminal fused anti CD3 scFv 4.15.64 and protease linker (FIG. 5B, SEQ ID NOs 1, 3 and 6).

ID7611: FolR1 16D5 2+1 IgG, classic format (anti-idiotypic scFv 4.32.63 - non-cleavable GS linker - CD3 - N-terminal fused to FolR1 VH - inert Fc) with N-terminal fused anti CD3 scFv 4.32.63 and protease linker (FIG. 5D, SEQ ID NOs 1, 3 and 5).

Anti-idiotypic (ID) binder sequences were obtained by RACE-PCR (rapid amplification of cDNA ends) from RNA of Hybridoma cells. Hybridoma cells were obtained by immunization of mice. Single chain Fv (ScFv) sequence synthesis was ordered at Invitrogen including the necessary restriction sites for cloning. Six different anti-idiotypic CH2527 binders were compared for their affinities (FIG. 2, result Biacore-Analytics (AG M. Schräml) at 25°C / 37°C (Analyt: MAK<CEA/CD3>rH)) and four of them were cloned as N-terminal fusions at the HC of CD3 - FolR1 16D5 TCB.

The anti-ID single chain Fv DNA sequences were subcloned in frame with the CD3 VH chain pre-inserted into

the respective recipient mammalian expression vector. Protein expression is driven by an MPSV promoter and a synthetic polyA signal sequence is present at the 3' end of the coding sequence (CDS). In addition each vector contains an EBV OriP sequence.

The molecules were produced by co-transfecting HEK293-EBNA cells growing in suspension with the mammalian expression vectors using polyethylenimine (PEI). The cells were transfected with the corresponding expression vectors in a 1:3:2 ratio ("vector heavy chain hole (VH-CH1-CH2-CH3)" : "common light chain (CLC)" : "vector heavy chain knob (scFv-VH-CH1-VH-CH1-CH2-CH3)").

For transfection HEK293 EBNA cells were cultivated in serum free ExCell culture medium containing 6 mM L-glutamine and 250 mg/l G418. For the production in 600 ml tubespin flasks (max. working volume 400 mL) 800 million HEK293 EBNA cells were seeded 24 hours before transfection without G418. For transfection 800 mio cells were centrifuged for 5 min at 210 × g and supernatant was replaced by 40 ml pre-warmed CD CHO medium containing 6mM L-Glutamine. Expression vectors were mixed with 40 ml CD CHO medium containing 6mM L-Glutamine to a total amount of 400 μg DNA. After addition of 1080 μl PEI solution (2.7 μg/ml) the mixture was vortexed for 15 s and subsequently incubated for 10 min at room temperature. Afterwards cells were mixed with the DNA/PEI solution, transferred to a 600 ml tubespin flask and incubated for 3 hours at 37°C in an incubator with a 5% $CO_2$ atmosphere. After incubation, 320 ml ExCell + 6mM L-glutamine + 5g/L Pepsoy + 1.0mM VPA + 3 g/l glucose medium was added and cells were cultivated for 24 hours prior to feeding with 7% Feed 7. After 6-7 days cultivation supernatant was collected for purification by centrifugation for 20 - 30 min at 210 × g (Sigma 8K centrifuge). The solution was sterile filtered (0.22 μm filter) and sodium azide in a final concentration of 0.01% w/v was added. The solution was kept at 4°C until purification. The secreted protein was purified from cell culture supernatants by affinity chromatography using ProteinA affinity chromatography, followed by one to two size exclusion chromatographic steps.

For affinity chromatography supernatant was loaded on a HiTrap Protein A FF column (CV = 5 mL, GE Healthcare) equilibrated with 25 ml 20 mM sodium phosphate, 20 mM sodium citrate, 0.5M sodium chloride, 0.01% Tween-20 pH 7.5. Unbound protein was removed by washing with at least 10 column volumes 20 mM sodium phosphate, 20 mM sodium citrate, 0.5M sodium chloride, 0.01% Tween-20 pH 7.5 and target protein was eluted in 20 column volumes (gradient from 0 % - 100 %) 20 mM sodium citrate, 0.5M sodium chloride, 0.01% Tween-20 pH 2.5.

Protein solution was neutralized by adding 1/10 of 2 M Tris pH 10.5. Target protein was concentrated with Amicon®Ultra-15 Ultracel 30K (Merck Millipore Ltd.) to a volume of 4 ml maximum prior loading on a HiLoad Superdex 200 column (GE Healthcare) equilibrated with 20 mM histidine, 140 mM sodium chloride, pH 6.0, 0.01% Tween20.

For analytics after size exclusion chromatography the purity and molecular weight of the molecules in the single fractions were analyzed by SDS-PAGE in the absence of a reducing agent and staining with Coomassie (InstantBlue™, Expedeon). The NuPAGE® Pre-Cast gel system (4-12% Bis-Tris, Invitrogen or 3-8%Tris-Acetate, Invitrogen) was used according to the manufacturer's instruction.

The protein concentration of purified protein samples was determined by measuring the optical density (OD) at 280 nm divided by the molar extinction coefficient calculated on the basis of the amino acid sequence.

Purity and molecular weight of the molecules after the final purification step were analyzed by CE-SDS analyses in the presence and absence of a reducing agent. The Caliper LabChip GXII system (Caliper Lifescience) was used according to the manufacturer's instruction.

The aggregate content of the molecules was analyzed using a TSKgel G3000 SW XL analytical size-exclusion column (Tosoh) in 25 mM K2HPO4, 125 mM NaCl, 200 mM L-arginine monohydrocloride, 0.02% (w/v) NaN3, pH 6.7 running buffer at 25°C. The final quality of all molecules was good, with ≥ 92 % monomer content.

TABLE 2. Summary of production and purification of protease activated TCB molecules.

| Molecule | Titer [mg/l] | Yield [mg/l] | Analytical SEC (HMW/Monomer/LMW) [%] |
|---|---|---|---|
| 1 | 33 | 3.7 | 0.98/92.7/6.32 |
| 2 | 11 | 0.55 | 3.76/96.24/0 |
| 3 | 12.9 | 0.89 | 2.9/93.82/2.19 |
| 4 | 6.7 | 0.35 | 4.59/95.41/0 |

**Example 5**

**Transient expression of protease activated TCBs.**

Different plasmid ratios used for transfection were compared by size exclusion chromatograpy as the knob chain was suspected to be expressed in lower levels compared to the hole chain and the light chain. As shown in FIG.

6 and 7, using a plasmid ratio of 1 (hole): 2 (knob): 3 (CLC) (FIG. 7) instead of 1(hole): 1 (knob): 3 (CLC) (FIG. 6) increased the yield of correct molecule (left peak) and decreased the amount of hole hole homodimers (right peak).

**Example 6**

**Cleavage and stability of Protease activated TCB.**

Protease activated TCBs were analyzed by capillary electrophoresis. Comparison of untreated sample and treated sample showed that the anti-idiotype scFc moiety was completely cleaved off after treatment with rh-Matriptase/ST14. Analysis of samples incubated for 48 h at 37°C confirmed stability of the molecules in formulation buffer (FIG. 12A-D).

**Example 7**

**Cell killing using target cell lines that express different levels of FolR1.**

T-cell-mediated cell killing induced by protease activated TCB molecules was assessed using target cell lines expressing different levels of FolR1 (FIG. 13). Human PBMCs were used as effector cells and cell killing was detected at 48 h of incubation with the protease activated TCB molecules. Human Peripheral blood mononuclear cells (PBMCs) were isolated from fresh taken blood or from buffy coats obtained from healthy human donors. For fresh blood 50 ml Leucosep tubes (GreinerBioOne) were used for preparation. For enriched lymphocyte preparations (buffy coats) Histopaque-1077 density preparation was used. Blood/buffy coat was diluted 1:1 with sterile PBS and layered over Histopaque gradient (Sigma, #H8889). After centrifugation (450 × g, 30 minutes, w/o break, room temperature), the plasma above the PBMC-containing interphase was discarded and PBMCs transferred in a new falcon tube subsequently filled with 50 ml of PBS. The mixture was centrifuged (400 × g, 10 minutes, room temperature), the supernatant discarded and the PBMC pellet resuspended in 2 ml ACK buffer for Erythrocytes lysis. After incubation at 37°C for about 2 -3 minutes the tubes were filled with sterile PBS to 50 ml and centrifuged at 350 × g for 10 minutes. This washing step was repeated once prior to resuspension of PBMCs in RPMI1640 medium containing 2% FCS and 1X GlutaMax at 37°C, 5% $CO_2$ in cell incubator until further use. Briefly, adherent target cells were harvested with Trypsin/EDTA, counted, checked for viability and resuspended at 0.4 $\times 10^6$ cells/ml in assay medium (RPMI1640, 2% FCS, 1X GlutaMax). Target cells were plated at a density of 20 000 cells/well using round-bottom 96-well plates. For the killing assay, the molecules were added at the indicated concentrations in triplicates. FolR1 16D5 TCB was included as positive control and an untargeted TCB molecule (binding to CD3 but not to a target cell antigen) was included as negative control. PBMCs were added to target cells at final E:T ratio of 10:1. Target cell killing was assessed after 48 h of incubation at 37°C, 5% $CO_2$ by quantification of LDH release into cell supernatants by apoptotic/necrotic cells (LDH detection kit, Roche Applied Science, #11 644 793 001). Maximal lysis of the target cells (= 100%) was achieved by incubation of target cells with 1% Triton X-100 1 h before LDH readout. Minimal lysis (= 0%) refers to target cells co-incubated with effector cells without any TCB.

The results (FIG. 14A, 15A, 16, 17, 18A, 19A and 20A) show that the protease activated TCB with anti-idiotypic CD3 scFv moiety N-terminally linked by a non-cleavable linker (#7676 and #7611, FIG. 5B and D, respectively) were able to significantly reduce cell lysis on Skov3 and HT29 cells. #7611 (FIG. 5D) led to reduced killing on Hela cells while anti-idiotypic CD3 scFv 4.15.64 in #7676 (FIG. 5B) was less efficient in reduction of cell lysis. This is in line with affinities of the anti-idiotypic CD3 scFv N moiety. The higher affinity scFv moiety masks more efficiently.

Comparable potency of treated and untreated TCBs suggests Matriptase expression of Hela and Skov3 cells. Expression of Matriptase seems to be lower in HT29 cells. Treatment of Mkn-45, a FolR1 negative cell line, shows only weak killing with all molecules used herein (FIG.15A).

**Example** 8

**T-cell activation after co-incubation of tumor cell lines with human PBMCs.**

T-cell activation mediated by protease activated TCB molecules was assessed on Hela, Skov3 and HT29 cells. Human PBMCs were used as effector cells and the T cell activation was detected at 48 h of incubation with target cells and the antibodies. Target cells were plated at a density of 20 000 cells/well using round-bottom 96-well plates. Molecules were added at the indicated concentrations in triplicates. FolR1 16D5 TCB was included as positive control and an untargeted TCB molecule (binding to CD3 but not to a target cell antigen) was included as negative control. PBMCs were added to target cells at final E:T ratio of 10:1. T- cell activation was assessed after 48 h of incubation at 37°C, 5% $CO_2$ by quantification of CD25 and CD69 on CD4 positive and CD8 positive T cells. T cell activation results are consistent with the results observed in the previous example assessing target cell killing (Example 7).

## Example 9

**T-cell activation mediated by protease-activated TCBs and target cell lines expressing low antigen levels.**

T-cell activation mediated by protease activated TCB molecules was assessed on HT29 cells expressing only low levels of FolR1 (FIG. 13). Human PBMCs isolated from buffy coat were used as effector cells. For enriched lymphocyte preparations (buffy coats) Histopaque-1077 density preparation was used. Buffy coat was diluted 1:1 with sterile PBS and layered over Histopaque gradient (Sigma, #H8889). After centrifugation (450 × g, 30 minutes, w/o break, room temperature), the plasma above the PBMC-containing interphase was discarded and PBMCs transferred in a new falcon tube subsequently filled with 50 ml of PBS. The mixture was centrifuged (400 × g, 10 minutes, room temperature), the supernatant discarded and the PBMC pellet resuspended in 2 ml ACK buffer for Erythrocytes lysis. After incubation at 37°C for about 2 -3 minutes the tubes were filled with sterile PBS to 50 ml and centrifuged at 350 × g for 10 minutes. This washing step was repeated once prior to resuspension of PBMCs in RPMI1640 medium containing 2% FCS and 1X GlutaMax at 37°C, 5% $CO_2$ in cell incubator until further use. Briefly, adherent target cells were harvested with Trypsin/EDTA, counted, assessed for viability and resuspended at 0.4 $\times 10^6$ cells/ml in assay medium (RPMI1640, 2% FCS, 1X GlutaMax). Target cells were plated at a density of 20 000 cells/well using round-bottom 96-well plates. Molecules were added at the indicated concentrations in triplicates. FolR1 16D5 TCB was included as positive control and an untargeted TCB molecule (binding to CD3 but not to a target cell antigen) was included as negative control. PBMCs were added to target cells at final E:T ratio of 10:1. T-cell activation was assessed after 48 h of incubation at 37°C, 5% $CO_2$ by quantification of CD25 and CD69 on CD4 positive and CD8-positive T cells. The potency of treated protease activated TCB is comparable to 16D5 TCB (6298). The 16D5 TCB (inverted format) show higher potency than the classic format. Masked TCBs with non-cleavable linker or without Matriptase pre-treatment do not induce T cell activation on this cell line. For cell lines with low or medium FolR1 expression levels both anti-idiotypic scFvs are sufficient in masking the CD3 Fab (FIG. 22A and B).

## Example 10

**T-cell activation mediated by protease activated TCB with primary cell line HRCEpiC.**

T-cell activation mediated by protease activated TCB molecules was assessed on primary Human Renal Cortical Epithelial Cell (ScienceCell) cells expressing only very little amounts of FolR1 (FIG. 13). Human PBMCs isolated from buffy coat were used as effector cells. For enriched lymphocyte preparations (buffy coats) Histopaque-1077 density preparation was used. Buffy coat was diluted 1:1 with sterile PBS and layered over Histopaque gradient (Sigma, #H8889). After centrifugation (450 × g, 30 minutes, without break at room temperature), the plasma above the PBMC-containing interphase was discarded and PBMCs transferred in a new falcon tube subsequently filled with 50 ml of PBS. The mixture was centrifuged (400 × g, 10 minutes, room temperature), the supernatant discarded and the PBMC pellet resuspended in 2 ml ACK buffer for Erythrocytes lysis. After incubation at 37°C for about 2 -3 minutes the tubes were filled with sterile PBS to 50 ml and centrifuged at 350 × g for 10 minutes. This washing step was repeated once prior to resuspension of PBMCs in RPMI1640 medium containing 2% FCS and 1X GlutaMax at 37°C, 5% $CO_2$ in cell incubator until further use. Briefly, adherent target cells were harvested with Trypsin/EDTA, counted, checked for viability and resuspended at 0.4 $\times 10^6$ cells/ml in assay medium (RPMI1640, 2% FCS, 1X GlutaMax). Target cells were plated at a density of 20 000 cells/well using round-bottom 96-well plates. Protease activatable TCB molecules were added at the indicated concentrations in triplicates. FolR1 16D5 TCB was included as positive control and an untargeted TCB molecule (binding to CD3 but not to a target cell antigen) was included as negative control. PBMCs were added to target cells at final E:T ratio of 10:1. T- cell activation was assessed after 48 h of incubation at 37°C, 5% $CO_2$ by quantification of CD25 and CD69 on CD4 positive and CD8 positive T cells. Masked 16D5 TCB does not induce T cell activation upon incubation with primary human renal cortical epithelial cells despite low level FolR1 expression at the highest concentration of 10.000 pM of TCB, demonstrating the effectiveness of the anti-idiotype masking moiety. Little T cell activation can be observed for the 16D5 TCBs (inverted and classic format) (FIG. 23).

## Example 11

**Anti-ID CD3 Fab masking CD3 binder of 16D5 TCB. Killing on Ovcar3 cells.**

T-cell-mediated target cell killing mediated by protease activated TCB molecules was assessed on OVCAR3 cells (FIG. 24). Human PBMCs were used as effector cells and cell killing was detected at 48 h of incubation with the molecules. Human Peripheral blood mononuclear cells (PBMCs) were isolated from fresh taken blood of a healthy donor. 50 ml Leucosep tubes (GreinerBioOne) were used for preparation. Blood was diluted 1:1 with sterile PBS and layered

over Histopaque gradient (Sigma, #H8889). After centrifugation (450 × g, 30 minutes, w/o break, room temperature), the plasma above the PBMC-containing interphase was discarded and PBMCs transferred in a new falcon tube subsequently filled with 50 ml of PBS. The mixture was centrifuged (400 × g, 10 minutes, room temperature), the supernatant discarded and the PBMC pellet resuspended in 2 ml ACK buffer for Erythrocytes lysis. After incubation at 37°C for about 2 -3 minutes the tubes were filled with sterile PBS to 50 ml and centrifuged at 350 × g for 10 minutes. This washing step was repeated once prior to resuspension of PBMCs in RPMI1640 medium containing 2% FCS and 1X GlutaMax at 37°C, 5% $CO_2$ in cell incubator until further use. Briefly, adherent target cells were harvested with Trypsin/EDTA, counted, checked for viability and resuspended at 0.4 $\times 10^6$ cells/ml in assay medium (RPMI1640, 2% FCS, 1× GlutaMax). Target cells were plated at a density of 20 000 cells/well using round-bottom 96-well plates. For the killing assay, the molecules were added at the indicated concentrations in triplicates. FolR1 16D5 TCB was included as positive control and an untargeted TCB molecule (binding to CD3 but not to a target cell antigen) was included as negative control. PBMCs were added to target cells at final E:T ratio of 10:1. Target cell killing was assessed after 48 h of incubation at 37°C, 5% $CO_2$ by quantification of LDH release into cell supernatants by apoptotic/necrotic cells (LDH detection kit, Roche Applied Science, #11 644 793 001). Maximal lysis of the target cells (= 100%) was achieved by incubation of target cells and PBMCs with 1% Triton X-100 1 h before LDH readout. Minimal lysis (= 0%) refers to target cells co-incubated with effector cells without any TCB. The result (Figure 24) shows that protease activated TCB with anti-idiotypic CD3 4.15.64 crossed Fab N - terminally linked by a non-cleavable linker is not significantly masking the CD3 binder. Further, Ovcar3 cells appear to express Matriptase because untreated molecule also induces killing of these cells.

**Example 12**

**Killing on Skov3 and HeLa cells with three different human PBMC donors.**

T-cell killing mediated by protease activated TCB molecules was assessed on two different cell lines expressing different levels of FolR1 (Figs. 25-27). Human PBMCs were used as effector cells and cell killing was detected at 48 h of incubation with the molecules. Human Peripheral blood mononuclear cells (PBMCs) were isolated from buffy coats obtained from healthy human donors. For enriched lymphocyte preparations (buffy coats) Histopaque-1077 density preparation was used. Blood/buffy coat was diluted 1:1 with sterile PBS and layered over Histopaque gradient (Sigma, #H8889). After centrifugation (450 × g, 30 minutes, w/o break, room temperature), the plasma above the PBMC-containing interphase was discarded and PBMCs transferred in a new falcon tube subsequently filled with 50 ml of PBS. The mixture was centrifuged (400 × g, 10 minutes, room temperature), the supernatant discarded and the PBMC pellet resuspended in 2 ml ACK buffer for Erythrocytes lysis. After incubation at 37°C for about 2 -3 minutes the tubes were filled with sterile PBS to 50 ml and centrifuged at 350 × g for 10 minutes. This washing step was repeated once prior to resuspension of PBMCs in RPMI1640 medium containing 10% FCS and 1X GlutaMax. PBMCs were resuspended in RPMI1640 medium containing 10% FCS, 1X GlutaMax and 10 % DMSO. PBMCs were frozen overnight at -80 °C in Cool Cell boxes before they were transferred to liquid nitrogen. 24 h before assay start, PBMCs were thawed and kept in RPMI1640 medium containing 10% FCS and 1X GlutaMax at 37°C, 5% CO2 in cell incubator. The day before assay start adherent target cells were harvested with Trypsin/EDTA, counted, checked for viability and resuspended at 0.4 $\times 10^6$ cells/ml in appropriate medium. Target cells were plated at a density of 20 000 cells/well using flat-bottom 96-well plates. On the day of assay start PBMCs were counted and checked for viability. PBMC s were centrifuged at 350 g for 5 min and resupsended in assay medium (RPMI1640, 2% FCS, 1X GlutaMax). The medium of target cells was removed and PBMCs were added to the target cells before diluted antibodies were added at the indicated concentrations in triplicates. FolR1 16D5 TCB was included as positive control and an untargeted TCB molecule (binding to CD3 but not to a target cell antigen) was included as negative control. PBMCs were added to target cells at E:T ratio of 10:1. Target cell killing was assessed after 48 h of incubation at 37°C, 5% CO2 by quantification of LDH release into cell supernatants by apoptotic/necrotic cells (LDH detection kit, Roche Applied Science, #11 644 793 001). Maximal lysis of the target cells (= 100%) was achieved by incubation of target cells with 1% Triton X-100 2 h before LDH readout. Minimal lysis (= 0%) refers to target cells co-incubated with effector cells without any TCB.

The results (Figures 25-27) show that FolR1 TCB with scFv 4.32.63 N-terminally linked by a non-cleavable linker (Figure 5D) induced reduced killing on Hela cells at concentration of 100 pM and on Skov3 cells at a concentration of 10 nM. FolR1 TCB with scFv 4.15.64 N-terminally linked by a non-cleavable linker (Figure 5B) was less efficient in reducing killing on Skov3 cells at a concentration of 10 nM. The stronger mask, meaning the anti-idiotypic scFv 4.32.63 with the higher affinity, is more efficient in masking the CD3 binder than the weak anti-idiotypic scFv 4.15.64. Comparable potency of treated and untreated TCBs suggests protease, e.g. Matriptase, expression by Hela and Skov3 cells.

## Example 13

**Preparation of the HER1 binding antibody GA201 masked with an anti-idiotype GA201 scFv.**

The following molecules were prepared in this example:

1: GA201 IgG1 antibody with N-terminal fusion of an anti-idiotypic GA201 scFv and Matrix Metalloprotease site in glycine serine linker (SEQ ID NOs 32 and 34); and
2: HER1-binding IgG1 antibody GA201 (SEQ ID NOs 32 and 33).

Schematic illustrations thereof are shown in FIGs. 28 and 29. The GA201 anti-idiotypic (ID) binder sequence was obtained by RT-PCR (reverse transcription) from RNA of Hybridoma cells using degenerated primers binding to the ends of the variable light and heavy chain, respectively.. Hybridoma cells were obtained by immunization of mice. Single chain Fv (scFv) DNA sequence synthesis with flanking singular restriction endonuclease sites was ordered at Geneart and cloned as N-terminal fusion at the GA201 light chain.

A Roche expression vector was used for the construction of all heavy and light chain scFv fusion protein encoding expression plasmids. The vector is composed of the following elements:

- a hygromycin resistance gene as a selection marker,
- an origin of replication, oriP, of Epstein-Barr virus (EBV),
- an origin of replication from the vector pUC18 which allows replication of this plasmid in E. coli
- a beta-lactamase gene which confers ampicillin resistance in E. coli,
- the immediate early enhancer and promoter from the human cytomegalovirus (HCMV),
- the human 1-immunoglobulin polyadenylation ("poly A") signal sequence, and
- unique BamHI and XbaI restriction sites.

The molecules were produced by co-transfecting human embryonic kidney 293-F cells growing in suspension with the mammalian expression vectors using the FreeStyle™ 293 Expression System according to the manufacturer's instruction (Invitrogen, USA). Briefly, suspension FreeStyle™ 293-F cells were cultivated in FreeStyle™ 293 Expression medium at 37°C/8 % $CO_2$ and the cells were seeded in fresh medium at a density of 1-2 $\times$ $10^6$ viable cells/ml on the day of transfection. DNA-293fectin™ complexes were prepared in Opti-MEM I medium (Invitrogen, USA) using 325 μl of 293fectin™ (Invitrogen, Germany) and 250 μg of heavy ("GA201 heavy chain") and light chain ("anti-GA201 VH-VL scFv MMP cleavable linker G4S GA201 light chain" or "GA201 light chain") plasmid DNA in a 1:1 molar ratio for a 250 ml final transfection volume. Antibody containing cell culture supernatants were harvested 7 days after transfection by centrifugation at 14000 g for 30 minutes and filtered through a sterile filter (0.22 μm). Supernatants were stored at -20° C until purification.

The secreted protein was purified from cell culture supernatants by affinity chromatography using ProteinA affinity chromatography, followed by size exclusion chromatography. Briefly, sterile filtered cell culture supernatants were applied to a HiTrap ProteinA HP (5 ml) column equilibrated with PBS buffer (10 mM Na2HPO4, 1 mM KH2PO4, 137 mM NaCl and 2.7 mM KCl, pH 7.4). Unbound proteins were washed out with equilibration buffer. Antibody and antibody variants were eluted with 0.1 M citrate buffer, pH 2.8, and the protein containing fractions were neutralized with 0.1 ml 1 M Tris, pH 8.5. Then, the eluted protein fractions were pooled, concentrated with an Amicon Ultra centrifugal filter device (MWCO: 30 K, Millipore) to a volume of 3 ml and loaded on a Superdex200 HiLoad 120 ml 16/60 gel filtration column (GE Healthcare, Sweden) equilibrated with 20mM Histidin, 140 mM NaCl, pH 6.0. Fractions containing purified GA201-anti-GA201-scFv or GA201 with less than 5 % high molecular weight aggregates were pooled and stored as 1.0 mg/ml aliquots at -80°C.

For Protein analytics after size exclusion chromatography, the purity and molecular weight of the molecules in the single fractions were analyzed by SDS-PAGE in the absence of a reducing agent and staining with Coomassie (InstantBlue™, Expedeon). The NuPAGE® Pre-Cast gel system (4-12% Bis-Tris, Invitrogen or 3-8%Tris-Acetate, Invitrogen) was used according to the manufacturer's instruction.

The protein concentration of purified protein samples was determined by measuring the optical density (OD) at 280 nm divided by the molar extinction coefficient calculated on the basis of the amino acid sequence. Purity and molecular weight of the molecules after the final purification step were analyzed by CE-SDS analyses in the presence and absence of a reducing agent. The Caliper LabChip GXII system (Caliper Lifescience) was used according to the manufacturer's instruction.

The aggregate content of the molecules was analyzed by high-performance SEC using a Superdex 200 analytical size-exclusion column (GE Healthcare, Sweden) in 200 mM KH2PO4, 250 mM KCl, pH 7.0 running buffer at 25°C. 25 μg protein were injected on the column at a flow rate of 0.5 ml/min and eluted isocratic over 50 minutes.

The final purity of all molecules was ≥ 95 % monomer content as detected by high performance SEC. The molecular weight of the anti-idiotypic scFv masked GA201 was determined by CE-SDS analysis as 216.3 kDa under non reducing conditions (Figure 1A) and under reducing conditions as 58.3 kDa for the GA201 heavy chain and 60.3 kDa for the scFv linked GA201 light chain (Figure 30B), respectively. The molecular weight based on the amino acid sequence was calculated as 49.2 kDa for the heavy chain and 51.9 kDa for the scFv fused GA201 light chain, which indicates glycosylation of both chains in HEK293 cells.

TABLE 3. Summary of production and purification of protease-activated GA201 IgG (Figure 28) and GA201 (Figure 29) control molecules.

| Molecule | Supernatant | Protein A - Yield | SEC - Yield |
|---|---|---|---|
| 1 | 1.0 L | 1.3 mg | 0.4 mg |
| 2 | 1.0 L | 26.4 mg | 24 mg |

**Example 14**

**Masking effect of an anti-idiotypic scFv for GA201 IgG.**

The efficiency of masking the HER1 binding of GA201 by N-terminal fusion of an anti-idiotypic GA201 scFv was shown by FACS analysis on HER1 expressing H322M cells and Surface Plasmon Resonance (SPR) analysis on a HER1 coated chip surface. For proteolytic cleavage of GA201-anti-GA201-scFv recombinant active human MMP2 (Calbiochem) was used. 1 mg of GA201 anti-idiotypic scFv fused to GA201 by a glycine serine linker containing a MMP cleavage site was incubated with 1.2 μg MMP2 overnight at 37°C in PBS.

For FACS analysis of HER1 binding of cleaved and uncleaved GA201-anti-GA201-scFv, the non-small cell lung cancer line H322M was used. Cells were adjusted to $1\times10^6$/ml and distributed to a 96-well round-bottom plate. The molecules were added and incubated on ice for 30 minutes. Cells were washed once with FACS buffer (PBS + 2% FCS + 0.1% sodium azide) and re-suspended with a F(ab')2-goat anti-human IgG Fc secondary antibody FITC conjugate (ThermoFisher Scientific). After another 20 minutes on ice, cells were washed twice and re-suspended in FACS buffer and analyzed in a BD FACS Canto II. 10000 cells were measured and the median of the fluorescence signal was used for analysis. Before MMP-2 cleavage of GA201-anti-GA201-scFv no binding to HER1 on H322M cells was measurable, indicating complete masking of the GA201 binding domains by the anti-idiotypic scFv (Fig. 31). Binding of uncleaved GA201-anti-GA201-scFv was comparable to an unspecific isotype IgG control antibody (Fig. 31). In contrast, MMP cleavage of the anti-idiotypic scFv leads to activation of GA201 and binding to HER1 on H322M cells was restored to similar levels as the unmasked parental antibody GA201 (Fig. 31)

To confirm the FACS binding data of masked GA201 binding after MMP cleavage, we also performed a SPR experiment as second analytical method using a Biacore T100 instrument (GE Healthcare Biosciences AB, Uppsala, Sweden). HER1 was immobilized on the surface of a CM5 biosensorchip using standard amine-coupling chemistry. The HER1 extracellular domain was injected in sodium acetate, pH 5.0 at 1 μg/ml. Reference control flow cells were treated in the same way but with vehicle buffer only. GA201-anti-GA201-scFv, before and after an overnight MMP cleavage, and GA201 were diluted in 1xPBS pH 7.4, 0.05 % Tween20 Roche Diagnostics GmbH) and injected at increasing concentrations between 3.125 and 50 nM with a flow rate of 30 μl/min. The association phase was 3 minutes and the dissociation time was 10 minutes. HER1 binding was regenerated with an inject of 0.85 % phosphoric acid for 30 s at a flow rate of 5 μl/min. Kinetic rate constants and equilibrium dissociation constants were calculated by using the 1:1 Langmuir binding model within the Biaevaluation software. A $K_D$ value of 1 nM for binding of HER1 was determined for the GA201 parental unmasked antibody (Fig. 32). After an overnight MMP-2 incubation of GA201-anti-GA201-scFv, a $K_D$ value of 2 nM was measured with similar $k_a$ and $k_d$ rate constants for association and dissociation as the unmasked control antibody, indicating complete restoration of HER1 binding by protease cleavage (Fig. 32). Uncleaved GA201-anti-GA201-scFv did not show any binding to HER1 in SPR analysis (Fig. 32). In summary, we have demonstrated a complete loss of binding to HER1 by fusion of an anti-idiotypic scFv to the N-terminus of the IgG1 antibody GA201 with two independent analytical methods. Furthermore, binding to HER1 was fully restored by removal of the scFv through protease cleavage in the MMP cleavage site in the glycine serine linker.

**Example 15**

**Preparation of anti FolR1/anti-CD3 and antiMesothelin/anti-CD3 T cell bispecific (TCB) molecules with anti CD3 scFv**

Several T cell bispecific (TCB) molecules with various anti-idiotypic scFv were produced and are schematically depicted in FIGs. 33A-J with their respective ID number. The following molecules were prepared:

ID 8364: "FolR1 16D5 2+1 IgG, classic format (anti idiotypic scFv 4.32.63 - MMP9 - MK062 Matriptase site - CD3 - N-terminal fused to FolR1 VH - inert Fc) with N-terminal fused anti CD3 scFv 4.32.63 and MMP9 - MK062 protease linker " (FIG. 33A, SEQ ID NOs 1, 3 and 72).
ID 8363: "FolR1 16D5 2+1 IgG, classic format (anti idiotypic scFv 4.32.63 - Cathepsin S/B site - CD3 - N-terminal fused to FolR1 VH - inert Fc) with N-terminal fused anti CD3 scFv 4.32.63 and Cathepsin S/B protease linker" (FIG. 33B, SEQ ID NOs 1, 3 and 85).
ID 8365: "FolR1 16D5 2+1 IgG, inverted format, (anti idiotypic scFv 4.32.63 - MK062 Matriptase linker - CD3 - N-terminal fused to CD3 VL - inert Fc) with N-terminal fused anti CD3 scFv 4.32.63 and MK062 Matriptase linker" (FIG. 33C, SEQ ID NOs 1, 3, 73 and 74). ID 8366: "FolR1 16D5 2+1 IgG, inverted format, (anti idiotypic scFv 4.32.63 - non-cleavable GS linker - CD3 - N-terminal fused to CD3 VL - inert Fc) with N-terminal fused anti CD3 scFv 4.32.63 and non-cleavable GS linker" (FIG. 33D).
ID 8672: "aMesothelin 2+1 IgG, classic format, MSLN charged variants, CD3 crossed (anti idiotypic scFv 4.32.63 - MMP9 - MK062 Matriptase - CD3 - N-terminal fused to aMesothelin VH - inert Fc) with N-terminal fused anti CD3 scFv 4.32.63 and MMP9 - MK062 Matriptase" (FIG. 33E, SEQ ID NOs 77, 78, 81, 82).
ID 8673: "aMesothelin 2+1 IgG, classic format, MSLN charged variants, CD3 crossed (anti idiotypic scFv 4.32.63 -non-cleavable GS linker - CD3 - N-terminal fused to aMesothelin VH - inert Fc) with N-terminal fused anti CD3 scFv 4.32.63 non-cleavable GS linker" (FIG. 33F).
ID 8674: "aMesothelin 2+1 IgG, inverted format, MSLN charged variants, CD3 crossed (anti idiotypic scFv 4.32.63 - MMP9 - MK062 Matriptase - CD3 - N-terminal fused to CD3 VH - inert Fc) with N-terminal fused anti CD3 scFv 4.32.63 and MMP9 - MK062 Matriptase" (FIG. 33G, SEQ ID NOs 76, 77, 78, 79).
ID 8675: "aMesothelin 2+1 IgG, inverted format, MSLN charged variants, CD3 crossed (anti idiotypic scFv 4.32.63 - non-cleavable GS linker - CD3 - N-terminal fused to CD3 VH - inert Fc) with N-terminal fused anti CD3 scFv 4.32.63 and non-cleavable GS linker" (FIG. 33H).
ID 8505: "aMesothelin 2+1 IgG, inverted format, MSLN charged variants, CD3 (aMesothelin HC N-terminally fused to CD3 VL - inert Fc)" (FIG. 33I).
ID 8676: "aMesothelin 2+1 IgG, classic format, MSLN charged variants, CD3 crossed (aMesothelin IgG with CD3 - N-terminal fused to aMesothelin VH - inert Fc)" (FIG. 33J)

The variable domains were subcloned in frame with the pre-inserted domains into the respective recipient mammalian expression vector. Protein expression is driven by an MPSV promoter and a synthetic polyA signal sequence is present at the 3' end of the CDS. In addition each vector contains an EBV OriP sequence.

The molecules (except 8505, this molecule was produced by co-transfecting CHO cells growing in suspension with the mammalian expression vectors. Transient transfection was done at Evitria AG (Switzerland).) were produced by co-transfecting HEK293-EBNA cells growing in suspension with the mammalian expression vectors using polyethylenimine (PEI). For transfection HEK293 EBNA cells were cultivated in serum free ExCell culture medium containing 6 mM L-glutamine and 250 mg / 1 G418. For the production in 600 ml tubespin flasks (max. working volume 400 ml) 800 million HEK293 EBNA cells were seeded 24 hours before transfection without G418. For transfection 800 mio cells were centrifuged for 5 min at 210 $\times$ g and supernatant was replaced by 40 ml pre-warmed CD CHO medium containing 6mM L-Glutamine. Expression vectors were mixed with 40 ml CD CHO medium containing 6mM L-Glutamine to a total amount of 400 $\mu$g DNA. After addition of 1080 $\mu$l PEI solution (2.7 $\mu$g/ml) the mixture was vortexed for 15 s and subsequently incubated for 10 min at room temperature. Afterwards cells were mixed with the DNA/PEI solution, transferred to a 600 ml tubespin flask and incubated for 3 hours at 37°C in an incubator with a 5% $CO_2$ atmosphere. After incubation, 320 ml ExCell + 6mM L-glutamine + 5g/L Pepsoy + 1.0mM VPA + 3 g/l glucose medium was added and cells were cultivated for 24 hours prior to feeding with 7% Feed 7. After 6-7 days the cultivation supernatant was collected for purification by centrifugation for 20 - 30 min at 210 $\times$ g (Sigma 8K centrifuge). The solution was sterile filtered (0.22 $\mu$m filter) and sodium azide in a final concentration of 0.01% w/v was added. The solution was kept at 4°C until purification.

The secreted protein was purified from cell culture supernatants by affinity chromatography using ProteinA affinity chromatography, followed by one to two size exclusion chromatographic steps.

For affinity chromatography supernatant was loaded on a Protein A MabSelectSure (CV = 5 mL, GE Healthcare) equilibrated with 20 mM Sodium Citrate, 20 mM Sodium Phosphate, pH 7.5. Unbound protein was removed by washing

with at least 10 column volumes 20 mM Sodium Citrate, 20 mM Sodium Phosphate, pH 7.5 and target protein was eluted in 20 column volumes (gradient from 0 % - 100 %) 20 mM Sodium Citrate, 100mM Sodium Chloride, 100 mM Glycine, pH 3.0. Protein solution was neutralized by adding 1/10 of 0.5 M Na2HPO4 pH 8.0. Target protein was concentrated with Amicon®Ultra-15 Ultracel 30K (Merck Millipore Ltd.) to a volume of 4 ml maximum prior loading on a HiLoad Superdex 200 column (GE Healthcare) equilibrated with 20 mM Histidine, 140 mM NaCl, 0.01% Tween pH 6.0.

The protein concentration of purified protein samples was determined by measuring the optical density (OD) at 280 nm divided by the molar extinction coefficient calculated on the basis of the amino acid sequence.

Purity and molecular weight of the molecules after the final purification step were analyzed by CE-SDS analyses in the presence and absence of a reducing agent. The Caliper LabChip GXII system (Caliper Lifescience) was used according to the manufacturer's instruction.

The aggregate content of the molecules was analyzed using a TSKgel G3000 SW XL analytical size-exclusion column (Tosoh) in 25 mM K2HPO4, 125 mM NaCl, 200 mM L-arginine monohydrocloride, 0.02% (w/v) NaN3, pH 6.7 running buffer at 25°C.

The final quality of all molecules was good, with ≥ 95 % monomer content.

TABLE 4. Summary of production and purification of protease activated TCB molecules.

| Molecule | Titer [mg/l] | Yield [mg/l] | Analytical SEC (HMW/Monomer/LMW) [%] |
|---|---|---|---|
| 1 (8364) | 34.55 | 1.72 | 0.68/99.32/0 |
| 2 (8363) | 33.75 | 1.59 | 4.02/95.98/0 |
| 3 (8365) | 5.35 | 0.24 | 2.71/96.46/0.83 |
| 4 (8366) | 4.2 | 0.43 | 4.908/96.02/0 |
| 5 (8672) | 13.8 | 1.59 | 3.96/96.04/0 |
| 6 (8673) | 14 | 1.99 | 2.15/97.85/0 |
| 7 (8674) | 3.6 | 0.96 | 6.27/93.73/0 |
| 8 (8675) | 5.2 | 0.59 | 5.81/90.63/3.57 |
| 9 (8505) | 120 | 20.46 | 0.47/99.32/0.22 |
| 10 (8676) | 22.5 | 3.84 | 1.98/96.21/1.81 |

**Example 16**

**Quality control and stability - Capillary Electrophoresis SDS analysis of different TCB molecules.**

Purity and molecular weight of the molecules after the final purification step were analyzed by CE-SDS analyses in the presence and absence of a reducing agent. The Caliper LabChip GXII system (Caliper Lifescience) was used according to the manufacturer's instruction. Comparison of untreated molecules (stored at 4 °C), treated molecules (treated with appropriate recombinant protease (R&D Systems) for 24 h at 37 °C and molecule incubated for 72 h at 37 °C (FIGs. 34, 35A and 35B).

Comparison of the untreated and treated molecule shows complete cleavage of the anti ID scFv after rh-Matriptase/ST14 treatment for the inverted format containing MK062 Matriptase linker but incomplete cleavage of MMP9-MK062 Matriptase linker. rhCathepsin B and rhCathepsin S treatment is incomplete as well. The conditions for the purified enzymes have not been optimal. Molecules incubated at 37 °C for 72 h are running on the same height than pure molecules suggesting that the molecules are stable at 37 °C for the time of *in vitro* assay duration. Pre-stained protein Marker Mark 12 (Invitrogen) was used for estimation of correct molecule weight.

**Example 17**

**Comparison of different linkers and formats of Protease activated FolR1 TCBs**

Jurkat NFAT activation assay. Jurkat NFAT activation assay for comparison of different formats and linkers of protease activated TCB. Jurkat-NFAT reporter cell line (Promega) is a human acute lymphatic leukemia reporter cell line with a NFAT promoter, expressing human CD3ε. If the TCB binds the tumor target and the CD3 binder (crosslinkage) binds the CD3ε Luciferase expression can be measured in Luminescence after addition of One-Glo substrate (Promega).

20.000 target cells were seeded in 96-well white walled clear bottom plate (Greiner BioOne) in 50 ul / well Jurkat medium (RPMI1640, 2g/l Glucose, 2 g/l NaHCO3, 10 % FCS, 25 mM HEPES, 2 mM L-Glutamin, 1 × NEAA, 1 × Sodium-pyruvate) without Hygromycine. Plates were incubated for about 20 hours at 37 °C. Jurkat-NFAT reporter cells were harvested and viability was assessed using ViCell. Cells were resuspended in Jurkat medium without Hygromycine and 50 μl per well (50.000 cells / well) were added. The E:T ratio was 2.5:1 (based on cell number seeded). Antibodies were diluted in Jurkat medium without Hygromycine and 50 ul / well were added. Cells were incubated at 37 °C for 6 h in a humidified incubator before they were taken out of the incubator for about 10 min to adapt to room temperature prior to Luminescence read out. 50 μl/well of ONE-Glo solution were added to wells and incubated for 10 min at room temperature in the dark. Luminescence was detected using WALLAC Victor3 ELISA reader (PerkinElmer2030), 1 sec/well as detection time. Comparison of the pretreated protease activated TCB (8364, grey filled squares) and FolR1 TCB (black triangles pointing down) showed that potency after cleavage is recovered completely. No Luminescence was detectable for cells incubated with the masked TCB (containing a GS non-cleavable linker, grey triangles pointing up) and the non-targeted TCB control (empty triangle pointing down) for both cell lines in this concentration range. The dotted line shows the Luminescence of target cells and effector cells without any TCB (FIGs. 36A and 36B).

**Example 18**

**Tumor cell cytotoxicity mediated by different formats of protease activated TCB**

T-cell killing mediated by protease activated TCB molecules was assessed on cell lines expressing different levels of FolR1. Human Peripheral blood mononuclear cells (PBMCs) were isolated from buffy coats obtained from healthy human donors. Buffy coat was diluted 1: 1 with sterile PBS and layered over Histopaque gradient (Sigma, #H8889). After centrifugation (450 × g, 30 minutes, w/o break, room temperature) the PBMC-containing interphase was transferred in a new falcon tube that was subsequently filled with 50 ml of PBS. The mixture was centrifuged (400 × g, 10 minutes, room temperature), the supernatant was discarded and the PBMC pellet was resuspended in 2 ml ACK buffer for Erythrocytes lysis. After incubation for about 2 - 3 minutes at 37 °C the tubes were filled with sterile PBS to 50 ml and centrifuged for 10 minutes at 350 × g. This washing step was repeated once prior to resuspension of PBMCs in RPMI1640 medium containing 10% FCS, 1X GlutaMax and 10% DMSO. PBMCs were slowly frozen in CoolCell® Cell Freezing Containers (BioCision) at - 80 °C and then transferred to liquid nitrogen. One day before assay start adherent target cells were harvested with Trypsin/EDTA, counted, checked for viability and resuspended in assay medium (RPMI1640, 2% FCS, 1X GlutaMax). Target cells were plated at a density of 20 000 cells/well using 96-well flat-bottom plates and incubated for about 20 h at 37 °C in a humidified incubator. About 20 h before assay start PBMCs were thawed in RPMI1640 medium (10 % FCS, 1X GlutaMax). PBMCs were centrifuged at 350 g for 7 min. The pellet was resuspended in fresh medium (RPMI1640, 10% FCS, 1X GlutaMax) and incubated for max 24 h at 37 °C in a humidified incubator. On the day of the assay start PBMCs were harvested and centrifuged at 350 g for 7 min. The pellet was resuspended in assay medium and 0.2 mio PBMCs in 100 ul / well (E:T 10:1, based on the number of seeded target cells) were added to the target cells. The molecules were diluted in assay medium (RPMI1640, 2% FCS, 1X GlutaMax) and 50 ul / well were added at the indicated concentrations in triplicates before the plates were incubated for about 48 h at 37 °C in a humidified incubator. Target cell killing was assessed after 48 h of incubation at 37 °C, 5% CO2 by quantification of LDH release into cell supernatants by apoptotic/necrotic cells (LDH detection kit, Roche Applied Science, #11 644 793 001). Maximal lysis of the target cells (= 100%) was achieved by incubation of target cells with 1% Triton X-100 20 h before LDH readout. Minimal lysis (= 0%) refers to target cells co-incubated with effector cells without any TCB.

The results (FIGs.37A and 37B) show the comparison of two different formats of the Protease activated TCBs both containing the anti idiotypic CD3 scFv 4.32.63 linked with a MK062 Matriptase linker. The inverted format of the protease activated TCB (8365, grey circles) seems to be more potent in killing (HeLa and Skov-3 target cells) than the classic format of the protease activated TCB (8408, dark grey triangles pointing up). However the inverted molecule containing the non-cleavable linker (8366, light grey squares) is less efficient in masking than the classic molecule (8409, dark grey triangles pointing down).

FIG. 37C HeLa target cell cytotoxicity. Comparison of classic Protease activated TCB containing the anti idiotypic CD3 scFv 4.32.63 and GS linkers with different protease sites. Protease activated TCB containing the MMP9-Matriptase MK062 linker (8364, grey squares) reaches the potency of FolR1 TCB (light grey triangles pointing down) whereas the protease activated TCB containing only Matriptase MK062 (light grey rhomb) is less potent in killing HeLa cells. Molecules containing Cathepsin site (grey circles) or non-cleavable linker (black triangles pointing down) are comparable.

FIG. 37D Skov-3 target cell cytotoxicity. Comparison of classic Protease activated TCB containing the anti idiotypic CD3 scFv 4.32.63 and GS linkers with different protease sites. Protease activated TCB containing the MMP9-Matriptase MK062 linker (8364, grey squares) nearly reaches the potency of FolR1 TCB (light grey triangles pointing down) whereas the protease activated TCB containing only Matriptase MK062 (light grey rhomb) is less potent in killing Skov-3 cells. The molecule containing Cathepsin site (grey circles) is less potent than the molecule containing only the

Matriptase MK062 site and the molecule containing the non-cleavable linker (black triangles pointing down) only induces killing below 10% in the indicated concentration range for Skov-3 cells.

**Example 19**

**T-cell activation after co-incubation of human renal epithelial cortical cells or human bronchial epithelial cells with TCBs and human PBMCs**

T-cell activation mediated by protease activated TCB molecules was assessed for HRCEpi (Human renal cortical epithelial cells) and HBEpiC (human bronchial epithelial cells expressing only little amounts of FolR1.Human PBMCs were used as effector cells and T cell activation markers were stained after 48 h of incubation with the molecules and cells. Human Peripheral blood mononuclear cells (PBMCs) were isolated from buffy coats obtained from healthy human donors. Buffy coat was diluted 1:1 with sterile PBS and layered over Histopaque gradient (Sigma, #H8889). After centrifugation (450 × g, 30 minutes, w/o break, room temperature) the PBMC-containing interphase was transferred in a new falcon tube subsequently filled with 50 ml of PBS. The mixture was centrifuged (400 × g, 10 minutes, room temperature), the supernatant was discarded and the PBMC pellet was resuspended in 2 ml ACK buffer for Erythrocytes lysis. After incubation for about two minutes at 37 °C the tubes were filled with sterile PBS to 50 ml and centrifuged for 10 minutes at 350 × g. This washing step was repeated once prior to resuspension of PBMCs in RPMI1640 medium containing 10 % FCS, 1 × GlutaMax and 10 % DMSO. PBMCs were slowly frozen in CoolCell® Cell Freezing Containers (BioCision) at - 80 °C and then transferred to liquid nitrogen. One day before the assay was started adherent target cells were harvested with Trypsin/EDTA, counted, checked for viability and resuspended in assay medium (RPMI1640, 2 % FCS, 1 × GlutaMax). Target cells were plated at a density of 20 000 cells/well using 96-well flat-bottom plates and incubated for about 20 h at 37 °C in a humidified incubator. About 20 h before assay start PBMCs were thawed in RPMI1640 medium (10 % FCS, 1 × GlutaMax). PBMCs were centrifuged for 7 min at 350 g. The pellet was resuspended in fresh medium (RPMI1640, 10 % FCS, 1 × GlutaMax) and incubated for max 24 h at 37 °C in a humidified incubator. On the day of the assay start PBMCs were harvested and centrifuged for 7 min at 350 g. The pellet was resuspended in assay medium and 0.2 mio PBMCs in 100 ul / well (E:T 10:1, based on the number of seeded target cells) were added to the target cells. The molecules were diluted in assay medium (RPMI1640, 2 % FCS, 1 × GlutaMax) and added at the indicated concentrations in triplicates before the plates were incubated for about 48 h at 37 °C in a humidified incubator.

T- cell activation was assessed after 48 h of incubation at 37 °C, 5 % $CO_2$ by quantification of CD25 and CD69 on CD4 positive and CD8 positive T cells. FolR1 16D5 TCB (6298) and an untargeted TCB (binding to CD3 but not to a target cell antigen, 7235) were included as controls. Each point represents the mean value of triplicates of three different human PBMC donors. Standard deviation is indicated in error bars. Unpaired t test was used for statistical analysis. The results show an increase in CD69 for CD8 positive cells for the FolR1 TCB that is significantly higher than the median fluorescence intensity for the protease activated TCBs (FIGs 38A and 38B).

**Example 20**

**Tumor cell cytotoxicity mediated by different formats of protease activated Mesothelin (MSLN) TCB**

T-cell killing mediated by protease activated TCB molecules was assessed on cell lines expressing different levels of Mesothelin (MSLN). Human Peripheral blood mononuclear cells (PBMCs) were isolated from buffy coats obtained from healthy human donors. Buffy coat was diluted 1:1 with sterile PBS and layered over Histopaque gradient (Sigma, #H8889). After centrifugation (450 × g, 30 minutes, w/o break, room temperature) the PBMC-containing interphase was transferred in a new falcon tube subsequently filled with 50 ml of PBS. The mixture was centrifuged (400 × g, 10 minutes, room temperature), the supernatant was discarded and the PBMC pellet was resuspended in 2 ml ACK buffer for Erythrocytes lysis. After incubation for about two minutes at 37 °C the tubes were filled with sterile PBS to 50 ml and centrifuged for 10 minutes at 350 × g. This washing step was repeated once prior to resuspension of PBMCs in RPMI1640 medium containing 10 % FCS, 1 X GlutaMax and 10 % DMSO. PBMCs were slowly frozen in CoolCell® Cell Freezing Containers (BioCision) at - 80 °C and then transferred to liquid nitrogen. Adherent target cells were harvested with Trypsin/EDTA, counted, checked for viability and resuspended in assay medium (RPMI1640, 2 % FCS, 1 X GlutaMax) one day before the assay was started. Target cells were plated at a density of 20 000 cells/well using 96-well flat-bottom plates and incubated for about 20 h at 37 °C in a humidified incubator. PBMCs were thawed in RPMI1640 medium (10 % FCS, 1 X GlutaMax) about 20 h before assay start. PBMCs were centrifuged for 7 min at 350 g. The pellet was resuspended in fresh medium (RPMI1640, 10% FCS, 1 X GlutaMax) and incubated for max 24 h at 37 °C in a humidified incubator. On the day of the assay start PBMCs were harvested and centrifuged for 7 min at 350 g. The pellet was resuspended in assay medium and 0.2 mio PBMCs in 100 ul / well (E:T 10:1, based on the number of seeded target cells) were added to the target cells. The molecules were diluted in assay medium (RPMI1640, 2 %

FCS, 1 X GlutaMax) and added at the indicated concentrations in triplicates before the plates were incubated for about 48 h at 37 °C in a humidified incubator. Target cell killing was assessed after 48 h of incubation at 37 °C, 5% CO2 by quantification of LDH release into cell supernatants by apoptotic/necrotic cells (LDH detection kit, Roche Applied Science, #11 644 793 001). Maximal lysis of the target cells (= 100%) was achieved by incubation of target cells with 1% Triton X-100 20 h before LDH readout. Minimal lysis (= 0%) refers to target cells co-incubated with effector cells without any TCB.

The results (FIGs. 39A and 39B) show target cell killing mediated by Protease activated MSLN TCB (8672) for NCI H596 and AsPC-1 cell lines. The protease activated TCBs nearly reaches the potency of MSLN TCB (8676) for NCI H596 and AsPC-1. The molecule containing the non-cleavable GS linker (8673) does not induce killing in the indicated concentration range for both cell lines.

**Example 21**

**Jurkat-NFAT reporter assay to monitor target expression (FOLR1 TCB) and protease activity (Protease activated FOLR1 TCB) in primary tumor samples**

The intention of this assay was to show tumor target antigen (FolR1) expression and activity of tumor specific proteases like MMP9, Matriptase or Cathepsin in human tumor samples.

Jurkat-NFAT reporter cell line (Promega) is a human acute lymphatic leukemia reporter cell line with a NFAT promoter, expressing human CD3ε. Luciferase expression can be measured, if the T cell bispecific molecule binds the tumor target and the CD3ε (crosslinkage). Luminescence is measured after addition of One-Glo substrate (Promega).

Primary tumor samples were received from Indivumed GmbH, Germany. Samples were shipped over night in transport medium. About 24 h after surgery the sample was cut in small pieces. 96-well white walled, flat (clear) bottom plate was prepared by adding 18ul cold Matrigel (Matrigel (734-1101, Corning/VWR). Plate was incubated for 2 min at 37 °C before tumor pieces were added (triplicates). 33 ul of cold Matrigel were added per well and plate was incubated again for 2 min at 37 °C. 50 ul of antibody dilution (in Jurkat medium without Hygromycine but containing 2X Penicillin/Streptomycine) was added per well and plate was incubated for about 48 hours at 37 °C, 5 % $CO_2$.

Jurkat-NFAT reporter cells were harvested and viability was assessed using ViCell. Cells were centrifuged at 350 × g, 7 min before they were resuspended in Jurkat medium without Hygromycine and 50 μl per well (50.000 cells / well) were added. Plate was incubated for 5 h at 37 °C in a humidified incubator before it was taken out for Luminescence read out. 80 ul of each well were transferred into a white walled 96-well plate. 27 μl/well of ONE-Glo solution were added to each well and incubated for 10 min at room temperature in the dark. Luminescence was detected using WALLAC Victor3 ELISA reader (PerkinElmer2030), 1 sec/well as detection time. Jurkat NFAT reporter cells are activated after co-incubation with FolR1 TCB (6298) and Protease activated FolR1 TCB containing MMP9-Matriptase cleavage site (8364). Protease activated FolR1 TCBs (8363, 8408) and control TCBs (8409, 7235) do not induce Luciferase expression. The dotted line indicates the baseline Luminescence for Jurkat NFAT cells co-incubated with tumor (FIG. 40).

**Example 22**

**Serum stability of protease activated TCBs**

Capillary electrophoresis of protease activated TCBs after incubation in human serum.

Molecules were incubated for 0 or 14 days in human IgG depleted serum at 37 °C in a humidified incubator (5 % CO2). All molecules were purified by affinity chromatography (ProteinA) and then analyzed by Capillary electrophoresis.

100 ug of each molecule was added either in buffer (Histidine buffer (Bichsel) with 0.01% Tween-20) or in human serum (IgG depleted, SP1839, TL-15216, 16FSP63814). The concentration of the molecules was higher than 2 mg/ml and the final concentration was 0.5 mg/ml. The pretreatment for one molecule (8408) was done with rhMatriptase (R&D Systems) for 24 h at 37 °C, 5 % CO2 in a humidified incubator (otherwise pH of serum could change). The samples for day 0 were directly frozen in liquid nitrogen and stored at - 80 °C until analysis. Samples for day 14 were incubated for 14 days at 37 °C, 5 % CO2 in a humidified incubator until they were also snap frozen.

Prior to CE-SDS analysis all samples were purified via HPLC affinity chromatography (Agilent technologies 1200series, column: Upchurch scientific C-130B, packaging material: Applied Biosystems POROS 20A 60 μl, buffer: 10 mM Tris, 50 mM Glycine, 500 mM NaCl pH 8.0 und pH 2.0, injection volume: 100 μl, flow rate 1 ml / min, collection: peak based, neutralization: 0.5 M Na-phosphate pH 8.0 10 %volume). Protease activated TCB is stable in human IgG depleted serum for a minimum of 14 days (FIGs. 41A-C).

**Example 23**

**Design of anti Her2/anti-CD3 and antiFolR1/anti-CD3 T cell bispecific (TCB) molecules with anti CD3 scFv**

Several T cell bispecific (TCB) molecules designed and are schematically depicted in FIGs. 42A-F with their respective ID number. The following molecules were designed:

ID 8955: "Herceptarg 2+1 IgG, classic format, Herceptarg charged variants, CD3 crossed (anti idiotypic scFv 4.32.63 - MMP9 - MK062 Matriptase - CD3 - N-terminal fused to Herceptarg VH - inert Fc) with N-terminal fused anti CD3 scFv 4.32.63 and MMP9 - MK062 Matriptase " (FIG. 42A, SEQ ID NOs 81, 132, 133 and 136).
ID 8957: "Herceptarg 2+1 IgG, classic format, Herceptarg charged variants, CD3 crossed (anti idiotypic scFv 4.32.63 -non cleavable GS linker - CD3 - N-terminal fused to Herceptarg VH - inert Fc) with N-terminal fused anti CD3 scFv 4.32.63 and non cleavable GS linker " (FIG. 42B, SEQ ID NOs 81, 132, 133 and 135).
ID 8959: "Herceptarg 2+1 IgG, classic format, Herceptarg charged variants, CD3 crossed (Herceptarg IgG with CD3 - N-terminal fused to Herceptarg VH - inert Fc)" (FIG. 42C, SEQ ID NOs 81, 132, 133 and 134).
ID 8997: "FolR1 36F2 2+1 IgG, classic format, FolR1 36F2 charged variants, CD3 crossed (anti idiotypic scFv 4.32.63 - MMP9 - MK062 Matriptase - CD3 - N-terminal fused to FolR1 36F2 VH - inert Fc) with N-terminal fused anti CD3 scFv 4.32.63 and MMP9 - MK062 Matriptase " (FIGs. 42D, SEQ ID NOs 81, 137, 138 and 139).
ID 8998: "FolR1 36F2 2+1 IgG, classic format, FolR1 36F2 charged variants, CD3 crossed (anti idiotypic scFv 4.32.63 - non cleavable GS linker - CD3 - N-terminal fused to FolR1 36F2 VH - inert Fc) with N-terminal fused anti CD3 scFv 4.32.63 and non cleavable GS linker " (FIGs. 42E, SEQ ID NOs 81, 137, 138 and 140).
ID 8996: "FolR1 36F2 2+1 IgG, classic format, FolR1 36F2 charged variants, CD3 crossed (FolR1 36F2 IgG with CD3 - N-terminal fused to FolR1 36F2 VH - inert Fc)" (FIG. 42F, SEQ ID NOs 81, 137, 138 and 141).

The variable domains were subcloned in frame with the pre-inserted domains into the respective recipient mammalian expression vector. Protein expression is driven by an MPSV or CMV (for Herceptarg) promoter and a synthetic polyA signal sequence is present at the 3' end of the CDS. In addition each vector contains an EBV OriP sequence.

**Example 24**

**Primary cell cytotoxicity mediated by Protease activated FolR1 TCB**

T-cell killing mediated by protease activated FolR1 TCB molecule was assessed on primary cell lines expressing low levels of FolR1 (FIG. 43). Human Peripheral blood mononuclear cells (PBMCs) were isolated from buffy coats obtained from healthy human donors. Buffy coat was diluted 1:1 with sterile PBS and layered over Histopaque gradient (Sigma, #H8889). After centrifugation (450 × g, 30 minutes, w/o break, room temperature) the PBMC-containing interphase was transferred in a new falcon tube that was subsequently filled with 50 ml of PBS. The mixture was centrifuged (400 × g, 10 minutes, room temperature), the supernatant was discarded and the PBMC pellet was resuspended in 2 ml ACK buffer for Erythrocytes lysis. After incubation for about 2 - 3 minutes at 37 °C the tubes were filled with sterile PBS to 50 ml and centrifuged for 10 minutes at 350 × g. This washing step was repeated once prior to resuspension of PBMCs in RPMI1640 medium containing 10% FCS, 1X GlutaMax and 10% DMSO. PBMCs were slowly frozen in CoolCell® Cell Freezing Containers (BioCision) at - 80 °C and then transferred to liquid nitrogen. One day before assay start adherent target cells were harvested with Trypsin/EDTA, counted, checked for viability and resuspended in assay medium (RPMI1640, 2% FCS, 1X GlutaMax). Target cells were plated at a density of 20 000 cells/well using 96-well flat-bottom plates and incubated for about 20 h at 37 °C in a humidified incubator. About 20 h before assay start PBMCs were thawed in RPMI1640 medium (10 % FCS, 1X GlutaMax). PBMCs were centrifuged at 350 g for 7 min. The pellet was resuspended in fresh medium (RPMI1640, 10% FCS, 1X GlutaMax) and incubated for max 24 h at 37 °C in a humidified incubator. On the day of the assay start PBMCs were harvested and centrifuged at 350 g for 7 min. The pellet was resuspended in assay medium and 0.2 mio PBMCs in 100 ul / well (E:T 10:1, based on the number of seeded target cells) were added to the target cells. The molecules were diluted in assay medium (RPMI1640, 2 % FCS, 1 × GlutaMax) and 50 ul / well were added at the indicated concentrations in triplicates before the plates were incubated for about 48 h, 72 h or 96 h at 37 °C in a humidified incubator. Target cell killing was assessed after 48 h, 72 h and 96 h of incubation at 37 °C, 5% $CO_2$ by quantification of LDH release into cell supernatants by apoptotic/necrotic cells (LDH detection kit, Roche Applied Science, #11 644 793 001). Maximal lysis of the target cells (= 100%) was achieved by incubation of target cells with 1% Triton X-100 20 h before LDH readout. Minimal lysis (= 0%) refers to target cells co-incubated with effector cells without any TCB.

Human Bronchial Epithelial Cell toxicity mediated by human PBMCs and 100 nM or 10 nM of FolR1 TCB is

higher compared to Protease activated TCB.

**Example 25**

**FolR1 negative target cell cytotoxicity mediated by Protease activated FolR1 TCB**

T-cell killing mediated by protease activated FolR1 TCB molecule was assessed on FolR1 negative Mkn-45 cell line (FIG. 44). Human Peripheral blood mononuclear cells (PBMCs) were isolated from buffy coats obtained from healthy human donors. Buffy coat was diluted 1:1 with sterile PBS and layered over Histopaque gradient (Sigma, #H8889). After centrifugation (450 × g, 30 minutes, w/o break, room temperature) the PBMC-containing interphase was transferred in a new falcon tube that was subsequently filled with 50 ml of PBS. The mixture was centrifuged (400 × g, 10 minutes, room temperature), the supernatant was discarded and the PBMC pellet was resuspended in 2 ml ACK buffer for Erythrocytes lysis. After incubation for about 2 - 3 minutes at 37 °C the tubes were filled with sterile PBS to 50 ml and centrifuged for 10 minutes at 350 × g. This washing step was repeated once prior to resuspension of PBMCs in RPMI1640 medium containing 10% FCS, 1X GlutaMax and 10% DMSO. PBMCs were slowly frozen in CoolCell® Cell Freezing Containers (BioCision) at - 80 °C and then transferred to liquid nitrogen. One day before assay start adherent target cells were harvested with Trypsin/EDTA, counted, checked for viability and resuspended in assay medium (RPMI1640, 2% FCS, 1X GlutaMax). Target cells were plated at a density of 20 000 cells/well using 96-well flat-bottom plates and incubated for about 20 h at 37 °C in a humidified incubator. About 20 h before assay start PBMCs were thawed in RPMI1640 medium (10 % FCS, 1X GlutaMax). PBMCs were centrifuged at 350 g for 7 min. The pellet was resuspended in fresh medium (RPMI1640, 10% FCS, 1X GlutaMax) and incubated for max 24 h at 37 °C in a humidified incubator. On the day of the assay start PBMCs were harvested and centrifuged at 350 g for 7 min. The pellet was resuspended in assay medium and 0.2 mio PBMCs in 100 ul / well (E:T 10:1, based on the number of seeded target cells) were added to the target cells. The molecules were diluted in assay medium (RPMI1640, 2 % FCS, 1 X GlutaMax) and 50 ul / well were added at the indicated concentrations in triplicates before the plates were incubated for about 48 h and 72 h at 37 °C in a humidified incubator. Target cell killing was assessed after 48 h, 72 h and 96 h of incubation at 37 °C, 5% CO2 by quantification of LDH release into cell supernatants by apoptotic/necrotic cells (LDH detection kit, Roche Applied Science, #11 644 793 001). Maximal lysis of the target cells (= 100%) was achieved by incubation of target cells with 1% Triton X-100 20 h before LDH readout. Minimal lysis (= 0%) refers to target cells co-incubated with effector cells without any TCB. Protease activated TCB did not induce target cell killing at 100 nM.

**EXAMPLARY SEQUENCES**

| Construct | Amino acid Sequence | SEQ ID No |
|---|---|---|
| **LC Common light chain pETR13197** | QAVVTQEPSLTVSPGGTVTLTCGSSTGAVTTSNYANWVQEK PGQAFRGLIGGTNKRAPGTPARFSGSLLGGKAALTLSGAQP EDEAEYYCALWYSNLWVFGGGTKLTVLGQPKAAPSVTLFPP SSEELQANKATLVCLISDFYPGAVTVAWKADSSPVKAGVETT TPSKQSNNKYAASSYLSLTPEQWKSHRSYSCQVTHEGSTVE KTVAPTECS | 1 |

(continued)

| Construct | Amino acid Sequence | SEQ ID No |
|---|---|---|
| **anti CD3 (CH2527 VH_ 3-23(12) VL7-46(13)) scFv15-Matriptase MK062 CH2527 VH3_23-VH12 CH1 FolR1 16D5 VH CH1 hum Fc knob PG LALA, pETR15422**<br>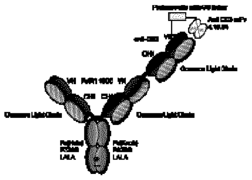 | QIQLVQSGPELKKPGETVRISCKASGYTFTDYSIHWVKQAPG<br>KCLKWMGWINTETGEPAYADDFKGRFAFSLETSASTAYLQI<br>NNLKNEDTATFFCAHPYDYDVLDYWGQGTSVTVSSGGGGS<br>GGGGSGGGGSGGGGSDTVLTQSPASLGVSLGQRATISCRA<br>SKSVSTSNYSYIHWYQQKPGQPPKLLIKYVSYLESGVPARFS<br>GSGSGTDFTLNIHPVEEEDAATYYCQHSREFPWTFGCGTKL<br>EIK**GGGGSGGGGSRQARVVNGGGGGSGGGGSGGGGS**EV<br>QLLESGGGLVQPGGSLRLSCAASGFTFSTYAMNWVRQAPG<br>KGLEWVSRIRSKYNNYATYYADSVKGRFTISRDDSKNTLYLQ<br>MNSLRAEDTAVYYCVRHGNFGNSYVSWFAYWGQGTLVTVS<br>SASTKGPSVFPLAPSSKSTSGGTAALGCLVKDYFPEPVTVS<br>WNSGALTSGVHTFPAVLQSSGLYSLSSVVTVPSSSLGTQTYI<br>CNVNHKPSNTKVDKKVEPKSCDGGGGSGGGGSEVQLVES<br>GGGLVKPGGSLRLSCAASGFTFSNAWMSWVRQAPGKGLE<br>WVGRIKSKTDGGTTDYAAPVKGRFTISRDDSKNTLYLQMNS<br>LKTEDTAVYYCTTPWEWSWYDYWGQGTLVTVSSASTKGPS<br>VFPLAPSSKSTSGGTAALGCLVKDYFPEPVTVSWNSGALTS<br>GVHTFPAVLQSSGLYSLSSVVTVPSSSLGTQTYICNVNHKPS<br>NTKVDKKVEPKSCDKTHTCPPCPAPEAAGGPSVFLFPPKPK<br>DTLMISRTPEVTCVVVDVSHEDPEVKFNWYVDGVEVHNAKT<br>KPREEQYNSTYRVVSVLTVLHQDWLNGKEYKCKVSNKALGA<br>PIEKTISKAKGQPREPQVYTLPPCRDELTKNQVSLWCLVKGF<br>YPSDIAVEWESNGQPENNYKTTPPVLDSDGSFFLYSKLTVD<br>KSRWQQGNVFSCSVMHEALHNHYTQKSLSLSPGK | 2 |
| **FolR1 16D5 VH CH1 Fc hole P329G LALA HRYF, pETR15214** | EVQLVESGGGLVKPGGSLRLSCAASGFTFSNAWMSWVRQA<br>PGKGLEWVGRIKSKTDGGTTDYAAPVKGRFTISRDDSKNTL<br>YLQMNSLKTEDTAVYYCTTPWEWSWYDYWGQGTLVTVSSA<br>STKGPSVFPLAPSSKSTSGGTAALGCLVKDYFPEPVTVSWN<br>SGALTSGVHTFPAVLQSSGLYSLSSVVTVPSSSLGTQTYICN<br>VNHKPSNTKVDKKVEPKSCDKTHTCPPCPAPEAAGGPSVFL<br>FPPKPKDTLMISRTPEVTCVVVDVSHEDPEVKFNWYVDGVE<br>VHNAKTKPREEQYNSTYRVVSVLTVLHQDWLNGKEYKCKVS<br>NKALGAPIEKTISKAKGQPREPQVCTLPPSRDELTKNQVSLS<br>CAVKGFYPSDIAVEWESNGQPENNYKTTPPVLDSDGSFFLV<br>SKLTVDKSRWQQGNVFSCSVMHEALHNRFTQKSLSLSPGK | 3 |

(continued)

| Construct | Amino acid Sequence | SEQ ID No |
|---|---|---|
| **anti CD3 (CH2527 VH_ 3-23(12) VL7-46(13)) scFv 4.32.63 Matriptase MK062 CH2527 VH3_23-VH12 CH1 FolR1 16D5 VH CH1 hum Fc knob PG LALA, pETR15599**<br>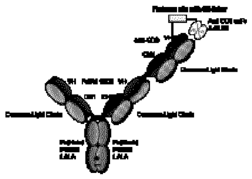 | QVQLKESGPGLVAPSQSLSITCTVSGFSLTSYGVSWVRQPP<br>GKCLEWLGIIWGDGSTNYHSALISRLSISKDNSKSQVFLKLNS<br>LQTDDTATYYCAKGITTVVDDYYAMDYWGQGTSVTVSSGG<br>GGSGGGGSGGGGSGGGGSDIQMTQSPASLSASVGETVTIT<br>CRASENIDSYLAWYQQKQGKSPQLLVYAATFLADDVPSRFS<br>GSGSGTQYSLKINSLQSEDVARYYCQHYYSTPYTFGCGTKL<br>EIK**GGGGSGGGGSRQARVVNGGGGGSGGGGSGGGGS**EV<br>QLLESGGGLVQPGGSLRLSCAASGFTFSTYAMNWVRQAPG<br>KGLEWVSRIRSKYNNYATYYADSVKGRFTISRDDSKNTLYLQ<br>MNSLRAEDTAVYYCVRHGNFGNSYVSWFAYWGQGTLVTVS<br>SASTKGPSVFPLAPSSKSTSGGTAALGCLVKDYFPEPVTVS<br>WNSGALTSGVHTFPAVLQSSGLYSLSSVVTVPSSSLGTQTYI<br>CNVNHKPSNTKVDKKVEPKSCDGGGGSGGGGSEVQLVES<br>GGGLVKPGGSLRLSCAASGFTFSNAWMSWVRQAPGKGLE<br>WVGRIKSKTDGGTTDYAAPVKGRFTISRDDSKNTLYLQMNS<br>LKTEDTAVYYCTTPWEWSWYDYWGQGTLVTVSSASTKGPS<br>VFPLAPSSKSTSGGTAALGCLVKDYFPEPVTVSWNSGALTS<br>GVHTFPAVLQSSGLYSLSSVVTVPSSSLGTQTYICNVNHKPS<br>NTKVDKKVEPKSCDKTHTCPPCPAPEAAGGPSVFLFPPKPK<br>DTLMISRTPEVTCVVVDVSHEDPEVKFNWYVDGVEVHNAKT<br>KPREEQYNSTYRVVSVLTVLHQDWLNGKEYKCKVSNKALGA<br>PIEKTISKAKGQPREPQVYTLPPCRDELTKNQVSLWCLVKGF<br>YPSDIAVEWESNGQPENNYKTTPPVLDSDGSFFLYSKLTVD<br>KSRWQQGNVFSCSVMHEALHNHYTQKSLSLSPGK | 4 |
| **anti CD3 (CH2527 VH_ 3-23(12) VL7-46(13)) scFv = 4.32.63 non-cleavable linker CH2527 VH3_23-VH12 CH1 FolR1 16D5 VH CH1 hum Fc knob PG LALA, pETR15603**<br>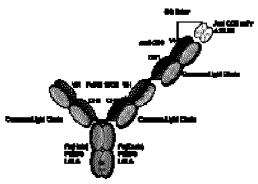 | QVQLKESGPGLVAPSQSLSITCTVSGFSLTSYGVSWVRQPP<br>GKCLEWLGIIWGDGSTNYHSALISRLSISKDNSKSQVFLKLNS<br>LQTDDTATYYCAKGITTVVDDYYAMDYWGQGTSVTVSSGG<br>GGSGGGGSGGGGSGGGGSDIQMTQSPASLSASVGETVTIT<br>CRASENIDSYLAWYQQKQGKSPQLLVYAATFLADDVPSRFS<br>GSGSGTQYSLKINSLQSEDVARYYCQHYYSTPYTFGCGTKL<br>EIKGGGGSGGGGSGGGGSGGGGGGGSGGGGSGGGGSEV<br>QLLESGGGLVQPGGSLRLSCAASGFTFSTYAMNWVRQAPG<br>KGLEWVSRIRSKYNNYATYYADSVKGRFTISRDDSKNTLYLQ<br>MNSLRAEDTAVYYCVRHGNFGNSYVSWFAYWGQGTLVTVS<br>SASTKGPSVFPLAPSSKSTSGGTAALGCLVKDYFPEPVTVS<br>WNSGALTSGVHTFPAVLQSSGLYSLSSVVTVPSSSLGTQTYI<br>CNVNHKPSNTKVDKKVEPKSCDGGGGSGGGGSEVQLVES<br>GGGLVKPGGSLRLSCAASGFTFSNAWMSWVRQAPGKGLE<br>WVGRIKSKTDGGTTDYAAPVKGRFTISRDDSKNTLYLQMNS<br>LKTEDTAVYYCTTPWEWSWYDYWGQGTLVTVSSASTKGPS<br>VFPLAPSSKSTSGGTAALGCLVKDYFPEPVTVSWNSGALTS<br>GVHTFPAVLQSSGLYSLSSVVTVPSSSLGTQTYICNVNHKPS<br>NTKVDKKVEPKSCDKTHTCPPCPAPEAAGGPSVFLFPPKPK<br>DTLMISRTPEVTCVVVDVSHEDPEVKFNWYVDGVEVHNAKT<br>KPREEQYNSTYRVVSVLTVLHQDWLNGKEYKCKVSNKALGA<br>PIEKTISKAKGQPREPQVYTLPPCRDELTKNQVSLWCLVKGF<br>YPSDIAVEWESNGQPENNYKTTPPVLDSDGSFFLYSKLTVD<br>KSRWQQGNVFSCSVMHEALHNHYTQKSLSLSPGK | 5 |

(continued)

| Construct | Amino acid Sequence | SEQ ID No |
|---|---|---|
| **anti CD3 (CH2527 VH_ 3-23(12) VL7-46(13)) scFv15 non-cleavable linker CH2527 VH3_23-VH12 CH1 FolR1 16D5 VH CH1 hum Fc knob PG LALA, pETR14759** 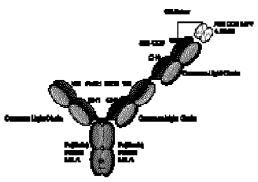 | QIQLVQSGPELKKPGETVRISCKASGYTFTDYSIHWVKQAPG<br>KCLKWMGWINTETGEPAYADDFKGRFAFSLETSASTAYLQI<br>NNLKNEDTATFFCAHPYDYDVLDYWGQGTSVTVSSGGGGS<br>GGGGSGGGGSGGGGSDTVLTQSPASLGVSLGQRATISCRA<br>SKSVSTSNYSYIHWYQ<br>QKPGQPPKLLIKYVSYLESGVPARFSGSGSGTDFTLNIHPVE<br>EEDAATYYCQHSREFPWTFGCGTKLEIKGGGGSGGGGSGG<br>GGSGGGGGGGSGGGGSGGGGSEVQLLESGGGLVQPGGS<br>LRLSCAASGFTFSTYAMNWVRQAPGKGLEWVSRIRSKYNN<br>YATYYADSVKGRFTISRDDSKNTLYLQMNSLRAEDTAVYYCV<br>RHGNFGNSYVSWFAYWGQGTLVTVSSASTKGPSVFPLAPS<br>SKSTSGGTAALGCLVKDYFPEPVTVSWNSGALTSGVHTFPA<br>VLQSSGLYSLSSVVTVPSSSLGTQTYICNVNHKPSNTKVDKK<br>VEPKSCDGGGGSGGGGSEVQLVESGGGLVKPGGSLRLSC<br>AASGFTFSNAWMSWVRQAPGKGLEWVGRIKSKTDGGTTDY<br>AAPVKGRFTISRDDSKNTLYLQMNSLKTEDTAVYYCTTPWE<br>WSWYDYWGQGTLVTVSSASTKGPSVFPLAPSSKSTSGGTA<br>ALGCLVKDYFPEPVTVSWNSGALTSGVHTFPAVLQSSGLYS<br>LSSVVTVPSSSLGTQTYICNVNHKPSNTKVDKKVEPKSCDKT<br>HTCPPCPAPEAAGGPSVFLFPPKPKDTLMISRTPEVTCVVVD<br>VSHEDPEVKFNWYVDGVEVHNAKTKPREEQYNSTYRVVSV<br>LTVLHQDWLNGKEYKCKVSNKALGAPIEKTISKAKGQPREPQ<br>VYTLPPCRDELTKNQVSLWCLVKGFYPSDIAVEWESNGQPE<br>NNYKTTPPVLDSDGSFFLYSKLTVDKSRWQQGNVFSCSVM<br>HEALHNHYTQKSLSLSPGK | 6 |
| MK062 Protease linker | GGGGSGGGGSRQARVVNGGGGGSGGGGSGGGGS | 7 |
| Combined NF9/Mat5 linker | GGGGSVHMPLGFLGPGRSRGSFPGGGGS | 8 |
| Combined MK062 MMP9 | GGGGSGGGGSRQARVVNGGGGGSVPLSLYSGGGGGSGG<br>GGS | 9 |
| Combined MK062 MMP9 | GGGGSGGGGSRQARVVNGVPLSLYSGGGGGSGGGGS | 10 |
| **H2527 CDR H1 Kabat** | TYAMN | 11 |
| **CH2527 CDR H2 Kabat** | RIRSKYNNYATYYADSVKG | 12 |
| **CH2527 CDR H3 Kabat** | HGNFGNSYVSWFAY | 13 |
| **FolR1 CDR H1 Kabat** | NAWMS | 14 |
| **FolR1 CDR H2 Kabat** | RIKSKTDGGTTDYAAPVKG | 15 |
| **FolR1 CDR H3 Kabat** | PWEWSWYDY | 16 |
| **CLC CDR1 L1 Kabat** | GSSTGAVTTSNYAN | 17 |
| **CLC CDR L2 Kabat** | GTNKRAP | 18 |
| **CLC CDR L3 Kabat** | ALWYSNLWV | 19 |
| **Anti-ID 4.15.64 CDR H1 Kabat** | DYSIH | 20 |
| **Anti-ID 4.15.64 CDR H2 Kabat** | WINTETGEPAYADDFKG | 21 |
| **Anti-ID 4.15.64 CDR H3 Kabat** | PYDYDVLDY | 22 |
| **Anti-ID 4.15.64 CDR L1 Kabat** | RASKSVSTSNYSYIH | 23 |
| **Anti-ID 4.15.64 CDR L2 Kabat** | YVSYLES | 24 |

(continued)

| Construct | Amino acid Sequence | SEQ ID No |
|---|---|---|
| **Anti-ID 4.15.64 CDR L3 Kabat** | QHSREFPWT | 25 |
| **Anti-ID 4.32.63 CDR H1 Kabat** | SYGVS | 26 |
| **A nti-ID 4.32.63 CDR H2 Kabat** | IIWGDGSTNYHSALIS | 27 |
| **Anti-ID 4.32.63 CDR H3 Kabat** | GITTVVDDYYAMDY | 28 |
| **Anti-ID 4.32.63 CDR L1 Kabat** | RASENIDSYLA | 29 |
| **Anti-ID 4.32.63 CDR L2 Kabat** | AATFLAD | 30 |
| **Anti-ID 4.32.63 CDR L3 Kabat** | QHYYSTPYT | 31 |
| anti HER1 (GA201 heavy chain, pUC-Exp-GA201-HC) 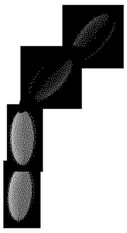 | QVQLVQSGAEVKKPGSSVKVSCKASGFTFTDYKIHWVRQAP GQGLEWMGYFNPNSGYSTYAQKFQGRVTITADKSTSTAYM ELSSLRSEDTAVYYCARLSPGGYYVMDAWGQGTTVTVSSA STKGPSVFPLAPSSKSTSGGTAALGCLVKDYFPEPVTVSWN SGALTSGVHTFPAVLQSSGLYSLSSVVTVPSSSLGTQTYICN VNHKPSNTKVDKKVEPKSCDKTHTCPPCPAPELLGGPSVFL FPPKPKDTLMISRTPEVTCVVVDVSHEDPEVKFNWYVDGVE VHNAKTKPREEQYNSTYRVVSVLTVLHQDWLNGKEYKCKVS NKALPAPIEKTISKAKGQPREPQVYTLPPSRDELTKNQVSLT CLVKGFYPSDIAVEWESNGQPENNYKTTPPVLDSDGSFFLY SKLTVDKSRWQQGNVFSCSVMHEALHNHYTQKSLSLSPGK | 32 |
| anti HER1 (GA201 light chain, pUC-Exp-GA201-LC) 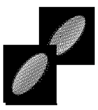 | DIQMTQSPSSLSASVGDRVTITCRASQGINNYLNWYQQKPG KAPKRLIYNTNNLQTGVPSRFSGSGSGTEFTLTISSLQPEDF ATYYCLQHNSFPTFGQGTKLEIKRTVAAPSVFIFPPSDEQLKS GTASVVCLLNNFYPREAKVQWKVDNALQSGNSQESVTEQD SKDSTYSLSSTLTLSKADYEKHKVYACEVTHQGLSSPVTKSF NRGEC | 33 |
| anti HER1 (anti-GA201 VH-VL scFv MMP cleavable linker G4S GA201 light chain, pUC-I_ GA201_MMP_LC) 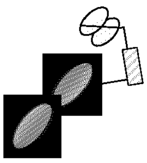 | EVQLEQSGPVLVKPGTSVKMSCKASGYTFTDYYINWIIQSHG KCLEWIGVINPDSGGTDYNQNFKGKATLTVDKSSTTAYMELT SLTSEDSAVYYCARRDSYGFDYWGQGTTLTVSSGGGGSGG GGSGGGGSGGGGSDIVLTQTPKFLLVPAGDRITMTCKASLS VTNDVAWYQQKPGQSPKLLLYYASNRNAGVPDRFTGSGYG TDFTFTITTLQAEDLAVYFCQQDYTSPPTFGCGTKLEIRGGG GSGGGGSGPLGLWSQGGGGSGGGGSGGGGSGGDIQMTQ SPSSLSASVGDRVTITCRASQGINNYLNWYQQKPGKAPKRLI YNTNNLQTGVPSRFSGSGSGTEFTLTISSLQPEDFATYYCLQ HNSFPTFGQGTKLEIKRTVAAPSVFIFPPSDEQLKSGTASVV CLLNNFYPREAKVQWKVDNALQSGNSQESVTEQDSKDSTY SLSSTLTLSKADYEKHKVYACEVTHQGLSSPVTKSFNRGEC | 34 |
| MMP Protease linker | GGGGSGGGGSGPLGLWSQGGGGSGGGGSGGGGSGG | 35 |
| Protease recognition site 1 | RQARVVNG | 36 |
| Protease recognition site 2 | VHMPLGFLGPGRSRGSFP | 37 |
| Protease recognition site 3 | RQARVVNGXXXXXVPLSLYSG | 38 |

(continued)

| Construct | Amino acid Sequence | SEQ ID No |
|---|---|---|
| Protease recognition site 4 | RQARVVNGVPLSLYSG | 39 |
| Protease recognition site 5 | PLGLWSQ | 40 |
| 4.15.64 Anti-idiotypic scFv | QIQLVQSGPELKKPGETVRISCKASGYTFTDYSIHWVKQAPGKCLKWMGWINTETGEPAYADDFKGRFAFSLETSASTAYLQINNLKNEDTATFFCAHPYDYDVLDYWGQGTSVTVSSGGGGSGGGGSGGGGSGGGGSDTVLTQSPASLGVSLGQRATISCRASKSVSTSNYSYIHWYQQKPGQPPKLLIKYVSYLESGVPARFSGSGSGTDFTLNIHPVEEEDAATYYCQHSREFPWTFGCGTKLEIK | 41 |
| 4.32.63 Anti-idiotypic scFv | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIK | 42 |
| Anti-CD3 variable heavy chain (VH) | EVQLLESGGGLVQPGGSLRLSCAASGFTFSTYAMNWVRQAPGKGLEWVSRIRSKYNNYATYYADSVKGRFTISRDDSKNTLYLQMNSLRAEDTAVYYCVRHGNFGNSYVSWFAYWGQGTLVTVSS | 43 |
| CD3 heavy chain (VH)_ CDR1 | TYAMN | 44 |
| CD3 heavy chain (VH)_ CDR2 | RIRSKYNNYATYYADSVKG | 45 |
| CD3 heavy chain (VH)_ CDR3 | HGNFGNSYVSWFAY | 46 |
| Anti-FolR1 16D5 variable region | EVQLVESGGGLVKPGGSLRLSCAASGFTFSNAWMSWVRQAPGKGLEWVGRIKSKTDGGTTDYAAPVKGRFTISRDDSKNTLYLQMNSLKTEDTAVYYCTTPWEWSWYDYWGQGTLVTVSS | 47 |
| anti-idiotypic GA201 CDR H1 Kabat | DYYIN | 48 |
| anti-idiotypic GA201 CDR H2 Kabat | VINPDSGGTDYNQNFKG | 49 |
| anti-idiotypic GA201 CDR H3 Kabat | RDSYGFDY | 50 |
| anti-idiotypic GA201 CDR L1 Kabat | KASLSVTNDVA | 51 |
| anti-idiotypic GA201 CDR L2 Kabat | YASNRNA | 52 |
| anti-idiotypic GA201 CDR L3 Kabat | QQDYTSPPT | 53 |

(continued)

| Construct | Amino acid Sequence | SEQ ID No |
|---|---|---|
| hu CD3□ | MQSGTHWRVLGLCLLSVGVWG**QDGNEEMGGITQTPYKVSI SGTTVILTCPQYPGSEILWQHNDKNIGGDEDDKNIGSDEDHL SLKEFSELEQSGYYVCYPRGSKPEDANFYLYLRARVCENC MEMD**VMSVATIVIVDICITGGLLLLVYYWSKNRKAKAKPVTRG AGAGGRQRGQNKERPPPVPNPDYEPIRKGQRDLYSGLNQR RI | 54 |
| **LC Common light chain pETR13197** V region | QAVVTQEPSLTVSPGGTVTLTC<u>GSSTGAVTTSNYAN</u>WVQEK PGQAFRGLIG<u>GTNKRAP</u>GTPARFSGSLLGGKAALTLSGAQP EDEAEYYC<u>ALWYSNLWV</u>FGGGTKLTVL | 55 |
| GA201 CDR H1 Kabat | DYKIH | 56 |
| GA201 CDR H2 Kabat | YFNPNSGYSTYAQKFQG | 57 |
| GA201 CDR H3 Kabat | LSPGGYYVMDA | 58 |
| GA201 CDR L1 Kabat | RASQGINNYLN | 59 |
| GA201 CDR L2 Kabat | NTNNLQT | 60 |
| GA201 CDR L3 Kabat | LQHNSFPT | 61 |
| **LC Common light chain pETR13197** | CAGGCCGTCGTGACCCAGGAACCCAGCCTGACAGTGTCTCCTGGC GGCACCGTGACCCTGACATGTGGCAGTTCTACAGGCGCCGTGACC ACCAGCAACTACGCCAACTGGGTGCAGGAAAAGCCCGGCCAGGCC TTCAGAGGACTGATCGGCGGCACCAACAAGAGAGCCCCTGGCACC CCTGCCAGATTCAGCGGATCTCTGCTGGGAGGAAAGGCCGCCCTG ACACTGTCTGGCGCCCAGCCAGAAGATGAGGCCGAGTACTACTGC GCCCTGTGGTACAGCAACCTGTGGGTGTTCGGCGGAGGCACCAAG CTGACAGTCCTAGGTCAACCCAAGGCTGCCCCCAGCGTGACCCTG TTCCCCCCCAGCAGCGAGGAACTGCAGGCCAACAAGGCCACCCTG GTCTGCCTGATCAGCGACTTCTACCCAGGCGCCGTGACCGTGGCC TGGAAGGCCGACAGCAGCCCCGTGAAGGCCGGCGTGGAGACCAC CACCCCCAGCAAGCAGAGCAACAACAAGTACGCCGCCAGCAGCTA CCTGAGCCTGACCCCCGAGCAGTGGAAGAGCCACAGGTCCTACAG CTGCCAGGTGACCCACGAGGGCAGCACCGTGGAGAAAACCGTGG CCCCCACCGAGTGCAGCTGA | 62 |

(continued)

| Construct | Amino acid Sequence | SEQ ID No |
|---|---|---|
| **anti CD3 (CH2527 VH_ 3-23(12) VL7-46(13)) scFv15-Matriptase MK062 CH2527 VH3_23-VH12 CH1 FolR1 16D5 VH CH1 hum Fc knob PG LALA, pETR15422** 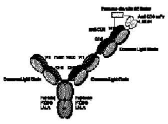 | CAGATCCAGCTGGTGCAGAGCGGCCCTGAGCTGAAGAAACCCGGC<br>GAGACAGTGCGGATCAGCTGCAAGGCCAGCGGCTACACCTTCACC<br>GACTACAGCATCCACTGGGTCAAGCAGGCCCCTGGCAAGTGCCTG<br>AAGTGGATGGGCTGGATCAACACCGAGACAGGCGAGCCCGCCTAC<br>GCCGACGATTTCAAGGGCAGATTCGCCTTCAGCCTGGAAACCAGC<br>GCCAGCACCGCCTACCTGCAGATCAACAACCTGAAGAACGAGGAC<br>ACCGCCACCTTTTTCTGCGCCCACCCCTACGACTACGACGTGCTG<br>GATTATTGGGGCCAGGGCACCAGCGTGACCGTGTCTAGCGGAGGC<br>GGAGGATCTGGCGGCGGAGGAAGTGGCGGAGGGGGATCTGGGG<br>GAGGCGGATCTGATACCGTGCTGACACAGAGCCCTGCCAGCCTGG<br>GAGTGTCCCTGGGACAGAGAGCCACCATCAGCTGTCGGGCCAGCA<br>AGAGCGTGTCCACCAGCAACTACAGCTATATCCACTGGTATCAGCA<br>GAAGCCCGGCCAGCCCCCCAAGCTGCTGATCAAATACGTGTCCTA<br>CCTGGAAAGCGGCGTGCCCGCCAGATTTTCTGGCTCTGGCAGCGG<br>CACCGACTTCACCCTGAACATCCACCCCGTGGAAGAGGAAGATGC<br>CGCCACCTACTACTGCCAGCACAGCAGAGAGTTCCCTTGGACCTTC<br>GGCTGCGGCACCAAGCTGGAAATCAAAGGCGGGGGAGGCTCCGG<br>AGGCGGCGGAAGTAGACAGGCCAGAGTCGTGAACGGGGGAGGGG<br>GGGGAAGTGGGGGCGGAGGCAGTGGGGGGGGAGGATCCGAGGT<br>GCAGCTGCTGGAATCTGGCGGCGGACTGGTGCAGCCTGGCGGAT<br>CTCTGAGACTGAGCTGTGCCGCCAGCGGCTTCACCTTCAGCACCT<br>ACGCCATGAACTGGGTGCGCCAGGCCCCTGGCAAAGGCCTGGAAT<br>GGGTGTCCCGGATCAGAAGCAAGTACAACAACTACGCCACCTACTA<br>CGCCGACAGCGTGAAGGGCCGGTTCACCATCAGCCGGGACGACA<br>GCAAGAACACCCTGTACCTGCAGATGAACAGCCTGCGGGCCGAGG<br>ACACCGCCGTGTACTATTGTGTGCGGCACGGCAACTTCGGCAACA<br>GCTATGTGTCTTGGTTTGCCTACTGGGGCCAGGGCACCCTCGTGA<br>CCGTGTCAAGCGCTAGCACAAAGGGCCCTAGCGTGTTCCCTCTGG<br>CCCCCAGCAGCAAGAGCACAAGCGGCGGAACAGCCGCCCTGGGC<br>TGCCTCGTGAAGGACTACTTCCCCGAGCCCGTGACAGTGTCTTGG<br>AACAGCGGAGCCCTGACAAGCGGCGTGCACACCTTCCCTGCCGTG<br>CTGCAGAGCAGCGGCCTGTACTCCCTGAGCAGCGTGGTCACCGTG<br>CCTAGCAGCAGCCTGGGCACCCAGACCTACATCTGCAACGTGAAC<br>CACAAGCCCAGCAACACCAAAGTGGACAAGAAGGTGGAGCCCAAG<br>AGCTGTGATGGCGGAGGAGGGTCCGGAGGCGGAGGATCCGAGGT<br>GCAATTGGTTGAATCTGGTGGTGGTCTGGTAAAACCGGGCGGTTC<br>CCTGCGTCTGAGCTGCGCGGCTTCCGGATTCACCTTCTCCAACGC<br>GTGGATGAGCTGGGTTCGCCAGGCCCCGGGCAAAGGCCTCGAGT<br>GGGTTGGTCGTATCAAGTCTAAAACTGACGGTGGCACCACGGATTA<br>CGCGGCTCCAGTTAAAGGTCGTTTTACCATTTCCCGCGACGATAGC<br>AAAAACACTCTGTATCTGCAGATGAACTCTCTGAAAACTGAAGACAC<br>CGCAGTCTACTACTGTACTACCCCGTGGGAATGGTCTTGGTACGAT<br>TATTGGGGCCAGGGCACGCTGGTTACGGTGTCTAGCGCTAGTACC | 63 |

(continued)

| Construct | Amino acid Sequence | SEQ ID No |
|---|---|---|
| | AAGGGCCCCAGCGTGTTCCCCCTGGCACCCAGCAGCAAGAGCACA<br>TCTGGCGGAACAGCCGCTCTGGGCTGTCTGGTGAAAGACTACTTC<br>CCCGAGCCCGTGACCGTGTCTTGGAACTCTGGCGCCCTGACCAGC<br>GGCGTGCACACCTTTCCAGCCGTGCTGCAGAGCAGCGGCCTGTAC<br>TCCCTGTCCTCCGTGGTCACCGTGCCCTCTAGCTCCCTGGGAACA<br>CAGACATATATCTGTAATGTCAATCACAAGCCTTCCAACACCAAAGT<br>CGATAAGAAAGTCGAGCCCAAGAGCTGCGACAAAACTCACACATG<br>CCCACCGTGCCCAGCACCTGAAGCTGCAGGGGGACCGTCAGTCTT<br>CCTCTTCCCCCCAAAACCCAAGGACACCCTCATGATCTCCCGGACC<br>CCTGAGGTCACATGCGTGGTGGTGGACGTGAGCCACGAAGACCCT<br>GAGGTCAAGTTCAACTGGTACGTGGACGGCGTGGAGGTGCATAAT<br>GCCAAGACAAAGCCGCGGGAGGAGCAGTACAACAGCACGTACCGT<br>GTGGTCAGCGTCCTCACCGTCCTGCACCAGGACTGGCTGAATGGC<br>AAGGAGTACAAGTGCAAGGTCTCCAACAAAGCCCTCGGCGCCCCC<br>ATCGAGAAAACCATCTCCAAAGCCAAAGGGCAGCCCCGAGAACCA<br>CAGGTGTACACCCTGCCCCCATGCCGGGATGAGCTGACCAAGAAC<br>CAGGTCAGCCTGTGGTGCCTGGTCAAAGGCTTCTATCCCAGCGAC<br>ATCGCCGTGGAGTGGGAGAGCAATGGGCAGCCGGAGAACAACTAC<br>AAGACCACGCCTCCCGTGCTGGACTCCGACGGCTCCTTCTTCCTCT<br>ACAGCAAGCTCACCGTGGACAAGAGCAGGTGGCAGCAGGGGAAC<br>GTCTTCTCATGCTCCGTGATGCATGAGGCTCTGCACAACCACTACA<br>CGCAGAAGAGCCTCTCCCTGTCTCCGGGTAAATGA | |
| **FolR1 16D5 VH CH1 Fc hole P329G LALA HRYF, pETR15214** | GAGGTGCAATTGGTTGAATCTGGTGGTGGTCTGGTAAAACCGGGC<br>GGTTCCCTGCGTCTGAGCTGCGCGGCTTCCGGATTCACCTTCTCC<br>AACGCGTGGATGAGCTGGGTTCGCCAGGCCCCGGGCAAAGGCCT<br>CGAGTGGGTTGGTCGTATCAAGTCTAAAACTGACGGTGGCACCAC<br>GGATTACGCGGCTCCAGTTAAAGGTCGTTTTACCATTTCCCGCGAC<br>GATAGCAAAAACACTCTGTATCTGCAGATGAACTCTCTGAAAACTGA<br>AGACACCGCAGTCTACTACTGTACTACCCCGTGGGAATGGTCTTGG<br>TACGATTATTGGGGCCAGGGCACGCTGGTTACGGTGTCTTCCGCT<br>AGCACCAAGGGCCCCTCCGTGTTCCCCCTGGCCCCCAGCAGCAAG<br>AGCACCAGCGGCGGCACAGCCGCTCTGGGCTGCCTGGTCAAGGA<br>CTACTTCCCCGAGCCCGTGACCGTGTCCTGGAACAGCGGAGCCCT<br>GACCTCCGGCGTGCACACCTTCCCCGCCGTGCTGCAGAGTTCTGG<br>CCTGTATAGCCTGAGCAGCGTGGTCACCGTGCCTTCTAGCAGCCT<br>GGGCACCCAGACCTACATCTGCAACGTGAACCACAAGCCCAGCAA<br>CACCAAGGTGGACAAGAAGGTGGAGCCCAAGAGCTGCGACAAAAC<br>TCACACATGCCCACCGTGCCCAGCACCTGAAGCTGCAGGGGGACC<br>GTCAGTCTTCCTCTTCCCCCCAAAACCCAAGGACACCCTCATGATC<br>TCCCGGACCCCTGAGGTCACATGCGTGGTGGTGGACGTGAGCCAC<br>GAAGACCCTGAGGTCAAGTTCAACTGGTACGTGGACGGCGTGGAG<br>GTGCATAATGCCAAGACAAAGCCGCGGGAGGAGCAGTACAACAGC<br>ACGTACCGTGTGGTCAGCGTCCTCACCGTCCTGCACCAGGACTGG<br>CTGAATGGCAAGGAGTACAAGTGCAAGGTCTCCAACAAAGCCCTC<br>GGCGCCCCCATCGAGAAAACCATCTCCAAAGCCAAAGGGCAGCCC<br>CGAGAACCACAGGTGTGCACCCTGCCCCCATCCCGGGATGAGCTG<br>ACCAAGAACCAGGTCAGCCTCTCGTGCGCAGTCAAAGGCTTCTATC<br>CCAGCGACATCGCCGTGGAGTGGGAGAGCAATGGGCAGCCGGAG<br>AACAACTACAAGACCACGCCTCCCGTGCTGGACTCCGACGGCTCC<br>TTCTTCCTCGTGAGCAAGCTCACCGTGGACAAGAGCAGGTGGCAG<br>CAGGGGAACGTCTTCTCATGCTCCGTGATGCATGAGGCTCTGCAC<br>AACCGCTTCACGCAGAAGAGCCTCTCCCTGTCTCCGGGTAAATGA | 64 |

(continued)

| Construct | Amino acid Sequence | SEQ ID No |
|---|---|---|
| **anti CD3 (CH2527 VH_ 3-23(12) VL7-46(13)) scFv 4.32.63 Matriptase MK062 CH2527 VH3_23-VH12 CH1 FolR1 16D5 VH CH1 hum Fc knob PG LALA, pETR15599** 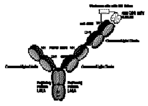 | CAAGTGCAGCTGAAAGAGTCCGGCCCTGGACTGGTGGCCCCTAGC<br>CAGAGCCTGAGCATCACCTGTACCGTGTCCGGCTTCAGCCTGACC<br>AGCTACGGCGTGTCATGGGTGCGCCAGCCTCCAGGCAAGTGTCTG<br>GAATGGCTGGGCATCATCTGGGGCGACGGCAGCACCAATTACCAC<br>AGCGCCCTGATCAGCAGACTGAGCATCTCCAAGGACAACAGCAAG<br>AGCCAGGTGTTCCTGAAGCTGAACAGCCTGCAGACCGACGACACC<br>GCCACCTACTACTGCGCCAAGGGCATCACCACCGTGGTGGACGAC<br>TACTACGCTATGGACTACTGGGGCCAGGGCACCAGCGTGACAGTG<br>TCTAGCGGAGGCGGAGGATCTGGCGGCGGAGGAAGTGGCGGAGG<br>GGGATCTGGGGGAGGCGGAAGCGATATCCAGATGACCCAGAGCC<br>CTGCCAGCCTGTCTGCCTCTGTGGGCGAGACAGTGACCATCACAT<br>GCCGGGCCAGCGAGAACATCGACAGCTACCTGGCCTGGTATCAGC<br>AGAAGCAGGGCAAGAGCCCCCAGCTGCTGGTGTACGCCGCCACCT<br>TTCTGGCCGACGATGTGCCCAGCAGATTCAGCGGCAGCGGAAGCG<br>GCACACAGTACAGCCTGAAGATCAACTCCCTGCAGAGCGAGGACG<br>TGGCCCGGTACTACTGCCAGCACTACTACAGCACCCCCTACACCTT<br>CGGCTGCGGCACCAAGCTGGAAATCAAAGGCGGGGGAGGCTCCG<br>GAGGCGGCGGAAGTAGACAGGCCAGAGTCGTGAACGGGGGAGGG<br>GGGGGAAGTGGGGGCGGAGGCAGTGGGGGCGGAGGATCCGAGG<br>TGCAGCTGCTGGAATCTGGCGGCGGACTGGTGCAGCCTGGCGGA<br>TCTCTGAGACTGAGCTGTGCCGCCAGCGGCTTCACCTTCAGCACC<br>TACGCCATGAACTGGGTGCGCCAGGCCCCTGGCAAAGGCCTGGAA<br>TGGGTGTCCCGGATCAGAAGCAAGTACAACAACTACGCCACCTACT<br>ACGCCGACAGCGTGAAGGGCCGGTTCACCATCAGCCGGGACGAC<br>AGCAAGAACACCCTGTACCTGCAGATGAACAGCCTGCGGGCCGAG<br>GACACCGCCGTGTACTATTGTGTGCGGCACGGCAACTTCGGCAAC<br>AGCTATGTGTCTTGGTTTGCCTACTGGGGCCAGGGCACCCTCGTG<br>ACCGTGTCAAGCGCTAGCACAAAGGGCCCTAGCGTGTTCCCTCTG<br>GCCCCCAGCAGCAAGAGCACAAGCGGCGGAACAGCCGCCCTGGG<br>CTGCCTCGTGAAGGACTACTTCCCCGAGCCCGTGACAGTGTCTTG | 65 |

(continued)

| Construct | Amino acid Sequence | SEQ ID No |
|---|---|---|
| | GAACAGCGGAGCCCTGACAAGCGGCGTGCACACCTTCCCTGCCGT GCTGCAGAGCAGCGGCCTGTACTCCCTGAGCAGCGTGGTCACCGT GCCTAGCAGCAGCCTGGGCACCCAGACCTACATCTGCAACGTGAA CCACAAGCCCAGCAACACCAAAGTGGACAAGAAGGTGGAGCCCAA GAGCTGTGATGGCGGAGGAGGGTCCGGGGGCGGAGGATCCGAG GTGCAATTGGTTGAATCTGGTGGTGGTCTGGTAAAACCGGGCGGT TCCCTGCGTCTGAGCTGCGCGGCTTCCGGGTTCACCTTCTCCAAC GCGTGGATGAGCTGGGTTCGCCAGGCCCCGGGCAAAGGCCTCGA GTGGGTTGGTCGTATCAAGTCTAAAACTGACGGTGGCACCACGGA TTACGCGGCTCCAGTTAAAGGTCGTTTTACCATTTCCCGCGACGAT AGCAAAAACACTCTGTATCTGCAGATGAACTCTCTGAAAACTGAAG ACACCGCAGTCTACTACTGTACTACCCCGTGGGAATGGTCTTGGTA CGATTATTGGGGCCAGGGCACGCTGGTTACGGTGTCTAGCGCTAG TACCAAGGGCCCCAGCGTGTTCCCCCTGGCACCCAGCAGCAAGAG CACATCTGGCGGAACAGCCGCTCTGGGCTGTCTGGTGAAAGACTA CTTCCCCGAGCCCGTGACCGTGTCTTGGAACTCTGGCGCCCTGAC CAGCGGCGTGCACACCTTTCCAGCCGTGCTGCAGAGCAGCGGCCT GTACTCCCTGTCCTCCGTGGTCACCGTGCCCTCTAGCTCCCTGGG AACACAGACATATATCTGTAATGTCAATCACAAGCCTTCCAACACCA AAGTCGATAAGAAAGTCGAGCCCAAGAGCTGCGACAAAACTCACAC ATGCCCACCGTGCCCAGCACCTGAAGCTGCAGGGGGACCGTCAGT CTTCCTCTTCCCCCCAAAACCCAAGGACACCCTCATGATCTCCCGG ACCCCTGAGGTCACATGCGTGGTGGTGGACGTGAGCCACGAAGAC CCTGAGGTCAAGTTCAACTGGTACGTGGACGGCGTGGAGGTGCAT AATGCCAAGACAAAGCCGCGGGAGGAGCAGTACAACAGCACGTAC CGTGTGGTCAGCGTCCTCACCGTCCTGCACCAGGACTGGCTGAAT GGCAAGGAGTACAAGTGCAAGGTCTCCAACAAAGCCCTCGGCGCC CCCATCGAGAAAACCATCTCCAAAGCCAAAGGGCAGCCCCGAGAA CCACAGGTGTACACCCTGCCCCCATGCCGGGATGAGCTGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTT CCTCTACAGCAAGCTCACCGTGGACAAGAGCAGGTGGCAGCAGGG GAACGTCTTCTCATGCTCCGTGATGCATGAGGCTCTGCACAACCAC TACACGCAGAAGAGCCTCTCCCTGTCTCCGGGTAAATGA | |

(continued)

| Construct | Amino acid Sequence | SEQ ID No |
|---|---|---|
| anti CD3 (CH2527 VH_ 3-23(12) VL7-46(13)) scFv 4.32.63 non-cleavable linker CH2527 VH3_23-VH12 CH1 FolR1 16D5 VH CH1 hum Fc knob PG LALA, pETR15603 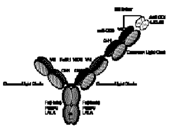 | CAAGTGCAGCTGAAAGAGTCCGGCCCTGGACTGGTGGCCCCTAGC<br>CAGAGCCTGAGCATCACCTGTACCGTGTCCGGCTTCAGCCTGACC<br>AGCTACGGCGTGTCATGGGTGCGCCAGCCTCCAGGCAAGTGTCTG<br>GAATGGCTGGGCATCATCTGGGGCGACGGCAGCACCAATTACCAC<br>AGCGCCCTGATCAGCAGACTGAGCATCTCCAAGGACAACAGCAAG<br>AGCCAGGTGTTCCTGAAGCTGAACAGCCTGCAGACCGACGACACC<br>GCCACCTACTACTGCGCCAAGGGCATCACCACCGTGGTGGACGAC<br>TACTACGCTATGGACTACTGGGGCCAGGGCACCAGCGTGACAGTG<br>TCTAGCGGAGGCGGAGGATCTGGCGGCGGAGGAAGTGGCGGAGG<br>GGGATCTGGGGGAGGCGGAAGCGATATCCAGATGACCCAGAGCC<br>CTGCCAGCCTGTCTGCCTCTGTGGGCGAGACAGTGACCATCACAT<br>GCCGGGCCAGCGAGAACATCGACAGCTACCTGGCCTGGTATCAGC<br>AGAAGCAGGGCAAGAGCCCCCAGCTGCTGGTGTACGCCGCCACCT<br>TTCTGGCCGACGATGTGCCCAGCAGATTCAGCGGCAGCGGAAGCG<br>GCACACAGTACAGCCTGAAGATCAACTCCCTGCAGAGCGAGGACG<br>TGGCCCGGTACTACTGCCAGCACTACTACAGCACCCCCTACACCTT<br>CGGCTGCGGCACCAAGCTGGAAATCAAAGGCGGGGGAGGCTCCG<br>GAGGCGGCGGAAGTGGAGGCGGCGGAAGTGGCGGAGGCGGAGG<br>GGGGGGAAGTGGGGGCGGAGGCAGTGGGGGGGGAGGATCCGAG<br>GTGCAGCTGCTGGAATCTGGCGGCGGACTGGTGCAGCCTGGCGG<br>ATCTCTGAGACTGAGCTGTGCCGCCAGCGGCTTCACCTTCAGCAC<br>CTACGCCATGAACTGGGTGCGCCAGGCCCCTGGCAAAGGCCTGGA<br>ATGGGTGTCCCGGATCAGAAGCAAGTACAACAACTACGCCACCTAC<br>TACGCCGACAGCGTGAAGGGCCGGTTCACCATCAGCCGGGACGA<br>CAGCAAGAACACCCTGTACCTGCAGATGAACAGCCTGCGGGCCGA<br>GGACACCGCCGTGTACTATTGTGTGCGGCACGGCAACTTCGGCAA<br>CAGCTATGTGTCTTGGTTTGCCTACTGGGGCCAGGGCACCCTCGT<br>GACCGTGTCAAGCGCTAGCACAAAGGGCCCTAGCGTGTTCCCTCT<br>GGCCCCCAGCAGCAAGAGCACAAGCGGCGGAACAGCCGCCCTGG<br>GCTGCCTCGTGAAGGACTACTTCCCCGAGCCCGTGACAGTGTCTT<br>GGAACAGCGGAGCCCTGACAAGCGGCGTGCACACCTTCCCTGCC<br>GTGCTGCAGAGCAGCGGCCTGTACTCCCTGAGCAGCGTGGTCACC<br>GTGCCTAGCAGCAGCCTGGGCACCCAGACCTACATCTGCAACGTG<br>AACCACAAGCCCAGCAACACCAAAGTGGACAAGAAGGTGGAGCCC<br>AAGAGCTGTGATGGCGGAGGAGGGTCCGGAGGCGGAGGCTCCGA<br>GGTGCAATTGGTTGAATCTGGTGGTGGTCTGGTAAAACCGGGCGG<br>TTCCCTGCGTCTGAGCTGCGCGGCTTCCGGATTCACCTTCTCCAAC<br>GCGTGGATGAGCTGGGTTCGCCAGGCCCCGGGCAAAGGCCTCGA<br>GTGGGTTGGTCGTATCAAGTCTAAAACTGACGGTGGCACCACGGA<br>TTACGCGGCTCCAGTTAAAGGTCGTTTTACCATTTCCCGCGACGAT<br>AGCAAAAACACTCTGTATCTGCAGATGAACTCTCTGAAAACTGAAG<br>ACACCGCAGTCTACTACTGTACTACCCCGTGGGAATGGTCTTGGTA<br>CGATTATTGGGGCCAGGGCACGCTGGTTACGGTGTCTAGCGCTAG<br>TACCAAGGGCCCCAGCGTGTTCCCCCTGGCACCCAGCAGCAAGAG<br>CACATCTGGCGGAACAGCCGCTCTGGGCTGTCTGGTGAAAGACTA<br>CTTCCCCGAGCCCGTGACCGTGTCTTGGAACTCTGGCGCCCTGAC<br>CAGCGGCGTGCACACCTTTCCAGCCGTGCTGCAGAGC<br>AGCGGCCTGTACTCCCTGTCCTCCGTGGTCACCGTGCCCTCTAGC<br>TCCCTGGGAACACAGACATATATCTGTAATGTCAATCACAAGCCTTC<br>CAACACCAAAGTCGATAAGAAAGTCGAGCCCAAGAGCTGCGACAA<br>AACTCACACATGCCCACCGTGCCCAGCACCTGAAGCTGCAGGGGG<br>ACCGTCAGTCTTCCTCTTCCCCCCAAAACCCAAGGACACCCTCATG<br>ATCTCCCGGACCCCTGAGGTCACATGCGTGGTGGTGGACGTGAGC<br>CACGAAGACCCTGAGGTCAAGTTCAACTGGTACGTGGACGGCGTG<br>GAGGTGCATAATGCCAAGACAAAGCCGCGGGAGGAGCAGTACAAC<br>AGCACGTACCGTGTGGTCAGCGTCCTCACCGTCCTGCACCAGGAC<br>TGGCTGAATGGCAAGGAGTACAAGTGCAAGGTCTCCAACAAAGCC<br>CTCGGCGCCCCCATCGAGAAAACCATCTCCAAAGCCAAAGGGCAG<br>CCCCGAGAACCACAGGTGTACACCCTGCCCCCATGCCGGGATGAG<br>CTGACCAAGAACCAGGTCAGCCTGTGGTGCCTGGTCAAAGGCTTC | 66 |

(continued)

| Construct | Amino acid Sequence | SEQ ID No |
|---|---|---|
| | TATCCCAGCGACATCGCCGTGGAGTGGGAGAGCAATGGGCAGCC GGAGAACAACTACAAGACCACGCCTCCCGTGCTGGACTCCGACGG CTCCTTCTTCCTCTACAGCAAGCTCACCGTGGACAAGAGCAGGTGG CAGCAGGGGAACGTCTTCTCATGCTCCGTGATGCATGAGGCTCTG CACAACCACTACACGCAGAAGAGCCTCTCCCTGTCTCCGGGTAAAT GA | |

(continued)

| Construct | Amino acid Sequence | SEQ ID No |
|---|---|---|
| **anti CD3 (CH2527 VH_ 3-23(12) VL7-46(13)) scFv15 non-cleavable linker CH2527 VH3_23-VH12 CH1 FolR1 16D5 VH CH1 hum Fc knob PG LALA, pETR14759** 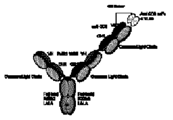 | CAGATCCAGCTGGTGCAGAGCGGCCCTGAGCTGAAGAAACCCGGC<br>GAGACAGTGCGGATCAGCTGCAAGGCCAGCGGCTACACCTTCACC<br>GACTACAGCATCCACTGGGTCAAGCAGGCCCCTGGCAAGTGCCTG<br>AAGTGGATGGGCTGGATCAACACCGAGACAGGCGAGCCCGCCTAC<br>GCCGACGATTTCAAGGGCAGATTCGCCTTCAGCCTGGAAACCAGC<br>GCCAGCACCGCCTACCTGCAGATCAACAACCTGAAGAACGAGGAC<br>ACCGCCACCTTTTTCTGCGCCCACCCCTACGACTACGACGTGCTG<br>GATTATTGGGGCCAGGGCACCAGCGTGACCGTGTCTAGCGGAGGC<br>GGAGGATCTGGCGGCGGAGGAAGTGGCGGAGGGGGATCTGGGG<br>GAGGCGGATCTGATACCGTGCTGACACAGAGCCCTGCCAGCCTGG<br>GAGTGTCCCTGGGACAGAGAGCCACCATCAGCTGTCGGGCCAGCA<br>AGAGCGTGTCCACCAGCAACTACAGCTATATCCACTGGTATCAGCA<br>GAAGCCCGGCCAGCCCCCCAAGCTGCTGATCAAATACGTGTCCTA<br>CCTGGAAAGCGGCGTGCCCGCCAGATTTTCTGGCTCTGGCAGCGG<br>CACCGACTTCACCCTGAACATCCACCCCGTGGAAGAGGAAGATGC<br>CGCCACCTACTACTGCCAGCACAGCAGAGAGTTCCCTTGGACCTTC<br>GGCTGCGGCACCAAGCTGGAAATCAAAGGCGGGGGAGGCTCCGG<br>AGGCGGCGGAAGTGGAGGCGGCGGAAGTGGCGGAGGCGGAGGG<br>GGGGGAAGTGGGGGCGGAGGCAGTGGGGGGGGAGGATCCGAGG<br>TGCAGCTGCTGGAATCTGGCGGCGGACTGGTGCAGCCTGGCGGA<br>TCTCTGAGACTGAGCTGTGCCGCCAGCGGCTTCACCTTCAGCACC<br>TACGCCATGAACTGGGTGCGCCAGGCCCCTGGCAAAGGCCTGGAA<br>TGGGTGTCCCGGATCAGAAGCAAGTACAACAACTACGCCACCTACT<br>ACGCCGACAGCGTGAAGGGCCGGTTCACCATCAGCCGGGACGAC<br>AGCAAGAACACCCTGTACCTGCAGATGAACAGCCTGCGGGCCGAG<br>GACACCGCCGTGTACTATTGTGTGCGGCACGGCAACTTCGGCAAC<br>AGCTATGTGTCTTGGTTTGCCTACTGGGGCCAGGGCACCCTCGTG<br>ACCGTGTCAAGCGCTAGCACAAAGGGCCCTAGCGTGTTCCCTCTG<br>GCCCCCAGCAGCAAGAGCACAAGCGGCGGAACAGCCGCCCTGGG<br>CTGCCTCGTGAAGGACTACTTCCCCGAGCCCGTGACAGTGTCTTG<br>GAACAGCGGAGCCCTGACAAGCGGCGTGCACACCTTCCCTGCCGT<br>GCTGCAGAGCAGCGGCCTGTACTCCCTGAGCAGCGTGGTCACCGT<br>GCCTAGCAGCAGCCTGGGCACCCAGACCTACATCTGCAACGTGAA<br>CCACAAGCCCAGCAACACCAAAGTGGACAAGAAGGTGGAGCCCAA<br>GAGCTGTGATGGCGGAGGAGGGTCCGGAGGCGGAGGCTCCGAGG<br>TGCAATTGGTTGAATCTGGTGGTGGTCTGGTAAAACCGGGCGGTTC<br>CCTGCGTCTGAGCTGCGCGGCTTCCGGATTCACCTTCTCCAACGC<br>GTGGATGAGCTGGGTTCGCCAGGCCCCGGGCAAAGGCCTCGAGT<br>GGGTTGGTCGTATCAAGTCTAAAACTGACGGTGGCACCACGGATTA<br>CGCGGCTCCAGTTAAAGGTCGTTTTACCATTTCCCGCGACGATAGC<br>AAAAACACTCTGTATCTGCAGATGAACTCTCTGAAAACTGAAGACAC<br>CGCAGTCTACTACTGTACTACCCCGTGGGAATGGTCTTGGTACGAT<br>TATTGGGGCCAGGGCACGCTGGTTACGGTGTCTAGCGCTAGTACC<br>AAGGGCCCCAGCGTGTTCCCCCTGGCACCCAGCAGCAAGAGCACA<br>TCTGGCGGAACAGCCGCTCTGGGCTGTCTGGTGAAAGACTACTTC<br>CCCGAGCCCGTGACCGTGTCTTGGAACTCTGGCGCCCTGACCAGC<br>GGCGTGCACACCTTTCCAGCCGTGCTGCAGAGCAGCGGCCTGTAC<br>TCCCTGTCCTCCGTGGTCACCGTGCCCTCTAGCTCCCTGGGAACA<br>CAGACATATATCTGTAATGTCAATCACAAGCCTTCCAACACCAAAGT<br>CGATAAGAAAGTCGAGCCCAAGAGCTGCGACAAAACTCACACATG<br>CCCACCGTGCCCAGCACCTGAAGCTGCAGGGGGACCGTCAGTCTT<br>CCTCTTCCCCCCAAAACCCAAGGACACCCTCATGATCTCCCGGACC<br>CCTGAGGTCACATGCGTGGTGGTGGACGTGAGCCACGAAGACCCT<br>GAGGTCAAGTTCAACTGGTACGTGGACGGCGTGGAGGTGCATAAT<br>GCCAAGACAAAGCCGCGGGAGGAGCAGTACAACAGCACGTACCGT<br>GTGGTCAGCGTCCTCACCGTCCTGCACCAGGACTGGCTGAATGGC<br>AAGGAGTACAAGTGCAAGGTCTCCAACAAAGCCCTCGGCGCCCCC<br>ATCGAGAAAACCATCTCCAAAGCCAAAGGGCAGCCCCGAGAACCA<br>CAGGTGTACACCCTGCCCCCATGCCGGGATGAGCTGACCAAGAAC<br>CAGGTCAGCCTGTGGTGCCTGGTCAAAGGCTTCTATCCCAGCGAC | 67 |

(continued)

| Construct | Amino acid Sequence | SEQ ID No |
|---|---|---|
| | ATCGCCGTGGAGTGGGAGAGCAATGGGCAGCCGGAGAACAACTAC AAGACCACGCCTCCCGTGCTGGACTCCGACGGCTCCTTCTTCCTCT ACAGCAAGCTCACCGTGGACAAGAGCAGGTGGCAGCAGGGGAAC GTCTTCTCATGCTCCGTGATGCATGAGGCTCTGCACAACCACTACA CGCAGAAGAGCCTCTCCCTGTCTCCGGGTAAATGA | |
| MK062 Protease linker | GGCGGGGGAGGCTCCGGAGGCGGCGGAAGTAGACAGGCCAGAG TCGTGAACGGGGGAGGGGGGGGAAGTGGGGGCGGAGGCAGTGG GGGCGGAGGATCC | 68 |
| anti HER1 (GA201 heavy chain, pUC-Exp-GA201-HC) | CAGGTGCAGCTGGTCCAGAGCGGCGCCGAGGTGAAGAAACCCGG GTCCTCTGTCAAGGTGTCATGCAAGGCTAGCGGATTCACCTTTACA GACTACAAAATCCACTGGGTTAGGCAGGCACCTGGCCAAGGACTC GAATGGATGGGGTATTTCAACCCAAATTCCGGCTACTCTACCTATG CCCAGAAGTTTCAGGGAAGAGTGACTATTACAGCTGATAAGAGTAC CAGCACTGCATACATGGAGCTGTCCTCTCTTCGCTCAGAGGACACC GCCGTCTACTATTGTGCTCGGCTGAGCCCCGGTGGCTACTATGTG ATGGATGCATGGGGGCAGGGAACAACCGTAACAGTGTCCTCTGCG TCGACTAAGGGCCCTTCAGTTTTTCCACTCGCCCCCAGTAGCAAGT CCACATCTGGGGGTACCGCTGCCCTGGGCTGCCTTGTGAAAGACT ATTTCCCTGAACCAGTCACTGTGTCATGGAATAGCGGAGCCCTGAC CTCCGGTGTACACACATTCCCCGCTGTGTTGCAGTCTAGTGGCCTG TACAGCCTCTCCTCTGTTGTGACCGTCCCTTCAAGCTCCCTGGGGA CACAGACCTATATCTGTAACGTGAATCATAAGCCATCTAACACTAAA GTAGATAAAAAAGTGGAGCCCAAGAGTTGCGACAAAACACACACCT GTCCCCCTTGCCCAGCCCCCGAGCTTCTGGGAGGCCCTAGCGTCT TTCTCTTCCCACCCAAGCCTAAGGATACTCTGATGATATCCAGGAC CCCAGAAGTTACATGCGTGGTCGTGGACGTCTCACACGAGGACCC CGAAGTGAAATTTAACTGGTACGTTGATGGTGTGGAAGTCCATAAT GCCAAGACCAAGCCTAGAGAGGAGCAATACAACAGTACATATCGC GTGGTAAGCGTGTTGACCGTTCTCCACCAGGACTGGCTCAATGGG AAAGAATACAAGTGTAAAGTGTCCAACAAAGCTCTGCCAGCACCCA TCGAGAAGACTATTTCTAAGGCCAAAGGCCAGCCCCGGGAGCCTC AGGTCTATACACTTCCACCCTCAAGGGATGAACTGACCAAGAACCA AGTGAGCTTGACTTGCCTGGTAAAGGGGTTCTACCCTTCCGACATC GCTGTGGAGTGGGAGTCTAATGGACAACCAGAAAACAATTACAAAA CCACACCCCCTGTCCTCGACAGTGATGGCAGCTTTTTCCTGTATAG CAAACTTACCGTTGACAAGTCCAGATGGCAGCAGGGAAACGTGTTC TCATGTAGCGTCATGCACGAAGCTTTGCATAACCACTACACACAGA AAAGCCTCAGCCTGAGTCCAGGGAAG | 69 |
| anti HER1 (GA201 light chain, pUC-Exp-GA201-LC) | GACATCCAAATGACCCAGTCACCTAGTAGCCTCTCCGCCTCTGTTG GCGACAGGGTGACAATTACATGCAGAGCTTCACAGGGTATCAACAA TTACCTGAACTGGTATCAGCAGAAACCAGGGAAGGCCCCCAAGCG CTTGATATATAACACCAATAACCTGCAAACTGGCGTCCCTAGCCGG TTCTCCGGATCTGGTAGTGGCACCGAATTTACACTCACCATCAGCT CCCTGCAGCCAGAGGATTTCGCCACATACTATTGTCTTCAGCATAA TTCTTTCCCCACCTTTGGGCAAGGAACTAAACTGGAGATTAAGCGT ACTGTCGCCGCTCCCTCTGTGTTCATTTTTCCTCCAAGTGATGAGC AGCTCAAAAGCGGTACCGCATCCGTTGTGTGCCTGCTTAACAACTT CTATCCCCGGGAAGCCAAGGTCCAATGGAAGGTGGACAATGCTCT GCAGTCAGGAAACAGTCAGGAGAGCGTAACCGAGCAGGATTCCAA AGACTCTACTTACTCATTGAGCTCCACCCTGACACTCTCTAAGGCA GACTATGAAAAGCATAAAGTGTACGCCTGTGAGGTTACCCACCAGG GCCTGAGTAGCCCTGTGACAAAGTCCTTCAATAGGGGAGAGTGC | 70 |

(continued)

| Construct | Amino acid Sequence | SEQ ID No |
|---|---|---|
| HER1 (anti-GA201 VH-VL scFv MMP cleavable linker G4S GA201 light chain, pUC-I_ GA201_MM P_LC) | GAGGTTCAGCTGGAGCAGTCAGGACCTGTGCTGGTGAAGCCTGGG ACTTCAGTGAAGATGTCCTGTAAGGCTTCTGGATACACATTCACTG ACTACTATATAAACTGGATAATACAGAGCCATGGAAAGTGTCTTGAG TGGATTGGAGTTATTAATCCTGACAGCGGTGGTACTGACTACAACC AGAACTTCAAGGGCAAGGCCACATTGACTGTTGACAAGTCCTCCAC CACAGCCTACATGGAACTCACTAGCCTGACATCTGAGGACTCTGCA GTCTATTATTGTGCAAGAAGGGATTCTTACGGCTTTGACTACTGGG GCCAAGGCACCACTCTCACAGTCTCCTCAGGCGGAGGTGGCTCAG GGGGAGGCGGTAGCGGCGGAGGTGGCTCAGGGGGAGGCGGTAG CGACATTGTGCTGACCCAGACTCCCAAATTCCTGCTTGTGCCAGCA GGAGACAGGATTACCATGACCTGCAAGGCCAGTCTGAGTGTGACT AATGATGTAGCTTGGTATCAACAGAAACCAGGGCAGTCTCCTAAAC TGCTGTTATACTATGCATCCAATCGCAACGCTGGAGTCCCTGATCG CTTCACTGGCAGTGGATATGGGACGGATTTCACTTTCACCATCACC ACTTTGCAGGCTGAAGACCTGGCAGTTTATTTCTGTCAGCAGGATT ATACCTCTCCTCCGACGTTCGGTTGTGGCACCAAGCTAGAAATCCG TGGTGGCGGCGGTTCTGGCGGAGGGGGTTCTGGCCCCCTGGGGC TATGGAGCCAGGGTGGCGGCGGTTCTGGCGGAGGGGGTTCTGGC GGTGGTGGCTCTGGCGGTGACATCCAAATGACCCAGTCACCTAGT AGCCTCTCCGCCTCTGTTGGCGACAGGGTGACAATTACATGCAGA GCTTCACAGGGTATCAACAATTACCTGAACTGGTATCAGCAGAAAC CAGGGAAGGCCCCCAAGCGCTTGATATATAACACCAATAACCTGCA AACTGGCGTCCCTAGCCGGTTCTCCGGATCTGGTAGTGGCACCGA ATTTACACTCACCATCAGCTCCCTGCAGCCAGAGGATTTCGCCACA TACTATTGTCTTCAGCATAATTCTTTCCCCACCTTTGGGCAAGGAAC TAAACTGGAGATTAAGCGTACTGTCGCCGCTCCCTCTGTGTTCATT TTTCCTCCAAGTGATGAGCAGCTCAAAAGCGGTACCGCATCCGTTG TGTGCCTGCTTAACAACTTCTATCCCCGGGAAGCCAAGGTCCAATG GAAGGTGGACAATGCTCTGCAGTCAGGAAACAGTCAGGAGAGCGT AACCGAGCAGGATTCCAAAGACTCTACTTACTCATTGAGCTCCACC CTGACACTCTCTAAGGCAGACTATGAAAAGCATAAAGTGTACGCCT GTGAGGTTACCCACCAGGGCCTGAGTAGCCCTGTGACAAAGTCCT TCAATAGGGGAGAGTGC | 71 |

| Construct | Amino acid Sequence | SEQ ID No |
|---|---|---|
| anti CD3 (CH2527 VH_3-23 (12) VL7-46(13)) scFv 4.32.63 MMP9 Matriptase MK062 CH2527 VH3_23-VH12 CH1 FolR1 16D5 VH CH1 hum Fc knob PG LALA, pETR16546 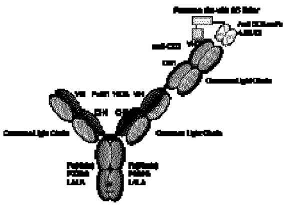 | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIKGGGGSVHMPLGFLGPRQARVVNGGGGGSGGGGSEVQLLESGGGLVQPGGSLRLSCAASGFTFSTYAMNWVRQAPGKGLEWVSRIRSKYNNYATYYADSVKGRFTISRDDSKNTLYLQMNSLRAEDTAVYYCVRHGNFGNSYVSWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSEVQLVESGGGLVKPGGSLRLSCAASGFTFSNAWMSWVRQAPGKGLEWVGRIKSKTDGGTTDYAAPVKGRFTISRDDSKNTLYLQMNSLKTEDTAVYYCTTPWEWSWYDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 72 |
| FolR1 16D5 HC CH2527-VH3_23-12 HC Fc knob PG LALA,<br>pCON999 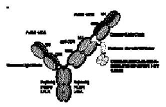 | EVQLVESGGGLVKPGGSLRLSCAASGFTFSNAWMSWVRQAPGKGLEWVGRIKSKTDGGTTDYAAPVKGRFTISRDDSKNTLYLQMNSLKTEDTAVYYCTTPWEWSWYDYWGQGTLVTVSSA<br><br>STKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSEVQLLESGGGLVQPGGSLRLSCAASGFTFSTYAMNWVRQAPGKGLEWVSRIRSKYNNYATYYADSVKGRFTISRDDSKNTLYLQMNSLRAEDTAVYYCVRHGNFGNSYVSWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 73 |

(continued)

| Construct | Amino acid Sequence | SEQ ID No |
|---|---|---|
| anti ID CD3 scFv 4.32.63 MK062 protease site CD3 VL CLambda, pETR16544 | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIKGGGGSGGGGSRQARVVNGGGGGSGGGGSGGGGSQAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS | 74 |
| anti ID CD3 scFv 4.32.63 non-cleavable linker CD3 VL CLambda, pETR16545 | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIKGGGGSGGGGSGGGGSGGGGGGGSGGGGSGGGGSQAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS | 75 |
| aMSLN RG7787 VH CH1 EE CD3 CH2527-VH3_23-12 VL CH1 Fc knob PG LALA, pETR15445 | QVQLVQSGAEVKKPGASVKVSCKASGYSFTGYTMNWVRQAPGQGLEWMGLITPYNGASSYNQKFRGKATMTVDTSTSTVYMELSSLRSEDTAVYYCARGGYDGRGFDYWGQGTLVTVSSASTKGPSVFPLAPSSKSTSGGTAALGCLVEDYFPEPVTVSWNSGALTSGVHTFPAVLQSSGLYSLSSVVTVPSSSLGTQTYICNVNHKPSNTKVDEKVEPKSCDGGGGSGGGGSQAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 76 |
| aMSLN RG7787 VH CH1EE Fc hole P329G LALA, pETR15444 | QVQLVQSGAEVKKPGASVKVSCKASGYSFTGYTMNWVRQAPGQGLEWMGLITPYNGASSYNQKFRGKATMTVDTSTSTVYMELSSLRSEDTAVYYCARGGYDGRGFDYWGQGTLVTVSSA | 77 |

(continued)

| Construct | Amino acid Sequence | SEQ ID No |
|---|---|---|
| | STKGPSVFPLAPSSKSTSGGTAALGCLVEDYFPEPVTVSWN SGALTSGVHTFPAVLQSSGLYSLSSVVTVPSSSLGTQTYICN VNHKPSNTKVDEKVEPKSCDKTHTCPPCPAPEAAGGPSVFL FPPKPKDTLMISRTPEVTCVVVDVSHEDPEVKFNWYVDGVE VHNAKTKPREEQYNSTYRVVSVLTVLHQDWLNGKEYKCKVS NKALGAPIEKTISKAKGQPREPQVCTLPPSRDELTKNQVSLS CAVKGFYPSDIAVEWESNGQPENNYKTTPPVLDSDGSFFLV SKLTVDKSRWQQGNVFSCSVMHEALHNHYTQKSLSLSPGK | |
| aMSLN RG7787 VL Ck RK, pETR15443 | DIQMTQSPSSLSASVGDRVTITCSASSSVSYMHWYQQKSGK APKLLIYDTSKLASGVPSRFSGSGSGTDFTLTISSLQPEDFAT YYCQQWSKHPLTFGQGTKLEIKRTVAAPSVFIFPPSDRKLKS GTASVVCLLNNFYPREAKVQWKVDNALQSGNSQESVTEQD SKDSTYSLSSTLTLSKADYEKHKVYACEVTHQGLSSPVTKSF NRGEC | 78 |
| anti ID CH2527 4.32.63 CD3 CH2527 VH 23-12 Ck, MMP9-MK062 site, pETR16758 | QVQLKESGPGLVAPSQSLSITCTVSGFSLTSYGVSWVRQPP GKCLEWLGIIWGDGSTNYHSALISRLSISKDNSKSQVFLKLNS LQTDDTATYYCAKGITTVVDDYYAMDYWGQGTSVTVSSGG GGSGGGGSGGGGSGGGGSDIQMTQSPASLSASVGETVTIT CRASENIDSYLAWYQQKQGKSPQLLVYAATFLADDVPSRFS GSGSGTQYSLKINSLQSEDVARYYCQHYYSTPYTFGCGTKL EIKGGGGSVHMPLGFLGPRQARVVNGGGGGSGGGGSEVQ LLESGGGLVQPGGSLRLSCAASGFTFSTYAMNWVRQAPGK GLEWVSRIRSKYNNYATYYADSVKGRFTISRDDSKNTLYLQ MNSLRAEDTAVYYCVRHGNFGNSYVSWFAYWGQGTLVTVS SASVAAPSVFIFPPSDEQLKSGTASVVCLLNNFYPREAKVQW KVDNALQSGNSQESVTEQDSKDSTYSLSSTLTLSKADYEKH KVYACEVTHQGLSSPVTKSFNRGEC | 79 |
| anti ID CH2527 4.32.63 CD3 CH2527 VH 23-12 Ck, non-cleavable linker, pETR16759 | QVQLKESGPGLVAPSQSLSITCTVSGFSLTSYGVSWVRQPP GKCLEWLGIIWGDGSTNYHSALISRLSISKDNSKSQVFLKLNS LQTDDTATYYCAKGITTVVDDYYAMDYWGQGTSVTVSSGG GGSGGGGSGGGGSGGGGSDIQMTQSPASLSASVGETVTIT CRASENIDSYLAWYQQKQGKSPQLLVYAATFLADDVPSRFS GSGSGTQYSLKINSLQSEDVARYYCQHYYSTPYTFGCGTKL EIKGGGGSGGGGSGGGGSGGGGGGGSGGGGSGGGGSEV QLLESGGGLVQPGGSLRLSCAASGFTFSTYAMNWVRQAPG KGLEWVSRIRSKYNNYATYYADSVKGRFTISRDDSKNTLYLQ MNSLRAEDTAVYYCVRHGNFGNSYVSWFAYWGQGTLVTVS SASVAAPSVFIFPPSDEQLKSGTASVVCLLNNFYPREAKVQW KVDNALQSGNSQESVTEQDSKDSTYSLSSTLTLSKADYEKH KVYACEVTHQGLSSPVTKSFNRGEC | 80 |
| CD3 CH2527 VH 23-12 - Ck, pETR13811 | EVQLLESGGGLVQPGGSLRLSCAASGFTFSTYAMNWVRQA PGKGLEWVSRIRSKYNNYATYYADSVKGRFTISRDDSKNTLY LQMNSLRAEDTAVYYCVRHGNFGNSYVSWFAYWGQGTLVT VSSASVAAPSVFIFPPSDEQLKSGTASVVCLLNNFYPREAKV QWKVDNALQSGNSQESVTEQDSKDSTYSLSSTLTLSKADYE KHKVYACEVTHQGLSSPVTKSFNRGEC | 81 |

(continued)

| Construct | Amino acid Sequence | SEQ ID No |
|---|---|---|
| anti CD3 (CH2527 VH_3-23 (12) VL7-46(13)) scFv 4.32.63 MMP9 Matriptase MK062 aMSLN VH CH1 EE CH2527-VL7_46-13CH1 hum Fc knob PG LALA, pETR16751 | QVQLKESGPGLVAPSQSLSITCTVSGFSLTSYGVSWVRQPP GKCLEWLGIIWGDGSTNYHSALISRLSISKDNSKSQVFLKLNS LQTDDTATYYCAKGITTVVDDYYAMDYWGQGTSVTVSSGG GGSGGGGSGGGGSGGGGSDIQMTQSPASLSASVGETVTIT CRASENIDSYLAWYQQKQGKSPQLLVYAATFLADDVPSRFS GSGSGTQYSLKINSLQSEDVARYYCQHYYSTPYTFGCGTKL EIKGGGGSVHMPLGFLGPRQARVVNGGGGGSGGGGSQAV VTQEPSLTVSPGGTVTLTCGSSTGAVTTSNYANWVQEKPG QAFRGLIGGTNKRAPGTPARFSGSLLGGKAALTLSGAQPED EAEYYCALWYSNLWVFGGGTKLTVLSSASTKGPSVFPLAPS SKSTSGGTAALGCLVKDYFPEPVTVSWNSGALTSGVHTFPA VLQSSGLYSLSSVVTVPSSSLGTQTYICNVNHKPSNTKVDKK VEPKSCDGGGGSGGGGSQVQLVQSGAEVKKPGASVKVSC KASGYSFTGYTMNWVRQAPGQGLEWMGLITPYNGASSYNQ KFRGKATMTVDTSTSTVYMELSSLRSEDTAVYYCARGGYDG RGFDYWGQGTLVTVSSASTKGPSVFPLAPSSKSTSGGTAAL GCLVEDYFPEPVTVSWNSGALTSGVHTFPAVLQSSGLYSLS SVVTVPSSSLGTQTYICNVNHKPSNTKVDEKVEPKSCDKTHT CPPCPAPEAAGGPSVFLFPPKPKDTLMISRTPEVTCVVVDVS HEDPEVKFNWYVDGVEVHNAKTKPREEQYNSTYRVVSVLT VLHQDWLNGKEYKCKVSNKALGAPIEKTISKAKGQPREPQV YTLPPCRDELTKNQVSLWCLVKGFYPSDIAVEWESNGQPEN NYKTTPPVLDSDGSFFLYSKLTVDKSRWQQGNVFSCSVMH EALHNHYTQKSLSLSPGK | 82 |
| anti CD3 (CH2527 VH_3-23 (12) VL7-46(13)) scFv 4.32.63 non-cleavable linker aMSLN VH CH1 EE CH2527-VL7_46-13 CH1 hum Fc knob PG LALA, pETR16752 | QVQLKESGPGLVAPSQSLSITCTVSGFSLTSYGVSWVRQPP GKCLEWLGIIWGDGSTNYHSALISRLSISKDNSKSQVFLKLNS LQTDDTATYYCAKGITTVVDDYYAMDYWGQGTSVTVSSGG GGSGGGGSGGGGSGGGGSDIQMTQSPASLSASVGETVTIT CRASENIDSYLAWYQQKQGKSPQLLVYAATFLADDVPSRFS GSGSGTQYSLKINSLQSEDVARYYCQHYYSTPYTFGCGTKL EIKGGGGSGGGGSGGGGSGGGGGGGSGGGGSGGGGSQ AVVTQEPSLTVSPGGTVTLTCGSSTGAVTTSNYANWVQEKP GQAFRGLIGGTNKRAPGTPARFSGSLLGGKAALTLSGAQPE DEAEYYCALWYSNLWVFGGGTKLTVLSSASTKGPSVFPLAP SSKSTSGGTAALGCLVKDYFPEPVTVSWNSGALTSGVHTFP AVLQSSGLYSLSSVVTVPSSSLGTQTYICNVNHKPSNTKVDK KVEPKSCDGGGGSGGGGSQVQLVQSGAEVKKPGASVKVS CKASGYSFTGYTMNWVRQAPGQGLEWMGLITPYNGASSYN QKFRGKATMTVDTSTSTVYMELSSLRSEDTAVYYCARGGYD GRGFDYWGQGTLVTVSSASTKGPSVFPLAPSSKSTSGGTA ALGCLVEDYFPEPVTVSWNSGALTSGVHTFPAVLQSSGLYS LSSVVTVPSSSLGTQTYICNVNHKPSNTKVDEKVEPKSCDKT HTCPPCPAPEAAGGPSVFLFPPKPKDTLMISRTPEVTCVVVD VSHEDPEVKFNWYVDGVEVHNAKTKPREEQYNSTYRVVSV LTVLHQDWLNGKEYKCKVSNKALGAPIEKTISKAKGQPREPQ VYTLPPCRDELTKNQVSLWCLVKGFYPSDIAVEWESNGQPE NNYKTTPPVLDSDGSFFLYSKLTVDKSRWQQGNVFSCSVM HEALHNHYTQKSLSLSPGK | 83 |

(continued)

| Construct | Amino acid Sequence | SEQ ID No |
|---|---|---|
| CH2527 XFab aMSLN RG7787 HC EE Fc knob PG LALA, pETR16764 | QAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSQVQLVQSGAEVKKPGASVKVSCKASGYSFTGYTMNWVRQAPGQGLEWMGLITPYNGASSYNQKFRGKATMTVDTSTSTVYMELSSLRSEDTAVYYCARGGYDGRGFDYWGQGTLVTVSSASTKGPSVFPLAPSSKSTSGGTAALGCLVEDYFPEPVTVSWNSGALTSGVHTFPAVLQSSGLYSLSSVVTVPSSSLGTQTYICNVNHKPSNTKVDEKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 84 |
| anti CD3 (CH2527 VH_3-23 (12) VL7-46(13)) scFv 4.32.63 Cathepsin S/B site CH2527 VH3_23-VH12 CH1 FolR1 16D5 VH CH1 hum Fc knob PG LALA, pETR16550 | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIKGGGGSGGGGSGGGGSFVGGTGGGGSGGGGSGGSEVQLLESGGGLVQPGGSLRLSCAASGFTFSTYAMNWVRQAPGKGLEWVSRIRSKYNNYATYYADSVKGRFTISRDDSKNTLYLQMNSLRAEDTAVYYCVRHGNFGNSYVSWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSEVQLVESGGGLVKPGGSLRLSCAASGFTFSNAWMSWVRQAPGKGLEWVGRIKSKTDGGTTDYAAPVKGRFTISRDDSKNTLYLQMNSLKTEDTAVYYCTTPWEWSWYDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 85 |
| Combined MMP9 MK062, 33 AA for CD3 | GGGGS**VHMPLGFLGPRQARVVNG**GGGGSGGGGS | 86 |
| Combined MMP9 MK062, 35 AA for Her1 | GGGGS**VHMPLGFLGPRQARVVNG**GGGGSGGGGSGG | 87 |
| Cathepsin S/B | GGGGSGGGGSGGGGS**FVGGTG**GGGSGGGGSGGS | 88 |
| KKAAPVNG | GGGGSGGGGS**KKAAPVNG**GGGGSGGGGSGGGGS | 89 |
| PMAKKVNG | GGGGSGGGGS**PMAKKVNG**GGGGSGGGGSGGGGS | 90 |
| QARAKVNG | GGGGSGGGGS**QARAKVNGG**GGGSGGGGSGGGGS | 91 |

(continued)

| Construct | Amino acid Sequence | SEQ ID No |
|---|---|---|
| MMP9 | GGGGSGGGGS**VHMPLGFLGP**GGGGSGGGGSGGS | 92 |
| QARAK | GGGGSGGGGS**QARAK**GGGGSGGGGSGGGGSGGS | 93 |
| MMP9-PMAKK | GGGGSVHMPLGFLGP**PMAKK**GGGGSGGGGSGGS | 94 |
| KKAAP | GGGGSGGGGS**KKAAP**GGGGSGGGGSGGGGSGGS | 95 |
| PMAKK | GGGGSGGGGS**PMAKK**GGGGSGGGGSGGGGSGGS | 96 |
| Protease recognition site 6 | VHMPLGFLGPRQARVVNG | 97 |
| Protease recognition site 7 | FVGGTG | 98 |
| Protease recognition site 8 | KKAAPVNG | 99 |
| Protease recognition site 9 | PMAKKVNG | 100 |
| Protease recognition site 10 | QARAKVNG | 101 |
| Protease recognition site 11 | VHMPLGFLGP | 102 |
| Protease recognition site 12 | QARAK | 103 |
| Protease recognition site 13 | VHMPLGFLGPPMAKK | 104 |
| Protease recognition site 14 | KKAAP | 105 |
| Protease recognition site 15 | PMAKK | 106 |
| aMSLN CDR H1 Kabat | GYTMN | 107 |
| aMSLN CDR H2 Kabat | LITPYNGASSYNQKFRG | 108 |
| aMSLN CDR H3 Kabat | GGYDGRGFDY | 109 |
| aMSLN CDR L1 Kabat | SASSSVSYMH | 110 |
| aMSLN CDR L2 Kabat | DTSKLAS | 111 |
| aMSLN CDR L3 Kabat | QQWSKHPLT | 112 |
| aMSLN VH | QVQLVQSGAEVKKPGASVKVSCKASGYSFTGYTMNWVRQA PGQGLEWMGLITPYNGASSYNQKFRGKATMTVDTSTSTVY MELSSLRSEDTAVYYCARGGYDGRGFDYWGQGTLVTVSS | 113 |
| aMSLN VL | DIQMTQSPSSLSASVGDRVTITCSASSSVSYMHWYQQKSGK APKLLIYDTSKLASGVPSRFSGSGSGTDFTLTISSLQPEDFAT YYCQQWSKHPLTFGQGTKLEIK | 114 |
| aHER1 VH | QVQLVQSGAEVKKPGSSVKVSCKASGFTFTDYKIHWVRQAP GQGLEWMGYFNPNSGYSTYAQKFQGRVTITADKSTSTAYM ELSSLRSEDTAVYYCARLSPGGYYVMDAWGQGTTVTVSS | 115 |
| aHER1 VL | DIQMTQSPSSLSASVGDRVTITCRASQGINNYLNWYQQKPGKA PKRLIYNTNNLQTGVPSRFSGSGSGTEFTLTISSLQPEDFATYYC LQHNSFPTFGQGTKLEIK | 116 |

| Construct | DNA Sequence | SEQ ID No |
|---|---|---|
| LC Common light chain pETR13197 | CAGGCCGTCGTGACCCAGGAACCCAGCCTGACAGTGTCTCCTGGC GGCACCGTGACCCTGACATGTGGCAGTTCTACAGGCGCCGTGACC ACCAGCAACTACGCCAACTGGGTGCAGGAAAAGCCCGGCCAGGCC TTCAGAGGACTGATCGGCGGCACCAACAAGAGAGCCCCTGGCACC CCTGCCAGATTCAGCGGATCTCTGCTGGGAGGAAAGGCCGCCCTG ACACTGTCTGGCGCCCAGCCAGAAGATGAGGCCGAGTACTACTGC GCCCTGTGGTACAGCAACCTGTGGGTGTTCGGCGGAGGCACCAAG CTGACAGTCCTAGGTCAACCCAAGGCTGCCCCCAGCGTGACCCTG TTCCCCCCCAGCAGCGAGGAACTGCAGGCCAACAAGGCCACCCTG GTCTGCCTGATCAGCGACTTCTACCCAGGCGCCGTGACCGTGGCC TGGAAGGCCGACAGCAGCCCCGTGAAGGCCGGCGTGGAGACCAC CACCCCCAGCAAGCAGAGCAACAACAAGTACGCCGCCAGCAGCTA CCTGAGCCTGACCCCCGAGCAGTGGAAGAGCCACAGGTCCTACAG CTGCCAGGTGACCCACGAGGGCAGCACCGTGGAGAAAACCGTGG CCCCCACCGAGTGCAGCTGA | 117 |
| anti CD3 (CH2527 VH_3-23(12) VL7-46 (13)) scFv 4.32.63 MMP9 Matriptase MK062 CH2527 VH3_23-VH12 CH1 FolR1 16D5 VH CH1 hum Fc knob PG LALA, pETR16546 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGAGGCGGCGGAAGTG TGCACATGCCCCTGGGCTTCCTGGGCCCCAGACAGGCCAGAGTCG | 118 |

(continued)

| Construct | DNA Sequence | SEQ ID No |
|---|---|---|
| 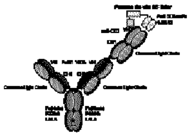 | TGAACGGGGGGGGCGGAGGCAGTGGGGGGGGAGGATCCGAGGT<br>GCAGCTGCTGGAATCTGGCGGCGGACTGGTGCAGCCTGGCGGAT<br>CTCTGAGACTGAGCTGTGCCGCCAGCGGCTTCACCTTCAGCACCT<br>ACGCCATGAACTGGGTGCGCCAGGCCCCTGGCAAAGGCCTGGAAT<br>GGGTGTCCCGGATCAGAAGCAAGTACAACAACTACGCCACCTACTA<br>CGCCGACAGCGTGAAGGGCCGGTTCACCATCAGCCGGGACGACA<br>GCAAGAACACCCTGTACCTGCAGATGAACAGCCTGCGGGCCGAGG<br>ACACCGCCGTGTACTATTGTGTGCGGCACGGCAACTTCGGCAACA<br>GCTATGTGTCTTGGTTTGCCTACTGGGGCCAGGGCACCCTCGTGA<br>CCGTGTCAAGCGCTAGCACAAAGGGCCCTAGCGTGTTCCCTCTGG<br>CCCCCAGCAGCAAGAGCACAAGCGGCGGAACAGCCGCCCTGGGC<br>TGCCTCGTGAAGGACTACTTCCCCGAGCCCGTGACAGTGTCTTGG<br>AACAGCGGAGCCCTGACAAGCGGCGTGCACACCTTCCCTGCCGTG<br>CTGCAGAGCAGCGGCCTGTACTCCCTGAGCAGCGTGGTCACCGTG<br>CCTAGCAGCAGCCTGGGCACCCAGACCTACATCTGCAACGTGAAC<br>CACAAGCCCAGCAACACCAAAGTGGACAAGAAGGTGGAGCCCAAG<br>AGCTGTGATGGCGGAGGAGGGTCCGGGGGCGGAGGATCCGAGGT<br>GCAATTGGTTGAATCTGGTGGTGGTCTGGTAAAACCGGGCGGTTC<br>CCTGCGTCTGAGCTGCGCGGCTTCCGGGTTCACCTTCTCCAACGC<br>GTGGATGAGCTGGGTTCGCCAGGCCCCGGGCAAAGGCCTCGAGT<br>GGGTTGGTCGTATCAAGTCTAAAACTGACGGTGGCACCACGGATTA<br>CGCGGCTCCAGTTAAAGGTCGTTTTACCATTTCCCGCGACGATAGC<br>AAAAACACTCTGTATCTGCAGATGAACTCTCTGAAAACTGAAGACAC<br>CGCAGTCTACTACTGTACTACCCCGTGGGAATGGTCTTGGTACGAT<br>TATTGGGGCCAGGGCACGCTGGTTACGGTGTCTAGCGCTAGTACC<br>AAGGGCCCCAGCGTGTTCCCCCTGGCACCCAGCAGCAAGAGCACA<br>TCTGGCGGAACAGCCGCTCTGGGCTGTCTGGTGAAAGACTACTTC<br>CCCGAGCCCGTGACCGTGTCTTGGAACTCTGGCGCCCTGACCAGC<br>GGCGTGCACACCTTTCCAGCCGTGCTGCAGAGCAGCGGCCTGTAC<br>TCCCTGTCCTCCGTGGTCACCGTGCCCTCTAGCTCCCTGGGAACA<br>CAGACATATATCTGTAATGTCAATCACAAGCCTTCCAACACCAAAGT<br>CGATAAGAAAGTCGAGCCCAAGAGCTGCGACAAAACTCACACATG<br>CCCACCGTGCCCAGCACCTGAAGCTGCAGGGGGACCGTCAGTCTT<br>CCTCTTCCCCCCAAAACCCAAGGACACCCTCATGATCTCCCGGACC<br>CCTGAGGTCACATGCGTGGTGGTGGACGTGAGCCACGAAGACCCT<br>GAGGTCAAGTTCAACTGGTACGTGGACGGCGTGGAGGTGCATAAT<br>GCCAAGACAAAGCCGCGGGAGGAGCAGTACAACAGCACGTACCGT<br>GTGGTCAGCGTCCTCACCGTCCTGCACCAGGACTGGCTGAATGGC<br>AAGGAGTACAAGTGCAAGGTCTCCAACAAAGCCCTCGGCGCCCCC<br>ATCGAGAAAACCATCTCCAAAGCCAAAGGGCAGCCCCGAGAACCA<br>CAGGTGTACACCCTGCCCCCATGCCGGGATGAGCTGACCAAGAAC<br>CAGGTCAGCCTGTGGTGCCTGGTCAAAGGCTTCTATCCCAGCGAC<br>ATCGCCGTGGAGTGGGAGAGCAATGGGCAGCCGGAGAACAACTAC<br>AAGACCACGCCTCCCGTGCTGGACTCCGACGGCTCCTTCTTCCTCT<br>ACAGCAAGCTCACCGTGGACAAGAGCAGGTGGCAGCAGGGGAAC<br>GTCTTCTCATGCTCCGTGATGCATGAGGCTCTGCACAACCACTACA<br>CGCAGAAGAGCCTCTCCCTGTCTCCGGGTAAATGA | |

(continued)

| Construct | DNA Sequence | SEQ ID No |
|---|---|---|
| FolR1 16D5 HC CH2527-VH3_23-12 HC Fc knob PG LALA, pCON999 | GAGGTGCAATTGGTTGAATCTGGTGGTGGTCTGGTAAAACCGGGC GGTTCCCTGCGTCTGAGCTGCGCGGCTTCCGGATTCACCTTCTCC AACGCGTGGATGAGCTGGGTTCGCCAGGCCCCGGGCAAAGGCCT CGAGTGGGTTGGTCGTATCAAGTCTAAAACTGACGGTGGCACCAC GGATTACGCGGCTCCAGTTAAAGGTCGTTTTACCATTTCCCGCGAC GATAGCAAAAACACTCTGTATCTGCAGATGAACTCTCTGAAAACTGA AGACACCGCAGTCTACTACTGTACTACCCCGTGGGAATGGTCTTGG TACGATTATTGGGGCCAGGGCACGCTGGTTACGGTGTCTTCCGCT AGCACAAAGGGCCCTAGCGTGTTCCCTCTGGCCCCCAGCAGCAAG AGCACAAGCGGCGGAACAGCCGCCCTGGGCTGCCTCGTGAAGGA CTACTTCCCCGAGCCCGTGACAGTGTCTTGGAACAGCGGAGCCCT GACAAGCGGCGTGCACACTTTCCCTGCCGTGCTGCAGAGCAGCGG CCTGTACTCCCTGAGCAGCGTGGTCACCGTGCCTAGCAGCAGCCT GGGCACCCAGACCTACATCTGCAACGTGAACCACAAGCCCAGCAA CACCAAAGTGGACAAGAAGGTGGAGCCCAAGAGCTGTGATGGCGG AGGAGGGTCCGGAGGCGGAGGATCCGAGGTGCAGCTGCTGGAAT CTGGCGGCGGACTGGTGCAGCCTGGCGGATCTCTGAGACTGAGCT GTGCCGCCAGCGGCTTCACCTTCAGCACCTACGCCATGAACTGGG TGCGCCAGGCCCCTGGCAAAGGCCTGGAATGGGTGTCCCGGATCA GAAGCAAGTACAACAACTACGCCACCTACTACGCCGACAGCGTGA AGGGCCGGTTCACCATCAGCCGGGACGACAGCAAGAACACCCTGT ACCTGCAGATGAACAGCCTGCGGGCCGAGGACACCGCCGTGTACT ATTGTGTGCGGCACGGCAACTTCGGCAACAGCTATGTGTCTTGGTT TGCCTACTGGGGCCAGGGCACCCTCGTGACCGTGTCAAGCGCTAG TACCAAGGGCCCCAGCGTGTTCCCCCTGGCACCCAGCAGCAAGAG CACATCTGGCGGAACAGCCGCTCTGGGCTGTCTGGTGAAAGACTA CTTCCCCGAGCCCGTGACCGTGTCTTGGAACTCTGGCGCCCTGAC CAGCGGCGTGCACACCTTTCCAGCCGTGCTGCAGAGCAGCGGCCT GTACTCCCTGTCCTCCGTGGTCACCGTGCCCTCTAGCTCCCTGGG AACACAGACATATATCTGTAATGTCAATCACAAGCCTTCCAACACCA AAGTCGATAAGAAAGTCGAGCCCAAGAGCTGCGACAAAACTCACAC ATGCCCACCGTGCCCAGCACCTGAAGCTGCAGGGGGACCGTCAGT CTTCCTCTTCCCCCCAAAACCCAAGGACACCCTCATGATCTCCCGG ACCCCTGAGGTCACATGCGTGGTGGTGGACGTGAGCCACGAAGAC CCTGAGGTCAAGTTCAACTGGTACGTGGACGGCGTGGAGGTGCAT AATGCCAAGACAAAGCCGCGGGAGGAGCAGTACAACAGCACGTAC CGTGTGGTCAGCGTCCTCACCGTCCTGCACCAGGACTGGCTGAAT GGCAAGGAGTACAAGTGCAAGGTCTCCAACAAAGCCCTCGGCGCC CCCATCGAGAAAACCATCTCCAAAGCCAAAGGGCAGCCCCGAGAA CCACAGGTGTACACCCTGCCCCCATGCCGGGATGAGCTGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTT CCTCTACAGCAAGCTCACCGTGGACAAGAGCAGGTGGCAGCAGGG GAACGTCTTCTCATGCTCCGTGATGCATGAGGCTCTGCACAACCAC TACACGCAGAAGAGCCTCTCCCTGTCTCCGGGTAAATGA | 119 |

(continued)

| Construct | DNA Sequence | SEQ ID No |
|---|---|---|
| anti ID CD3 scFv 4.32.63 MK062 protease site CD3 VL CLambda, pETR16544 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGCGGGGGAGGCTCCG GAGGCGGCGGAAGTAGACAGGCCAGAGTCGTGAACGGGGGAGGG GGGGGAAGTGGGGGCGGAGGCAGTGGGGGCGGAGGATCCCAGG CCGTCGTGACCCAGGAACCCAGCCTGACAGTGTCTCCTGGCGGCA CCGTGACCCTGACATGTGGCAGTTCTACAGGCGCCGTGACCACCA GCAACTACGCCAACTGGGTGCAGGAAAAGCCCGGCCAGGCCTTCA GAGGACTGATCGGCGGCACCAACAAGAGAGCCCCTGGCACCCCT GCCAGATTCAGCGGATCTCTGCTGGGAGGAAAGGCCGCCCTGACA CTGTCTGGCGCCCAGCCAGAAGATGAGGCCGAGTACTACTGCGCC CTGTGGTACAGCAACCTGTGGGTGTTCGGCGGAGGCACCAAGCTG ACAGTCCTAGGTCAACCCAAGGCTGCCCCCAGCGTGACCCTGTTC CCCCCCAGCAGCGAGGAACTGCAGGCCAACAAGGCCACCCTGGT CTGCCTGATCAGCGACTTCTACCCAGGCGCCGTGACCGTGGCCTG GAAGGCCGACAGCAGCCCCGTGAAGGCCGGCGTGGAGACCACCA CCCCCAGCAAGCAGAGCAACAACAAGTACGCCGCCAGCAGCTACC TGAGCCTGACCCCCGAGCAGTGGAAGAGCCACAGGTCCTACAGCT GCCAGGTGACCCACGAGGGCAGCACCGTGGAGAAAACCGTGGCC CCCACCGAGTGCAGCTGA | 120 |

(continued)

| Construct | DNA Sequence | SEQ ID No |
|---|---|---|
| anti ID CD3 scFv 4.32.63 non-cleavable linker CD3 VL CLambda, pETR16545 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGCGGGGGAGGCTCCG GAGGCGGCGGAAGTGGAGGCGGCGGAAGTGGCGGAGGCGGAGG GGGGGGAAGTGGGGGCGGAGGCAGTGGGGGGGGAGGATCCCAG GCCGTCGTGACCCAGGAACCCAGCCTGACAGTGTCTCCTGGCGGC ACCGTGACCCTGACATGTGGCAGTTCTACAGGCGCCGTGACCACC AGCAACTACGCCAACTGGGTGCAGGAAAAGCCCGGCCAGGCCTTC AGAGGACTGATCGGCGGCACCAACAAGAGAGCCCCTGGCACCCCT GCCAGATTCAGCGGATCTCTGCTGGGAGGAAAGGCCGCCCTGACA CTGTCTGGCGCCCAGCCAGAAGATGAGGCCGAGTACTACTGCGCC CTGTGGTACAGCAACCTGTGGGTGTTCGGCGGAGGCACCAAGCTG ACAGTCCTAGGTCAACCCAAGGCTGCCCCCAGCGTGACCCTGTTC CCCCCCAGCAGCGAGGAACTGCAGGCCAACAAGGCCACCCTGGT CTGCCTGATCAGCGACTTCTACCCAGGCGCCGTGACCGTGGCCTG GAAGGCCGACAGCAGCCCCGTGAAGGCCGGCGTGGAGACCACCA CCCCCAGCAAGCAGAGCAACAACAAGTACGCCGCCAGCAGCTACC TGAGCCTGACCCCCGAGCAGTGGAAGAGCCACAGGTCCTACAGCT GCCAGGTGACCCACGAGGGCAGCACCGTGGAGAAAACCGTGGCC CCCACCGAGTGCAGCTGA | 121 |
| aMSLN RG7787 VH CH1 EE CD3 CH2527-VH3_23-12 VL CH1 Fc knob PG LALA, pETR15445 | CAGGTGCAGCTGGTGCAGTCTGGCGCCGAAGTGAAGAAACCAGGC GCCAGCGTGAAGGTGTCCTGCAAGGCCAGCGGCTACAGCTTCACC GGCTACACCATGAACTGGGTGCGCCAGGCTCCTGGACAGGGCCTG GAATGGATGGGCCTGATCACCCCCTACAACGGCGCCAGCAGCTAC AACCAGAAGTTCCGGGGCAAGGCCACCATGACCGTGGACACCAGC ACCTCCACCGTGTATATGGAACTGAGCAGCCTGCGGAGCGAGGAC ACCGCCGTGTACTATTGTGCCAGAGGCGGCTACGACGGCAGAGGC TTCGATTATTGGGGCCAGGGCACCCTCGTGACCGTGTCCTCTGCTA GCACCAAGGGCCCCTCCGTGTTTCCTCTGGCCCCTTCCAGCAAGT CCACCTCTGGCGGAACTGCCGCTCTGGGCTGCCTGGTGGAAGATT ACTTCCCCGAGCCCGTGACCGTGTCCTGGAATTCTGGCGCTCTGA CCTCCGGCGTGCACACCTTTCCAGCTGTGCTGCAGTCCTCCGGCC TGTACTCCCTGTCCTCCGTCGTGACAGTGCCCTCCAGCTCTCTGGG CACCCAGACCTACATCTGCAACGTGAACCACAAGCCCTCCAACACC AAGGTGGACGAGAAGGTGGAACCCAAGTCCTGCGACGGTGGCGG AGGTTCCGGAGGCGGAGGATCCCAGGCTGTCGTGACCCAGGAAC CCTCCCTGACAGTGTCTCCTGGCGGCACCGTGACCCTGACCTGTG GATCTTCTACCGGCGCTGTGACCACCTCCAACTACGCCAATTGGGT GCAGGAAAAGCCCGGCCAGGCCTTCAGAGGACTGATCGGCGGCA | 122 |

(continued)

| Construct | DNA Sequence | SEQ ID No |
|---|---|---|
| | CCAACAAGAGAGCCCCTGGCACCCCTGCCAGATTCTCCGGTTCTC TGCTGGGCGGCAAGGCTGCCCTGACTCTGTCTGGTGCTCAGCCTG AGGACGAGGCCGAGTACTACTGCGCCCTGTGGTACTCCAACCTGT GGGTGTTCGGCGGAGGCACCAAGCTGACCGTGCTGTCCAGCGCTT CCACCAAGGGACCCAGTGTGTTCCCCCTGGCCCCCAGCTCCAAGT CTACATCCGGTGGCACAGCTGCCCTGGGATGTCTCGTGAAGGACT ACTTTCCTGAGCCTGTGACAGTGTCTTGGAACAGCGGAGCCCTGA CCAGCGGAGTGCACACATTCCCTGCAGTGCTGCAGAGCAGCGGCC TGTATAGCCTGAGCAGCGTCGTGACCGTGCCTTCCTCTAGCCTGG GAACACAGACATATATCTGTAATGTGAATCATAAGCCCAGTAATACC AAAGTGGATAAGAAAGTGGAACCTAAGAGCTGCGATAAGACCCACA CCTGTCCCCCCTGCCCTGCTCCTGAAGCTGCTGGTGGCCCTAGCG TGTTCCTGTTCCCCCCAAAGCCCAAGGACACCCTGATGATCTCCCG GACCCCCGAAGTGACCTGCGTGGTGGTGGATGTGTCCCACGAGGA CCCTGAAGTGAAGTTCAATTGGTACGTGGACGGCGTGGAAGTGCA CAACGCCAAGACCAAGCCTAGAGAGGAACAGTACAACTCCACCTA CCGGGTGGTGTCCGTGCTGACAGTGCTGCACCAGGACTGGCTGAA CGGCAAAGAGTACAAGTGCAAGGTGTCCAACAAGGCCCTGGGCGC TCCCATCGAAAAGACCATCTCCAAGGCCAAGGGCCAGCCCCGGGA ACCCCAGGTGTACACCCTGCCCCCATGCCGGGATGAGCTGACCAA GAACCAGGTCAGCCTGTGGTGCCTGGTCAAAGGCTTCTATCCCAG CGACATCGCCGTGGAGTGGGAGAGCAATGGGCAGCCGGAGAACA ACTACAAGACCACGCCTCCCGTGCTGGACTCCGACGGCTCCTTCTT CCTCTACAGCAAGCTCACCGTGGACAAGAGCAGGTGGCAGCAGGG GAACGTCTTCTCATGCTCCGTGATGCATGAGGCTCTGCACAACCAC TACACGCAGAAGAGCCTCTCCCTGTCTCCGGGTAAATGA | |

(continued)

| Construct | DNA Sequence | SEQ ID No |
|---|---|---|
| aMSLN RG7787 VH CH1EE Fc hole P329G LALA, pETR15444 | CAGGTGCAGCTGGTGCAGTCTGGCGCCGAAGTGAAGAAACCAGGC GCCAGCGTGAAGGTGTCCTGCAAGGCCAGCGGCTACAGCTTCACC GGCTACACCATGAACTGGGTGCGCCAGGCTCCTGGACAGGGCCTG GAATGGATGGGCCTGATCACCCCCTACAACGGCGCCAGCAGCTAC AACCAGAAGTTCCGGGGCAAGGCCACCATGACCGTGGACACCAGC ACCTCCACCGTGTATATGGAACTGAGCAGCCTGCGGAGCGAGGAC ACCGCCGTGTACTATTGTGCCAGAGGCGGCTACGACGGCAGAGGC TTCGATTATTGGGGCCAGGGCACCCTCGTGACCGTGTCCTCTGCTA GCACCAAGGGCCCCTCCGTGTTCCCCCTGGCCCCCAGCAGCAAGA GCACCAGCGGCGGCACAGCCGCTCTGGGCTGCCTGGTCGAGGAC TACTTCCCCGAGCCCGTGACCGTGTCCTGGAACAGCGGAGCCCTG ACCTCCGGCGTGCACACCTTCCCCGCCGTGCTGCAGAGTTCTGGC CTGTATAGCCTGAGCAGCGTGGTCACCGTGCCTTCTAGCAGCCTG GGCACCCAGACCTACATCTGCAACGTGAACCACAAGCCCAGCAAC ACCAAGGTGGACGAGAAGGTGGAGCCCAAGAGCTGCGACAAAACT CACACATGCCCACCGTGCCCAGCACCTGAAGCTGCAGGGGGACC GTCAGTCTTCCTCTTCCCCCCAAAACCCAAGGACACCCTCATGATC TCCCGGACCCCTGAGGTCACATGCGTGGTGGTGGACGTGAGCCAC GAAGACCCTGAGGTCAAGTTCAACTGGTACGTGGACGGCGTGGAG GTGCATAATGCCAAGACAAAGCCGCGGGAGGAGCAGTACAACAGC ACGTACCGTGTGGTCAGCGTCCTCACCGTCCTGCACCAGGACTGG CTGAATGGCAAGGAGTACAAGTGCAAGGTCTCCAACAAAGCCCTC GGCGCCCCCATCGAGAAAACCATCTCCAAAGCCAAAGGGCAGCCC CGAGAACCACAGGTGTGCACCCTGCCCCCATCCCGGGATGAGCTG ACCAAGAACCAGGTCAGCCTCTCGTGCGCAGTCAAAGGCTTCTATC CCAGCGACATCGCCGTGGAGTGGGAGAGCAATGGGCAGCCGGAG AACAACTACAAGACCACGCCTCCCGTGCTGGACTCCGACGGCTCC TTCTTCCTCGTGAGCAAGCTCACCGTGGACAAGAGCAGGTGGCAG CAGGGGAACGTCTTCTCATGCTCCGTGATGCATGAGGCTCTGCAC AACCACTACACGCAGAAGAGCCTCTCCCTGTCTCCGGGTAAATGA | 123 |
| aMSLN RG7787 VL Ck RK, pETR15443 | GACATCCAGATGACCCAGAGCCCCAGCAGCCTGTCTGCCAGCGTG GGCGACAGAGTGACCATCACCTGTAGCGCCAGCAGCAGCGTGTCC TACATGCACTGGTATCAGCAGAAGTCCGGCAAGGCCCCCAAGCTG CTGATCTACGACACCAGCAAGCTGGCCTCCGGCGTGCCCAGCAGA TTTTCTGGCAGCGGCTCCGGCACCGACTTCACCCTGACAATCAGCT CCCTCCAGCCCGAGGACTTCGCCACCTACTACTGCCAGCAGTGGT CCAAGCACCCCCTGACCTTTGGCCAGGGCACCAAGCTGGAAATCA AGCGTACGGTGGCTGCACCATCTGTCTTCATCTTCCCGCCATCTGA TCGGAAGTTGAAATCTGGAACTGCCTCTGTTGTGTGCCTGCTGAAT AACTTCTATCCCAGAGAGGCCAAAGTACAGTGGAAGGTGGATAACG CCCTCCAATCGGGTAACTCCCAGGAGAGTGTCACAGAGCAGGACA GCAAGGACAGCACCTACAGCCTCAGCAGCACCCTGACGCTGAGCA AAGCAGACTACGAGAAACACAAAGTCTACGCCTGCGAAGTCACCCA TCAGGGCCTGAGCTCGCCCGTCACAAAGAGCTTCAACAGGGGAGA GTGTTAG | 124 |

(continued)

| Construct | DNA Sequence | SEQ ID No |
|---|---|---|
| anti ID CH2527 4.32.63 CD3 CH2527 VH 23-12 Ck, MMP9-MK062 site, pETR16758 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGAGGCGGCGGAAGTG TGCACATGCCCCTGGGCTTCCTGGGCCCCAGACAGGCCAGAGTCG TGAACGGGGGGGGCGGAGGCAGTGGGGGGGGAGGATCCGAGGT GCAGCTGCTGGAATCTGGCGGCGGACTGGTGCAGCCTGGCGGAT CTCTGAGACTGAGCTGTGCCGCCAGCGGCTTCACCTTCAGCACCT ACGCCATGAACTGGGTGCGCCAGGCCCCTGGCAAAGGCCTGGAAT GGGTGTCCCGGATCAGAAGCAAGTACAACAACTACGCCACCTACTA CGCCGACAGCGTGAAGGGCCGGTTCACCATCAGCCGGGACGACA GCAAGAACACCCTGTACCTGCAGATGAACAGCCTGCGGGCCGAGG ACACCGCCGTGTACTATTGTGTGCGGCACGGCAACTTCGGCAACA GCTATGTGTCTTGGTTTGCCTACTGGGGCCAGGGCACCCTCGTGA CCGTGTCAAGCGCTAGCGTGGCCGCTCCCTCCGTGTTCATCTTCC CACCTTCCGACGAGCAGCTGAAGTCCGGCACCGCTTCTGTCGTGT GCCTGCTGAACAACTTCTACCCCCGCGAGGCCAAGGTGCAGTGGA AGGTGGACAACGCCCTGCAGTCCGGCAACAGCCAGGAATCCGTGA CCGAGCAGGACTCCAAGGACAGCACCTACTCCCTGTCCTCCACCC TGACCCTGTCCAAGGCCGACTACGAGAAGCACAAGGTGTACGCCT GCGAAGTGACCCACCAGGGCCTGTCTAGCCCCGTGACCAAGTCTT TCAACCGGGGCGAGTGCTGA | 125 |
| anti ID CH2527 4.32.63 CD3 CH2527 VH 23-12 Ck, non-cleavable linker, pETR16759 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG | 126 |

(continued)

| Construct | DNA Sequence | SEQ ID No |
|---|---|---|
| | GCACACAGTACAGCCTGAAGATCAACTCCCTGCAGAGCGAGGACGTGGCCCGGTACTACTGCCAGCACTACTACAGCACCCCCTACACCTTCGGCTGCGGCACCAAGCTGGAAATCAAAGGCGGGGGAGGCTCCGGAGGCGGCGGAAGTGGAGGCGGCGGAAGTGGCGGAGGCGGAGGGGGGGGAAGTGGGGGCGGAGGCAGTGGGGGGGGAGGATCCGAGGTGCAGCTGCTGGAATCTGGCGGCGGACTGGTGCAGCCTGGCGGATCTCTGAGACTGAGCTGTGCCGCCAGCGGCTTCACCTTCAGCACCTACGCCATGAACTGGGTGCGCCAGGCCCCTGGCAAAGGCCTGGAATGGGTGTCCCGGATCAGAAGCAAGTACAACAACTACGCCACCTACTACGCCGACAGCGTGAAGGGCCGGTTCACCATCAGCCGGGACGACAGCAAGAACACCCTGTACCTGCAGATGAACAGCCTGCGGGCCGAGGACACCGCCGTGTACTATTGTGTGCGGCACGGCAACTTCGGCAACAGCTATGTGTCTTGGTTTGCCTACTGGGGCCAGGGCACCCTCGTGACCGTGTCAAGCGCTAGCGTGGCCGCTCCCTCCGTGTTCATCTTCCCACCTTCCGACGAGCAGCTGAAGTCCGGCACCGCTTCTGTCGTGTGCCTGCTGAACAACTTCTACCCCCGCGAGGCCAAGGTGCAGTGGAAGGTGGACAACGCCCTGCAGTCCGGCAACAGCCAGGAATCCGTGACCGAGCAGGACTCCAAGGACAGCACCTACTCCCTGTCCTCCACCCTGACCCTGTCCAAGGCCGACTACGAGAAGCACAAGGTGTACGCCTGCGAAGTGACCCACCAGGGCCTGTCTAGCCCCGTGACCAAGTCTTTCAACCGGGGCGAGTGCTGA | |
| CD3 CH2527 VH 23-12 - Ck, pETR13811 | GAAGTGCAGCTGCTGGAATCCGGCGGAGGACTGGTGCAGCCTGGCGGATCTCTGAGACTGTCTTGTGCCGCCTCCGGCTTCACCTTCTCCACCTACGCCATGAACTGGGTGCGACAGGCTCCTGGCAAGGGCCTGGAATGGGTGTCCCGGATCAGATCCAAGTACAACAACTACGCCACCTACTACGCCGACTCCGTGAAGGGCCGGTTCACCATCTCTCGGGACGACTCCAAGAACACCCTGTACCTGCAGATGAACTCCCTGCGGGCCGAGGACACCGCCGTGTACTATTGTGTGCGGCACGGCAACTTCGGCAACTCCTATGTGTCTTGGTTTGCCTACTGGGGCCAGGGCACCCTCGTGACCGTGTCATCTGCTAGCGTGGCCGCTCCCTCCGTGTTCATCTTCCCACCTTCCGACGAGCAGCTGAAGTCCGGCACCGCTTCTGTCGTGTGCCTGCTGAACAACTTCTACCCCCGCGAGGCCAAGGTGCAGTGGAAGGTGGACAACGCCCTGCAGTCCGGCAACAGCCAGGAATCCGTGACCGAGCAGGACTCCAAGGACAGCACCTACTCCCTGTCCTCCACCCTGACCCTGTCCAAGGCCGACTACGAGAAGCACAAGGTGTACGCCTGCGAAGTGACCCACCAGGGCCTGTCTAGCCCCGTGACCAAGTCTTTCAACCGGGGCGAGTGCTGA | 127 |

(continued)

| Construct | DNA Sequence | SEQ ID No |
|---|---|---|
| anti CD3 (CH2527 VH_3-23(12) VL7-46 (13)) scFv 4.32.63 MMP9 Matriptase MK062 aMSLN VH CH1 EE CH2527-VL7_46-13 CH1 hum Fc knob PG LALA, pETR16751 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGAGGCGGCGGAAGTG TGCACATGCCCCTGGGCTTCCTGGGCCCCAGACAGGCCAGAGTCG TGAACGGGGGGGGCGGAGGCAGTGGGGGGGGAGGATCCCAGGC CGTCGTGACCCAGGAACCCAGCCTGACAGTGTCTCCTGGCGGCAC CGTGACCCTGACATGTGGCAGTTCTACAGGCGCCGTGACCACCAG CAACTACGCCAACTGGGTGCAGGAAAAGCCCGGCCAGGCCTTCAG AGGACTGATCGGCGGCACCAACAAGAGAGCCCCTGGCACCCCTGC | 128 |

(continued)

| Construct | DNA Sequence | SEQ ID No |
|---|---|---|
| | CAGATTCAGCGGATCTCTGCTGGGAGGAAAGGCCGCCCTGACACT GTCTGGCGCCCAGCCAGAAGATGAGGCCGAGTACTACTGCGCCCT GTGGTACAGCAACCTGTGGGTGTTCGGCGGAGGCACCAAGCTGAC AGTGCTGAGCAGCGCTTCCACCAAGGGACCCAGTGTGTTCCCCCT GGCCCCCAGCTCCAAGTCTACATCCGGTGGCACAGCTGCCCTGGG ATGTCTCGTGAAGGACTACTTTCCTGAGCCTGTGACAGTGTCTTGG AACAGCGGAGCCCTGACCAGCGGAGTGCACACATTCCCTGCAGTG CTGCAGAGCAGCGGCCTGTATAGCCTGAGCAGCGTCGTGACCGTG CCTTCCTCTAGCCTGGGAACACAGACATATATCTGTAATGTGAATCA TAAGCCCAGTAATACCAAAGTGGATAAGAAAGTGGAACCTAAGAGC TGCGATGGCGGAGGAGGGTCCGGAGGCGGAGGGTCCCAGGTGCA GCTGGTGCAGTCTGGCGCCGAAGTGAAGAAACCAGGCGCCAGCG TGAAGGTGTCCTGCAAGGCCAGCGGCTACAGCTTCACCGGCTACA CCATGAACTGGGTGCGCCAGGCTCCTGGACAGGGCCTGGAATGGA TGGGCCTGATCACCCCCTACAACGGCGCCAGCAGCTACAACCAGA AGTTCCGGGGCAAGGCCACCATGACCGTGGACACCAGCACCTCCA CCGTGTATATGGAACTGAGCAGCCTGCGGAGCGAGGACACCGCCG TGTACTATTGTGCCAGAGGCGGCTACGACGGCAGAGGCTTCGATT ATTGGGGCCAGGGCACCCTCGTGACCGTGTCCTCTGCTAGCACCA AGGGCCCCTCCGTGTTCCCCCTGGCCCCCAGCAGCAAGAGCACCA GCGGCGGCACAGCCGCTCTGGGCTGCCTGGTCGAGGACTACTTC CCCGAGCCCGTGACCGTGTCCTGGAACAGCGGAGCCCTGACCTCC GGCGTGCACACCTTCCCCGCCGTGCTGCAGAGTTCTGGCCTGTAT AGCCTGAGCAGCGTGGTCACCGTGCCTTCTAGCAGCCTGGGCACC CAGACCTACATCTGCAACGTGAACCACAAGCCCAGCAACACCAAG GTGGACGAGAAGGTGGAGCCCAAGAGCTGCGACAAAACTCACACA TGCCCACCGTGCCCAGCACCTGAAGCTGCAGGGGGACCGTCAGTC TTCCTCTTCCCCCCAAAACCCAAGGACACCCTCATGATCTCCCGGA CCCCTGAGGTCACATGCGTGGTGGTGGACGTGAGCCACGAAGACC CTGAGGTCAAGTTCAACTGGTACGTGGACGGCGTGGAGGTGCATA ATGCCAAGACAAAGCCGCGGGAGGAGCAGTACAACAGCACGTACC GTGTGGTCAGCGTCCTCACCGTCCTGCACCAGGACTGGCTGAATG GCAAGGAGTACAAGTGCAAGGTCTCCAACAAAGCCCTCGGCGCCC CCATCGAGAAAACCATCTCCAAAGCCAAAGGGCAGCCCCGAGAAC CACAGGTGTACACCCTGCCCCCATGCCGGGATGAGCTGACCAAGA ACCAGGTCAGCCTGTGGTGCCTGGTCAAAGGCTTCTATCCCAGCG ACATCGCCGTGGAGTGGGAGAGCAATGGGCAGCCGGAGAACAACT ACAAGACCACGCCTCCCGTGCTGGACTCCGACGGCTCCTTCTTCC TCTACAGCAAGCTCACCGTGGACAAGAGCAGGTGGCAGCAGGGGA ACGTCTTCTCATGCTCCGTGATGCATGAGGCTCTGCACAACCACTA CACGCAGAAGAGCCTCTCCCTGTCTCCGGGTAAATGA | |

(continued)

| Construct | DNA Sequence | SEQ ID No |
|---|---|---|
| anti CD3 (CH2527 VH_3-23(12) VL7-46 (13)) scFv 4.32.63 non-cleavable linker aMSLN VH CH1 EE CH2527-VL7_46-13 CH1 hum Fc knob PG LALA, pETR16752 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGCGGGGGAGGCTCCG GAGGCGGCGGAAGTGGAGGCGGCGGAAGTGGCGGAGGCGGAGG GGGGGGAAGTGGGGGCGGAGGCAGTGGGGGGGGAGGATCCCAG | 129 |

(continued)

| Construct | DNA Sequence | SEQ ID No |
|---|---|---|
| | GCCGTCGTGACCCAGGAACCCAGCCTGACAGTGTCTCCTGGCGGCACCGTGACCCTGACATGTGGCAGTTCTACAGGCGCCGTGACCACCAGCAACTACGCCAACTGGGTGCAGGAAAAGCCCGGCCAGGCCTTCAGAGGACTGATCGGCGGCACCAACAAGAGAGCCCCTGGCACCCCTGCCAGATTCAGCGGATCTCTGCTGGGAGGAAAGGCCGCCCTGACACTGTCTGGCGCCCAGCCAGAAGATGAGGCCGAGTACTACTGCGCCCTGTGGTACAGCAACCTGTGGGTGTTCGGCGGAGGCACCAAGCTGACAGTGCTGAGCAGCGCTTCCACCAAGGGACCCAGTGTGTTCCCCCTGGCCCCCAGCTCCAAGTCTACATCCGGTGGCACAGCTGCCCTGGGATGTCTCGTGAAGGACTACTTTCCTGAGCCTGTGACAGTGTCTTGGAACAGCGGAGCCCTGACCAGCGGAGTGCACACATTCCCTGCAGTGCTGCAGAGCAGCGGCCTGTATAGCCTGAGCAGCGTCGTGACCGTGCCTTCCTCTAGCCTGGGAACACAGACATATATCTGTAATGTGAATCATAAGCCCAGTAATACCAAAGTGGATAAGAAAGTGGAACCTAAGAGCTGCGATGGCGGAGGAGGGTCCGGAGGCGGAGGGTCCCAGGTGCAGCTGGTGCAGTCTGGCGCCGAAGTGAAGAAACCAGGCGCCAGCGTGAAGGTGTCCTGCAAGGCCAGCGGCTACAGCTTCACCGGCTACACCATGAACTGGGTGCGCCAGGCTCCTGGACAGGGCCTGGAATGGATGGGCCTGATCACCCCCTACAACGGCGCCAGCAGCTACAACCAGAAGTTCCGGGGCAAGGCCACCATGACCGTGGACACCAGCACCTCCACCGTGTATATGGAACTGAGCAGCCTGCGGAGCGAGGACACCGCCGTGTACTATTGTGCCAGAGGCGGCTACGACGGCAGAGGCTTCGATTATTGGGGCCAGGGCACCCTCGTGACCGTGTCCTCTGCTAGCACCAAGGGCCCCTCCGTGTTCCCCCTGGCCCCCAGCAGCAAGAGCACCAGCGGCGGCACAGCCGCTCTGGGCTGCCTGGTCGAGGACTACTTCCCCGAGCCCGTGACCGTGTCCTGGAACAGCGGAGCCCTGACCTCCGGCGTGCACACCTTCCCCGCCGTGCTGCAGAGTTCTGGCCTGTATAGCCTGAGCAGCGTGGTCACCGTGCCTTCTAGCAGCCTGGGCACCCAGACCTACATCTGCAACGTGAACCACAAGCCCAGCAACACCAAGGTGGACGAGAAGGTGGAGCCCAAGAGCTGCGACAAAACTCACACATGCCCACCGTGCCCAGCACCTGAAGCTGCAGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCGGCGCCCCCATCGAGAAAACCATCTCCAAAGCCAAAGGGCAGCCCCGAGAACCACAGGTGTACACCCTGCCCCCATGCCGGGATGAGCTGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAATGA | |

(continued)

| Construct | DNA Sequence | SEQ ID No |
|---|---|---|
| CH2527 XFab aMSLN RG7787 HC EE Fc knob PG LALA, pETR16764 | CAGGCCGTCGTGACCCAGGAACCCAGCCTGACAGTGTCTCCTGGC GGCACCGTGACCCTGACATGTGGCAGTTCTACAGGCGCCGTGACC ACCAGCAACTACGCCAACTGGGTGCAGGAAAAGCCCGGCCAGGCC TTCAGAGGACTGATCGGCGGCACCAACAAGAGAGCCCCTGGCACC CCTGCCAGATTCTCCGGTTCTCTGCTGGGCGGCAAGGCTGCCCTG ACTCTGTCTGGTGCTCAGCCTGAGGACGAGGCCGAGTACTACTGC GCCCTGTGGTACTCCAACCTGTGGGTGTTCGGCGGAGGCACCAAG CTGACCGTGCTGTCCAGCGCTTCCACCAAGGGACCCAGTGTGTTC CCCCTGGCCCCCAGCTCCAAGTCTACATCCGGTGGCACAGCTGCC CTGGGATGTCTCGTGAAGGACTACTTTCCTGAGCCTGTGACAGTGT CTTGGAACAGCGGAGCCCTGACCAGCGGAGTGCACACATTCCCTG CAGTGCTGCAGAGCAGCGGCCTGTATAGCCTGAGCAGCGTCGTGA CCGTGCCTTCCTCTAGCCTGGGAACACAGACATATATCTGTAATGT GAATCATAAGCCCAGTAATACCAAAGTGGATAAGAAAGTGGAACCT AAGAGCTGCGATGGCGGAGGAGGGTCCGGAGGCGGAGGGTCCCA GGTGCAGCTGGTGCAGTCTGGCGCCGAAGTGAAGAAACCAGGCG CCAGCGTGAAGGTGTCCTGCAAGGCCAGCGGCTACAGCTTCACCG GCTACACCATGAACTGGGTGCGCCAGGCTCCTGGACAGGGCCTGG AATGGATGGGCCTGATCACCCCCTACAACGGCGCCAGCAGCTACA ACCAGAAGTTCCGGGGCAAGGCCACCATGACCGTGGACACCAGCA CCTCCACCGTGTATATGGAACTGAGCAGCCTGCGGAGCGAGGACA CCGCCGTGTACTATTGTGCCAGAGGCGGCTACGACGGCAGAGGCT TCGATTATTGGGGCCAGGGCACCCTCGTGACCGTGTCCTCTGCTA GCACCAAGGGCCCCTCCGTGTTCCCCCTGGCCCCCAGCAGCAAGA GCACCAGCGGCGGCACAGCCGCTCTGGGCTGCCTGGTCGAGGAC TACTTCCCCGAGCCCGTGACCGTGTCCTGGAACAGCGGAGCCCTG ACCTCCGGCGTGCACACCTTCCCCGCCGTGCTGCAGAGTTCTGGC CTGTATAGCCTGAGCAGCGTGGTCACCGTGCCTTCTAGCAGCCTG GGCACCCAGACCTACATCTGCAACGTGAACCACAAGCCCAGCAAC ACCAAGGTGGACGAGAAGGTGGAGCCCAAGAGCTGCGACAAAACT CACACATGCCCACCGTGCCCAGCACCTGAAGCTGCAGGGGGACC GTCAGTCTTCCTCTTCCCCCCAAAACCCAAGGACACCCTCATGATC TCCCGGACCCCTGAGGTCACATGCGTGGTGGTGGACGTGAGCCAC GAAGACCCTGAGGTCAAGTTCAACTGGTACGTGGACGGCGTGGAG GTGCATAATGCCAAGACAAAGCCGCGGGAGGAGCAGTACAACAGC ACGTACCGTGTGGTCAGCGTCCTCACCGTCCTGCACCAGGACTGG CTGAATGGCAAGGAGTACAAGTGCAAGGTCTCCAACAAAGCCCTC GGCGCCCCCATCGAGAAAACCATCTCCAAAGCCAAAGGGCAGCCC CGAGAACCACAGGTGTACACCCTGCCCCCATGCCGGGATGAGCTG ACCAAGAACCAGGTCAGCCTGTGGTGCCTGGTCAAAGGCTTCTATC CCAGCGACATCGCCGTGGAGTGGGAGAGCAATGGGCAGCCGGAG AACAACTACAAGACCACGCCTCCCGTGCTGGACTCCGACGGCTCC TTCTTCCTCTACAGCAAGCTCACCGTGGACAAGAGCAGGTGGCAG CAGGGGAACGTCTTCTCATGCTCCGTGATGCATGAGGCTCTGCAC AACCACTACACGCAGAAGAGCCTCTCCCTGTCTCCGGGTAAATGA | 130 |

(continued)

| Construct | DNA Sequence | SEQ ID No |
|---|---|---|
| anti CD3 (CH2527 VH_3-23(12) VL7-46 (13)) scFv 4.32.63 Cathepsin S/B site CH2527 VH3_23-VH12 CH1 FolR1 16D5 VH CH1 hum Fc knob PG LALA, pETR16550 | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGCGGGGGAGGCTCCG GAGGCGGCGGAAGTGGAGGCGGCGGAAGTTTCGTGGGGGGGAC CGGGGGCGGAGGCAGTGGGGGGGGAGGATCCGGGGGATCCGAG GTGCAGCTGCTGGAATCTGGCGGCGGACTGGTGCAGCCTGGCGG ATCTCTGAGACTGAGCTGTGCCGCCAGCGGCTTCACCTTCAGCAC CTACGCCATGAACTGGGTGCGCCAGGCCCCTGGCAAAGGCCTGGA ATGGGTGTCCCGGATCAGAAGCAAGTACAACAACTACGCCACCTAC TACGCCGACAGCGTGAAGGGCCGGTTCACCATCAGCCGGGACGA CAGCAAGAACACCCTGTACCTGCAGATGAACAGCCTGCGGGCCGA GGACACCGCCGTGTACTATTGTGTGCGGCACGGCAACTTCGGCAA CAGCTATGTGTCTTGGTTTGCCTACTGGGGCCAGGGCACCCTCGT GACCGTGTCAAGCGCTAGCACAAAGGGCCCTAGCGTGTTCCCTCT GGCCCCCAGCAGCAAGAGCACAAGCGGCGGAACAGCCGCCCTGG GCTGCCTCGTGAAGGACTACTTCCCCGAGCCCGTGACAGTGTCTT | 131 |

(continued)

| Construct | DNA Sequence | SEQ ID No |
|---|---|---|
| | GGAACAGCGGAGCCCTGACAAGCGGCGTGCACACCTTCCCTGCC GTGCTGCAGAGCAGCGGCCTGTACTCCCTGAGCAGCGTGGTCACC GTGCCTAGCAGCAGCCTGGGCACCCAGACCTACATCTGCAACGTG AACCACAAGCCCAGCAACACCAAAGTGGACAAGAAGGTGGAGCCC AAGAGCTGTGATGGCGGAGGAGGGTCCGGGGGCGGAGGATCCGA GGTGCAATTGGTTGAATCTGGTGGTGGTCTGGTAAAACCGGGCGG TTCCCTGCGTCTGAGCTGCGCGGCTTCCGGGTTCACCTTCTCCAAC GCGTGGATGAGCTGGGTTCGCCAGGCCCCGGGCAAAGGCCTCGA GTGGGTTGGTCGTATCAAGTCTAAAACTGACGGTGGCACCACGGA TTACGCGGCTCCAGTTAAAGGTCGTTTTACCATTTCCCGCGACGAT AGCAAAAACACTCTGTATCTGCAGATGAACTCTCTGAAAACTGAAG ACACCGCAGTCTACTACTGTACTACCCCGTGGGAATGGTCTTGGTA CGATTATTGGGGCCAGGGCACGCTGGTTACGGTGTCTAGCGCTAG TACCAAGGGCCCCAGCGTGTTCCCCCTGGCACCCAGCAGCAAGAG CACATCTGGCGGAACAGCCGCTCTGGGCTGTCTGGTGAAAGACTA CTTCCCCGAGCCCGTGACCGTGTCTTGGAACTCTGGCGCCCTGAC CAGCGGCGTGCACACCTTTCCAGCCGTGCTGCAGAGCAGCGGCCT GTACTCCCTGTCCTCCGTGGTCACCGTGCCCTCTAGCTCCCTGGG AACACAGACATATATCTGTAATGTCAATCACAAGCCTTCCAACACCA AAGTCGATAAGAAAGTCGAGCCCAAGAGCTGCGACAAAACTCACAC ATGCCCACCGTGCCCAGCACCTGAAGCTGCAGGGGGACCGTCAGT CTTCCTCTTCCCCCCAAAACCCAAGGACACCCTCATGATCTCCCGG ACCCCTGAGGTCACATGCGTGGTGGTGGACGTGAGCCACGAAGAC CCTGAGGTCAAGTTCAACTGGTACGTGGACGGCGTGGAGGTGCAT AATGCCAAGACAAAGCCGCGGGAGGAGCAGTACAACAGCACGTAC CGTGTGGTCAGCGTCCTCACCGTCCTGCACCAGGACTGGCTGAAT GGCAAGGAGTACAAGTGCAAGGTCTCCAACAAAGCCCTCGGCGCC CCCATCGAGAAAACCATCTCCAAAGCCAAAGGGCAGCCCCGAGAA CCACAGGTGTACACCCTGCCCCCATGCCGGGATGAGCTGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTT CCTCTACAGCAAGCTCACCGTGGACAAGAGCAGGTGGCAGCAGGG GAACGTCTTCTCATGCTCCGTGATGCATGAGGCTCTGCACAACCAC TACACGCAGAAGAGCCTCTCCCTGTCTCCGGGTAAATGA | |

| Construct | Amino acid Sequence | SEQ ID No |
|---|---|---|
| pETR16859 Omnitarg aff.mat variant Fab cv - Fc hole PG LALA | EVQLVESGGGLVQPGGSLRLSCAASGFTFNDYTMDWVRQA PGKGLEWVADVNPNSGGSIVNRRFKGRFTLSVDRSKNTLYL QMNSLRAEDTAVYYCARNLGPFFYFDYWGQGTLVTVSSAS TKGPSVFPLAPSSKSTSGGTAALGCLVEDYFPEPVTVSWNS GALTSGVHTFPAVLQSSGLYSLSSVVTVPSSSLGTQTYICNV NHKPSNTKVDEKVEPKSCDKTHTCPPCPAPEAAGGPSVFLF PPKPKDTLMISRTPEVTCVVVDVSHEDPEVKFNWYVDGVEV HNAKTKPREEQYNSTYRVVSVLTVLHQDWLNGKEYKCKVS NKALGAPIEKTISKAKGQPREPQVCTLPPSRDELTKNQVSLS CAVKGFYPSDIAVEWESNGQPENNYKTTPPVLDSDGSFFLV SKLTVDKSRWQQGNVFSCSVMHEALHNHYTQKSLSLSPGK | 132 |

(continued)

| Construct | Amino acid Sequence | SEQ ID No |
|---|---|---|
| pETR16860 Herceptarg common CLkRK | DIQMTQSPSSLSASVGDRVTITCKASQDVSTAVAWYQQKPGKAPKLLIYSASFRYTGVPSRFSGSRSGTDFTLTISSLQPEDFATYYCQQHYTTPPTFGQGTKVEIKRTVAAPSVFIFPPSDRKLKSGTASVVCLLNNFYPREAKVQWKVDNALQSGNSQESVTEQDSKDSTYSLSSTLTLSKADYEKHKVYACEVTHQGLSSPVTKSFNRGEC | 133 |
| pETR17605 CD3 X Fab Herceptin HC charged variants Fc knob PG LALA | QAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSEVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGEGFYAMDYWGQGTLVTVSSASTKGPSVFPLAPSSKSTSGGTAALGCLVEDYFPEPVTVSWNSGALTSGVHTFPAVLQSSGLYSLSSVVTVPSSSLGTQTYICNVNHKPSNTKVDEKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 134 |
| pETR17606 anti CD3 (CH2527 VH_3-23(12) VL7-46(13)) scFv 4.32.63 non cleavable linker aHerceptin VH CH1 EE CH2527-VL7_46-13 CH1 hum Fc knob PG LALA | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIKGGGGSGGGGSGGGGSGGGGGGGSGGGGSGGGGSQAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSEVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGEGFYAMDYWGQGTLVTVSSASTKGPSVFPLAPSSKSTSGGTAALGCLVEDYFPEPVTVSWNSGALTSGVHTFPAVLQSSGLYSLSSVVTVPSSSLGTQTYICNVNHKPSNTKVDEKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 135 |

(continued)

| Construct | Amino acid Sequence | SEQ ID No |
|---|---|---|
| pETR17607 anti CD3 (CH2527 VH_3-23(12) VL7-46(13)) scFv 4.32.63 MMP9 Matriptase MK062 aHerceptin VH CH1 EE CH2527-VL7_46-13 CH1 hum Fc knob PG LALA | QVQLKESGPGLVAPSQSLSITCTVSGFSLTSYGVSWVRQPP GKCLEWLGIIWGDGSTNYHSALISRLSISKDNSKSQVFLKLNS LQTDDTATYYCAKGITTVVDDYYAMDYWGQGTSVTVSSGG GGSGGGGSGGGGSGGGGSDIQMTQSPASLSASVGETVTIT CRASENIDSYLAWYQQKQGKSPQLLVYAATFLADDVPSRFS GSGSGTQYSLKINSLQSEDVARYYCQHYYSTPYTFGCGTKL EIKGGGGSVHMPLGFLGPRQARVVNGGGGGSGGGGSQAV VTQEPSLTVSPGGTVTLTCGSSTGAVTTSNYANWVQEKPG QAFRGLIGGTNKRAPGTPARFSGSLLGGKAALTLSGAQPED EAEYYCALWYSNLWVFGGGTKLTVLSSASTKGPSVFPLAPS SKSTSGGTAALGCLVKDYFPEPVTVSWNSGALTSGVHTFPA VLQSSGLYSLSSVVTVPSSSLGTQTYICNVNHKPSNTKVDKK VEPKSCDGGGGSGGGGSEVQLVESGGGLVQPGGSLRLSC AASGFNIKDTYIHWVRQAPGKGLEWVARIYPTNGYTRYADS VKGRFTISADTSKNTAYLQMNSLRAEDTAVYYCSRWGGEGF YAMDYWGQGTLVTVSSASTKGPSVFPLAPSSKSTSGGTAAL GCLVEDYFPEPVTVSWNSGALTSGVHTFPAVLQSSGLYSLS SVVTVPSSSLGTQTYICNVNHKPSNTKVDEKVEPKSCDKTHT CPPCPAPEAAGGPSVFLFPPKPKDTLMISRTPEVTCVVVDVS HEDPEVKFNWYVDGVEVHNAKTKPREEQYNSTYRVVSVLT VLHQDWLNGKEYKCKVSNKALGAPIEKTISKAKGQPREPQV YTLPPCRDELTKNQVSLWCLVKGFYPSDIAVEWESNGQPEN NYKTTPPVLDSDGSFFLYSKLTVDKSRWQQGNVFSCSVMH EALHNHYTQKSLSLSPGK | 136 |
| FolR1 36F2 VH CH1 EE Fc hole PG LALA pETR14797 | QVQLVQSGAEVKKPGASVKVSCKASGYTFTSYYMHWVRQA PGQGLEWMGIINPSGGSTSYAQKFQGRVTMTHDTSTSTVY MELSSLRSEDTAVYYCARSFFTGFHLDYWGQGTLVTVSSAS TKGPSVFPLAPSSKSTSGGTAALGCLVEDYFPEPVTVSWNS GALTSGVHTFPAVLQSSGLYSLSSVVTVPSSSLGTQTYICNV NHKPSNTKVDEKVEPKSCDKTHTCPPCPAPEAAGGPSVFLF PPKPKDTLMISRTPEVTCVVVDVSHEDPEVKFNWYVDGVEV HNAKTKPREEQYNSTYRVVSVLTVLHQDWLNGKEYKCKVS NKALGAPIEKTISKAKGQPREPQVCTLPPSRDELTKNQVSLS CAVKGFYPSDIAVEWESNGQPENNYKTTPPVLDSDGSFFLV SKLTVDKSRWQQGNVFSCSVMHEALHNHYTQKSLSLSPGK | 137 |
| FolR1 36F2 VL Ck RK, pETR14798 | EIVLTQSPGTLSLSPGERATLSCRASQSVSSSYLAWYQQKP GQAPRLLIYGASSRATGIPDRFSGSGSGTDFTLTISRLEPEDF AVYYCQQYTNEHYYTFGQGTKVEIKRTVAAPSVFIFPPSDRK LKSGTASVVCLLNNFYPREAKVQWKVDNALQSGNSQESVTE QDSKDSTYSLSSTLTLSKADYEKHKVYACEVTHQGLSSPVTK SFNRGEC | 138 |

(continued)

| Construct | Amino acid Sequence | SEQ ID No |
|---|---|---|
| anti CD3 (CH2527 VH_3-23 (12) VL7-46(13)) scFv 4.32.63 MMP9 Matriptase MK062 aFolR1 36F2 VH CH1 EE CH2527-VL7_46-13CH1 hum Fc knob PG LALA pETR17621 | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIKGGGGSVHMPLGFLGPRQARVVNGGGGSGGGGSQAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSQVQLVQSGAEVKKPGASVKVSCKASGYTFTSYYMHWVRQAPGQGLEWMGIINPSGGSTSYAQKFQGRVTMTHDTSTSTVYMELSSLRSEDTAVYYCARSFFTGFHLDYWGQGTLVTVSSASTKGPSVFPLAPSSKSTSGGTAALGCLVEDYFPEPVTVSWNSGALTSGVHTFPAVLQSSGLYSLSSVVTVPSSSLGTQTYICNVNHKPSNTKVDEKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 139 |
| anti CD3 (CH2527 VH_3-23 (12) VL7-46(13)) scFv 4.32.63 non cleavable linker aFolR1 36F2 VH CH1 EE CH2527-VL7_46-13 CH1 hum Fc knob PG LALA pETR17622 | QVQLKESGPGLVAPSQSLSITCTVSGFSLTSYGVSWVRQPPGKCLEWLGIIWGDGSTNYHSALISRLSISKDNSKSQVFLKLNSLQTDDTATYYCAKGITTVVDDYYAMDYWGQGTSVTVSSGGGGSGGGGSGGGGSGGGGSDIQMTQSPASLSASVGETVTITCRASENIDSYLAWYQQKQGKSPQLLVYAATFLADDVPSRFSGSGSGTQYSLKINSLQSEDVARYYCQHYYSTPYTFGCGTKLEIKGGGGSGGGGSGGGGSGGGGGGGSGGGGSGGGGSQAVVTQEPSLTVSPGGTVTLTCGSSTGAVTTSNYANWVQEKPGQAFRGLIGGTNKRAPGTPARFSGSLLGGKAALTLSGAQPEDEAEYYCALWYSNLWVFGGGTKLTVLSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDGGGGSGGGGSQVQLVQSGAEVKKPGASVKVSCKASGYTFTSYYMHWVRQAPGQGLEWMGIINPSGGSTSYAQKFQGRVTMTHDTSTSTVYMELSSLRSEDTAVYYCARSFFTGFHLDYWGQGTLVTVSSASTKGPSVFPLAPSSKSTSGGTAALGCLVEDYFPEPVTVSWNSGALTSGVHTFPAVLQSSGLYSLSSVVTVPSSSLGTQTYICNVNHKPSNTKVDEKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNHYTQKSLSLSPGK | 140 |

(continued)

| Construct | Amino acid Sequence | SEQ ID No |
|---|---|---|
| FolR1 36F2 classic format: CH2527 XFab 36F2 HC EE Fc knob PG LALA pETR17623 | QAVVTQEPSLTVSPGGTVTLTCGSSTGAVTTSNYANWVQEK PGQAFRGLIGGTNKRAPGTPARFSGSLLGGKAALTLSGAQP EDEAEYYCALWYSNLWVFGGGTKLTVLSSASTKGPSVFPLA PSSKSTSGGTAALGCLVKDYFPEPVTVSWNSGALTSGVHTF PAVLQSSGLYSLSSVVTVPSSSLGTQTYICNVNHKPSNTKVD KKVEPKSCDGGGGSGGGGSQVQLVQSGAEVKKPGASVKV SCKASGYTFTSYYMHWVRQAPGQGLEWMGIINPSGGSTSY AQKFQGRVTMTHDTSTSTVYMELSSLRSEDTAVYYCARSFF TGFHLDYWGQGTLVTVSSASTKGPSVFPLAPSSKSTSGGTA ALGCLVEDYFPEPVTVSWNSGALTSGVHTFPAVLQSSGLYS LSSVVTVPSSSLGTQTYICNVNHKPSNTKVDEKVEPKSCDKT HTCPPCPAPEAAGGPSVFLFPPKPKDTLMISRTPEVTCVVVD VSHEDPEVKFNWYVDGVEVHNAKTKPREEQYNSTYRVVSV LTVLHQDWLNGKEYKCKVSNKALGAPIEKTISKAKGQPREPQ VYTLPPCRDELTKNQVSLWCLVKGFYPSDIAVEWESNGQPE NNYKTTPPVLDSDGSFFLYSKLTVDKSRWQQGNVFSCSVM HEALHNHYTQKSLSLSPGK | 141 |
| Herceptin/Omnitarg CDR H1 Kabat | DYTMD | 142 |
| Herceptin/Omnitarg CDR H2 Kabat | DVNPNSGGSIVNRRFKG | 143 |
| Herceptin/Omnitarg CDR H3 Kabat | NLGPFFYFDY | 144 |
| Perjeta CDR H1 Kabat | TSNYANW | 145 |
| Perjeta CDR H2 Kabat | GTNKRAPGTPARFSGSLLGG | 146 |
| Perjeta CDR H3 Kabat | TKLTV | 147 |
| CLC CDR L1 Kabat | KASQDVSTAVA | 148 |
| CLC CDR L2 Kabat | SASFRYT | 149 |
| CLC CDR L3 Kabat | QQHYTTPPT | 150 |
| 36F2 CDR H1 Kabat | SYYMH | 151 |
| 36F2 CDR H2 Kabat | IINPSGGSTSYAQKFQG | 152 |
| 36F2 CDR H3 Kabat | SFFTGFHLDY | 153 |
| 36F2 CDR L1 Kabat | RASQSVSSSYLA | 154 |
| 36F2 CDR L2 Kabat | GASSRAT | 155 |
| 36F2 CDR L3 Kabat | QQYTNEHYYT | 156 |
| Anti-FolR1 36F2 variable region VH | QVQLVQSGAEVKKPGASVKVSCKASGYTFTSYYMHWVRQA PGQGLEWMGIINPSGGSTSYAQKFQGRVTMTHDTSTSTVY MELSSLRSEDTAVYYCARSFFTGFHLDYWGQGTLVTVSS | 157 |
| Anti-FolR1 36F2 variable region VL | EIVLTQSPGTLSLSPGERATLSCRASQSVSSSYLAWYQQKP GQAPRLLIYGASSRATGIPDRFSGSGSGTDFTLTISRLEPEDF AVYYCQQYTNEHYYTFGQGTKVEIK | 158 |
| Herceptarg variable region VH1 | EVQLVESGGGLVQPGGSLRLSCAASGFTFNDYTMDWVRQA PGKGLEWVADVNPNSGGSIVNRRFKGRFTLSVDRSKNTLYL QMNSLRAEDTAVYYCARNLGPFFYFDYWGQGTLVTVSS | 159 |

(continued)

| Construct | Amino acid Sequence | SEQ ID No |
|---|---|---|
| Herceptarg variable region VH2 | EVQLVESGGGLVQPGGSLRLSCAASGFNIKDTYIHWVRQAP GKGLEWVARIYPTNGYTRYADSVKGRFTISADTSKNTAYLQ MNSLRAEDTAVYYCSRWGGEGFYAMDYWGQGTLVTVSS | 160 |
| Herceptarg common variable region VL | DIQMTQSPSSLSASVGDRVTITCKASQDVSTAVAWYQQKPG KAPKLLIYSASFRYTGVPSRFSGSRSGTDFTLTISSLQPEDFA TYYCQQHYTTPPTFGQGTKVEIK | 161 |

| Construct | DNA Sequence | SEQ ID No |
|---|---|---|
| pETR16859 Omnitarg aff.mat variant Fab cv - Fc hole PG LALA | GAAGTTCAGCTGGTTGAAAGCGGTGGTGGTCTGGTTCAGCCTGGT<br>GGTAGCCTGCGTCTGAGCTGTGCAGCAAGCGGTTTTACCTTTAACG<br>ATTATACCATGGATTGGGTTCGTCAGGCACCGGGTAAAGGTCTGGA<br>ATGGGTTGCAGATGTTAATCCGAATAGCGGTGGTAGCATTGTTAAC<br>CGTCGTTTTAAAGGTCGTTTTACCCTGAGCGTTGATCGTAGCAAAA<br>ATACCCTGTATCTGCAAATGAATAGTCTGCGTGCAGAGGATACCGC<br>AGTGTATTATTGTGCACGTAACCTGGGTCCGTTCTTCTACTTTGATT<br>ATTGGGGTCAGGGCACCCTGGTTACCGTTAGCAGCGCTAGCACCA<br>AGGGCCCCTCCGTGTTCCCCCTGGCCCCCAGCAGCAAGAGCACCA<br>GCGGCGGCACAGCCGCTCTGGGCTGCCTGGTCGAGGACTACTTC<br>CCCGAGCCCGTGACCGTGTCCTGGAACAGCGGAGCCCTGACCTCC<br>GGCGTGCACACCTTCCCCGCCGTGCTGCAGAGTTCTGGCCTGTAT<br>AGCCTGAGCAGCGTGGTCACCGTGCCTTCTAGCAGCCTGGGCACC<br>CAGACCTACATCTGCAACGTGAACCACAAGCCCAGCAACACCAAG<br>GTGGACGAGAAGGTGGAGCCCAAGAGCTGCGACAAAACTCACACA<br>TGCCCACCGTGCCCAGCACCTGAAGCTGCAGGGGGACCGTCAGTC<br>TTCCTCTTCCCCCCAAAACCCAAGGACACCCTCATGATCTCCCGGA<br>CCCCTGAGGTCACATGCGTGGTGGTGGACGTGAGCCACGAAGACC<br>CTGAGGTCAAGTTCAACTGGTACGTGGACGGCGTGGAGGTGCATA<br>ATGCCAAGACAAAGCCGCGGGAGGAGCAGTACAACAGCACGTACC<br>GTGTGGTCAGCGTCCTCACCGTCCTGCACCAGGACTGGCTGAATG<br>GCAAGGAGTACAAGTGCAAGGTCTCCAACAAAGCCCTCGGCGCCC<br>CCATCGAGAAAACCATCTCCAAAGCCAAAGGGCAGCCCCGAGAAC<br>CACAGGTGTGCACCCTGCCCCCATCCCGGGATGAGCTGACCAAGA<br>ACCAGGTCAGCCTCTCGTGCGCAGTCAAAGGCTTCTATCCCAGCG<br>ACATCGCCGTGGAGTGGGAGAGCAATGGGCAGCCGGAGAACAACT<br>ACAAGACCACGCCTCCCGTGCTGGACTCCGACGGCTCCTTCTTCC<br>TCGTGAGCAAGCTCACCGTGGACAAGAGCAGGTGGCAGCAGGGG<br>AACGTCTTCTCATGCTCCGTGATGCATGAGGCTCTGCACAACCACT<br>ACACGCAGAAGAGCCTCTCCCTGTCTCCGGGTAAATGA | 162 |

(continued)

| Construct | DNA Sequence | SEQ ID No |
|---|---|---|
| pETR16860 Herceptarg common CLkRK | GACATCCAGATGACCCAGAGCCCCAGCAGCCTGTCTGCCAGCGTG GGCGACAGAGTGACCATCACATGCAAGGCCAGCCAGGACGTGTCC ACAGCCGTGGCCTGGTATCAGCAGAAGCCTGGCAAGGCCCCCAAG CTGCTGATCTACAGCGCCAGCTTCCGGTACACCGGCGTGCCCAGC AGATTCAGCGGCAGCAGATCCGGCACCGACTTCACCCTGACCATC AGCTCCCTGCAGCCCGAGGACTTCGCCACCTACTACTGCCAGCAG CACTACACCACCCCCCCCACATTTGGCCAGGGCACCAAGGTGGAA ATCAAGCGTACGGTGGCTGCACCATCTGTCTTCATCTTCCCGCCAT CTGATCGGAAGTTGAAATCTGGAACTGCCTCTGTTGTGTGCCTGCT GAATAACTTCTATCCCAGAGAGGCCAAAGTACAGTGGAAGGTGGAT AACGCCCTCCAATCGGGTAACTCCCAGGAGAGTGTCACAGAGCAG GACAGCAAGGACAGCACCTACAGCCTCAGCAGCACCCTGACGCTG AGCAAAGCAGACTACGAGAAACACAAAGTCTACGCCTGCGAAGTCA CCCATCAGGGCCTGAGCTCGCCCGTCACAAAGAGCTTCAACAGGG GAGAGTGTTAG | 163 |
| pETR17606 anti CD3 (CH2527 VH_ 3-23(12) VL7-46 (13)) scFv 4.32.63 non cleavable linker | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG | 164 |

(continued)

| Construct | DNA Sequence | SEQ ID No |
|---|---|---|
| aHerceptin VH CH1 EE CH2527-VL7_46-13 CH1 hum Fc knob PG LALA | TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGCGGGGGAGGCTCCG GAGGCGGCGGAAGTGGAGGCGGCGGAAGTGGCGGAGGCGGAGG GGGGGGAAGTGGGGGCGGAGGCAGTGGGGGGGGAGGATCCCAG GCCGTCGTGACCCAGGAACCCAGCCTGACAGTGTCTCCTGGCGGC ACCGTGACCCTGACATGTGGCAGTTCTACAGGCGCCGTGACCACC AGCAACTACGCCAACTGGGTGCAGGAAAAGCCCGGCCAGGCCTTC AGAGGACTGATCGGCGGCACCAACAAGAGAGCCCCTGGCACCCCT GCCAGATTCAGCGGATCTCTGCTGGGAGGAAAGGCCGCCCTGACA CTGTCTGGCGCCCAGCCAGAAGATGAGGCCGAGTACTACTGCGCC CTGTGGTACAGCAACCTGTGGGTGTTCGGCGGAGGCACCAAGCTG ACAGTGCTGAGCAGCGCTTCCACCAAGGGACCCAGTGTGTTCCCC CTGGCCCCCAGCTCCAAGTCTACATCCGGTGGCACAGCTGCCCTG GGATGTCTCGTGAAGGACTACTTTCCTGAGCCTGTGACAGTGTCTT GGAACAGCGGAGCCCTGACCAGCGGAGTGCACACATTCCCTGCAG TGCTGCAGAGCAGCGGCCTGTATAGCCTGAGCAGCGTCGTGACCG TGCCTTCCTCTAGCCTGGGAACACAGACATATATCTGTAATGTGAAT CATAAGCCCAGTAATACCAAAGTGGATAAGAAAGTGGAACCTAAGA GCTGCGATGGCGGAGGAGGGTCCGGAGGCGGAGGGTCCGAGGT CCAGCTGGTCGAGTCTGGAGGAGGACTGGTGCAGCCAGGCGGAT CTCTGAGACTGAGCTGCGCCGCCAGCGGATTCAACATCAAGGACA CCTACATCCACTGGGTGAGGCAGGCCCCTGGAAAGGGACTGGAGT GGGTGGCCAGAATCTACCCCACCAACGGCTACACAAGATACGCCG ACAGCGTGAAGGGCAGATTCACCATCAGCGCCGACACCAGCAAGA ACACCGCCTACCTGCAGATGAACAGCCTGAGAGCCGAGGACACAG CCGTGTACTACTGCTCTAGATGGGGAGGCGAGGGCTTCTACGCCA TGGACTACTGGGGACAGGGCACACTGGTGACCGTGTCCAGCGCTA GCACCAAGGGCCCCTCCGTGTTCCCCCTGGCCCCCAGCAGCAAGA GCACCAGCGGCGGCACAGCCGCTCTGGGCTGCCTGGTCGAGGAC TACTTCCCCGAGCCCGTGACCGTGTCCTGGAACAGCGGAGCCCTG ACCTCCGGCGTGCACACCTTCCCCGCCGTGCTGCAGAGTTCTGGC CTGTATAGCCTGAGCAGCGTGGTCACCGTGCCTTCTAGCAGCCTG GGCACCCAGACCTACATCTGCAACGTGAACCACAAGCCCAGCAAC ACCAAGGTGGACGAGAAGGTGGAGCCCAAGAGCTGCGACAAAACT CACACATGCCCACCGTGCCCAGCACCTGAAGCTGCAGGGGGACC GTCAGTCTTCCTCTTCCCCCCAAAACCCAAGGACACCCTCATGATC TCCCGGACCCCTGAGGTCACATGCGTGGTGGTGGACGTGAGCCAC GAAGACCCTGAGGTCAAGTTCAACTGGTACGTGGACGGCGTGGAG GTGCATAATGCCAAGACAAAGCCGCGGGAGGAGCAGTACAACAGC ACGTACCGTGTGGTCAGCGTCCTCACCGTCCTGCACCAGGACTGG CTGAATGGCAAGGAGTACAAGTGCAAGGTCTCCAACAAAGCCCTC GGCGCCCCCATCGAGAAAACCATCTCCAAAGCCAAAGGGCAGCCC CGAGAACCACAGGTGTACACCCTGCCCCCATGCCGGGATGAGCTG ACCAAGAACCAGGTCAGCCTGTGGTGCCTGGTCAAAGGCTTCTATC CCAGCGACATCGCCGTGGAGTGGGAGAGCAATGGGCAGCCGGAG AACAACTACAAGACCACGCCTCCCGTGCTGGACTCCGACGGCTCC TTCTTCCTCTACAGCAAGCTCACCGTGGACAAGAGCAGGTGGCAG CAGGGGAACGTCTTCTCATGCTCCGTGATGCATGAGGCTCTGCAC AACCACTACACGCAGAAGAGCCTCTCCCTGTCTCCGGGTAAATGA | |

(continued)

| Construct | DNA Sequence | SEQ ID No |
|---|---|---|
| pETR17607 anti CD3 (CH2527 VH_ 3-23(12) | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC | 165 |

(continued)

| Construct | DNA Sequence | SEQ ID No |
|---|---|---|
| VL7-46(13)) scFv 4.32.63 MMP9 Matriptase MK062 aHerceptin VH CH1 EE CH2527-VL7_46-13 CH1 hum Fc knob PG LALA | AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG TGGCCCGGTACTACTGCCAGCACTACTACAGCACCCCCTACACCTT CGGCTGCGGCACCAAGCTGGAAATCAAAGGAGGCGGCGGAAGTG TGCACATGCCCCTGGGCTTCCTGGGCCCCAGACAGGCCAGAGTCG TGAACGGGGGGGGCGGAGGCAGTGGGGGGGGAGGATCCCAGGC CGTCGTGACCCAGGAACCCAGCCTGACAGTGTCTCCTGGCGGCAC CGTGACCCTGACATGTGGCAGTTCTACAGGCGCCGTGACCACCAG CAACTACGCCAACTGGGTGCAGGAAAAGCCCGGCCAGGCCTTCAG AGGACTGATCGGCGGCACCAACAAGAGAGCCCCTGGCACCCCTGC CAGATTCAGCGGATCTCTGCTGGGAGGAAAGGCCGCCCTGACACT GTCTGGCGCCCAGCCAGAAGATGAGGCCGAGTACTACTGCGCCCT GTGGTACAGCAACCTGTGGGTGTTCGGCGGAGGCACCAAGCTGAC AGTGCTGAGCAGCGCTTCCACCAAGGGACCCAGTGTGTTCCCCCT GGCCCCCAGCTCCAAGTCTACATCCGGTGGCACAGCTGCCCTGGG ATGTCTCGTGAAGGACTACTTTCCTGAGCCTGTGACAGTGTCTTGG AACAGCGGAGCCCTGACCAGCGGAGTGCACACATTCCCTGCAGTG CTGCAGAGCAGCGGCCTGTATAGCCTGAGCAGCGTCGTGACCGTG CCTTCCTCTAGCCTGGGAACACAGACATATATCTGTAATGTGAATCA TAAGCCCAGTAATACCAAAGTGGATAAGAAAGTGGAACCTAAGAGC TGCGATGGCGGAGGAGGGTCCGGAGGCGGAGGGTCCGAGGTCCA GCTGGTCGAGTCTGGAGGAGGACTGGTGCAGCCAGGCGGATCTCT GAGACTGAGCTGCGCCGCCAGCGGATTCAACATCAAGGACACCTA CATCCACTGGGTGAGGCAGGCCCCTGGAAAGGGACTGGAGTGGG TGGCCAGAATCTACCCCACCAACGGCTACACAAGATACGCCGACA GCGTGAAGGGCAGATTCACCATCAGCGCCGACACCAGCAAGAACA CCGCCTACCTGCAGATGAACAGCCTGAGAGCCGAGGACACAGCCG TGTACTACTGCTCTAGATGGGGAGGCGAGGGCTTCTACGCCATGG ACTACTGGGGACAGGGCACACTGGTGACCGTGTCCAGCGCTAGCA CCAAGGGCCCCTCCGTGTTCCCCCTGGCCCCCAGCAGCAAGAGCA CCAGCGGCGGCACAGCCGCTCTGGGCTGCCTGGTCGAGGACTAC TTCCCCGAGCCCGTGACCGTGTCCTGGAACAGCGGAGCCCTGACC TCCGGCGTGCACACCTTCCCCGCCGTGCTGCAGAGTTCTGGCCTG TATAGCCTGAGCAGCGTGGTCACCGTGCCTTCTAGCAGCCTGGGC ACCCAGACCTACATCTGCAACGTGAACCACAAGCCCAGCAACACCA AGGTGGACGAGAAGGTGGAGCCCAAGAGCTGCGACAAAACTCACA CATGCCCACCGTGCCCAGCACCTGAAGCTGCAGGGGGACCGTCA GTCTTCCTCTTCCCCCCAAAACCCAAGGACACCCTCATGATCTCCC GGACCCCTGAGGTCACATGCGTGGTGGTGGACGTGAGCCACGAA GACCCTGAGGTCAAGTTCAACTGGTACGTGGACGGCGTGGAGGTG CATAATGCCAAGACAAAGCCGCGGGAGGAGCAGTACAACAGCACG TACCGTGTGGTCAGCGTCCTCACCGTCCTGCACCAGGACTGGCTG AATGGCAAGGAGTACAAGTGCAAGGTCTCCAACAAAGCCCTCGGC GCCCCCATCGAGAAAACCATCTCCAAAGCCAAAGGGCAGCCCCGA GAACCACAGGTGTACACCCTGCCCCCATGCCGGGATGAGCTGACC AAGAACCAGGTCAGCCTGTGGTGCCTGGTCAAAGGCTTCTATCCCA GCGACATCGCCGTGGAGTGGGAGAGCAATGGGCAGCCGGAGAAC AACTACAAGACCACGCCTCCCGTGCTGGACTCCGACGGCTCCTTC TTCCTCTACAGCAAGCTCACCGTGGACAAGAGCAGGTGGCAGCAG GGGAACGTCTTCTCATGCTCCGTGATGCATGAGGCTCTGCACAACC ACTACACGCAGAAGAGCCTCTCCCTGTCTCCGGGTAAATGA | |

(continued)

| Construct | DNA Sequence | SEQ ID No |
|---|---|---|
| FolR1 36F2 VH CH1 EE Fc hole PG LALA pETR14797 | CAGGTGCAATTGGTTCAATCTGGTGCTGAAGTAAAAAAACCGGGCG CTTCCGTTAAAGTGAGCTGCAAAGCATCCGGATACACCTTCACTTC CTATTACATGCACTGGGTTCGTCAAGCCCCGGGCCAGGGTCTGGA ATGGATGGGCATCATTAACCCAAGCGGTGGCTCTACCTCCTACGC GCAGAAATTCCAGGGTCGCGTCACGATGACCCATGACACTAGCAC CTCTACCGTTTATATGGAGCTGTCCAGCCTGCGTTCTGAAGATACT GCAGTGTACTACTGTGCACGCTCTTTCTTCACTGGTTTCCATCTGG ACTATTGGGGTCAAGGCACCCTCGTAACGGTTTCTTCTGCTAGCAC CAAGGGCCCCTCCGTGTTCCCCCTGGCCCCCAGCAGCAAGAGCAC CAGCGGCGGCACAGCCGCTCTGGGCTGCCTGGTCGAGGACTACTT CCCCGAGCCCGTGACCGTGTCCTGGAACAGCGGAGCCCTGACCTC CGGCGTGCACACCTTCCCCGCCGTGCTGCAGAGTTCTGGCCTGTA TAGCCTGAGCAGCGTGGTCACCGTGCCTTCTAGCAGCCTGGGCAC CCAGACCTACATCTGCAACGTGAACCACAAGCCCAGCAACACCAA GGTGGACGAGAAGGTGGAGCCCAAGAGCTGCGACAAAACTCACAC ATGCCCACCGTGCCCAGCACCTGAAGCTGCAGGGGGACCGTCAGT CTTCCTCTTCCCCCCAAAACCCAAGGACACCCTCATGATCTCCCGG ACCCCTGAGGTCACATGCGTGGTGGTGGACGTGAGCCACGAAGAC CCTGAGGTCAAGTTCAACTGGTACGTGGACGGCGTGGAGGTGCAT AATGCCAAGACAAAGCCGCGGGAGGAGCAGTACAACAGCACGTAC CGTGTGGTCAGCGTCCTCACCGTCCTGCACCAGGACTGGCTGAAT GGCAAGGAGTACAAGTGCAAGGTCTCCAACAAAGCCCTCGGCGCC CCCATCGAGAAAACCATCTCCAAAGCCAAAGGGCAGCCCCGAGAA CCACAGGTGTGCACCCTGCCCCCATCCCGGGATGAGCTGACCAAG AACCAGGTCAGCCTCTCGTGCGCAGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTT CCTCGTGAGCAAGCTCACCGTGGACAAGAGCAGGTGGCAGCAGG GGAACGTCTTCTCATGCTCCGTGATGCATGAGGCTCTGCACAACCA CTACACGCAGAAGAGCCTCTCCCTGTCTCCGGGTAAATGA | 166 |
| FolR1 36F2 VL Ck RK, pETR14798 | GAAATCGTGTTAACGCAGTCTCCAGGCACCCTGTCTTTGTCTCCAG GGGAAAGAGCCACCCTCTCTTGCAGGGCCAGTCAGAGTGTTAGCA GCAGCTACTTAGCCTGGTACCAGCAGAAACCTGGCCAGGCTCCCA GGCTCCTCATCTATGGAGCATCCAGCAGGGCCACTGGCATCCCAG ACAGGTTCAGTGGCAGTGGATCCGGGACAGACTTCACTCTCACCAT CAGCAGACTGGAGCCTGAAGATTTTGCAGTGTATTACTGTCAGCAG TATACCAACGAACATTATTATACGTTCGGCCAGGGGACCAAAGTGG AAATCAAACGTACGGTGGCTGCACCATCTGTCTTCATCTTCCCGCC ATCTGATCGGAAGTTGAAATCTGGAACTGCCTCTGTTGTGTGCCTG CTGAATAACTTCTATCCCAGAGAGGCCAAAGTACAGTGGAAGGTGG ATAACGCCCTCCAATCGGGTAACTCCCAGGAGAGTGTCACAGAGC AGGACAGCAAGGACAGCACCTACAGCCTCAGCAGCACCCTGACGC TGAGCAAAGCAGACTACGAGAAACACAAAGTCTACGCCTGCGAAGT CACCCATCAGGGCCTGAGCTCGCCCGTCACAAAGAGCTTCAACAG GGGAGAGTGTTAG | 167 |

(continued)

| Construct | DNA Sequence | SEQ ID No |
|---|---|---|
| anti CD3 (CH2527 VH_3-23(12) VL7-46(13)) scFv 4.32.63 MMP9 Matriptase MK062 aFolR1 36F2 VH CH1 EE CH2527-VL7_46-13 CH1 hum Fc knob PG LALA | CAAGTGCAGCTGAAAGAGTCCGGCCCTGGACTGGTGGCCCCTAGC CAGAGCCTGAGCATCACCTGTACCGTGTCCGGCTTCAGCCTGACC AGCTACGGCGTGTCATGGGTGCGCCAGCCTCCAGGCAAGTGTCTG GAATGGCTGGGCATCATCTGGGGCGACGGCAGCACCAATTACCAC AGCGCCCTGATCAGCAGACTGAGCATCTCCAAGGACAACAGCAAG AGCCAGGTGTTCCTGAAGCTGAACAGCCTGCAGACCGACGACACC GCCACCTACTACTGCGCCAAGGGCATCACCACCGTGGTGGACGAC TACTACGCTATGGACTACTGGGGCCAGGGCACCAGCGTGACAGTG TCTAGCGGAGGCGGAGGATCTGGCGGCGGAGGAAGTGGCGGAGG GGGATCTGGGGGAGGCGGAAGCGATATCCAGATGACCCAGAGCC CTGCCAGCCTGTCTGCCTCTGTGGGCGAGACAGTGACCATCACAT GCCGGGCCAGCGAGAACATCGACAGCTACCTGGCCTGGTATCAGC AGAAGCAGGGCAAGAGCCCCCAGCTGCTGGTGTACGCCGCCACCT TTCTGGCCGACGATGTGCCCAGCAGATTCAGCGGCAGCGGAAGCG GCACACAGTACAGCCTGAAGATCAACTCCCTGCAGAGCGAGGACG | 168 |

(continued)

| Construct | DNA Sequence | SEQ ID No |
|---|---|---|
| pETR17621 | TGGCCCGGTACTACTGCCAGCACTACTACAGCACCCCCTACACCTTCGGCTGCGGCACCAAGCTGGAAATCAAAGGAGGCGGCGGAAGTGTGCACATGCCCCTGGGCTTCCTGGGCCCCAGACAGGCCAGAGTCGTGAACGGGGGGGGCGGAGGCAGTGGGGGGGGAGGATCCCAGGCCGTCGTGACCCAGGAACCCAGCCTGACAGTGTCTCCTGGCGGCACCGTGACCCTGACATGTGGCAGTTCTACAGGCGCCGTGACCACCAGCAACTACGCCAACTGGGTGCAGGAAAAGCCCGGCCAGGCCTTCAGAGGACTGATCGGCGGCACCAACAAGAGAGCCCCTGGCACCCCTGCCAGATTCAGCGGATCTCTGCTGGGAGGAAAGGCCGCCCTGACACTGTCTGGCGCCCAGCCAGAAGATGAGGCCGAGTACTACTGCGCCCTGTGGTACAGCAACCTGTGGGTGTTCGGCGGAGGCACCAAGCTGACAGTGCTGAGCAGCGCTTCCACCAAGGGACCCAGTGTGTTCCCCCTGGCCCCCAGCTCCAAGTCTACATCCGGTGGCACAGCTGCCCTGGGATGTCTCGTGAAGGACTACTTTCCTGAGCCTGTGACAGTGTCTTGGAACAGCGGAGCCCTGACCAGCGGAGTGCACACATTCCCTGCAGTGCTGCAGAGCAGCGGCCTGTATAGCCTGAGCAGCGTCGTGACCGTGCCTTCCTCTAGCCTGGGAACACAGACATATATCTGTAATGTGAATCATAAGCCCAGTAATACCAAAGTGGATAAGAAAGTGGAACCTAAGAGCTGCGATGGCGGAGGAGGGTCCGGAGGCGGAGGGTCCCAGGTGCAATTGGTTCAATCTGGTGCTGAAGTAAAAAAACCGGGCGCTTCCGTTAAAGTGAGCTGCAAAGCATCCGGATACACCTTCACTTCCTATTACATGCACTGGGTTCGTCAAGCCCCGGGCCAGGGTCTGGAATGGATGGGCATCATTAACCCAAGCGGTGGCTCTACCTCCTACGCGCAGAAATTCCAGGGTCGCGTCACGATGACCCATGACACTAGCACCTCTACCGTTTATATGGAGCTGTCCAGCCTGCGTTCTGAAGATACTGCAGTGTACTACTGTGCACGCTCTTTCTTCACTGGTTTCCATCTGGACTATTGGGGTCAAGGCACCCTCGTAACGGTTTCTTCTGCTAGCACCAAGGGCCCCTCCGTGTTCCCCCTGGCCCCCAGCAGCAAGAGCACCAGCGGCGGCACAGCCGCTCTGGGCTGCCTGGTCGAGGACTACTTCCCCGAGCCCGTGACCGTGTCCTGGAACAGCGGAGCCCTGACCTCCGGCGTGCACACCTTCCCCGCCGTGCTGCAGAGTTCTGGCCTGTATAGCCTGAGCAGCGTGGTCACCGTGCCTTCTAGCAGCCTGGGCACCCAGACCTACATCTGCAACGTGAACCACAAGCCCAGCAACACCAAGGTGGACGAGAAGGTGGAGCCCAAGAGCTGCGACAAAACTCACACATGCCCACCGTGCCCAGCACCTGAAGCTGCAGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCGGCGCCCCCATCGAGAAAACCATCTCCAAAGCCAAAGGGCAGCCCCGAGAACCACAGGTGTACACCCTGCCCCCATGCCGGGATGAGCTGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAATGA | |

(continued)

| Construct | DNA Sequence | SEQ ID No |
|---|---|---|
| anti CD3 (CH2527 VH_3-23(12) VL7-46(13)) scFv 4.32.63 non cleavable linker aFolR1 36F2 VH CH1 EE CH2527-VL7_46-13 CH1 hum Fc | AGAGTCCGGCCCTGGACTGGTGGCCCCTAGCCAGAGCCTGAGCAT CACCTGTACCGTGTCCGGCTTCAGCCTGACCAGCTACGGCGTGTC ATGGGTGCGCCAGCCTCCAGGCAAGTGTCTGGAATGGCTGGGCAT CATCTGGGGCGACGGCAGCACCAATTACCACAGCGCCCTGATCAG CAGACTGAGCATCTCCAAGGACAACAGCAAGAGCCAGGTGTTCCT GAAGCTGAACAGCCTGCAGACCGACGACACCGCCACCTACTACTG CGCCAAGGGCATCACCACCGTGGTGGACGACTACTACGCTATGGA CTACTGGGGCCAGGGCACCAGCGTGACAGTGTCTAGCGGAGGCG GAGGATCTGGCGGCGGAGGAAGTGGCGGAGGGGGATCTGGGGG AGGCGGAAGCGATATCCAGATGACCCAGAGCCCTGCCAGCCTGTC TGCCTCTGTGGGCGAGACAGTGACCATCACATGCCGGGCCAGCGA | 169 |

(continued)

| Construct | DNA Sequence | SEQ ID No |
|---|---|---|
| knob PG LALA pETR17622 | GAACATCGACAGCTACCTGGCCTGGTATCAGCAGAAGCAGGGCAA<br>GAGCCCCCAGCTGCTGGTGTACGCCGCCACCTTTCTGGCCGACGA<br>TGTGCCCAGCAGATTCAGCGGCAGCGGAAGCGGCACACAGTACAG<br>CCTGAAGATCAACTCCCTGCAGAGCGAGGACGTGGCCCGGTACTA<br>CTGCCAGCACTACTACAGCACCCCCTACACCTTCGGCTGCGGCAC<br>CAAGCTGGAAATCAAAGGCGGGGGAGGCTCCGGAGGCGGCGGAA<br>GTGGAGGCGGCGGAAGTGGCGGAGGCGGAGGGGGGGGAAGTGG<br>GGGCGGAGGCAGTGGGGGGGGAGGATCCCAGGCCGTCGTGACC<br>CAGGAACCCAGCCTGACAGTGTCTCCTGGCGGCACCGTGACCCTG<br>ACATGTGGCAGTTCTACAGGCGCCGTGACCACCAGCAACTACGCC<br>AACTGGGTGCAGGAAAAGCCCGGCCAGGCCTTCAGAGGACTGATC<br>GGCGGCACCAACAAGAGAGCCCCTGGCACCCCTGCCAGATTCAGC<br>GGATCTCTGCTGGGAGGAAAGGCCGCCCTGACACTGTCTGGCGCC<br>CAGCCAGAAGATGAGGCCGAGTACTACTGCGCCCTGTGGTACAGC<br>AACCTGTGGGTGTTCGGCGGAGGCACCAAGCTGACAGTGCTGAGC<br>AGCGCTTCCACCAAGGGACCCAGTGTGTTCCCCCTGGCCCCCAGC<br>TCCAAGTCTACATCCGGTGGCACAGCTGCCCTGGGATGTCTCGTG<br>AAGGACTACTTTCCTGAGCCTGTGACAGTGTCTTGGAACAGCGGAG<br>CCCTGACCAGCGGAGTGCACACATTCCCTGCAGTGCTGCAGAGCA<br>GCGGCCTGTATAGCCTGAGCAGCGTCGTGACCGTGCCTTCCTCTA<br>GCCTGGGAACACAGACATATATCTGTAATGTGAATCATAAGCCCAG<br>TAATACCAAAGTGGATAAGAAAGTGGAACCTAAGAGCTGCGATGGC<br>GGAGGAGGGTCTGGAGGCGGAGGGTCCCAGGTGCAATTGGTTCA<br>ATCTGGTGCTGAAGTAAAAAAACCGGGCGCTTCCGTTAAAGTGAGC<br>TGCAAAGCATCCGGATACACCTTCACTTCCTATTACATGCACTGGG<br>TTCGTCAAGCCCCGGGCCAGGGTCTGGAATGGATGGGCATCATTA<br>ACCCAAGCGGTGGCTCTACCTCCTACGCGCAGAAATTCCAGGGTC<br>GCGTCACGATGACCCATGACACTAGCACCTCTACCGTTTATATGGA<br>GCTGTCCAGCCTGCGTTCTGAAGATACTGCAGTGTACTACTGTGCA<br>CGCTCTTTCTTCACTGGTTTCCATCTGGACTATTGGGGTCAAGGCA<br>CCCTCGTAACGGTTTCTTCTGCTAGCACCAAGGGCCCCTCCGTGTT<br>CCCCCTGGCCCCCAGCAGCAAGAGCACCAGCGGCGGCACAGCCG<br>CTCTGGGCTGCCTGGTCGAGGACTACTTCCCCGAGCCCGTGACCG<br>TGTCCTGGAACAGCGGAGCCCTGACCTCCGGCGTGCACACCTTCC<br>CCGCCGTGCTGCAGAGTTCTGGCCTGTATAGCCTGAGCAGCGTGG<br>TCACCGTGCCTTCTAGCAGCCTGGGCACCCAGACCTACATCTGCAA<br>CGTGAACCACAAGCCCAGCAACACCAAGGTGGACGAGAAGGTGGA<br>GCCCAAGAGCTGCGACAAAACTCACACATGCCCACCGTGCCCAGC<br>ACCTGAAGCTGCAGGGGGACCGTCAGTCTTCCTCTTCCCCCCAAA<br>ACCCAAGGACACCCTCATGATCTCCCGGACCCCTGAGGTCACATG<br>CGTGGTGGTGGACGTGAGCCACGAAGACCCTGAGGTCAAGTTCAA<br>CTGGTACGTGGACGGCGTGGAGGTGCATAATGCCAAGACAAAGCC<br>GCGGGAGGAGCAGTACAACAGCACGTACCGTGTGGTCAGCGTCCT<br>CACCGTCCTGCACCAGGACTGGCTGAATGGCAAGGAGTACAAGTG<br>CAAGGTCTCCAACAAAGCCCTCGGCGCCCCCATCGAGAAAACCAT<br>CTCCAAAGCCAAAGGGCAGCCCCGAGAACCACAGGTGTACACCCT<br>GCCCCCATGCCGGGATGAGCTGACCAAGAACCAGGTCAGCCTGTG<br>GTGCCTGGTCAAAGGCTTCTATCCCAGCGACATCGCCGTGGAGTG<br>GGAGAGCAATGGGCAGCCGGAGAACAACTACAAGACCACGCCTCC<br>CGTGCTGGACTCCGACGGCTCCTTCTTCCTCTACAGCAAGCTCACC<br>GTGGACAAGAGCAGGTGGCAGCAGGGGAACGTCTTCTCATGCTCC<br>GTGATGCATGAGGCTCTGCACAACCACTACACGCAGAAGAGCCTC<br>TCCCTGTCTCCGGGTAAATGA | |

(continued)

| Construct | DNA Sequence | SEQ ID No |
|---|---|---|
| FolR1 36F2 classic format: CH2527 XFab 36F2 HC EE Fc knob PG LALA pETR17623 | CAGGCCGTCGTGACCCAGGAACCCAGCCTGACAGTGTCTCCTGGC<br>GGCACCGTGACCCTGACATGTGGCAGTTCTACAGGCGCCGTGACC<br>ACCAGCAACTACGCCAACTGGGTGCAGGAAAAGCCCGGCCAGGCC<br>TTCAGAGGACTGATCGGCGGCACCAACAAGAGAGCCCCTGGCACC<br>CCTGCCAGATTCAGCGGATCTCTGCTGGGAGGAAAGGCCGCCCTG<br>ACACTGTCTGGCGCCCAGCCAGAAGATGAGGCCGAGTACTACTGC<br>GCCCTGTGGTACAGCAACCTGTGGGTGTTCGGCGGAGGCACCAAG<br>CTGACAGTGCTGAGCAGCGCTTCCACCAAGGGACCCAGTGTGTTC<br>CCCCTGGCCCCCAGCTCCAAGTCTACATCCGGTGGCACAGCTGCC<br>CTGGGATGTCTCGTGAAGGACTACTTTCCTGAGCCTGTGACAGTGT<br>CTTGGAACAGCGGAGCCCTGACCAGCGGAGTGCACACATTCCCTG<br>CAGTGCTGCAGAGCAGCGGCCTGTATAGCCTGAGCAGCGTCGTGA<br>CCGTGCCTTCCTCTAGCCTGGGAACACAGACATATATCTGTAATGT<br>GAATCATAAGCCCAGTAATACCAAAGTGGATAAGAAAGTGGAACCT<br>AAGAGCTGCGATGGCGGAGGAGGGTCTGGAGGCGGAGGGTCCCA<br>GGTGCAATTGGTTCAATCTGGTGCTGAAGTAAAAAAACCGGGCGCT<br>TCCGTTAAAGTGAGCTGCAAAGCATCCGGATACACCTTCACTTCCT<br>ATTACATGCACTGGGTTCGTCAAGCCCCGGGCCAGGGTCTGGAAT<br>GGATGGGCATCATTAACCCAAGCGGTGGCTCTACCTCCTACGCGC<br>AGAAATTCCAGGGTCGCGTCACGATGACCCATGACACTAGCACCTC<br>TACCGTTTATATGGAGCTGTCCAGCCTGCGTTCTGAAGATACTGCA<br>GTGTACTACTGTGCACGCTCTTTCTTCACTGGTTTCCATCTGGACTA<br>TTGGGGTCAAGGCACCCTCGTAACGGTTTCTTCTGCTAGCACCAAG<br>GGCCCCTCCGTGTTCCCCCTGGCCCCCAGCAGCAAGAGCACCAGC<br>GGCGGCACAGCCGCTCTGGGCTGCCTGGTCGAGGACTACTTCCCC<br>GAGCCCGTGACCGTGTCCTGGAACAGCGGAGCCCTGACCTCCGG<br>CGTGCACACCTTCCCCGCCGTGCTGCAGAGTTCTGGCCTGTATAG<br>CCTGAGCAGCGTGGTCACCGTGCCTTCTAGCAGCCTGGGCACCCA<br>GACCTACATCTGCAACGTGAACCACAAGCCCAGCAACACCAAGGT<br>GGACGAGAAGGTGGAGCCCAAGAGCTGCGACAAAACTCACACATG<br>CCCACCGTGCCCAGCACCTGAAGCTGCAGGGGGACCGTCAGTCTT<br>CCTCTTCCCCCCAAAACCCAAGGACACCCTCATGATCTCCCGGACC<br>CCTGAGGTCACATGCGTGGTGGTGGACGTGAGCCACGAAGACCCT<br>GAGGTCAAGTTCAACTGGTACGTGGACGGCGTGGAGGTGCATAAT<br>GCCAAGACAAAGCCGCGGGAGGAGCAGTACAACAGCACGTACCGT<br>GTGGTCAGCGTCCTCACCGTCCTGCACCAGGACTGGCTGAATGGC<br>AAGGAGTACAAGTGCAAGGTCTCCAACAAAGCCCTCGGCGCCCCC<br>ATCGAGAAAACCATCTCCAAAGCCAAAGGGCAGCCCCGAGAACCA<br>CAGGTGTACACCCTGCCCCCATGCCGGGATGAGCTGACCAAGAAC<br>CAGGTCAGCCTGTGGTGCCTGGTCAAAGGCTTCTATCCCAGCGAC<br>ATCGCCGTGGAGTGGGAGAGCAATGGGCAGCCGGAGAACAACTAC<br>AAGACCACGCCTCCCGTGCTGGACTCCGACGGCTCCTTCTTCCTCT<br>ACAGCAAGCTCACCGTGGACAAGAGCAGGTGGCAGCAGGGGAAC<br>GTCTTCTCATGCTCCGTGATGCATGAGGCTCTGCACAACCACTACA<br>CGCAGAAGAGCCTCTCCCTGTCTCCGGGTAAATGA | 170 |

Although the foregoing invention has been described in some detail by way of illustration and example for purposes of clarity of understanding, the descriptions and examples should not be construed as limiting the scope of the invention.

**Claims**

1. A protease-activatable T cell activating bispecific molecule comprising

(a) a first antigen binding moiety capable of specific binding to CD3, wherein the first antigen binding moiety is an antibody or fragment thereof;
(b) a second antigen binding moiety capable of specific binding to a target cell antigen wherein the second antigen binding moiety is an antibody or fragment thereof; and
(c) a masking moiety covalently attached to the protease-activatable T cell activating bispecific molecule through a protease-cleavable linker, wherein the masking moiety is capable of specific binding to the idiotype of the first or the second antigen binding moiety thereby reversibly concealing the first or the second antigen binding moiety, wherein the masking moiety is an anti-idiotypic scFv.

2. The protease-activatable T cell activating bispecific molecule of claim 1, wherein the masking moiety is covalently attached to the first antigen binding moiety and reversibly conceals the first antigen binding moiety.

3. The protease-activatable T cell activating bispecific molecule of claim 1 or 2, wherein the masking moiety is covalently attached to the heavy chain variable region of the first antigen binding moiety.

4. The protease-activatable T cell activating bispecific molecule of any one of claims 1 to 3, wherein the second antigen binding moiety is capable of specific binding to a target cell antigen selected from the group consisting of FolRl, HER1, HER2 and Mesothelin.

5. The protease-activatable T cell activating bispecific molecule of any one of claims 1 to 4, wherein the first and the second antigen binding moiety are fused to each other, optionally via a peptide linker.

6. The protease-activatable T cell activating bispecific molecule of any one of claims 1 to 5, additionally comprising an Fc domain composed of a first and a second subunit capable of stable association.

7. The protease-activatable T cell activating bispecific molecule of claim 6, wherein the Fc domain is an IgG, specifically an $IgG_1$ or $IgG_4$, Fc domain.

8. The protease-activatable T cell activating bispecific molecule of any one of claims 6 or 7, wherein the Fc domain exhibits reduced binding affinity to an Fc receptor and/or reduced effector function, as compared to a native $IgG_1$ Fc domain.

9. The protease-activatable T cell activating bispecific molecule of any one of claims 1 to 8, wherein the masking moiety comprises a heavy chain variable region comprising:

(a) a heavy chain complementarity determining region CDR H1 amino acid sequence of SYGVS (SEQ ID NO:26);
(b) a CDR H2 amino acid sequence of IIWGDGSTNYHSALIS (SEQ ID NO:27);
(c) a CDR H3 amino acid sequence of GITTVVDDYYAMDY (SEQ ID NO:28); and a light chain variable region comprising:

(d) a light chain (CDR L)1 amino acid sequence of RASENIDSYLA (SEQ ID NO:29);
(e) a CDR L2 amino acid sequence of AATFLAD (SEQ ID NO:30); and
(f) a CDR L3 amino acid sequence of QHYYSTPYT (SEQ ID NO:31).

10. The protease-activatable T cell activating bispecific molecule of any one of claims 1 to 9, wherein the protease cleavable linker comprises at least one protease recognition sequence.

11. The protease-activatable T cell activating bispecific molecule of claim 10, wherein the protease cleavable linker comprises the protease recognition sequence RQARVVNG (SEQ ID NO:36).

12. The protease-activatable T cell activating bispecific molecule of any one of claims 1 to 11, wherein the first antigen binding moiety is capable of specific binding to CD3 and comprises a heavy chain variable region comprising:

a) a heavy chain complementarity determining region CDR H1 amino acid sequence of TYAMN (SEQ ID NO:44);

b) a CDR H2 amino acid sequence of RIRSKYNNYATYYADSVKG (SEQ ID NO:45); and
c) a CDR H3 amino acid sequence of HGNFGNSYVSWFAY (SEQ ID NO:46); and a light chain variable region comprising:

d) a light chain (CDR L)1 amino acid sequence of GSSTGAVTTSNYAN (SEQ ID NO:17);
e) a CDR L2 amino acid sequence of GTNKRAP (SEQ ID NO:18); and
f) a CDR L3 amino acid sequence of ALWYSNLWV (SEQ ID NO:19).

**13.** The protease-activatable T cell activating bispecific molecule of any one of claims 1 to 12, wherein the first antigen binding moiety is capable of specific binding to CD3 and comprises a heavy chain variable region comprising the amino acid sequence of SEQ ID NO: 43 and a light chain variable region comprising the amino acid sequence of SEQ ID NO: 55.

**14.** The protease-activatable T cell activating bispecific molecule of any one of claims 1 to 13, wherein the second antigen binding moiety is capable of specific binding to FolR1 and comprises a heavy chain variable region comprising:

a) a heavy chain complementarity determining region CDR H1 amino acid sequence of NAWMS (SEQ ID NO: 14);
b) a CDR H2 amino acid sequence of RIKSKTDGGTTDYAAPVKG (SEQ ID NO: 15); and
c) a CDR H3 amino acid sequence of PWEWSWYDY (SEQ ID NO: 16); and a light chain variable region comprising:
d) a light chain (CDR L)1 amino acid sequence of GSSTGAVTTSNYAN (SEQ ID NO: 17);
e) a CDR L2 amino acid sequence of GTNKRAP (SEQ ID NO: 18); and
f) a CDR L3 amino acid sequence of ALWYSNLWV (SEQ ID NO: 19).

**15.** An idiotype-specific polypeptide for reversibly concealing an anti-CD3 antigen binding site of a molecule, wherein the idiotype-specific polypeptide is an anti-idiotype scFv, wherein the idiotype-specific polypeptide is covalently attached to the molecule through a peptide linker.

**16.** The idiotype-specific polypeptide of claim 15, wherein the linker is a protease-cleavable linker.

**17.** The idiotype-specific polypeptide of claim 15 or of claim 16, wherein the peptide linker comprises at least one protease recognition site.

**18.** The idiotype-specific polypeptide of claim 17, wherein the protease cleavable linker comprises the protease recognition sequence RQARVVNG (SEQ ID NO:36).

**19.** The idiotype-specific polypeptide of any one of claims 17 or 18, wherein the molecule is a T-cell activating bispecific molecule.

**20.** The idiotype-specific polypeptide of any one of claims 15 to 19, comprising a heavy chain variable region comprising:

(a) a heavy chain complementarity determining region CDR H1 amino acid sequence of SYGVS (SEQ ID NO:26);
(b) a CDR H2 amino acid sequence of IIWGDGSTNYHSALIS (SEQ ID NO:27);
(c) a CDR H3 amino acid sequence of GITTVVDDYYAMDY (SEQ ID NO:28); and a light chain variable region comprising:

(d) a light chain (CDR L)1 amino acid sequence of RASENIDSYLA (SEQ ID NO:29);
(e) a CDR L2 amino acid sequence of AATFLAD (SEQ ID NO:30); and
(f) a CDR L3 amino acid sequence of QHYYSTPYT (SEQ ID NO:31).

**21.** A pharmaceutical composition comprising the protease-activatable T cell activating bispecific molecule of any one of claims 1 to 14 or the the idiotype-specific polypeptide of any one of claims 15 to 20 and a pharmaceutically acceptable carrier.

**22.** A protease-activatable T cell activating bispecific molecule of any one of claims 1 to 14, the idiotype-specific polypeptide of any one of claims 15 to 20 or the pharmaceutical composition of claim 21 for use as a medicament.

23. The protease-activatable T cell activating bispecific molecule for use according to claim 22, wherein the medicament is for treating or delaying progression of cancer, treating or delaying progression of an immune related disease, or enhancing or stimulating an immune response or function in an individual.

24. An isolated polynucleotide encoding the protease-activatable T cell activating bispecific antigen binding molecule of any one of claims 1 to 14 or the idiotype-specific polypeptide of any one of claims 15 to 20.

25. A vector, particularly an expression vector, comprising the polynucleotide of claim 24.

26. A host cell comprising the polynucleotide of claim 24 or the vector of claim 25.

27. A method of producing a protease-activatable T cell activating bispecific molecule or an idiotype-specific polypeptide, comprising the steps of a) culturing the host cell of claim 26 under conditions suitable for the expression of the protease-activatable T cell activating bispecific molecule or the idiotype-specific polypeptide and b) recovering the protease-activatable T cell activating bispecific molecule or the idiotype-specific polypeptide.

## Patentansprüche

1. Protease-aktivierbares T-Zell-aktivierendes bispezifisches Molekül, umfassend

   (a) eine erste antigenbindende Einheit, die zu spezifischer Bindung an CD3 fähig ist, wobei die erste antigenbindende Einheit ein Antikörper oder Fragment davon ist;
   (b) eine zweite antigenbindende Einheit, die zu spezifischer Bindung an ein Zielzellenantigen fähig ist, wobei die zweite antigenbindende Einheit ein Antikörper oder Fragment davon ist; und
   (c) eine Maskierungseinheit, die durch einen Protease-spaltbaren Linker kovalent an das Protease-aktivierbare T-Zell-aktivierende bispezifische Molekül gebunden ist, wobei die Maskierungseinheit zu spezifischer Bindung an den Idiotyp der ersten oder der zweiten antigenbindenden Einheit fähig ist, wodurch die erste oder die zweite antigenbindende Einheit reversibel verdeckt wird, wobei die Maskierungseinheit ein anti-idiotypisches scFv ist.

2. Protease-aktivierbares T-Zell-aktivierendes bispezifisches Molekül nach Anspruch 1, wobei die Maskierungseinheit kovalent an die erste antigenbindende Einheit gebunden ist und die erste antigenbindende Einheit reversibel verdeckt.

3. Protease-aktivierbares T-Zell-aktivierendes bispezifisches Molekül nach Anspruch 1 oder 2, wobei die Maskierungseinheit kovalent an die variable Region der schweren Kette der ersten antigenbindenden Einheit gebunden ist.

4. Protease-aktivierbares T-Zell-aktivierendes bispezifisches Molekül nach einem der Ansprüche 1 bis 3, wobei die zweite antigenbindende Einheit zu spezifischer Bindung an ein Zielzellenantigen fähig ist, das aus der Gruppe ausgewählt ist, die aus FolR1, HER1, HER2 und Mesothelin besteht.

5. Protease-aktivierbares T-Zell-aktivierendes bispezifisches Molekül nach einem der Ansprüche 1 bis 4, wobei die erste und die zweite antigenbindende Einheit gegebenenfalls über einen Peptid-Linker aneinander fusioniert sind.

6. Protease-aktivierbares T-Zell-aktivierendes bispezifisches Molekül nach einem der Ansprüche 1 bis 5, zusätzlich umfassend eine Fc-Domäne, die aus einer ersten und einer zweiten Untereinheit zusammengesetzt ist, die zu stabiler Assoziation fähig sind.

7. Protease-aktivierbares T-Zell-aktivierendes bispezifisches Molekül nach Anspruch 6, wobei die Fc-Domäne eine IgG-, speziell eine IgGi- oder $IgG_4$-Fc-Domäne ist.

8. Protease-aktivierbares T-Zell-aktivierendes bispezifisches Molekül nach einem der Ansprüche 6 oder 7, wobei die Fc-Domäne im Vergleich zu einer nativen $IgG_1$-Fc-Domäne reduzierte Bindungsaffinität zu einem Fc-Rezeptor und/oder reduzierte Effektorfunktion zeigt.

9. Protease-aktivierbares T-Zell-aktivierendes bispezifisches Molekül nach einem der Ansprüche 1 bis 8, wobei die Maskierungseinheit eine variable Region der schweren Kette, umfassend:

(a) eine Aminosäuresequenz von SYGVS der komplementaritätsbestimmenden Region der schweren Kette CDR H1 (SEQ ID NO:26);
(b) eine Aminosäuresequenz von IIWGDGSTNYHSALIS der CDR H2 (SEQ ID NO:27);
(c) eine Aminosäuresequenz von GITTVVDDYYAMDY der CDR H3 (SEQ ID NO:28); und eine variable Region der leichten Kette, umfassend:

(d) eine Aminosäuresequenz von RASENIDSYLA der leichten Kette (CDR L)1 (SEQ ID NO:29);
(e) eine Aminosäuresequenz von AATFLAD der CDR L2 (SEQ ID NO:30); und
(f) eine Aminosäuresequenz von QHYYSTPYT der CDR L3 (SEQ ID NO:31), umfasst.

**10.** Protease-aktivierbares T-Zell-aktivierendes bispezifisches Molekül nach einem der Ansprüche 1 bis 9, wobei der Protease-spaltbare Linker mindestens eine Proteaseerkennungssequenz umfasst.

**11.** Protease-aktivierbares T-Zell-aktivierendes bispezifisches Molekül nach Anspruch 10, wobei der Protease-spaltbare Linker die Proteaseerkennungssequenz RQARVVNG (SEQ ID NO:36) umfasst.

**12.** Protease-aktivierbares T-Zell-aktivierendes bispezifisches Molekül nach einem der Ansprüche 1 bis 11, wobei die erste antigenbindende Einheit zu spezifischer Bindung an CD3 fähig ist und eine variable Region der schweren Kette, umfassend:

a) eine Aminosäuresequenz von TYAMN der komplementaritätsbestimmenden Region der schweren Kette CDR H1 (SEQ ID NO:44);
b) eine Aminosäuresequenz von RIRSKYNNYATYYADSVKG der CDR H2 (SEQ ID NO:45); und
c) eine Aminosäuresequenz von HGNFGNSYVSWFAY der CDR H3 (SEQ ID NO:46); und eine variable Region der leichten Kette, umfassend:

d) eine Aminosäuresequenz von GSSTGAVTTSNYAN der leichten Kette (CDR L)1 (SEQ ID NO:17);
e) eine Aminosäuresequenz von GTNKRAP der CDR L2 (SEQ ID NO:18); und
f) eine Aminosäuresequenz von ALWYSNLWV der CDR L3 (SEQ ID NO:19), umfasst.

**13.** Protease-aktivierbares T-Zell-aktivierendes bispezifisches Molekül nach einem der Ansprüche 1 bis 12, wobei die erste antigenbindende Einheit zu spezifischer Bindung an CD3 fähig ist und eine variable Region der schweren Kette, umfassend die Aminosäuresequenz von SEQ ID NO:43, und eine variable Region der leichten Kette, umfassend die Aminosäuresequenz von SEQ ID NO:55, umfasst.

**14.** Protease-aktivierbares T-Zell-aktivierendes bispezifisches Molekül nach einem der Ansprüche 1 bis 13, wobei die zweite antigenbindende Einheit zu spezifischer Bindung an FolR1 fähig ist und eine variable Region der schweren Kette, umfassend:

a) eine Aminosäuresequenz von NAWMS der komplementaritätsbestimmenden Region der schweren Kette CDR H1 (SEQ ID NO:14);
b) eine Aminosäuresequenz von RIKSKTDGGTTDYAAPVKG der CDR H2 (SEQ ID NO:15); und
c) eine Aminosäuresequenz von PWEWSWYDY der CDR H3 (SEQ ID NO:16); und eine variable Region der leichten Kette, umfassend:

d) eine Aminosäuresequenz von GSSTGAVTTSNYAN der leichten Kette (CDR L)1 (SEQ ID NO:17);
e) eine Aminosäuresequenz von GTNKRAP der CDR L2 (SEQ ID NO:18); und
f) eine Aminosäuresequenz von ALWYSNLWV der CDR L3 (SEQ ID NO:19), umfasst.

**15.** Idiotyp-spezifisches Polypeptid zum reversiblen Verdecken einer Anti-CD3-Antigenbindungsstelle eines Moleküls, wobei das Idiotyp-spezifische Polypeptid ein Anti-Idiotyp-scFv ist, wobei das Idiotyp-spezifische Polypeptid durch einen Peptid-Linker kovalent an das Molekül gebunden ist.

**16.** Idiotyp-spezifisches Polypeptid nach Anspruch 15, wobei der Linker ein Proteasespaltbarer Linker ist.

**17.** Idiotyp-spezifisches Polypeptid nach Anspruch 15 oder nach Anspruch 16, wobei der Peptid-Linker mindestens eine Proteaseerkennungsstelle umfasst.

**18.** Idiotyp-spezifisches Polypeptid nach Anspruch 17, wobei der Protease-spaltbare Linker die Proteaseerkennungssequenz RQARVVNG (SEQ ID NO:36) umfasst.

**19.** Idiotyp-spezifisches Polypeptid nach einem der Ansprüche 17 oder 18, wobei das Molekül ein T-Zell-aktivierendes bispezifisches Molekül ist.

**20.** Idiotyp-spezifisches Polypeptid nach einem der Ansprüche 15 bis 19, umfassend eine variable Region der schweren Kette, umfassend:

(a) eine Aminosäuresequenz von SYGVS der komplementaritätsbestimmenden Region der schweren Kette CDR H1 (SEQ ID NO:26);
(b) eine Aminosäuresequenz von IIWGDGSTNYHSALIS der CDR H2 (SEQ ID NO:27);
(c) eine Aminosäuresequenz von GITTVVDDYYAMDY der CDR H3 (SEQ ID NO:28); und eine variable Region der leichten Kette, umfassend:

(d) eine Aminosäuresequenz von RASENIDSYLA der leichten Kette (CDR L)1 (SEQ ID NO:29);
(e) eine Aminosäuresequenz von AATFLAD der CDR L2 (SEQ ID NO:30); und
(f) eine Aminosäuresequenz von QHYYSTPYT der CDR L3 (SEQ ID NO:31).

**21.** Pharmazeutische Zusammensetzung, umfassend das Protease-aktivierbare T-Zell-aktivierende bispezifische Molekül nach einem der Ansprüche 1 bis 14 oder das Idiotyp-spezifische Polypeptid nach einem der Ansprüche 15 bis 20 und einen pharmazeutisch unbedenklichen Träger.

**22.** Protease-aktivierbares T-Zell-aktivierendes bispezifisches Molekül nach einem der Ansprüche 1 bis 14, Idiotyp-spezifisches Polypeptid nach einem der Ansprüche 15 bis 20 oder pharmazeutische Zusammensetzung nach Anspruch 21 zur Verwendung als ein Medikament.

**23.** Protease-aktivierbares T-Zell-aktivierendes bispezifisches Molekül zur Verwendung nach Anspruch 22, wobei das Medikament zur Behandlung oder Verzögerung des Fortschreitens von Krebs, Behandlung oder Verzögerung des Fortschreitens einer Immunkrankheit oder Verstärkung oder Stimulierung einer Immunantwort oder -funktion bei einem Individuum vorgesehen ist.

**24.** Isoliertes Polynukleotid, das für das Protease-aktivierbare T-Zell-aktivierende bispezifische antigenbindende Molekül nach einem der Ansprüche 1 bis 14 oder das Idiotyp-spezifische Polypeptid nach einem der Ansprüche 15 bis 20 kodiert.

**25.** Vektor, insbesondere ein Expressionsvektor, umfassend das Polynukleotid nach Anspruch 24.

**26.** Wirtszelle, umfassend das Polynukleotid nach Anspruch 24 oder den Vektor nach Anspruch 25.

**27.** Verfahren zum Produzieren eines Protease-aktivierbaren T-Zell-aktivierenden bispezifischen Moleküls oder eines Idiotyp-spezifischen Polypeptids, umfassend die Schritte a) Kultivieren der Wirtszelle nach Anspruch 26 unter Bedingungen, die für die Expression des Protease-aktivierbaren T-Zell-aktivierenden bispezifischen Moleküls oder des Idiotyp-spezifischen Polypeptids geeignet sind, und b) Gewinnen des Protease-aktivierbaren T-Zell-aktivierenden bispezifischen Moleküls oder des Idiotyp-spezifischen Polypeptids.

## Revendications

**1.** Molécule bispécifique d'activation de cellules T activable par une protéase comprenant

(a) une première fraction de liaison à l'antigène capable de se lier spécifiquement à CD3, dans laquelle la première fraction de liaison à l'antigène est un anticorps ou un fragment de celui-ci ;
(b) une seconde fraction de liaison à l'antigène capable de se lier spécifiquement à un antigène de cellule cible, dans laquelle la seconde fraction de liaison à l'antigène est un anticorps ou un fragment de celui-ci ; et
(c) une fraction de masquage liée de manière covalente à la molécule bispécifique d'activation de cellules T activable par une protéase par l'intermédiaire d'un lieur clivable par une protéase, dans laquelle la fraction de masquage est capable de se lier spécifiquement à l'idiotype de la première ou de la seconde fraction de liaison

à l'antigène cachant ainsi de manière réversible la première ou la seconde fraction de liaison à l'antigène, dans laquelle la fraction de masquage est un scFv anti-idiotypique.

2. Molécule bispécifique d'activation de cellules T activable par une protéase selon la revendication 1, dans laquelle la fraction de masquage est liée de manière covalente à la première fraction de liaison à l'antigène et cache de manière réversible la première fraction de liaison à l'antigène.

3. Molécule bispécifique d'activation de cellules T activable par une protéase selon la revendication 1 ou 2, dans laquelle la fraction de masquage est liée de manière covalente à la région variable de chaîne lourde de la première fraction de liaison à l'antigène.

4. Molécule bispécifique d'activation de cellules T activable par une protéase selon l'une quelconque des revendications 1 à 3, dans laquelle la seconde fraction de liaison à l'antigène est capable de se lier spécifiquement à un antigène de cellule cible choisi dans le groupe constitué par FolR1, HER1, HER2 et la mésothéline.

5. Molécule bispécifique d'activation de cellules T activable par une protéase selon l'une quelconque des revendications 1 à 4, dans laquelle la première et la seconde fraction de liaison à l'antigène sont fusionnées l'une à l'autre, éventuellement par le biais d'un lieur peptidique.

6. Molécule bispécifique d'activation de cellules T activable par une protéase selon l'une quelconque des revendications 1 à 5, comprenant en outre un domaine Fc composé d'une première et d'une seconde sous-unité capables d'une association stable.

7. Molécule bispécifique d'activation de cellules T activable par une protéase selon la revendication 6, dans laquelle le domaine Fc est un domaine Fc d'IgG, plus particulièrement d'$IgG_1$ ou d'$IgG_4$.

8. Molécule bispécifique d'activation de cellules T activable par une protéase selon l'une quelconque des revendications 6 ou 7, dans laquelle le domaine Fc présente une affinité de liaison à un récepteur Fc réduite et/ou une fonction effectrice réduite, par comparaison avec un domaine Fc d'$IgG_1$ native.

9. Molécule bispécifique d'activation de cellules T activable par une protéase selon l'une quelconque des revendications 1 à 8, dans laquelle la fraction de masquage comprend une région variable de chaîne lourde comprenant :

(a) une séquence d'acides aminés de région déterminant la complémentarité de chaîne lourde CDR-H1 de SYGVS (SEQ ID NO : 26) ;
(b) une séquence d'acides aminés de CDR-H2 de IIWGDGSTNYHSALIS (SEQ ID NO : 27) ;
(c) une séquence d'acides aminés de CDR-H3 de GITTVVDDYYAMDY (SEQ ID NO : 28) ; et une région variable de chaîne légère comprenant :

(d) une séquence d'acides aminés de chaîne légère (CDR-L)1 de RASENIDSYLA (SEQ ID NO : 29) ;
(e) une séquence d'acides aminés de CDR-L2 de AATFLAD (SEQ ID NO : 30) ; et
(f) une séquence d'acides aminés de CDR-L3 de QHYYSTPYT (SEQ ID NO : 31).

10. Molécule bispécifique d'activation de cellules T activable par une protéase selon l'une quelconque des revendications 1 à 9, dans laquelle le lieur clivable par une protéase comprend au moins une séquence de reconnaissance de protéase.

11. Molécule bispécifique d'activation de cellules T activable par une protéase selon la revendication 10, dans laquelle le lieur clivable par une protéase comprend la séquence de reconnaissance de protéase RQARVVNG (SEQ ID NO : 36).

12. Molécule bispécifique d'activation de cellules T activable par une protéase selon l'une quelconque des revendications 1 à 11, dans laquelle la première fraction de liaison à l'antigène est capable de se lier spécifiquement à CD3 et comprend une région variable de chaîne lourde comprenant :

a) une séquence d'acides aminés de région déterminant la complémentarité de chaîne lourde CDR-H1 de TYAMN (SEQ ID NO : 44) ;
b) une séquence d'acides aminés de CDR-H2 de RIRSKYNNYATYYADSVKG (SEQ ID NO : 45) ; et

c) une séquence d'acides aminés de CDR-H3 de HGNFGNSYVSWFAY (SEQ ID NO : 46) ; et une région variable de chaîne légère comprenant :

d) une séquence d'acides aminés de chaîne légère (CDR-L)1 de GSSTGAVTTSNYAN (SEQ ID NO : 17) ;
e) une séquence d'acides aminés de CDR-L2 de GTNKRAP (SEQ ID NO : 18) ; et
f) une séquence d'acides aminés de CDR-L3 de ALWYSNLWV (SEQ ID NO : 19).

**13.** Molécule bispécifique d'activation de cellules T activable par une protéase selon l'une quelconque des revendications 1 à 12, dans laquelle la première fraction de liaison à l'antigène est capable de se lier spécifiquement à CD3 et comprend une région variable de chaîne lourde comprenant la séquence d'acides aminés de SEQ ID NO : 43 et une région variable de chaîne légère comprenant la séquence d'acides aminés de SEQ ID NO : 55.

**14.** Molécule bispécifique d'activation de cellules T activable par une protéase selon l'une quelconque des revendications 1 à 13, dans laquelle la seconde fraction de liaison à l'antigène est capable de se lier spécifiquement à FolR1 et comprend une région variable de chaîne lourde comprenant :

a) une séquence d'acides aminés de région déterminant la complémentarité de chaîne lourde CDR-H1 de NAWMS (SEQ ID NO : 14) ;
b) une séquence d'acides aminés de CDR-H2 de RIKSKTDGGTTDYAAPVKG (SEQ ID NO : 15) ; et
c) une séquence d'acides aminés de CDR-H3 de PWEWSWYDY (SEQ ID NO : 16) ; et une région variable de chaîne légère comprenant :

d) une séquence d'acides aminés de chaîne légère (CDR-L)1 de GSSTGAVTTSNYAN (SEQ ID NO : 17) ;
e) une séquence d'acides aminés de CDR-L2 de GTNKRAP (SEQ ID NO : 18) ; et
f) une séquence d'acides aminés de CDR-L3 de ALWYSNLWV (SEQ ID NO : 19).

**15.** Polypeptide spécifique d'un idiotype pour cacher de manière réversible un site de liaison à l'antigène anti-CD3 d'une molécule, dans lequel le polypeptide spécifique d'un idiotype est un scFv anti-idiotype, dans lequel le polypeptide spécifique d'un idiotype est lié de manière covalente à la molécule par l'intermédiaire d'un lieur peptidique.

**16.** Polypeptide spécifique d'un idiotype selon la revendication 15, dans lequel le lieur est un lieur clivable par une protéase.

**17.** Polypeptide spécifique d'un idiotype selon la revendication 15 ou selon la revendication 16, dans lequel le lieur peptidique comprend
au moins un site de reconnaissance de protéase.

**18.** Polypeptide spécifique d'un idiotype selon la revendication 17, dans lequel le lieur clivable par une protéase comprend la séquence de reconnaissance de protéase RQARVVNG (SEQ ID NO : 36).

**19.** Polypeptide spécifique d'un idiotype selon l'une quelconque des revendications 17 ou 18, dans lequel la molécule est une molécule bispécifique d'activation de cellules T.

**20.** Polypeptide spécifique d'un idiotype selon l'une quelconque des revendications 15 à 19, comprenant une région variable de chaîne lourde comprenant :

(a) une séquence d'acides aminés de région déterminant la complémentarité de chaîne lourde CDR-H1 de SYGVS (SEQ ID NO : 26) ;
(b) une séquence d'acides aminés de CDR-H2 de IIWGDGSTNYHSALIS (SEQ ID NO : 27) ;
(c) une séquence d'acides aminés de CDR-H3 de GITTVVDDYYAMDY (SEQ ID NO : 28) ; et une région variable de chaîne légère comprenant :

(d) une séquence d'acides aminés de chaîne légère (CDR-L)1 de RASENIDSYLA (SEQ ID NO : 29) ;
(e) une séquence d'acides aminés de CDR-L2 de AATFLAD (SEQ ID NO : 30) ; et
(f) une séquence d'acides aminés de CDR-L3 de QHYYSTPYT (SEQ ID NO : 31).

**21.** Composition pharmaceutique comprenant la molécule bispécifique d'activation de cellules T activable par une protéase selon l'une quelconque des revendications 1 à 14 ou le polypeptide spécifique d'un idiotype selon l'une

quelconque des revendications 15 à 20 et un véhicule pharmaceutiquement acceptable.

**22.** Molécule bispécifique d'activation de cellules T activable par une protéase selon l'une quelconque des revendications 1 à 14, polypeptide spécifique d'un idiotype selon l'une quelconque des revendications 15 à 20 ou composition pharmaceutique selon la revendication 21 pour une utilisation comme médicament.

**23.** Molécule bispécifique d'activation de cellules T activable par une protéase pour une utilisation selon la revendication 22, dans laquelle le médicament est pour le traitement ou le ralentissement de la progression d'un cancer, le traitement ou le ralentissement de la progression d'une maladie immunitaire, ou l'amélioration ou la stimulation d'une réponse ou d'une fonction immunitaire chez un individu.

**24.** Polynucléotide isolé codant pour la molécule de liaison à l'antigène bispécifique d'activation de cellules T activable par une protéase selon l'une quelconque des revendications 1 à 14 ou le polypeptide spécifique d'un idiotype selon l'une quelconque des revendications 15 à 20.

**25.** Vecteur, en particulier vecteur d'expression, comprenant le polynucléotide selon la revendication 24.

**26.** Cellule hôte comprenant le polynucléotide selon la revendication 24 ou le vecteur selon la revendication 25.

**27.** Procédé de production d'une molécule bispécifique d'activation de cellules T activable par une protéase ou d'un polypeptide spécifique d'un idiotype, comprenant les étapes de a) culture de la cellule hôte selon la revendication 26 dans des conditions appropriées pour l'expression de la molécule bispécifique d'activation de cellules T activable par une protéase ou du polypeptide spécifique d'un idiotype et b) récupération de la molécule bispécifique d'activation de cellules T activable par une protéase ou du polypeptide spécifique d'un idiotype.

FIG. 1A

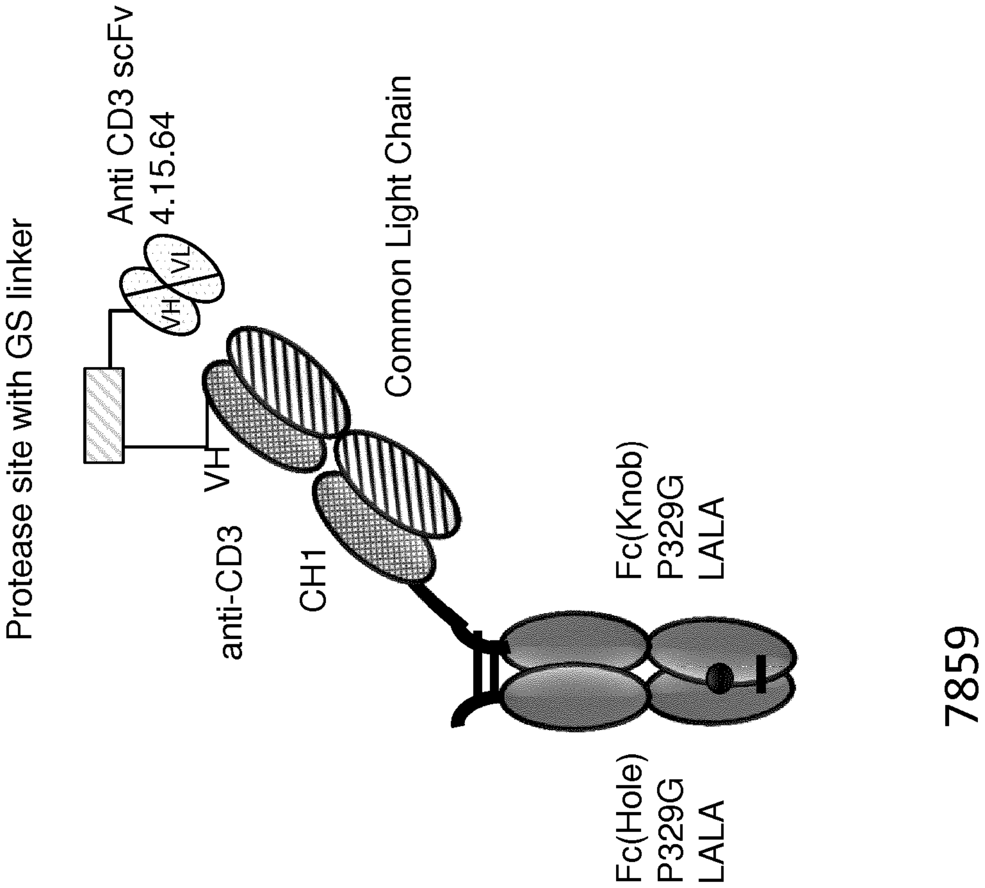
Protease site with GS linker
Anti CD3 scFv
4.15.64
VH
VL
Common Light Chain
VH
anti-CD3
CH1
Fc(Knob)
P329G
LALA
Fc(Hole)
P329G
LALA
7859

FIG. 1B

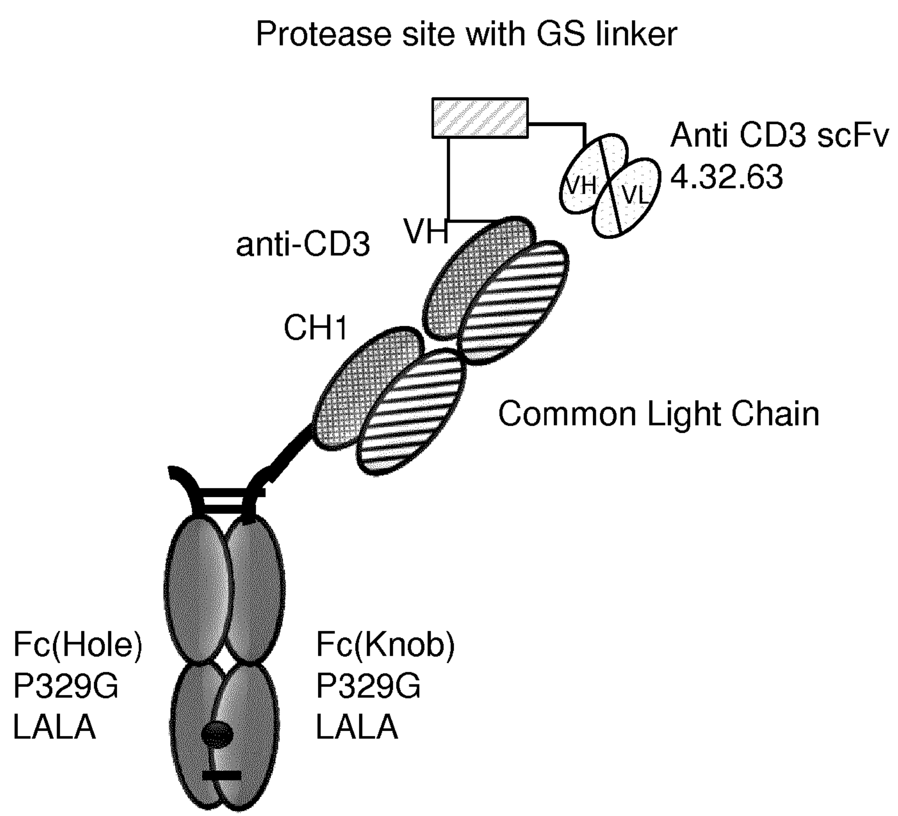
Protease site with GS linker
Anti CD3 scFv
4.32.63
VH
VL
anti-CD3
VH
CH1
Common Light Chain
Fc(Hole)
P329G
LALA
Fc(Knob)
P329G
LALA

7860

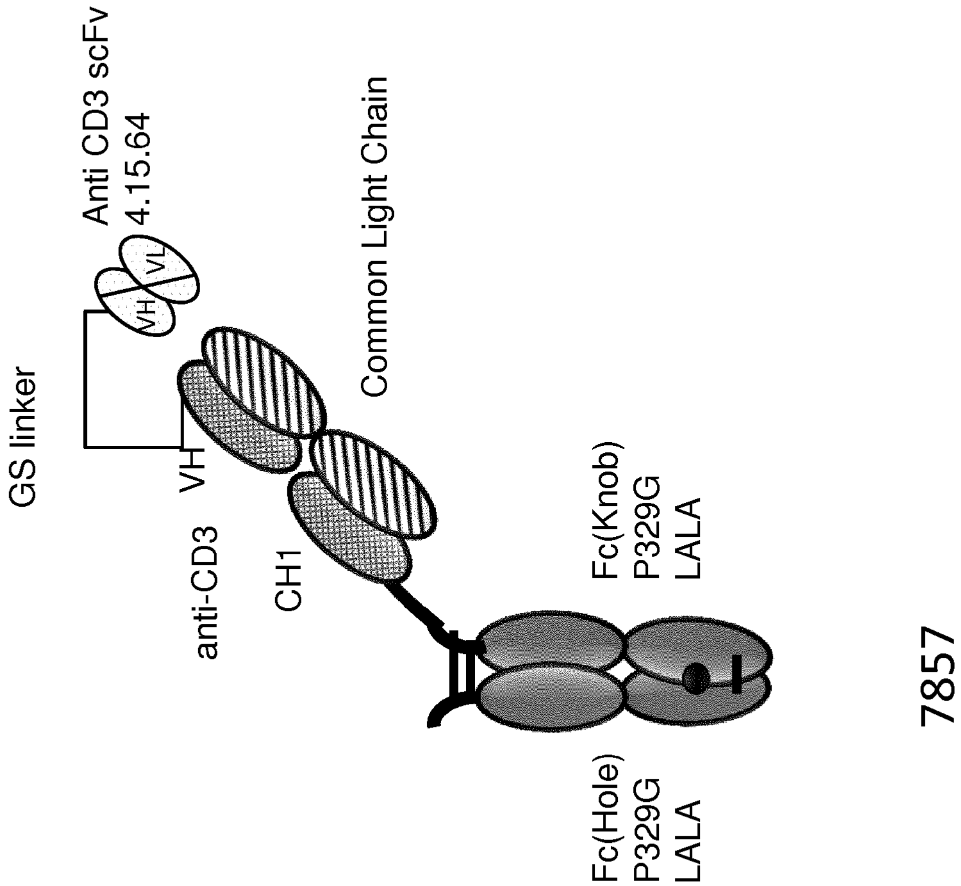
Anti CD3 scFv
4.15.64
VH
VL
GS linker
VH
anti-CD3
CH1
Common Light Chain
Fc(Knob)
P329G
LALA
Fc(Hole)
P329G
LALA
7857

FIG. 1C

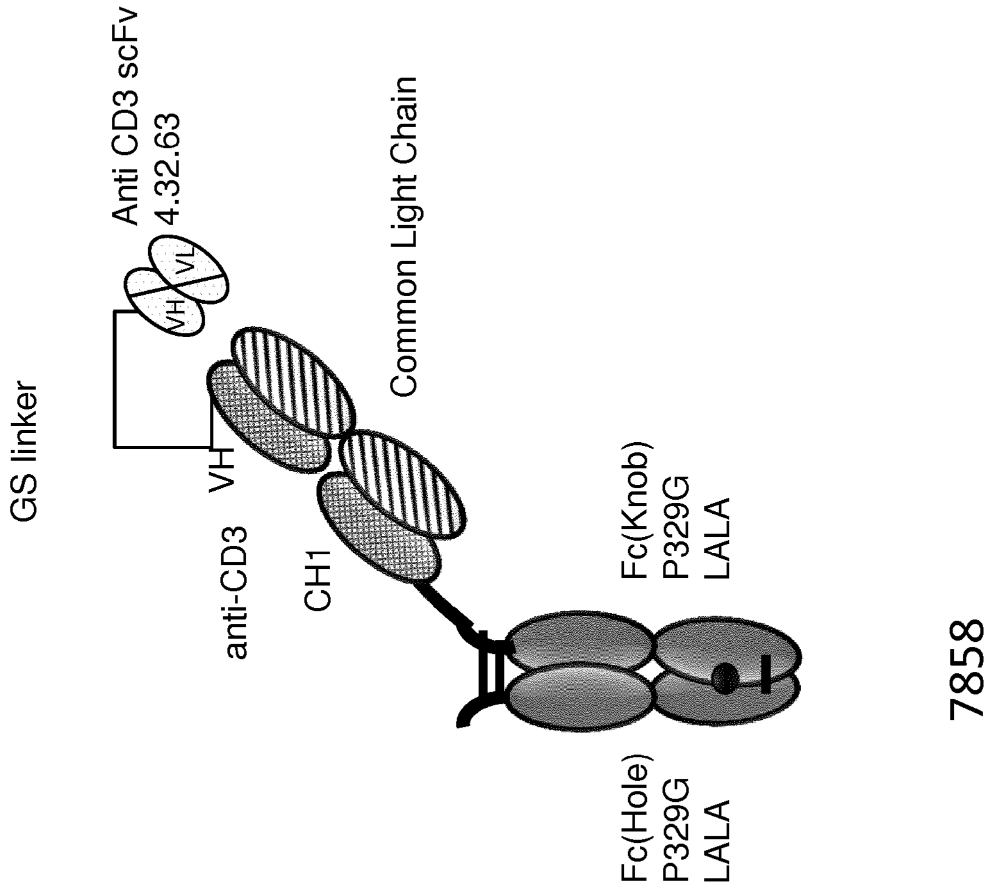
GS linker
Anti CD3 scFv
4.32.63
VH
VL
VH
anti-CD3
CH1
Common Light Chain
Fc(Knob)
P329G
LALA
Fc(Hole)
P329G
LALA
7858

FIG. 1D

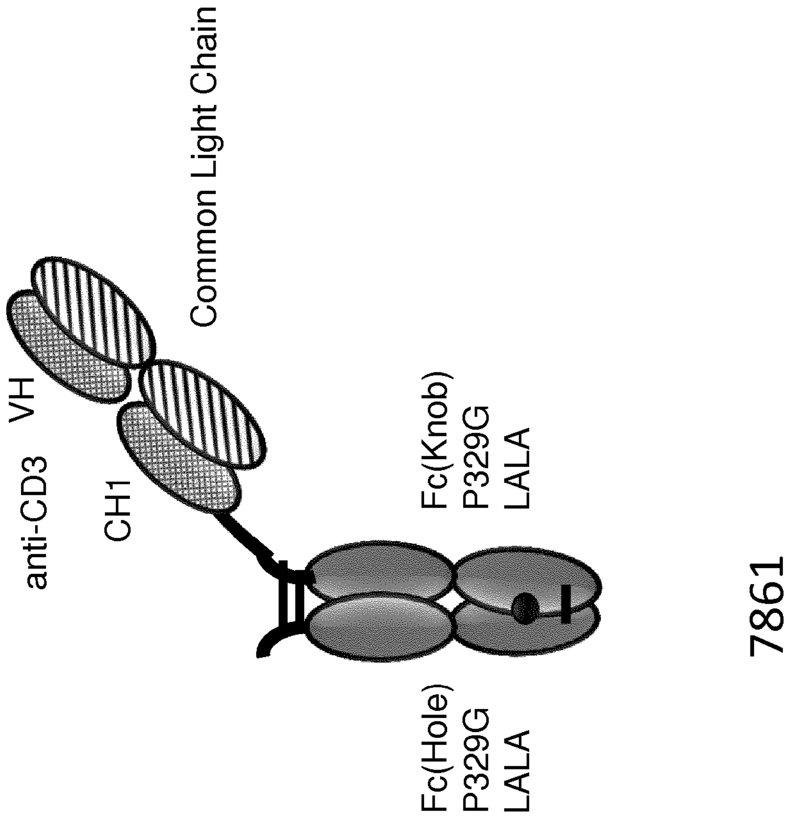
Common Light Chain
anti-CD3 VH
CH1
Fc(Knob)
P329G
LALA
Fc(Hole)
P329G
LALA
7861

FIG. 1E

FIG. 2

| Clone ID | Isotype | Temp. | $k_a$ (1/Ms) | $k_d$ (1/s) | $t_{1/2}$ diss. (min) | $K_D$ (nM) |
|---|---|---|---|---|---|---|
| 4.32.63 | IgG1, kappa | 25°C | 1.96E+05 | 1.05E-04 | 110 | 1 |
| | | 37°C | 3.46E+05 | 6.52E-04 | 18 | 2 |
| 4.35.77 | IgG1, kappa | 25°C | 1.66E+05 | 2.09E-04 | 55 | 1 |
| | | 37°C | 2.68E+05 | 6.96E-04 | 17 | 3 |
| 4.33.88 | IgG2b, kappa | 25°C | 2.71E+05 | 1.87E-03 | 6 | 7 |
| | | 37°C | 4.28E+05 | 3.24E-03 | 4 | 8 |
| 4.24.72 | IgG1, kappa | 25°C | 8.72E+04 | 1.18E-03 | 10 | 14 |
| | | 37°C | 1.55E+05 | 4.45E-03 | 3 | 29 |
| 4.21.78 | IgG1, kappa | 25°C | 1.31E+05 | 3.06E-03 | 4 | 23 |
| | | 37°C | 4.25E+05 | 9.28E-03 | 1 | 22 |
| 4.15.64 | IgG2b, kappa | 25°C | 2.61E+04 | 2.50E-03 | 5 | 96 |
| | | 37°C | 1.44E+05 | 1.35E-02 | 1 | 94 |

FIG. 3B

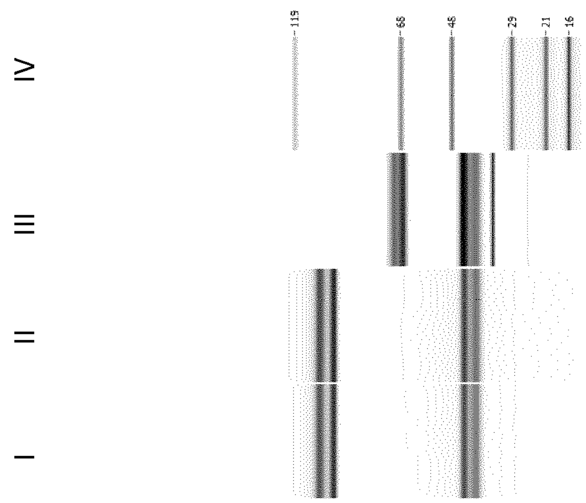


FIG. 3A

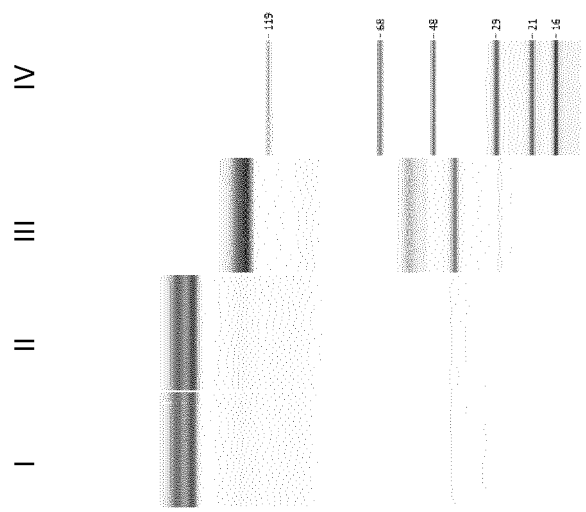

FIG. 3D

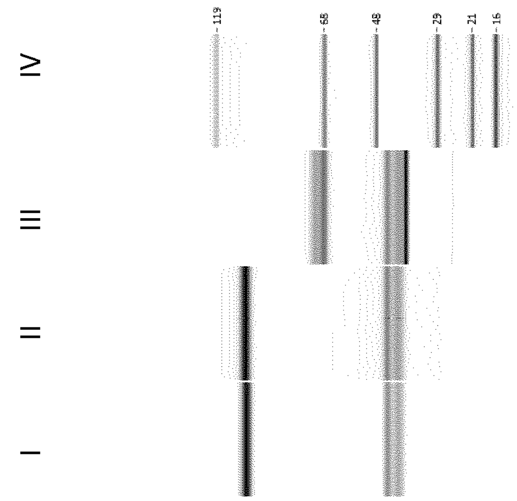


FIG. 3C

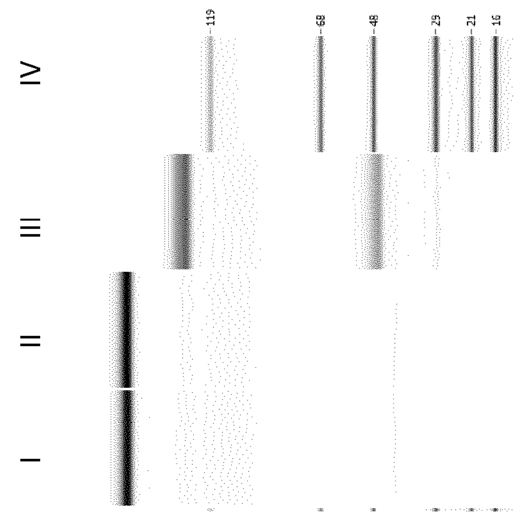

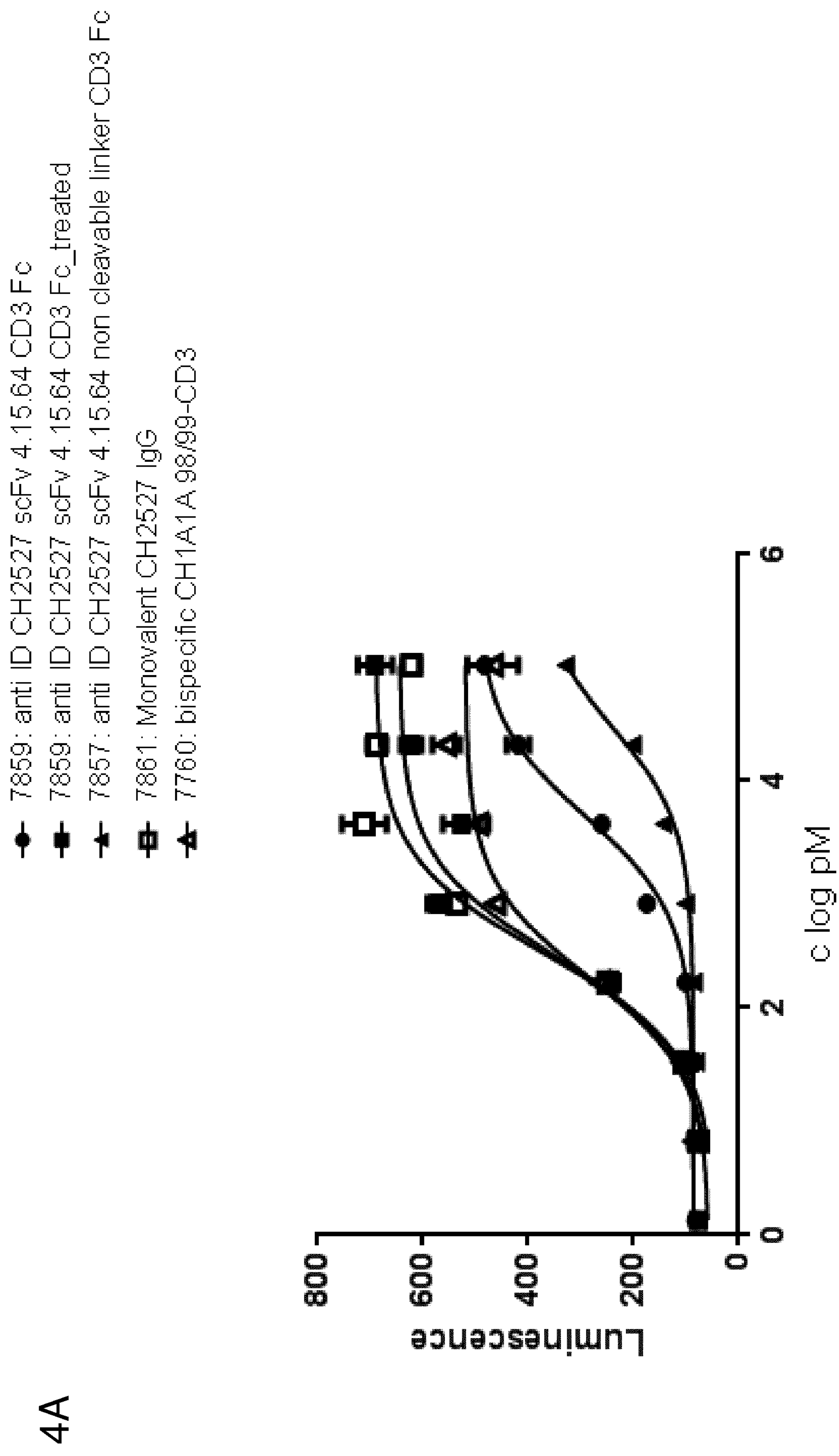
7859: anti ID CH2527 scFv 4.15.64 CD3 Fc
7859: anti ID CH2527 scFv 4.15.64 CD3 Fc_treated
7857: anti ID CH2527 scFv 4.15.64 non cleavable linker CD3 Fc
7861: Monovalent CH2527 IgG
7760: bispecific CH1A1A 98/99-CD3
Luminescence
800
600
400
200
0
0
2
4
6
c log pM

FIG. 4A

FIG. 4B

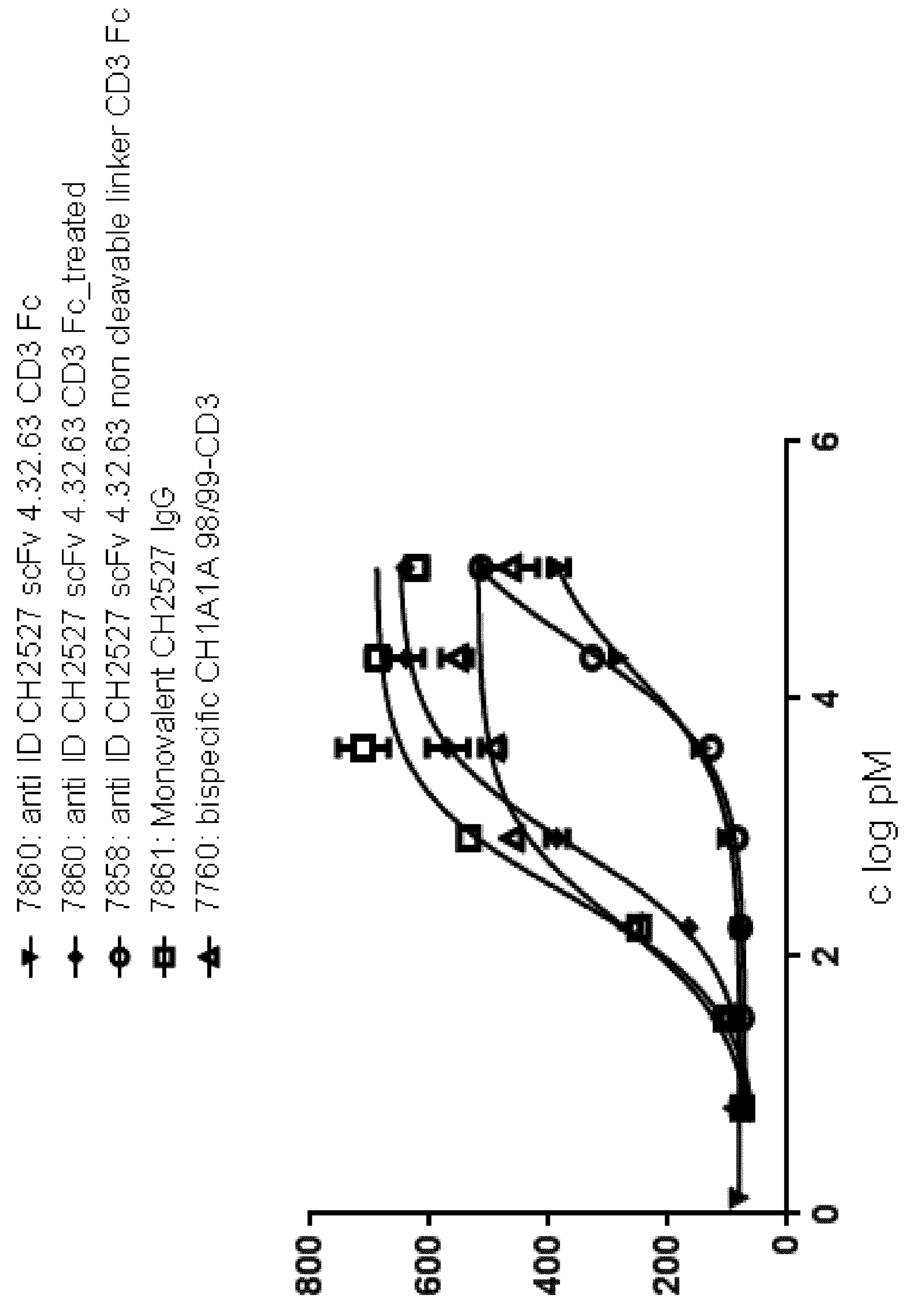
7860: anti ID CH2527 scFv 4.32.63 CD3 Fc
7860: anti ID CH2527 scFv 4.32.63 CD3 Fc_treated
7858: anti ID CH2527 scFv 4.32.63 non cleavable linker CD3 Fc
7861: Monovalent CH2527 IgG
7760: bispecific CH1A1A 98/99-CD3
Luminescence
800
600
400
200
0
0
2
4
6
c log pM

FIG. 4C

| EC50 | | |
|---|---|---|
| 7859 scFv 4.15.64 MK062 protease site | 4710 | 16 fold more than 7861 |
| 7861 | 286 | 16 fold less than 7859 |
| 7860 scFv 4.32.63 MK062 protease site | 15538 | 54 fold more than 7861 |
| 7861 | 286 | 54 fold less than 7860 |

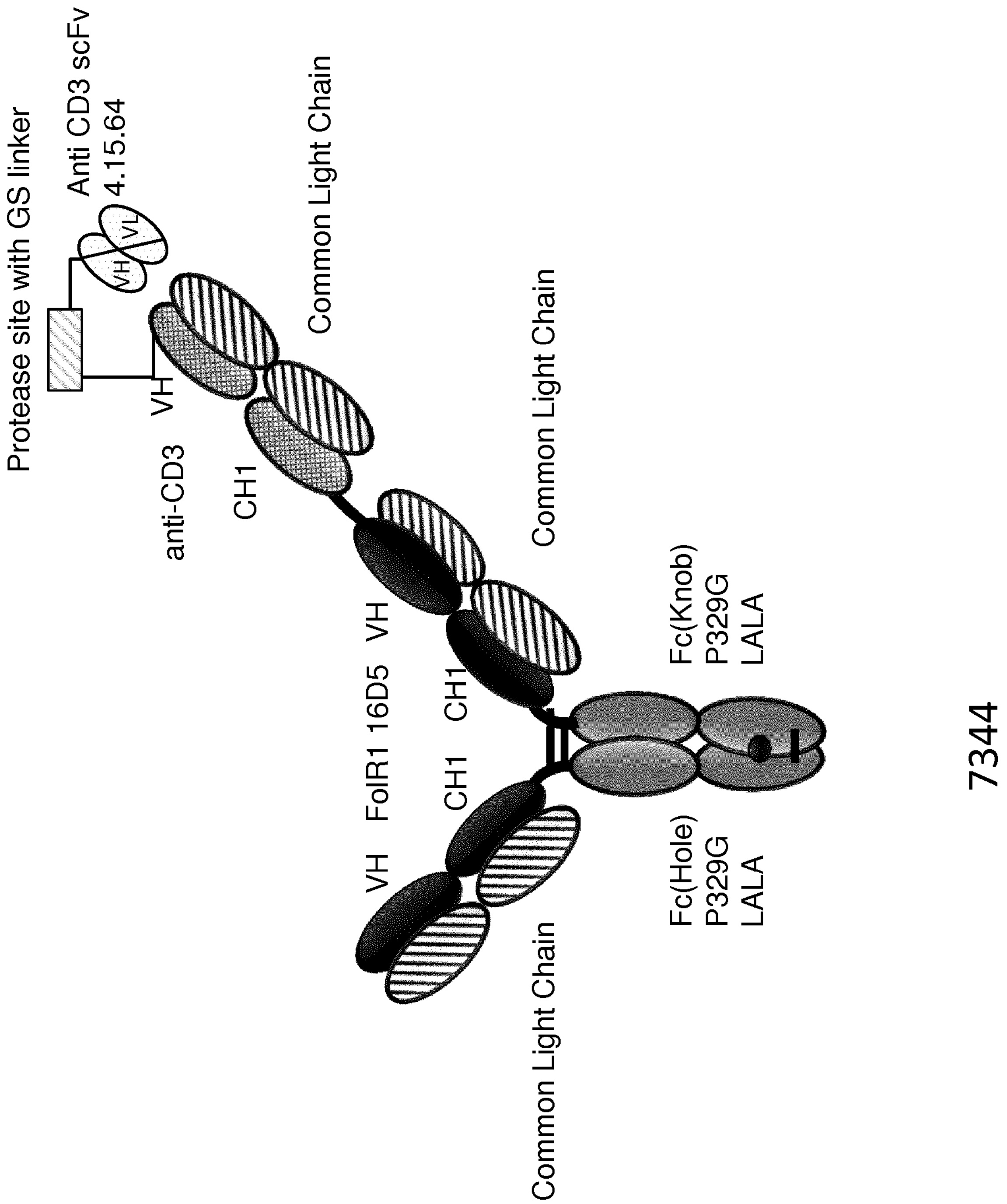
Protease site with GS linker
Anti CD3 scFv
4.15.64
VH
VL
Common Light Chain
anti-CD3
VH
CH1
Common Light Chain
Common Light Chain
FolR1 16D5
VH
CH1
CH1
VH
Fc(Knob)
P329G
LALA
Fc(Hole)
P329G
LALA
Common Light Chain
7344

FIG. 5A

FIG. 5B

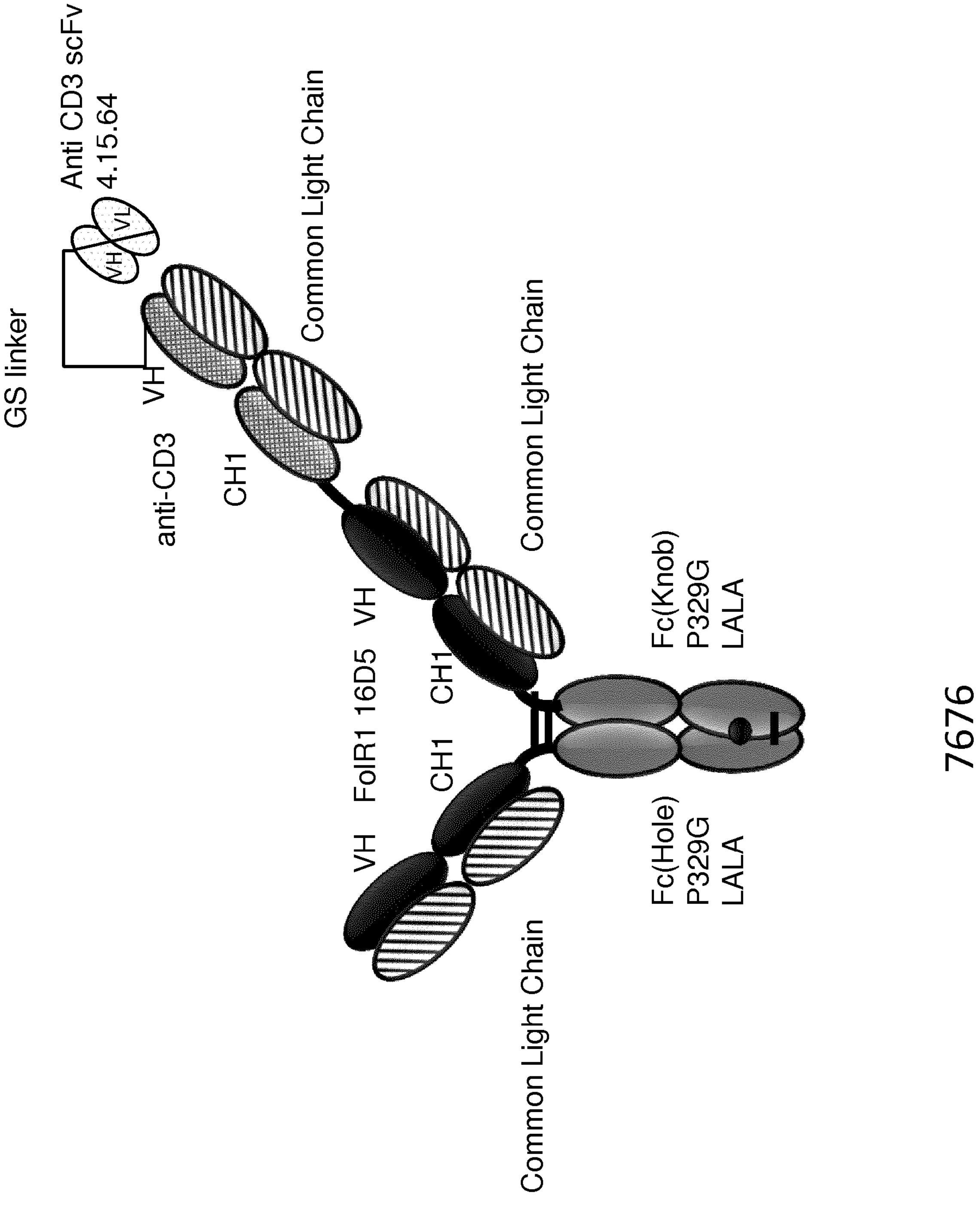
GS linker
Anti CD3 scFv
4.15.64
VH
VL
VH
anti-CD3
CH1
Common Light Chain
Common Light Chain
Common Light Chain
FolR1 16D5 VH
CH1
CH1
VH
Fc(Knob)
P329G
LALA
Fc(Hole)
P329G
LALA
Common Light Chain
7676

FIG. 5C

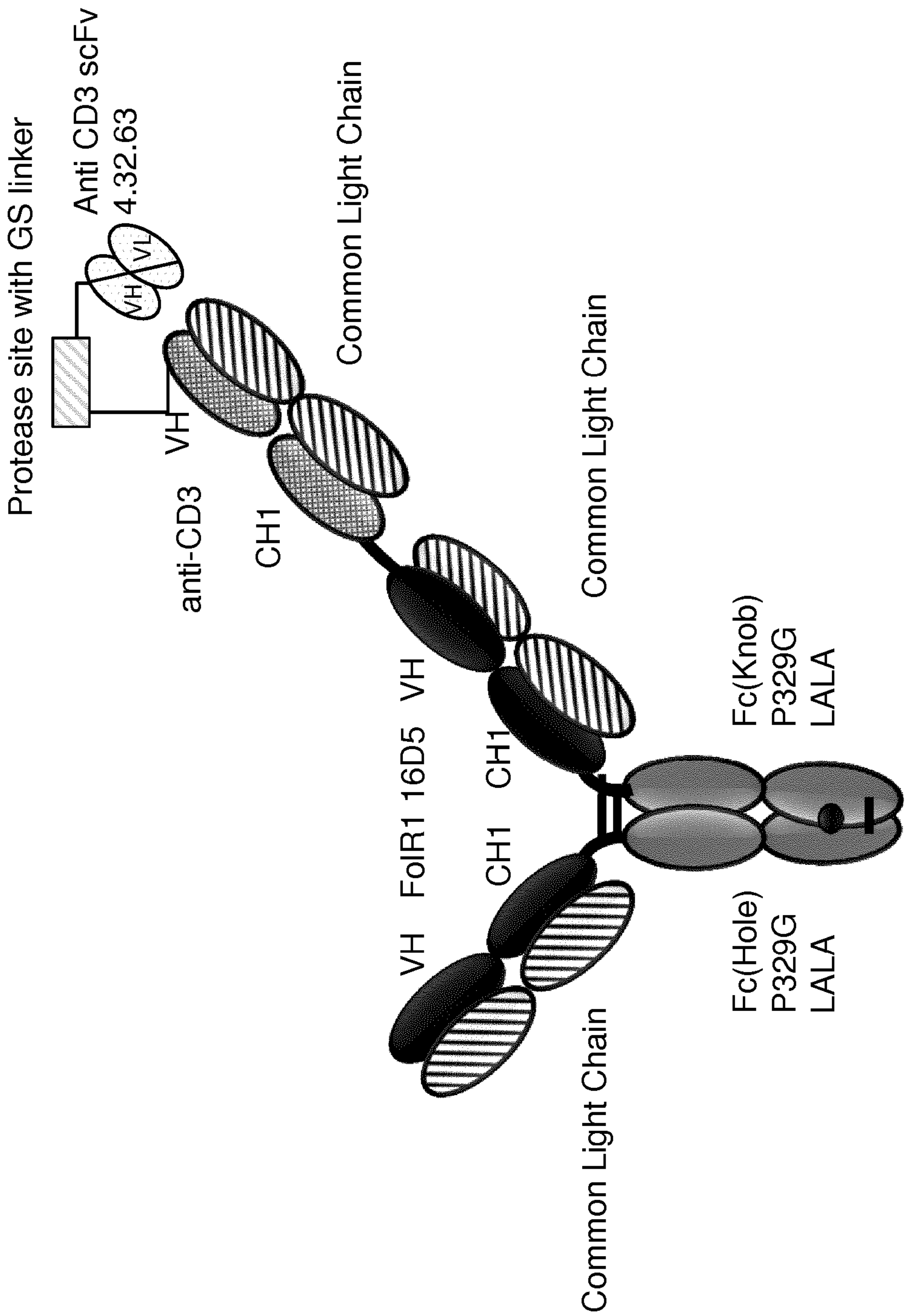
Protease site with GS linker
Anti CD3 scFv
4.32.63
VH
VL
Common Light Chain
anti-CD3
VH
CH1
Common Light Chain
FolR1 16D5 VH
CH1
Common Light Chain
VH
FolR1 16D5
CH1
Fc(Knob)
P329G
LALA
Fc(Hole)
P329G
LALA
Common Light Chain

7496

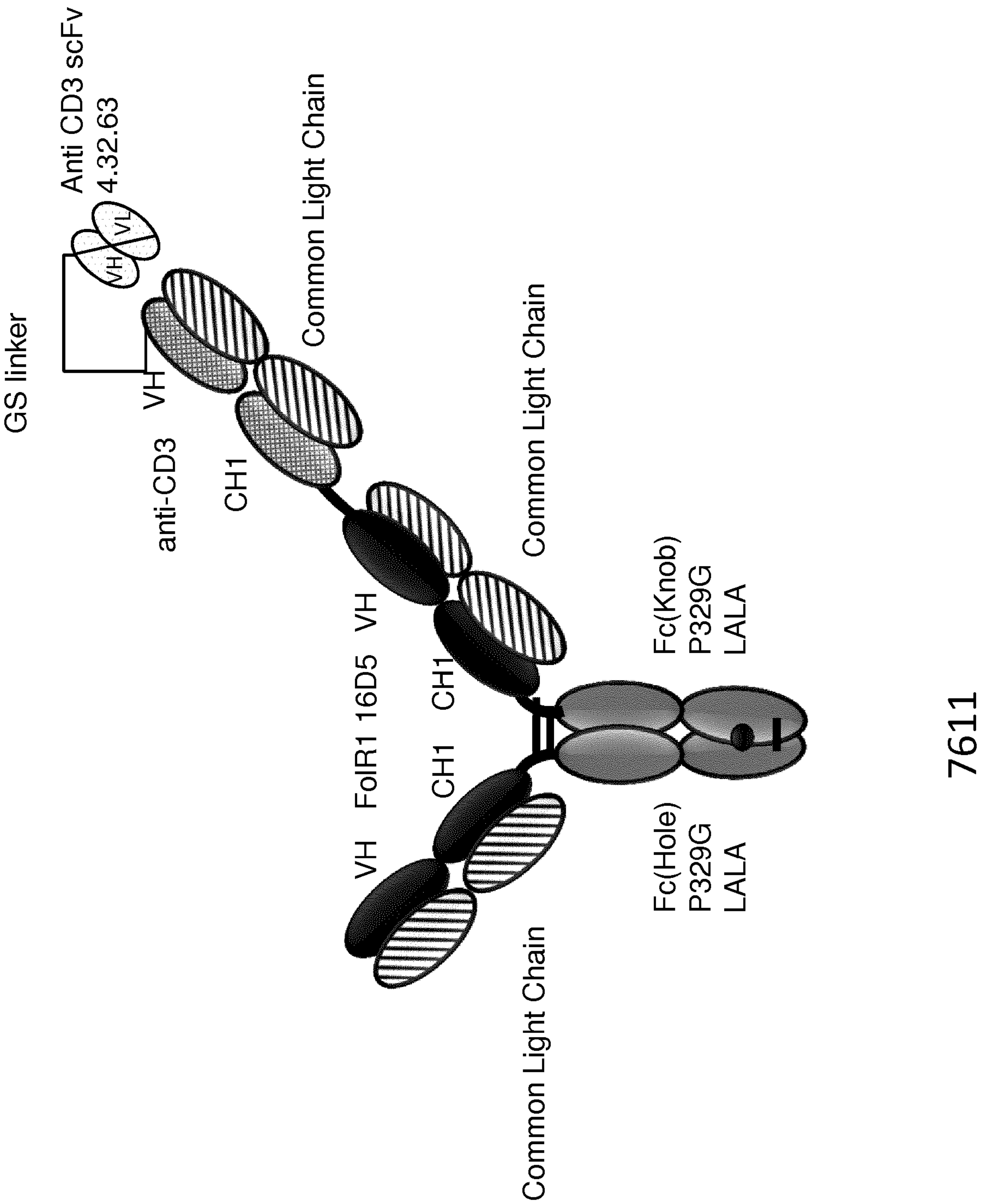
Anti CD3 scFv
4.32.63
VH
VL
GS linker
anti-CD3
VH
CH1
Common Light Chain
Common Light Chain
FolR1 16D5 VH
CH1
Common Light Chain
CH1
VH
Common Light Chain
Fc(Knob)
P329G
LALA
Fc(Hole)
P329G
LALA
7611

FIG. 5D

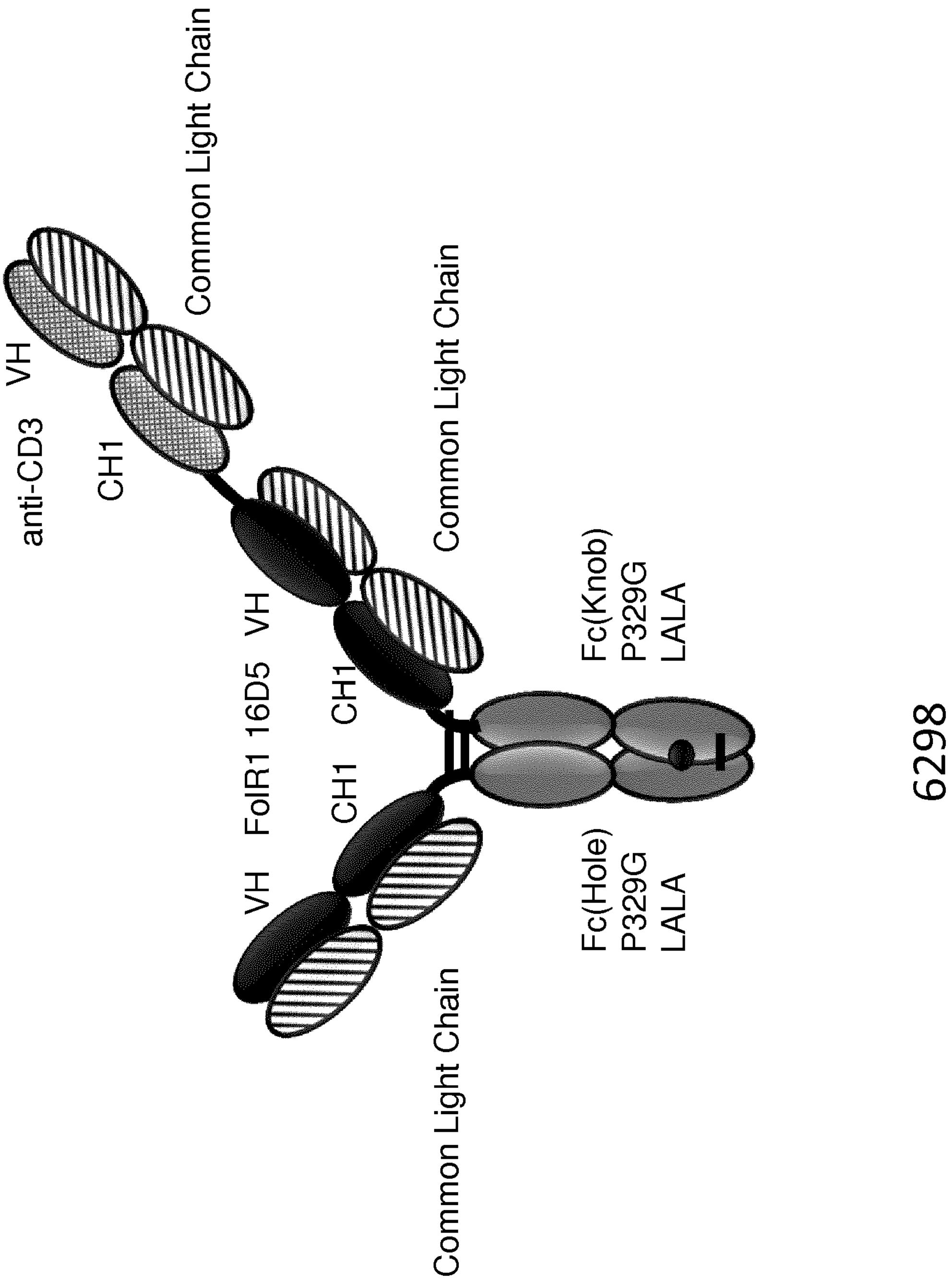
anti-CD3 VH
CH1
Common Light Chain
Common Light Chain
FolR1 16D5 VH
CH1
CH1
VH
Common Light Chain
Common Light Chain
Fc(Knob)
P329G
LALA
Fc(Hole)
P329G
LALA
6298

FIG. 5E

FIG. 5F

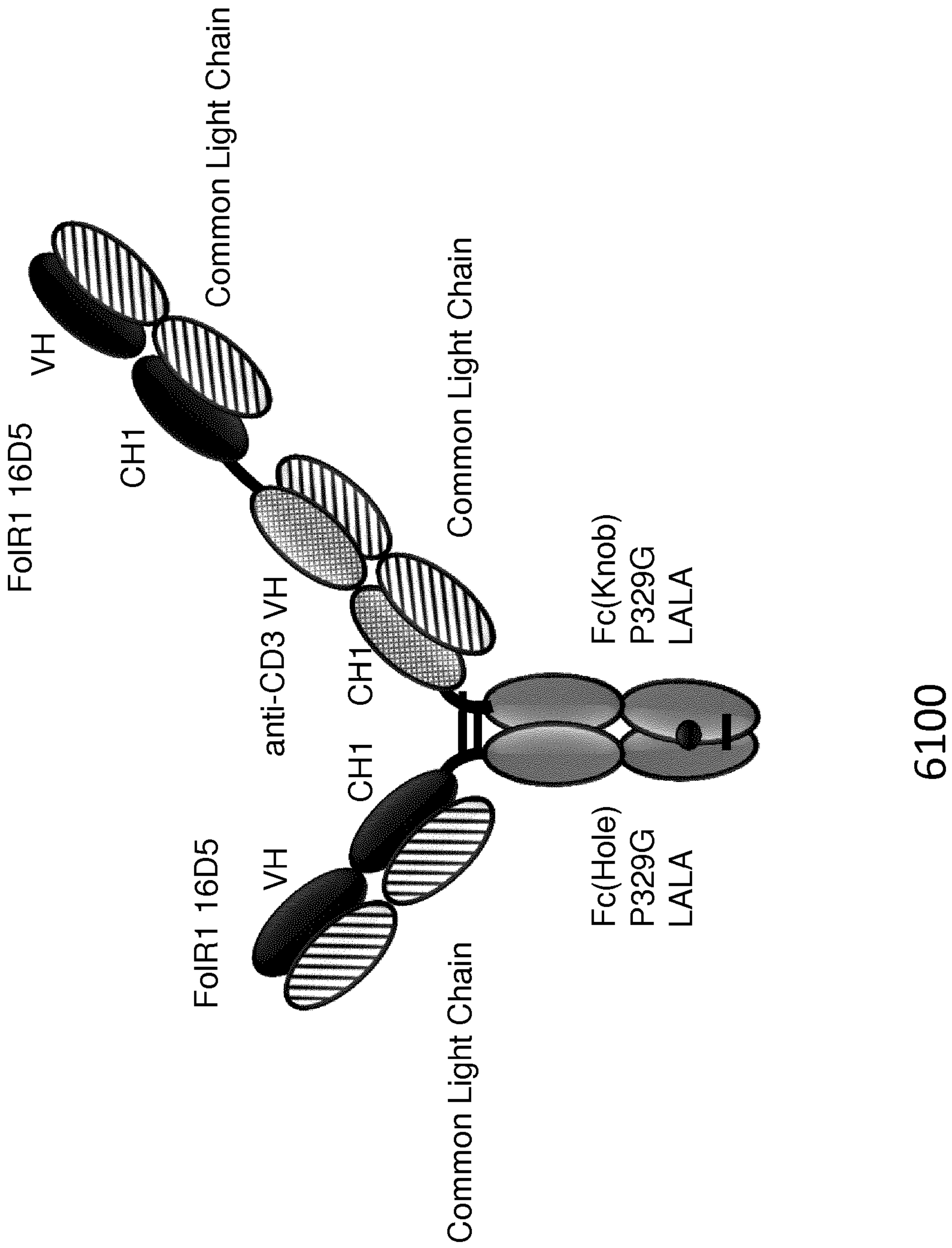
Common Light Chain
VH
FolR1 16D5
CH1
Common Light Chain
anti-CD3 VH
CH1
Fc(Knob)
P329G
LALA
CH1
VH
FolR1 16D5
Fc(Hole)
P329G
LALA
Common Light Chain
6100

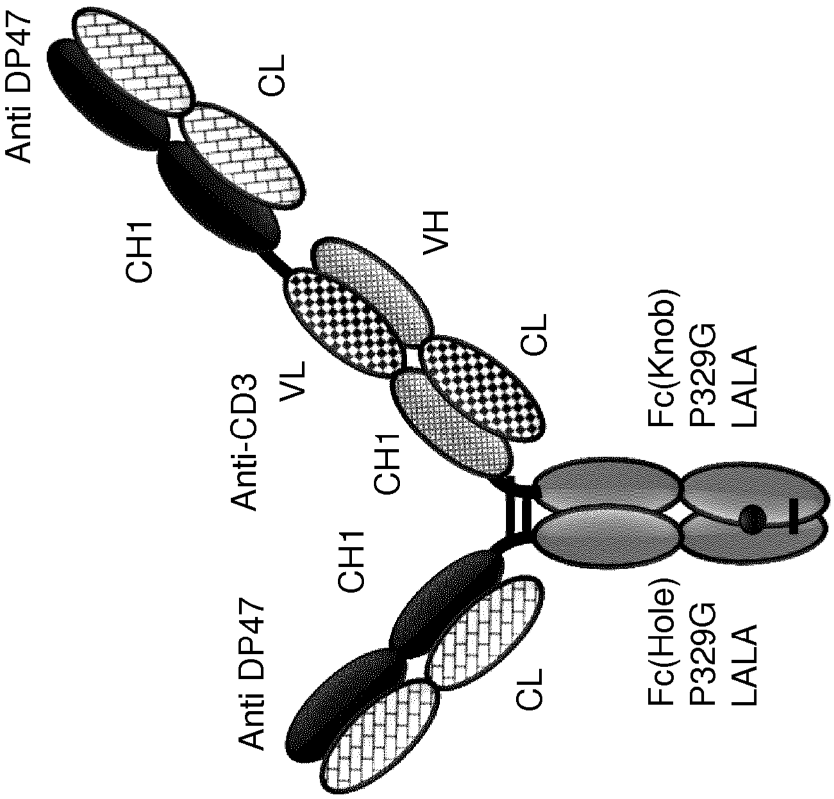
Anti DP47
CL
CH1
VH
Anti-CD3
VL
CL
CH1
Fc(Knob)
P329G
LALA
CH1
Anti DP47
CL
Fc(Hole)
P329G
LALA

6182

FIG. 5G

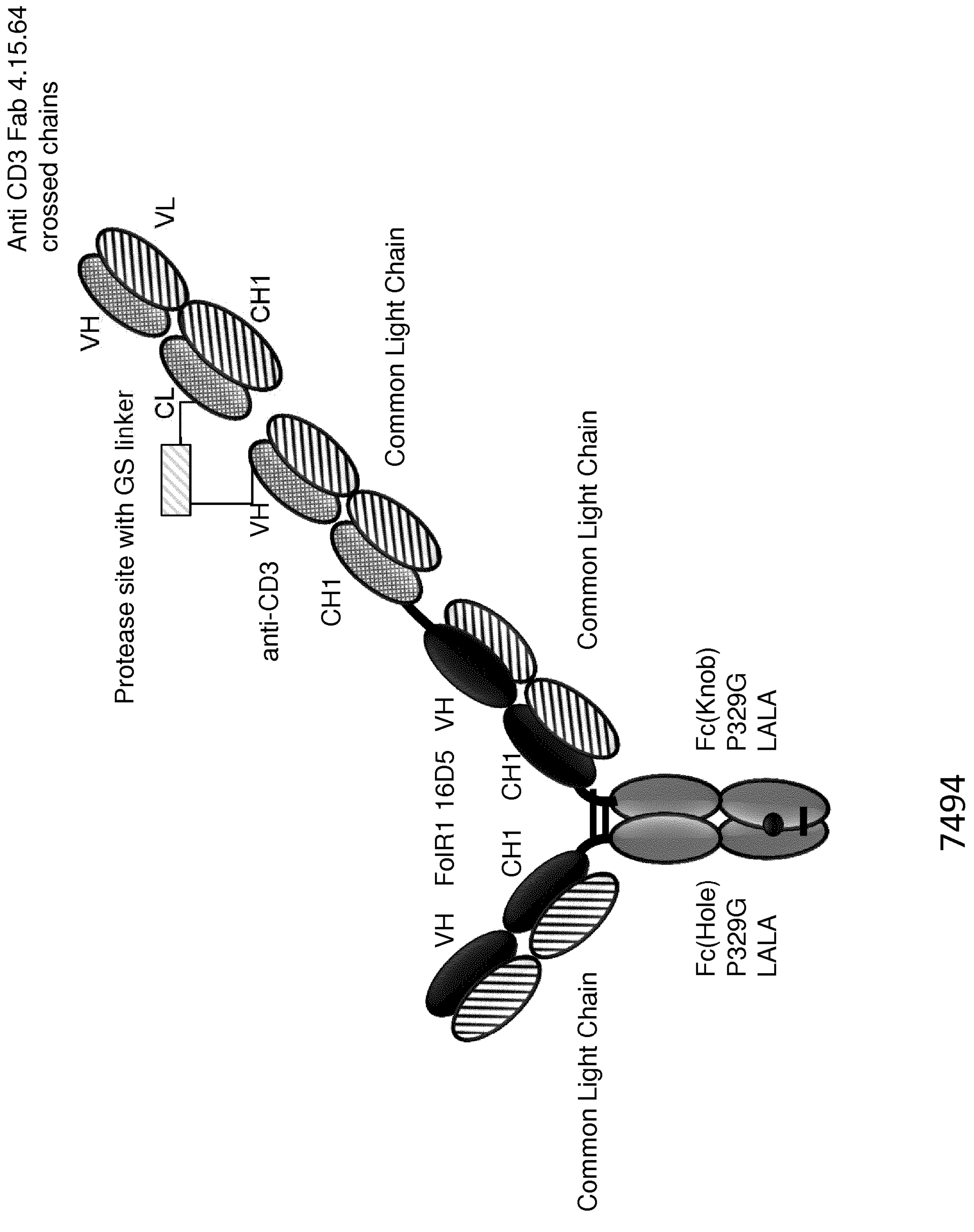
Anti CD3 Fab 4.15.64
crossed chains
VL
VH
CH1
CL
Protease site with GS linker
VH
anti-CD3
CH1
Common Light Chain
Common Light Chain
FolR1 16D5 VH
CH1
VH
CH1
Common Light Chain
Fc(Knob)
P329G
LALA
Fc(Hole)
P329G
LALA
7494

FIG. 5H

FIG. 6

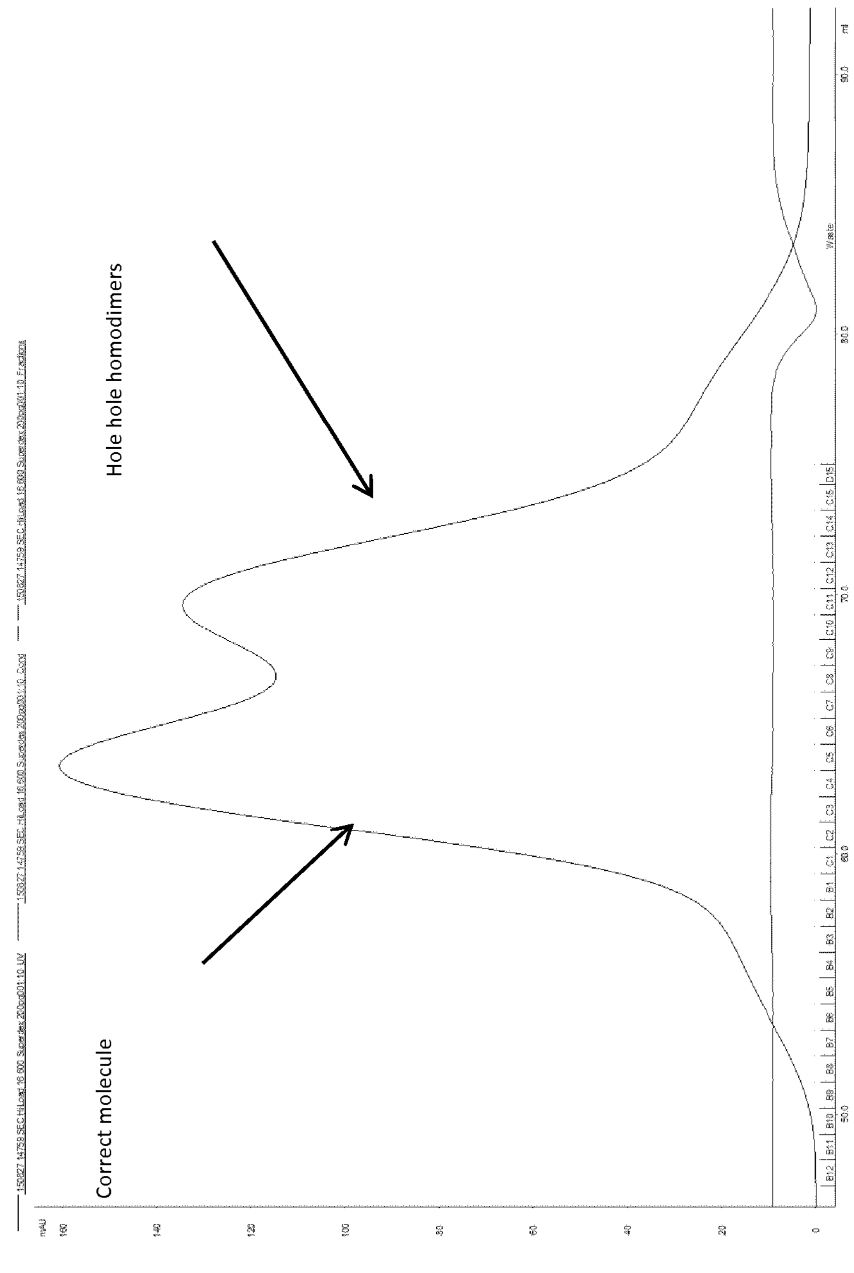
Correct molecule
Hole hole homodimers
mAU
160
140
120
100
80
60
40
20
0
50.0
60.0
70.0
80.0
90.0
ml
Waste

FIG. 7

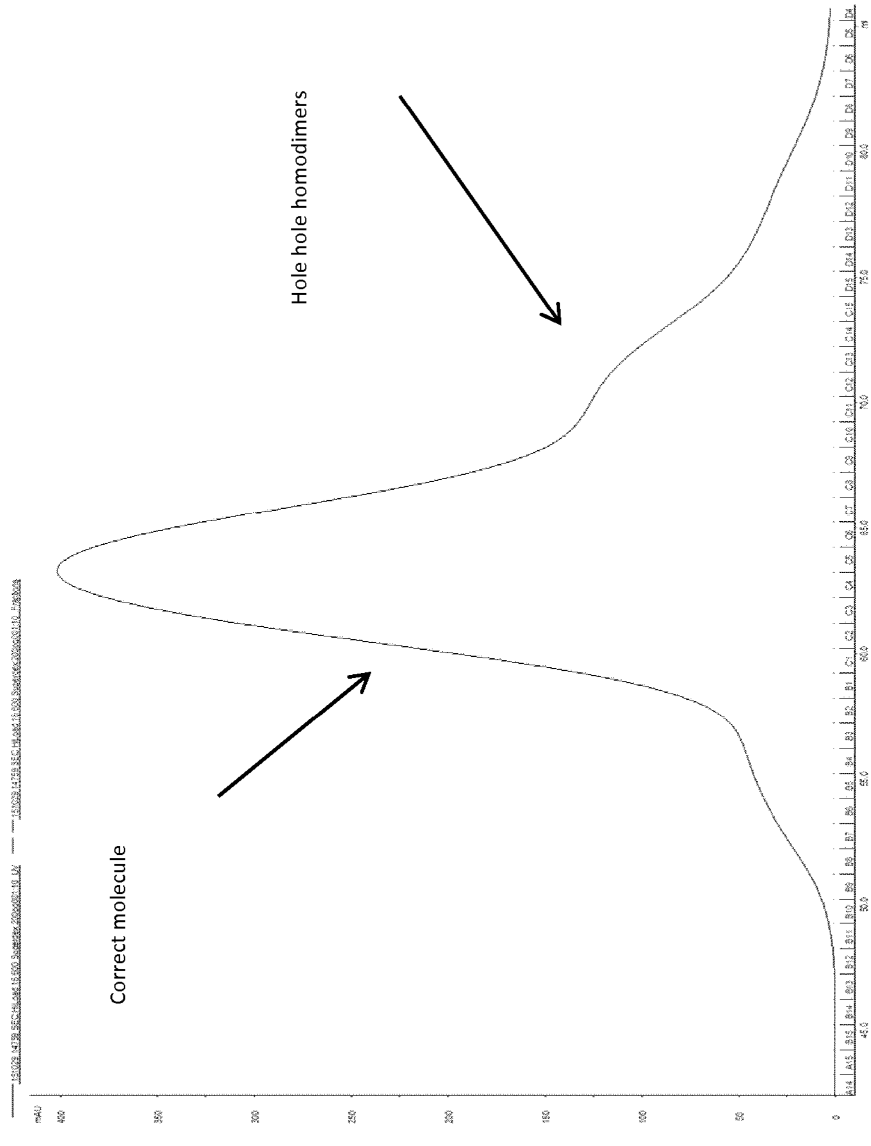

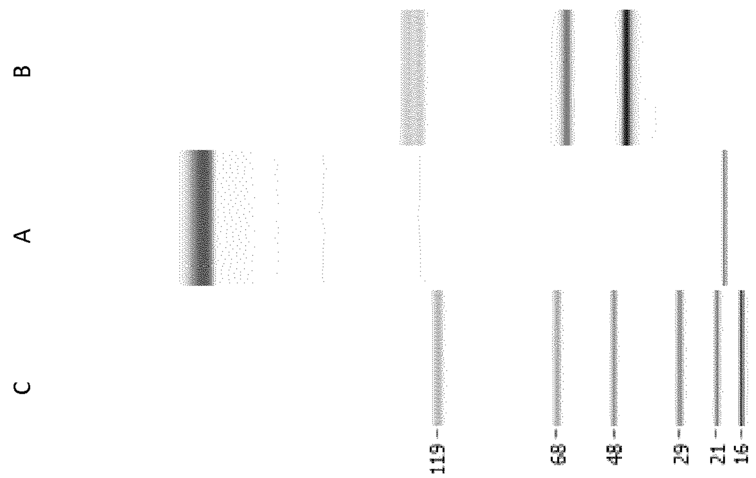


FIG. 8

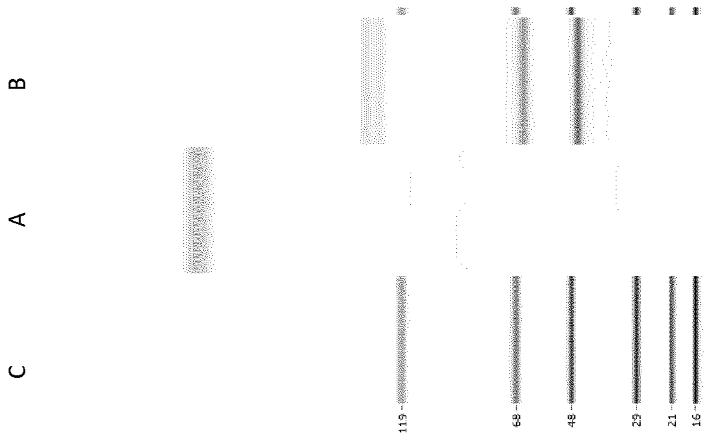


FIG. 9

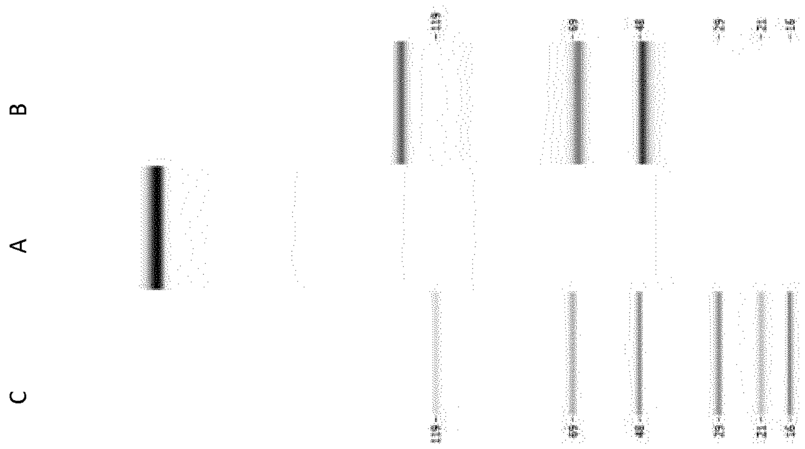


FIG. 10

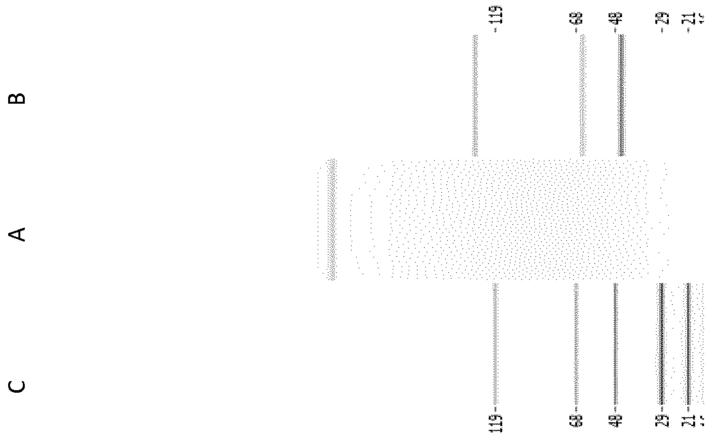


FIG. 11

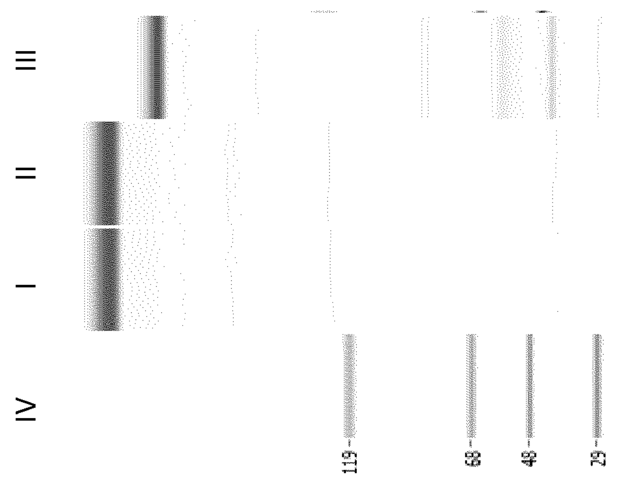


FIG. 12A

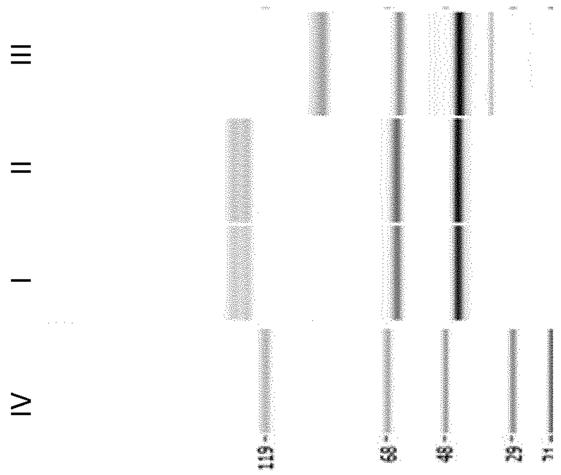


FIG. 12B

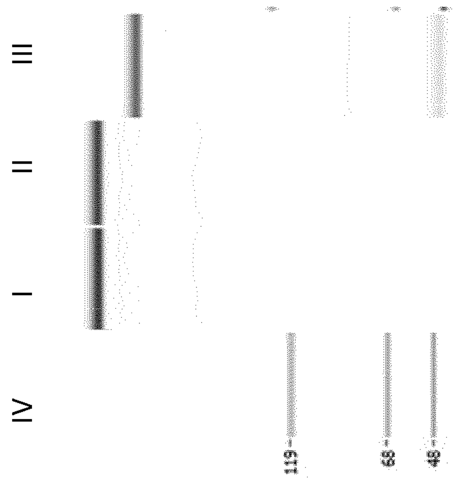


FIG. 12C

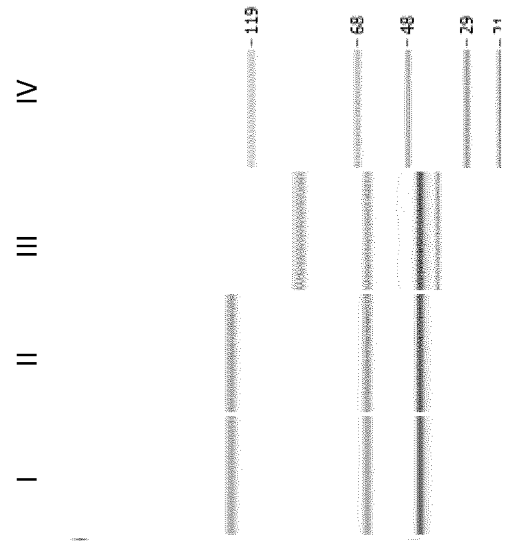


FIG. 12D

FIG. 13

| Human cell lines | FolR1 density |
|---|---|
| Hela | 2’240’716 |
| Skov3 | 91’510 |
| HT29 | 10’135 |
| MKN45 | 54 |
| Human Renal Cortical Epithelial Cells (HRCEpiC) | 312 |

FIG. 14A

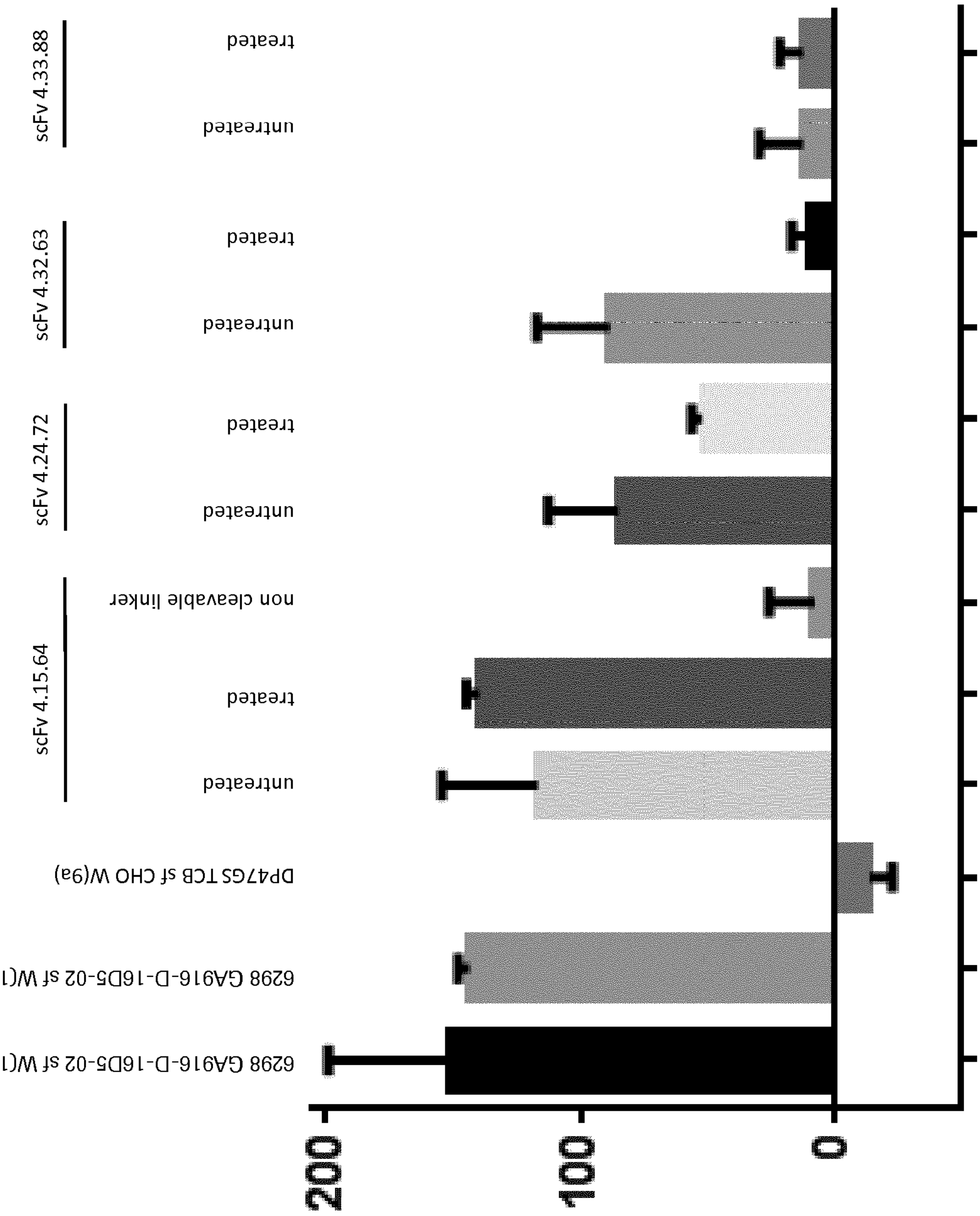
% killing
200
100
0
6298 GA916-D-16D5-02 sf W(1)
6298 GA916-D-16D5-02 sf W(1_treated)
DP47GS TCB sf CHO W(9a)
untreated
treated
non cleavable linker
scFv 4.15.64
untreated
treated
scFv 4.24.72
untreated
treated
scFv 4.32.63
untreated
treated
scFv 4.33.88

FIG. 14B

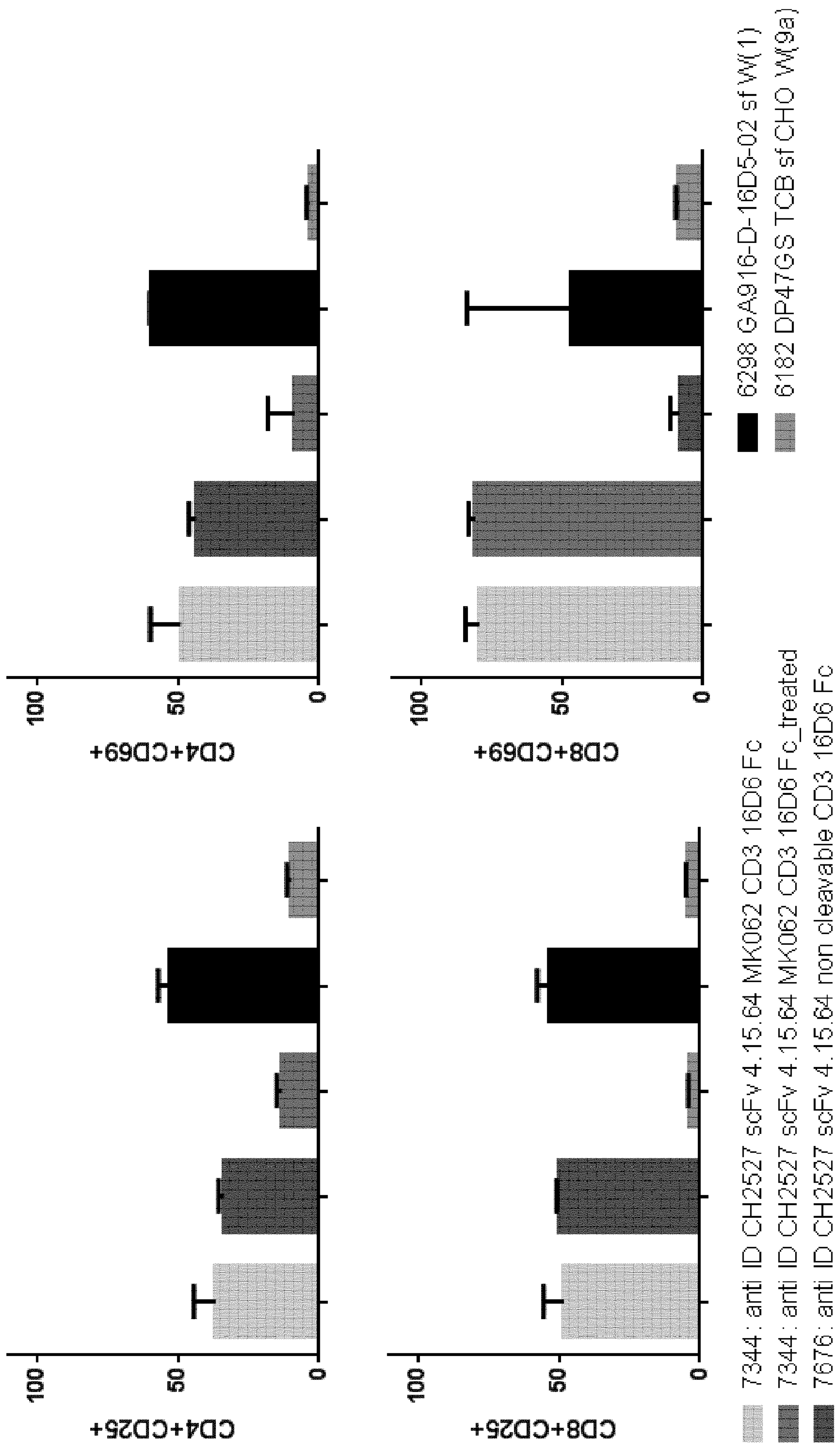
CD4+CD25+
CD8+CD25+
CD4+CD69+
CD8+CD69+
100
50
0
7344 : anti ID CH2527 scFv 4.15.64 MK062 CD3 16D6 Fc
7344 : anti ID CH2527 scFv 4.15.64 MK062 CD3 16D6 Fc_treated
7676 : anti ID CH2527 scFv 4.15.64 non cleavable CD3 16D6 Fc
6298 GA916-D-16D5-02 sf W(1)
6182 DP47GS TCB sf CHO W(9a)

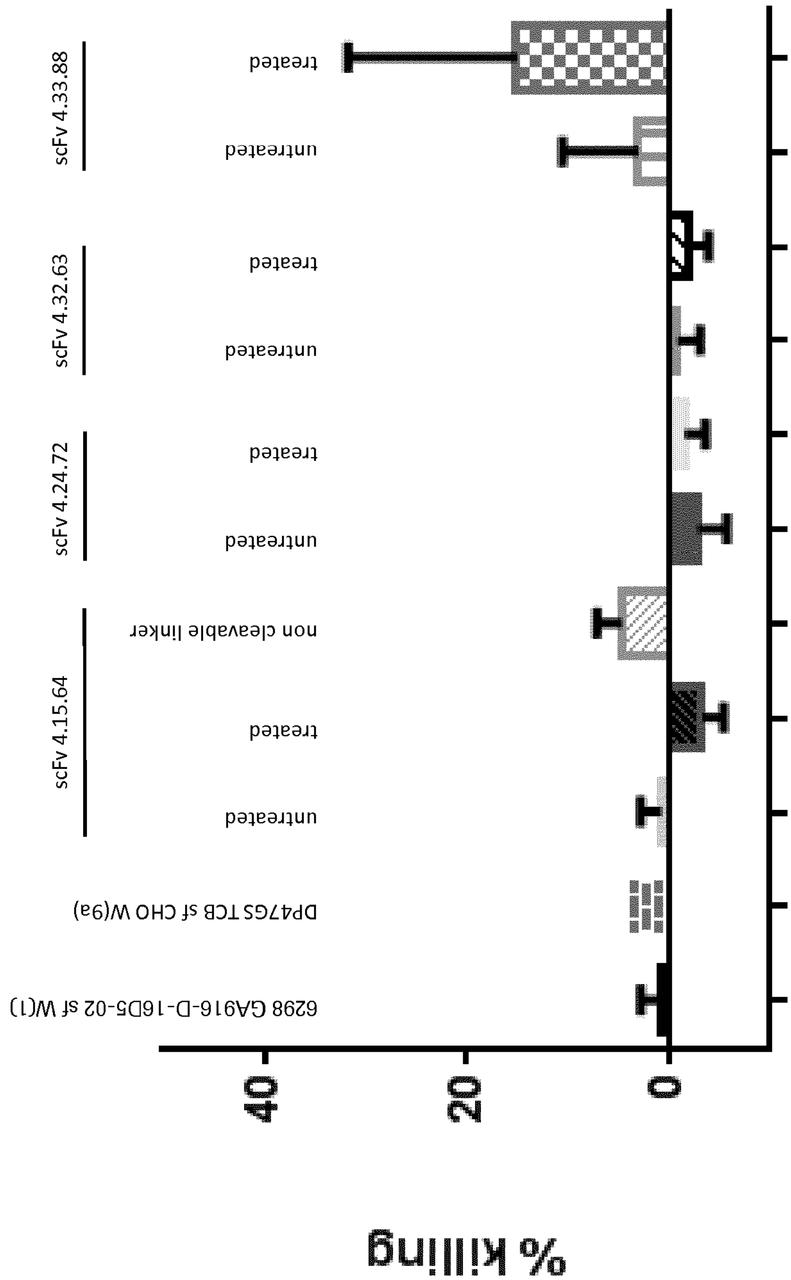
% killing
40
20
0
6298 GA916-D-16D5-02 sf W(1)
DP47GS TCB sf CHO W(9a)
untreated
treated
non cleavable linker
scFv 4.15.64
untreated
treated
scFv 4.24.72
untreated
treated
scFv 4.32.63
untreated
treated
scFv 4.33.88

FIG. 15A

FIG. 15B

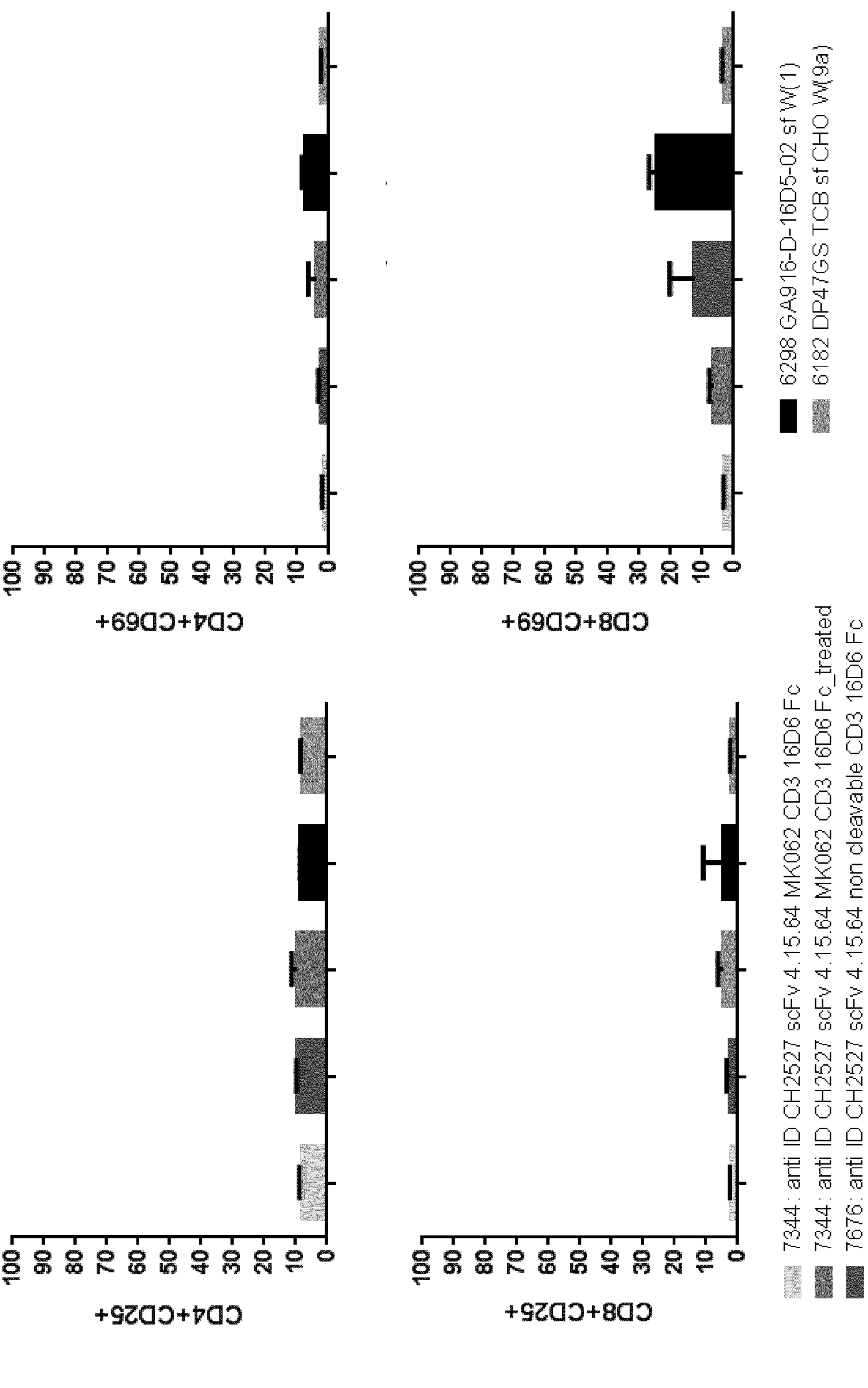
CD4+CD25+
CD4+CD69+
CD8+CD25+
CD8+CD69+
0
10
20
30
40
50
60
70
80
90
100
7344 : anti ID CH2527 scFv 4.15.64 MK062 CD3 16D6 Fc
7344: anti ID CH2527 scFv 4.15.64 MK062 CD3 16D6 Fc_treated
7676: anti ID CH2527 scFv 4.15.64 non cleavable CD3 16D6 Fc
6298 GA916-D-16D5-02 sf W(1)
6182 DP47GS TCB sf CHO W(9a)

FIG. 16

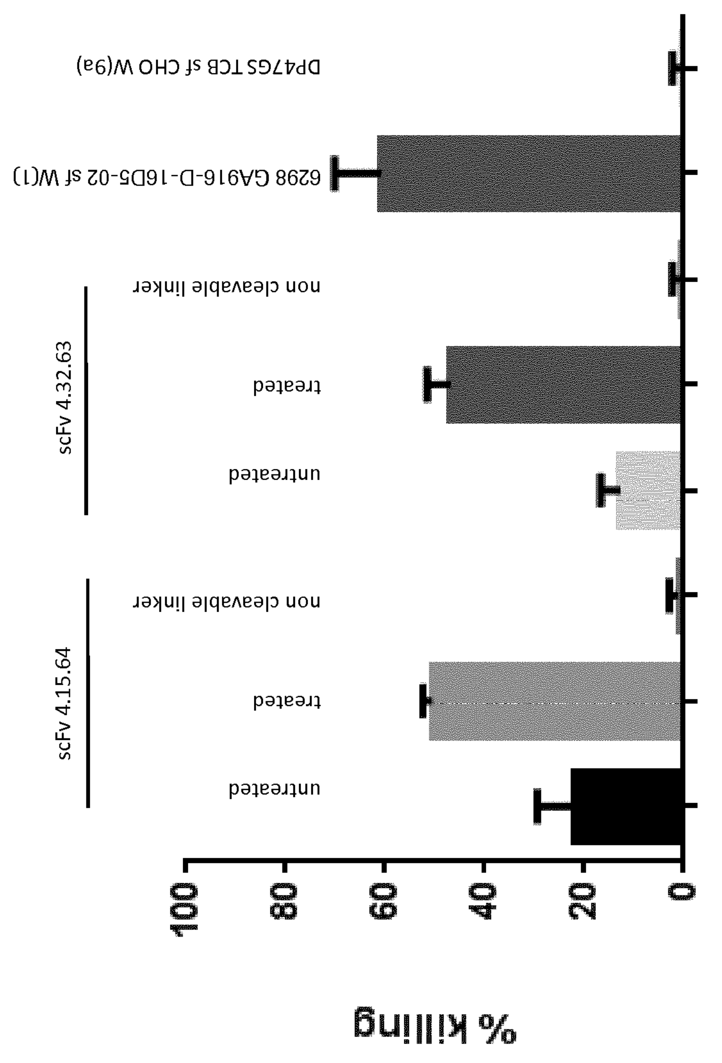
% killing
100
80
60
40
20
0
untreated
treated
non cleavable linker
scFv 4.15.64
untreated
treated
non cleavable linker
scFv 4.32.63
6298 GA916-D-16D5-02 sf W(1)
DP47GS TCB sf CHO W(9a)

FIG. 17

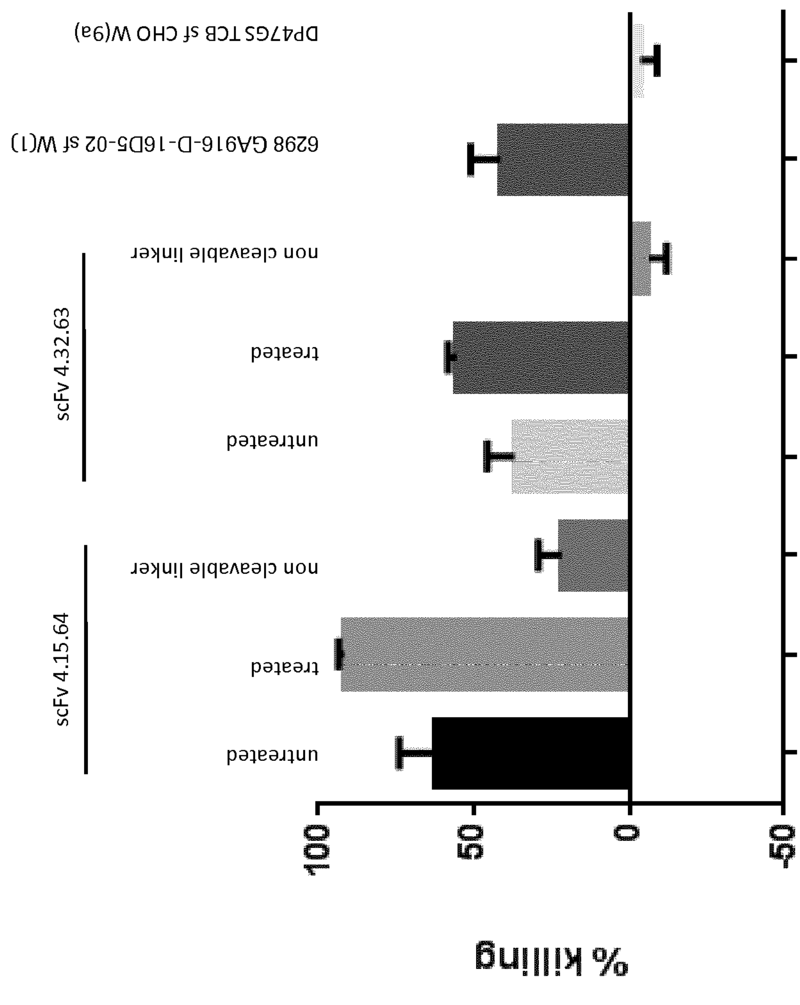
% killing
100
50
0
-50
untreated
treated
non cleavable linker
scFv 4.15.64
untreated
treated
non cleavable linker
scFv 4.32.63
6298 GA916-D-16D5-02 sf W(1)
DP47GS TCB sf CHO W(9a)

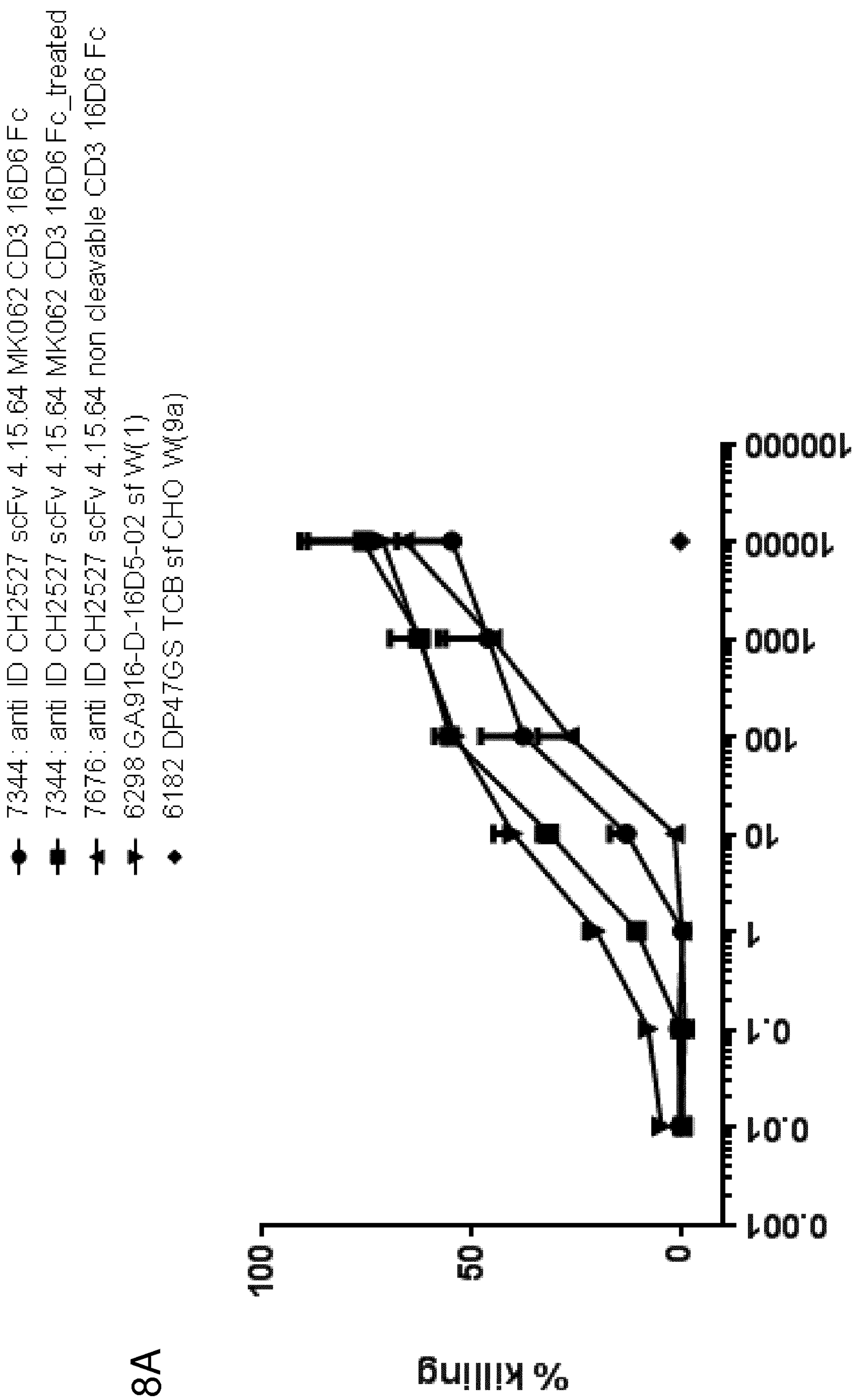
7344: anti ID CH2527 scFv 4.15.64 MK062 CD3 16D6 Fc
7344: anti ID CH2527 scFv 4.15.64 MK062 CD3 16D6 Fc_treated
7676: anti ID CH2527 scFv 4.15.64 non cleavable CD3 16D6 Fc
6298 GA916-D-16D5-02 sf W(1)
6182 DP47GS TCB sf CHO W(9a)
% killing
100
50
0
0.001
0.01
0.1
1
10
100
1000
10000
100000

FIG. 18A

FIG. 18B

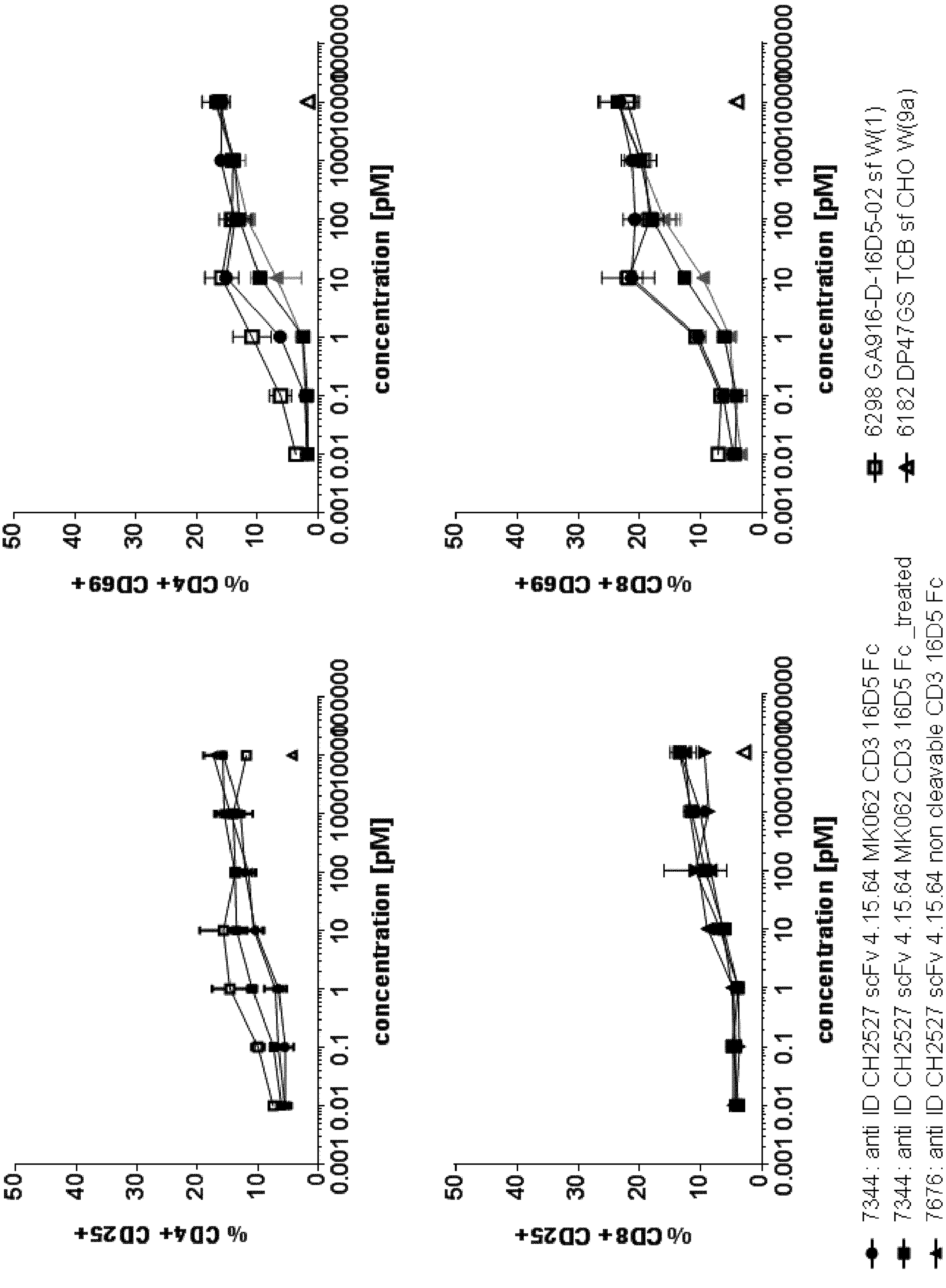
% CD4+ CD25+
% CD8+ CD25+
% CD4+ CD69+
% CD8+ CD69+
concentration [pM]
0.001 0.01 0.1 1 10 100 1000 10000 100000
0 10 20 30 40 50
7344 : anti ID CH2527 scFv 4.15.64 MK062 CD3 16D5 Fc
7344 : anti ID CH2527 scFv 4.15.64 MK062 CD3 16D5 Fc _treated
7676: anti ID CH2527 scFv 4.15.64 non cleavable CD3 16D5 Fc
6298 GA916-D-16D5-02 sf W(1)
6182 DP47GS TCB sf CHO W(9a)

FIG. 19A

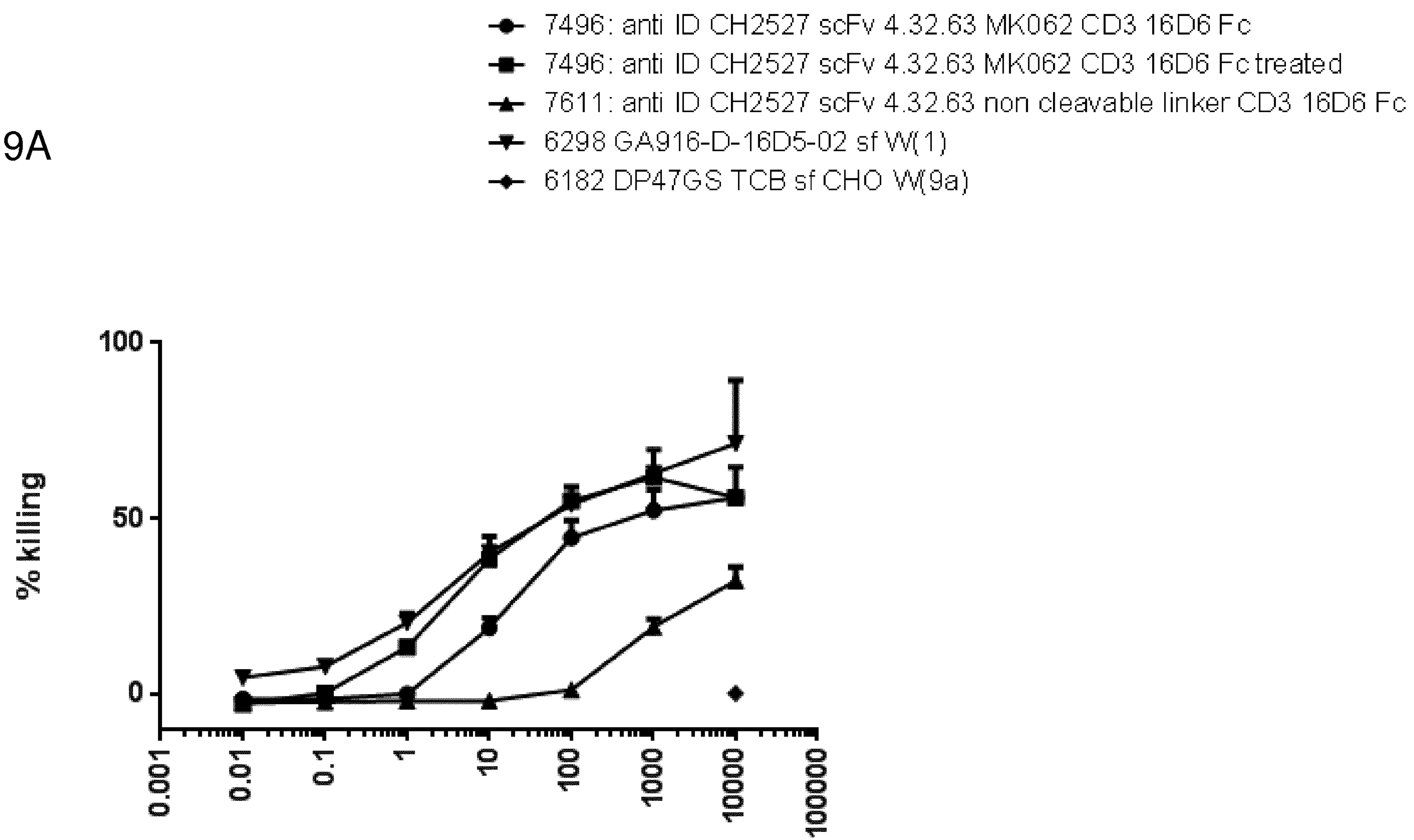
7496: anti ID CH2527 scFv 4.32.63 MK062 CD3 16D6 Fc
7496: anti ID CH2527 scFv 4.32.63 MK062 CD3 16D6 Fc treated
7611: anti ID CH2527 scFv 4.32.63 non cleavable linker CD3 16D6 Fc
6298 GA916-D-16D5-02 sf W(1)
6182 DP47GS TCB sf CHO W(9a)
% killing
100
50
0
0.001
0.01
0.1
1
10
100
1000
10000
100000

FIG. 19B

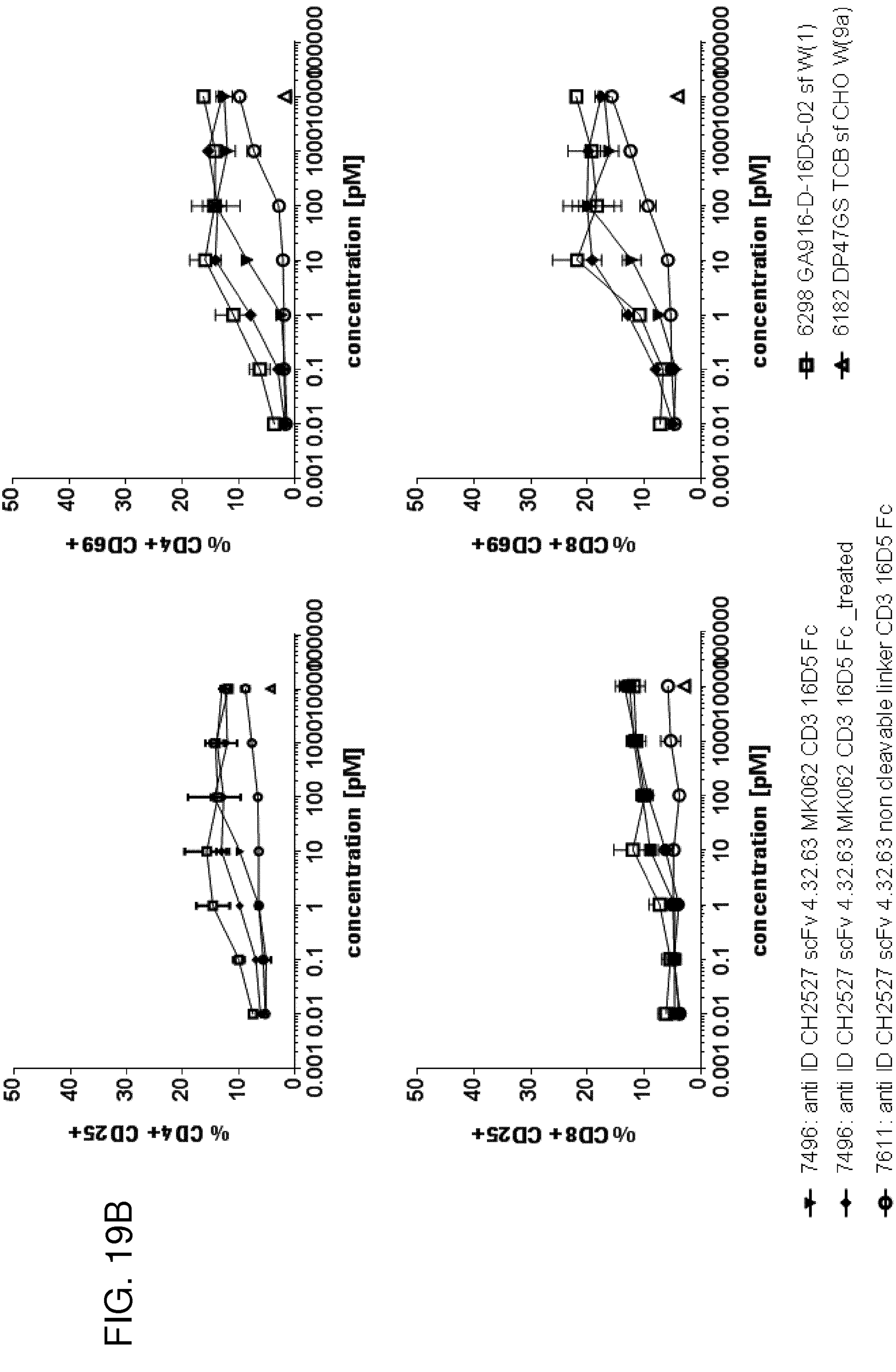
% CD4+ CD25+
% CD8+ CD25+
% CD4+ CD69+
% CD8+ CD69+
concentration [pM]
7496: anti ID CH2527 scFv 4.32.63 MK062 CD3 16D5 Fc
7496: anti ID CH2527 scFv 4.32.63 MK062 CD3 16D5 Fc _treated
7611: anti ID CH2527 scFv 4.32.63 non cleavable linker CD3 16D5 Fc
6298 GA916-D-16D5-02 sf W(1)
6182 DP47GS TCB sf CHO W(9a)

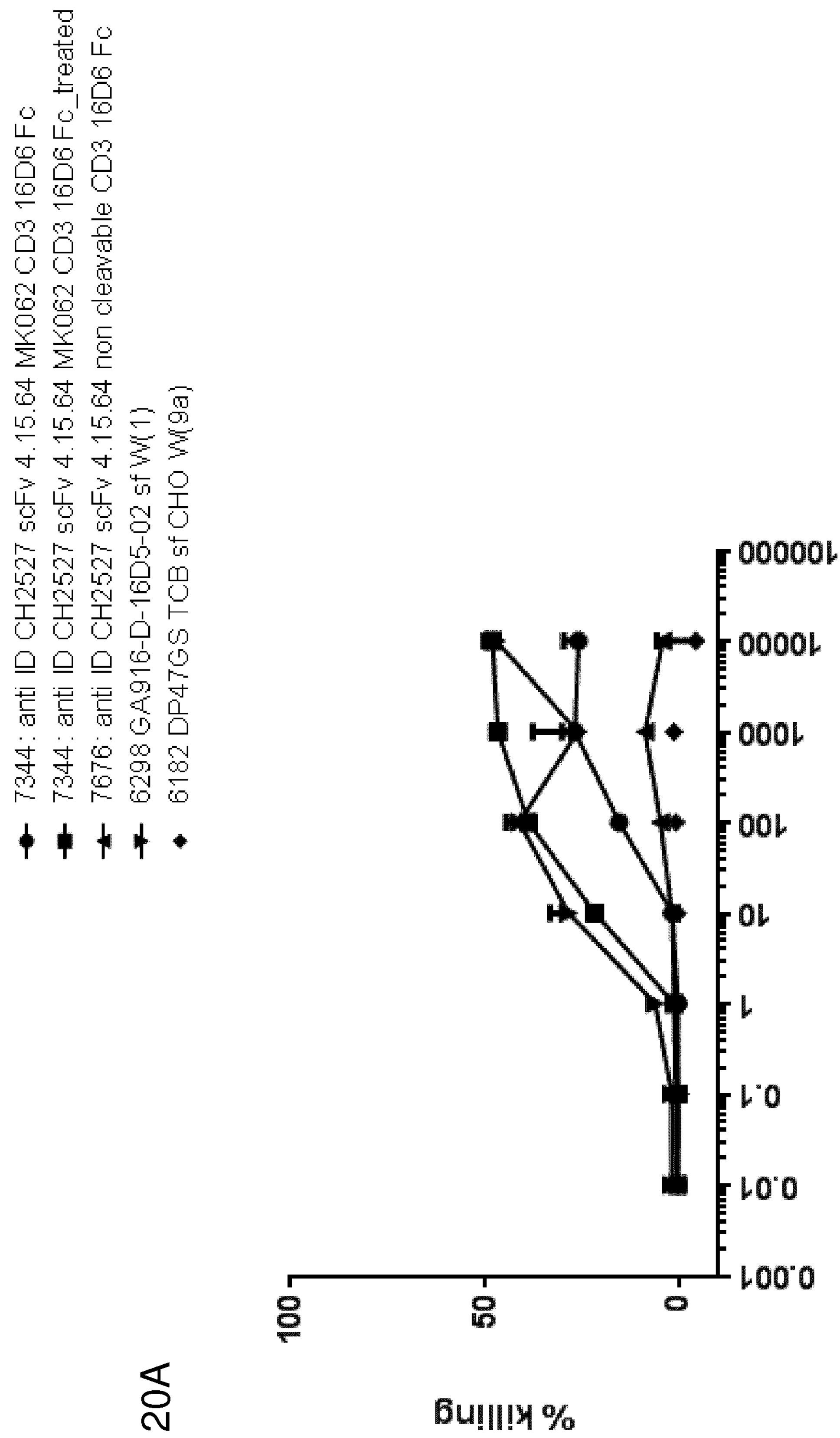
7344 : anti ID CH2527 scFv 4.15.64 MK062 CD3 16D6 Fc
7344 : anti ID CH2527 scFv 4.15.64 MK062 CD3 16D6 Fc_treated
7676 : anti ID CH2527 scFv 4.15.64 non cleavable CD3 16D6 Fc
6298 GA916-D-16D5-02 sf W(1)
6182 DP47GS TCB sf CHO W(9a)
% killing
100
50
0
0.001
0.01
0.1
1
10
100
1000
10000
100000

FIG. 20A

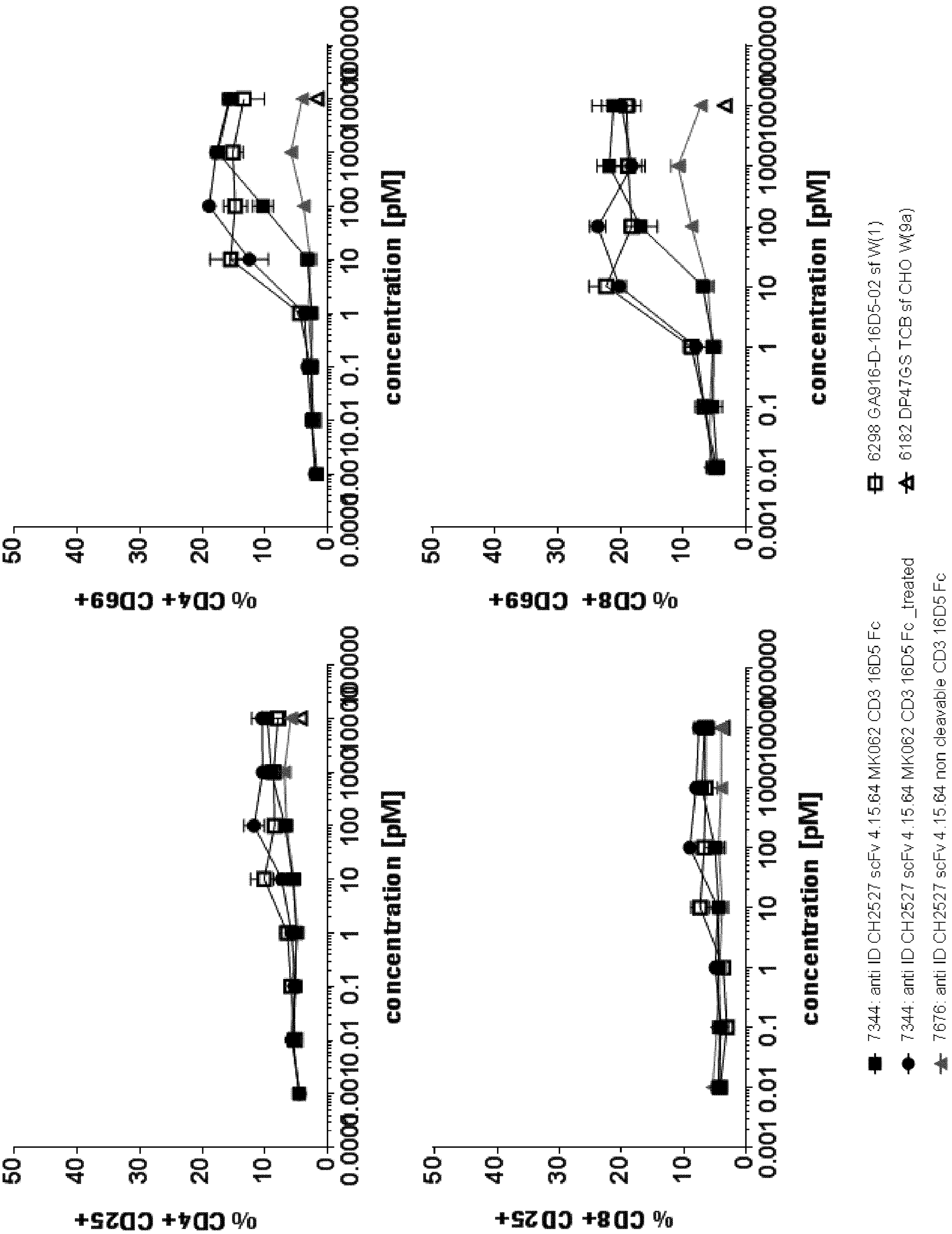

% CD4+ CD25+
% CD8+ CD25+
% CD4+ CD69+
% CD8+ CD69+
concentration [pM]
7344: anti ID CH2527 scFv 4.15.64 MK062 CD3 16D5 Fc
7344: anti ID CH2527 scFv 4.15.64 MK062 CD3 16D5 Fc_treated
7676: anti ID CH2527 scFv 4.15.64 non cleavable CD3 16D5 Fc
6298 GA916-D-16D5-02 sf W(1)
6182 DP47GS TCB sf CHO W(9a)


FIG. 20B

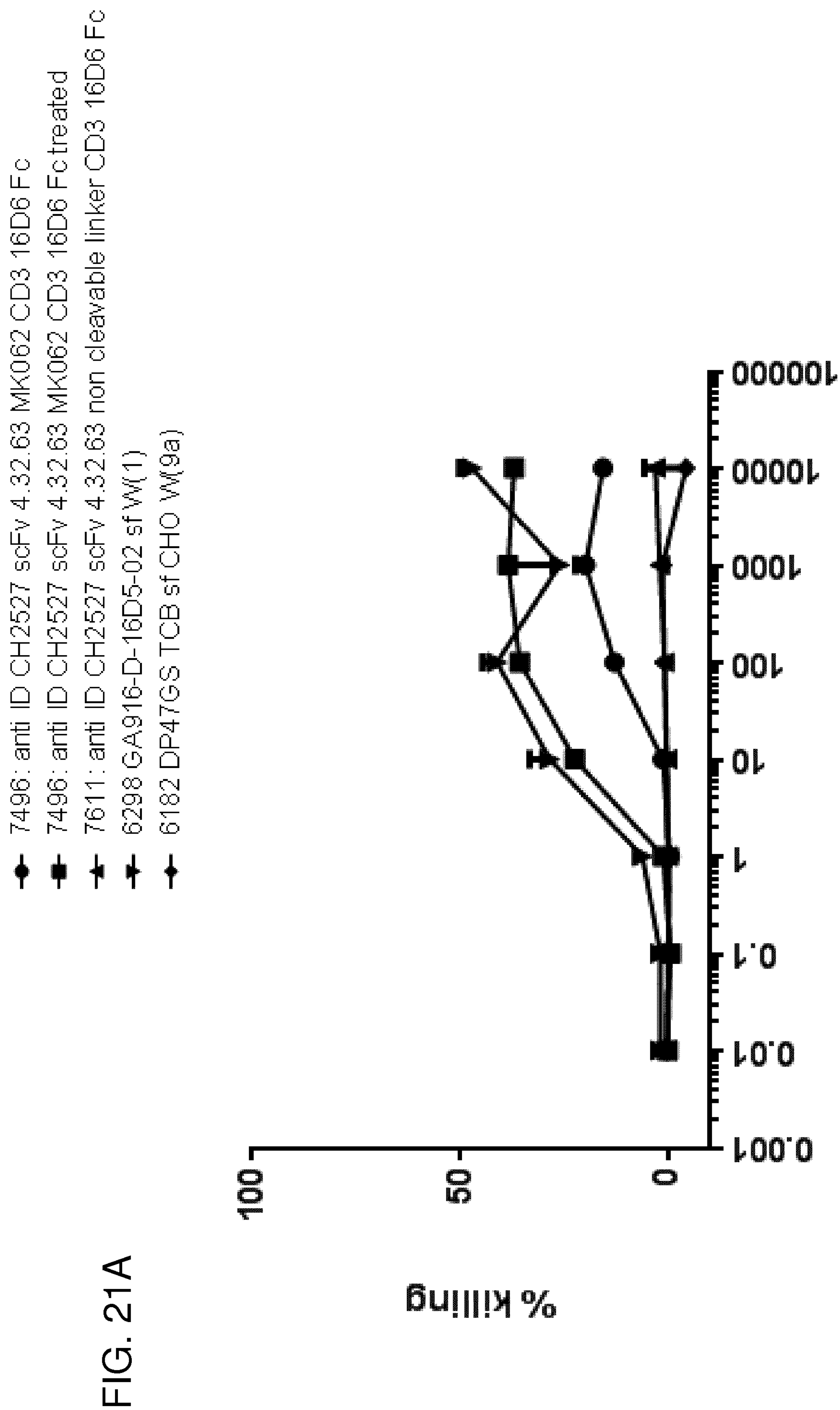
7496: anti ID CH2527 scFv 4.32.63 MK062 CD3 16D6 Fc
7496: anti ID CH2527 scFv 4.32.63 MK062 CD3 16D6 Fc treated
7611: anti ID CH2527 scFv 4.32.63 non cleavable linker CD3 16D6 Fc
6298 GA916-D-16D5-02 sf W(1)
6182 DP47GS TCB sf CHO W(9a)
% killing
100
50
0
0.001
0.01
0.1
1
10
100
1000
10000
100000

FIG. 21A

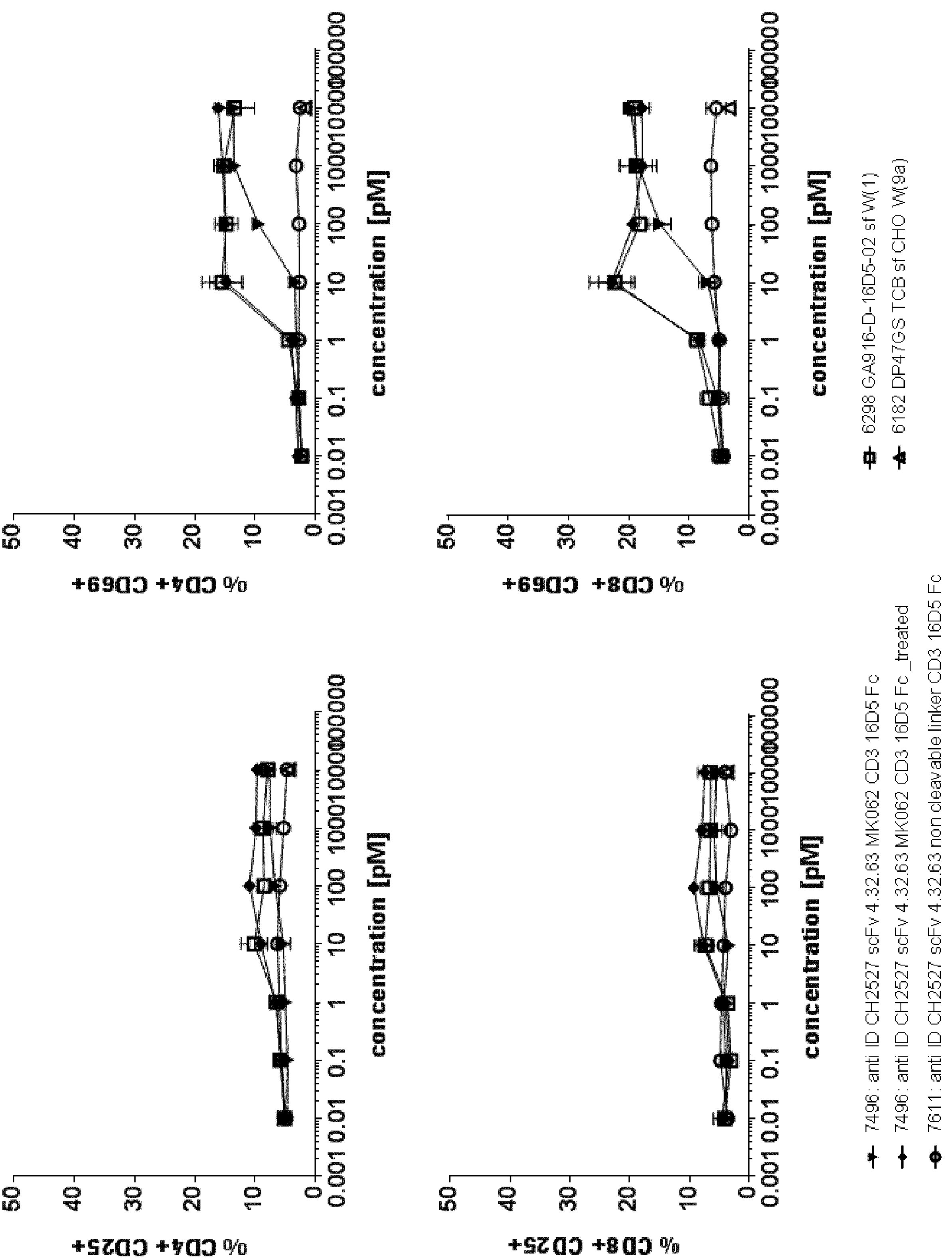
% CD4+ CD25+
% CD8+ CD25+
% CD4+ CD69+
% CD8+ CD69+
concentration [pM]
7496: anti ID CH2527 scFv 4.32.63 MK062 CD3 16D5 Fc
7496: anti ID CH2527 scFv 4.32.63 MK062 CD3 16D5 Fc _treated
7611: anti ID CH2527 scFv 4.32.63 non cleavable linker CD3 16D5 Fc
6298 GA916-D-16D5-02 sf W(1)
6182 DP47GS TCB sf CHO W(9a)

FIG. 21B

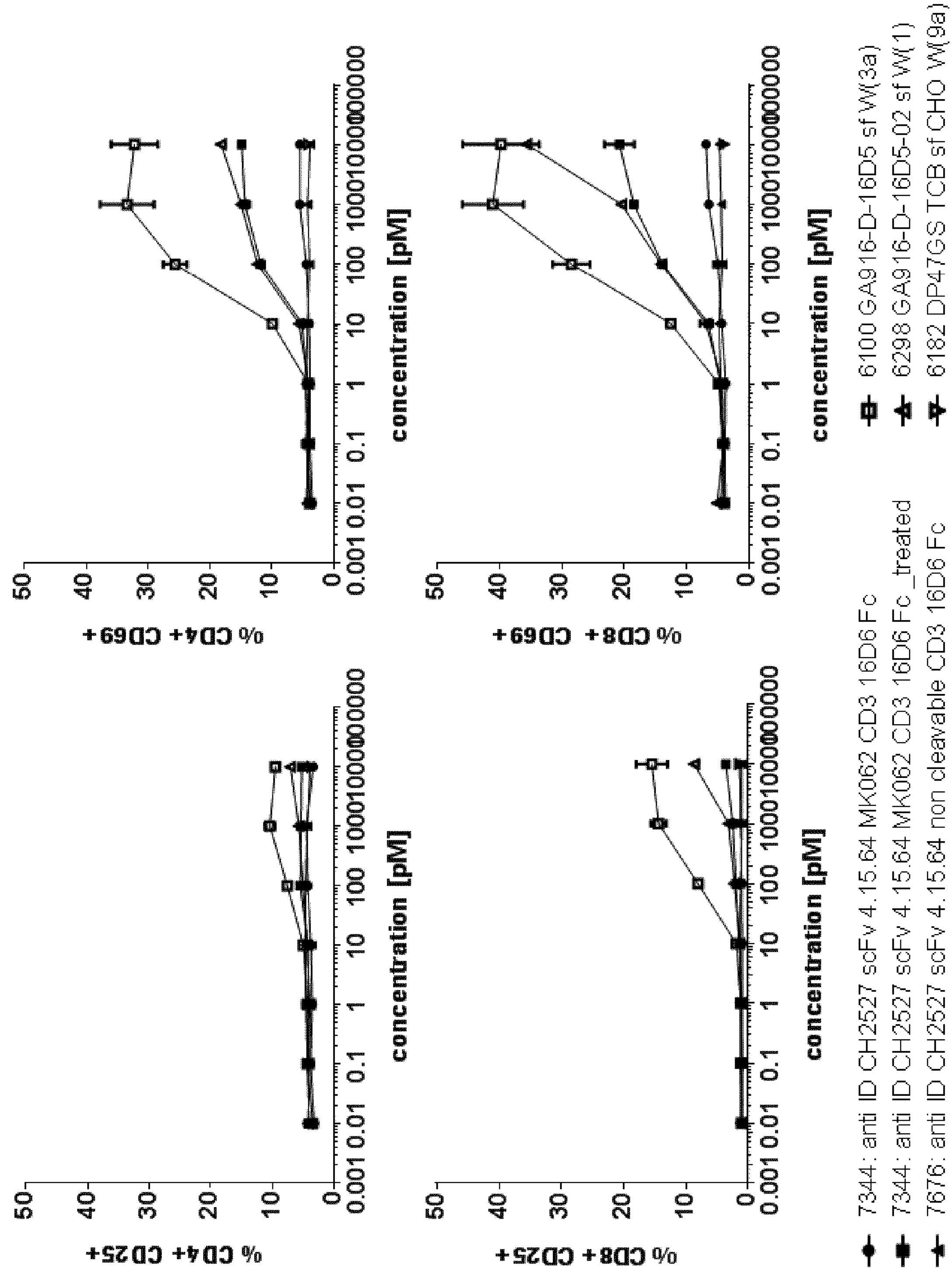
% CD4+ CD25+
% CD8+ CD25+
% CD4+ CD69+
% CD8+ CD69+
concentration [pM]
0.001 0.01 0.1 1 10 100 1000 10000 100000
0 10 20 30 40 50
7344: anti ID CH2527 scFv 4.15.64 MK062 CD3 16D6 Fc
7344: anti ID CH2527 scFv 4.15.64 MK062 CD3 16D6 Fc_treated
7676: anti ID CH2527 scFv 4.15.64 non cleavable CD3 16D6 Fc
6100 GA916-D-16D5 sf W(3a)
6298 GA916-D-16D5-02 sf W(1)
6182 DP47GS TCB sf CHO W(9a)

FIG. 22A

FIG. 22B

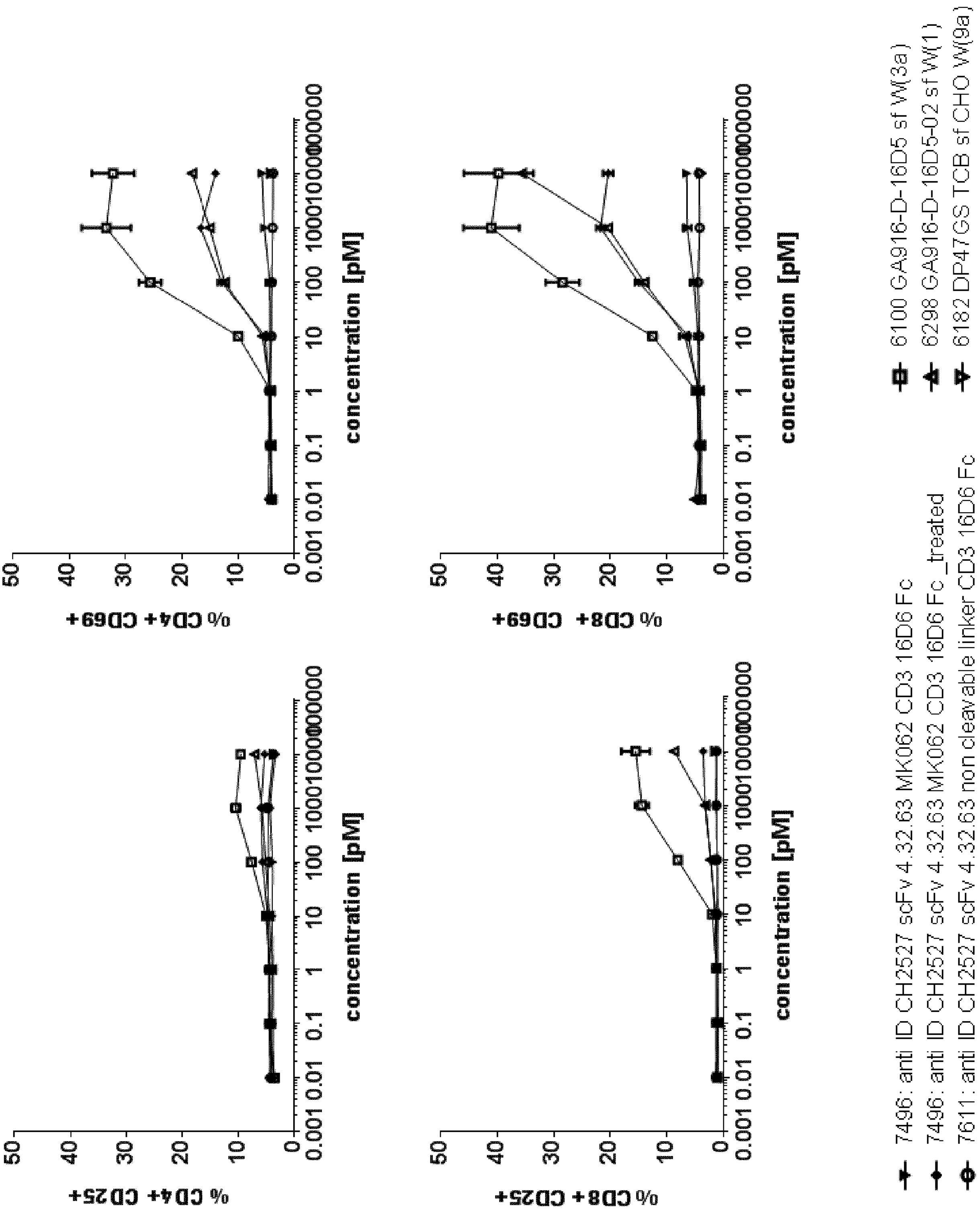
% CD4+ CD25+
% CD8+ CD25+
% CD4+ CD69+
% CD8+ CD69+
concentration [pM]
0.001 0.01 0.1 1 10 100 1000 10000 100000
0
10
20
30
40
50
7496: anti ID CH2527 scFv 4.32.63 MK062 CD3 16D6 Fc
7496: anti ID CH2527 scFv 4.32.63 MK062 CD3 16D6 Fc _treated
7611: anti ID CH2527 scFv 4.32.63 non cleavable linker CD3 16D6 Fc
6100 GA916-D-16D5 sf W(3a)
6298 GA916-D-16D5-02 sf W(1)
6182 DP47GS TCB sf CHO W(9a)

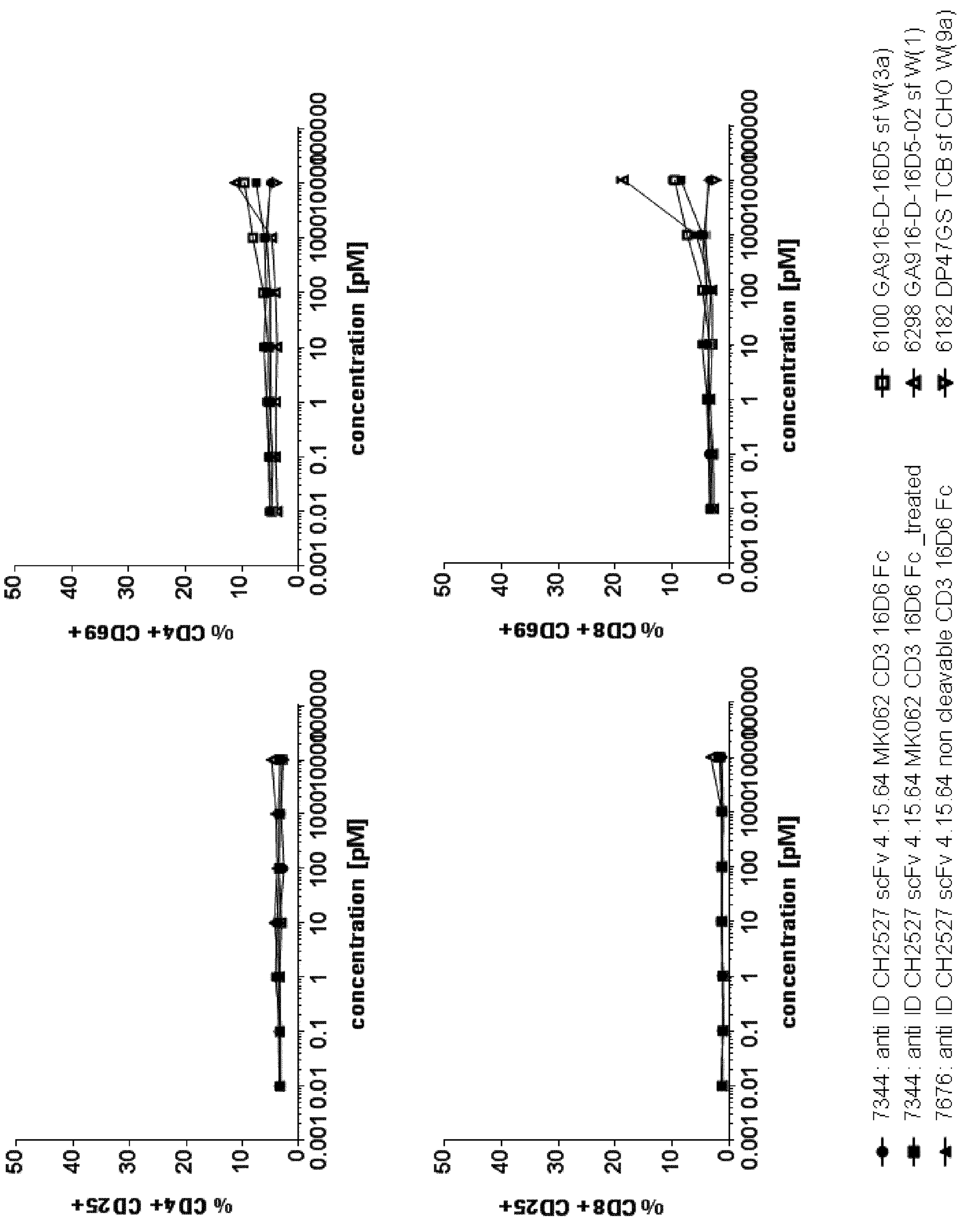
% CD4+ CD25+
% CD8+ CD25+
% CD4+ CD69+
% CD8+ CD69+
concentration [pM]
0.001 0.01 0.1 1 10 100 1000 10000 100000 1000000
0 10 20 30 40 50
7344: anti ID CH2527 scFv 4.15.64 MK062 CD3 16D6 Fc
7344: anti ID CH2527 scFv 4.15.64 MK062 CD3 16D6 Fc_treated
7676: anti ID CH2527 scFv 4.15.64 non cleavable CD3 16D6 Fc
6100 GA916-D-16D5 sf W(3a)
6298 GA916-D-16D5-02 sf W(1)
6182 DP47GS TCB sf CHO W(9a)

FIG. 23

FIG. 24

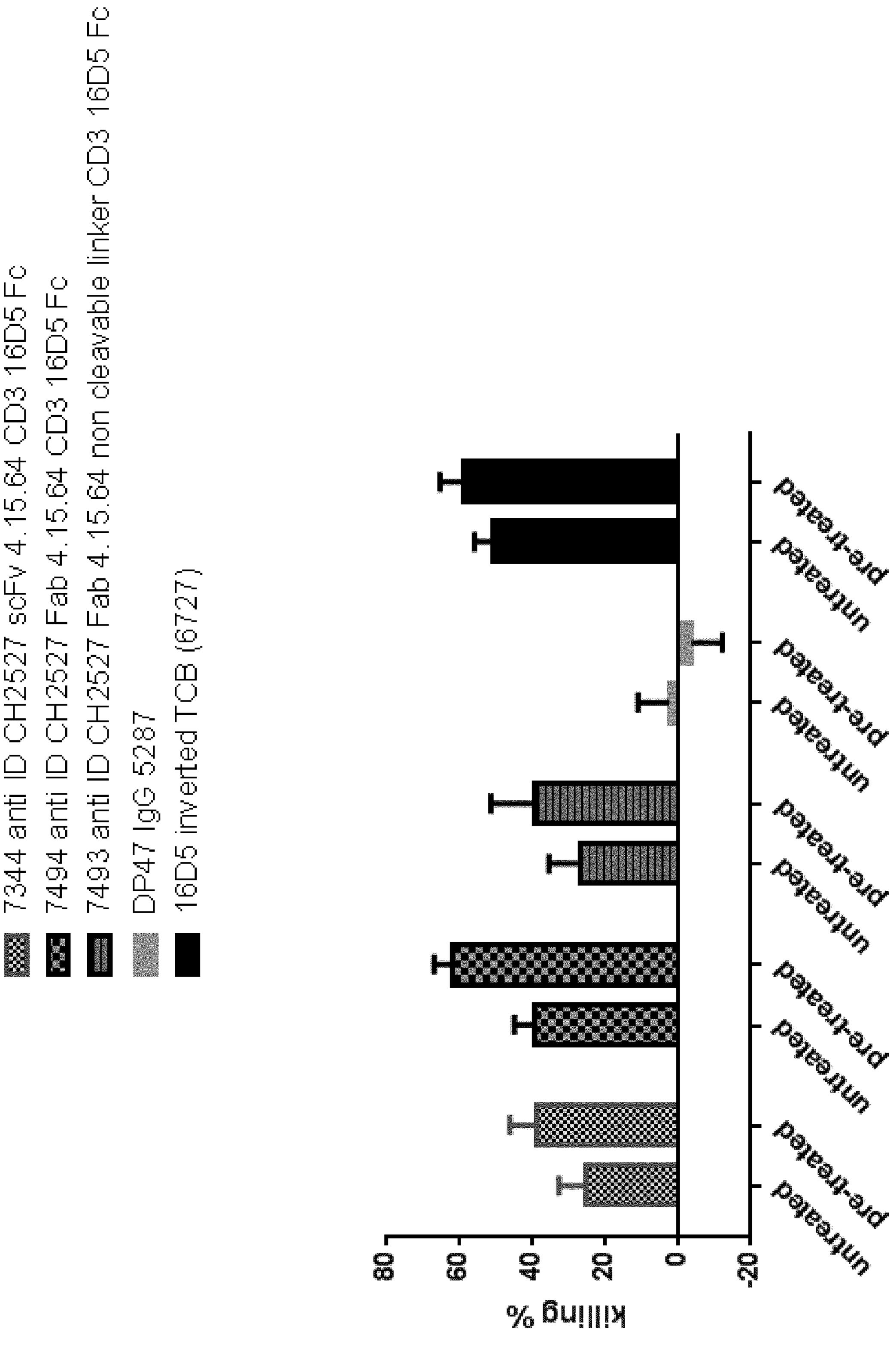
7344 anti ID CH2527 scFv 4.15.64 CD3 16D5 Fc
7494 anti ID CH2527 Fab 4.15.64 CD3 16D5 Fc
7493 anti ID CH2527 Fab 4.15.64 non cleavable linker CD3 16D5 Fc
DP47 IgG 5287
16D5 inverted TCB (6727)
Killing %
80
60
40
20
0
-20
untreated
pre-treated

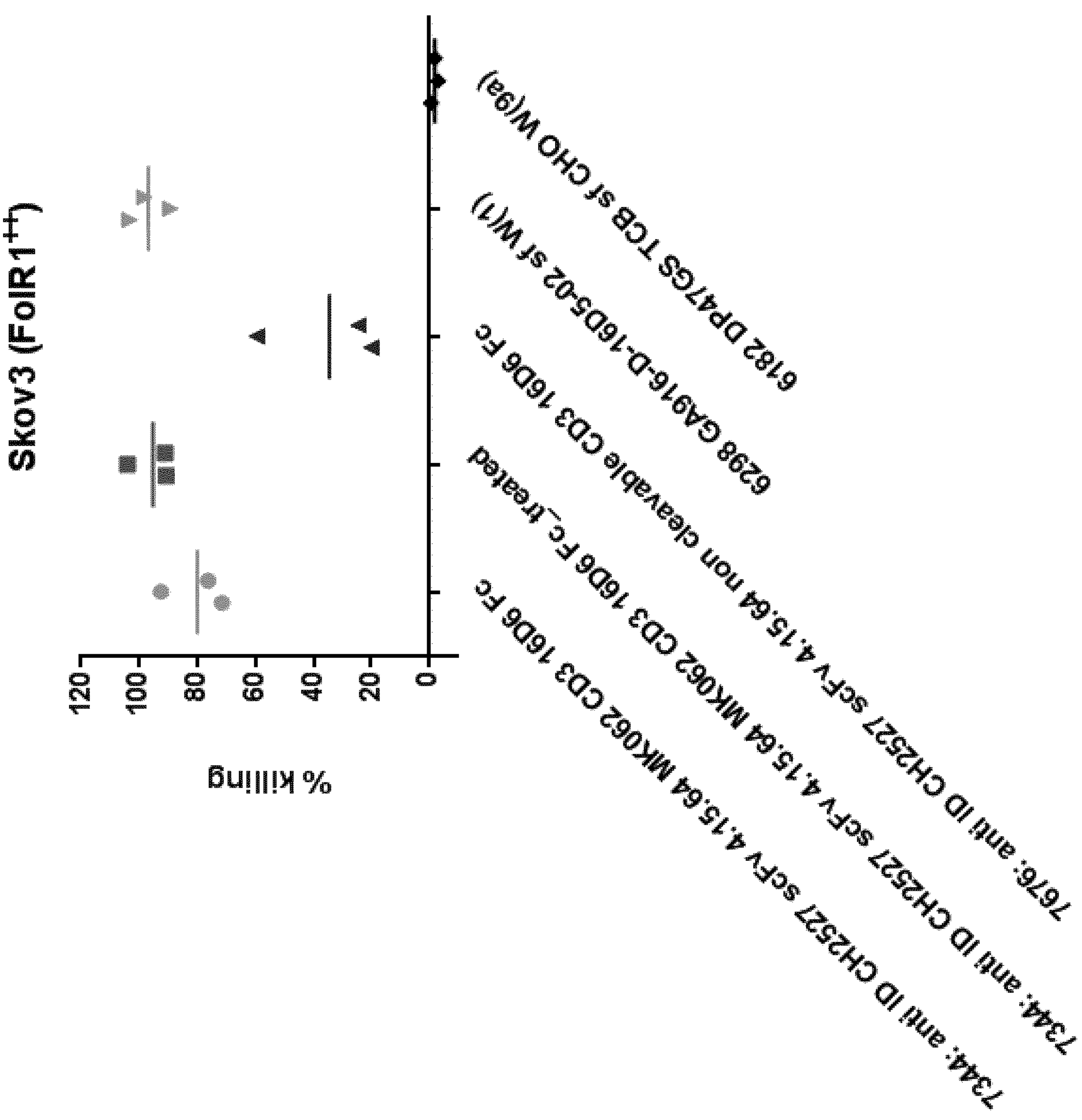
Skov3 (FolR1++)
% killing
120
100
80
60
40
20
0
7344: anti ID CH2527 scFv 4.15.64 MK062 CD3 16D6 Fc
7344: anti ID CH2527 scFv 4.15.64 MK062 CD3 16D6 Fc_treated
7676: anti ID CH2527 scFv 4.15.64 non cleavable CD3 16D6 Fc
6298 GA916-D-16D5-02 sf W(1)
6182 DP47GS TCB sf CHO W(9a)

FIG. 25

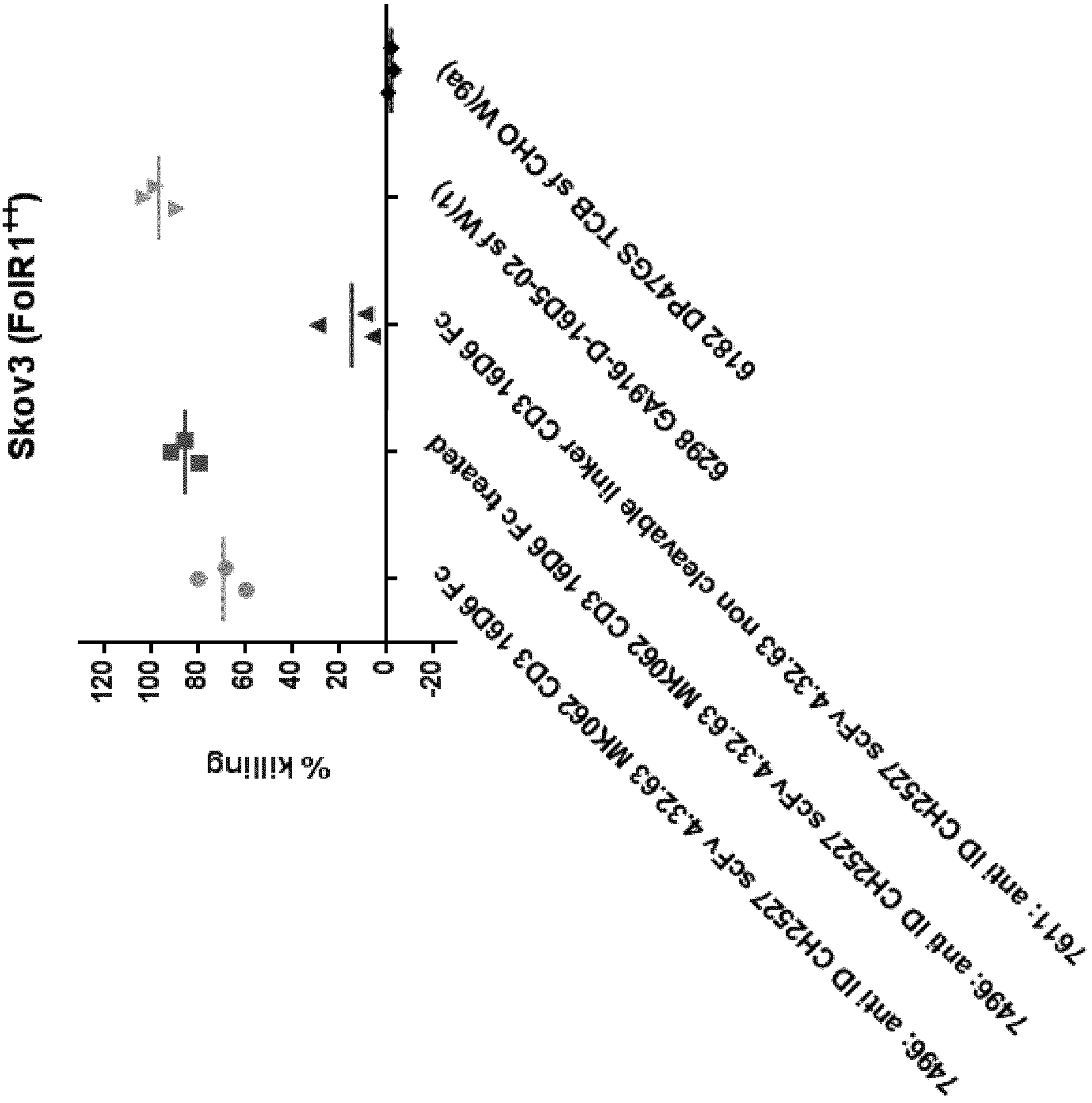
Skov3 (FolR1++)
% killing
120
100
80
60
40
20
0
-20
7496: anti ID CH2527 scFv 4.32.63 MK062 CD3 16D6 Fc
7496: anti ID CH2527 scFv 4.32.63 MK062 CD3 16D6 Fc treated
7611: anti ID CH2527 scFv 4.32.63 non cleavable linker CD3 16D6 Fc
6298 GA916-D-16D5-02 sf W(1)
6182 DP47GS TCB sf CHO W(9a)

FIG. 26

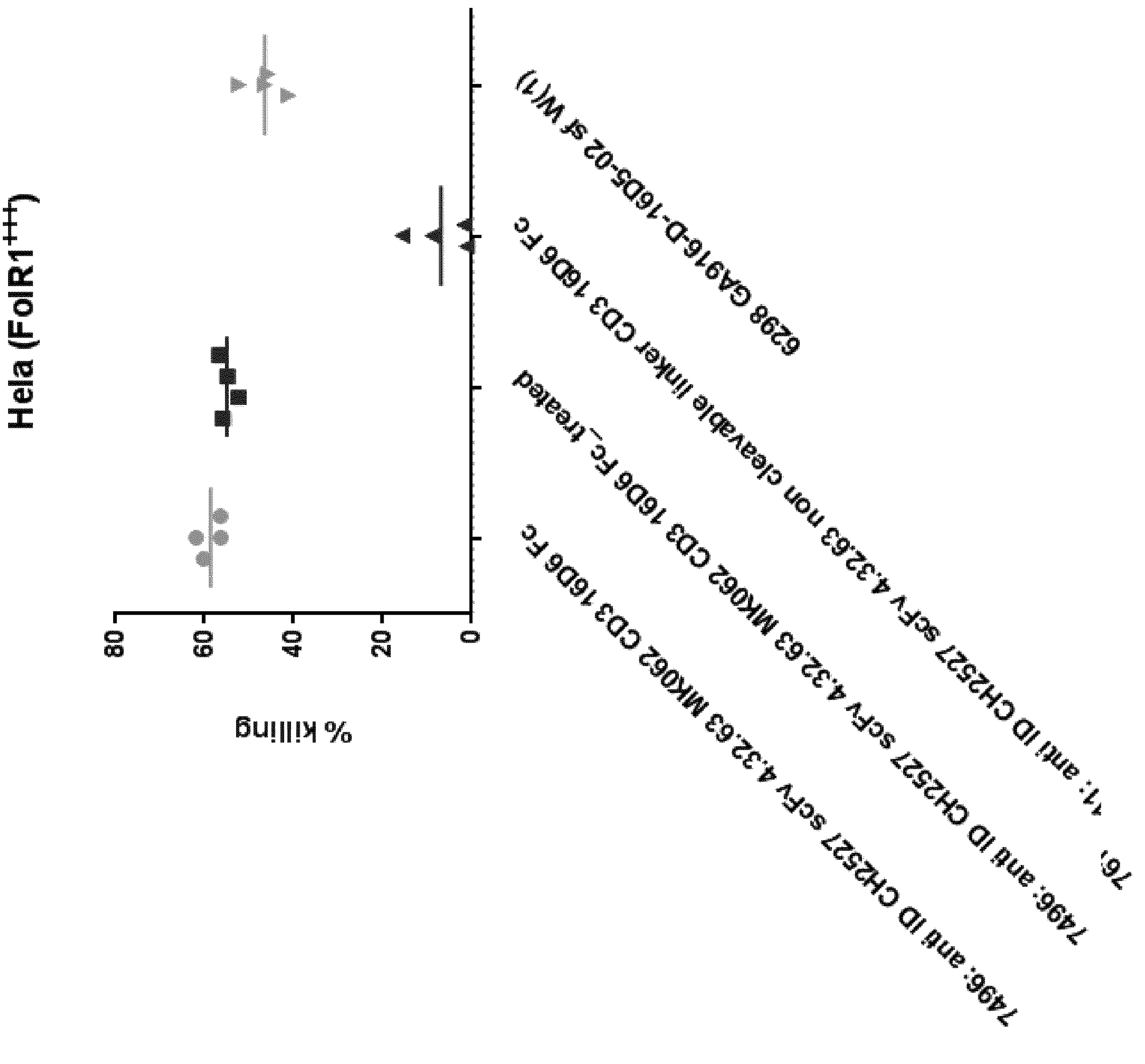
Hela (FolR1+++)
% killing
80
60
40
20
0
7496: anti ID CH2527 scFv 4.32.63 MK062 CD3 16D6 Fc
7496: anti ID CH2527 scFv 4.32.63 MK062 CD3 16D6 Fc_treated
anti ID CH2527 scFv 4.32.63 non cleavable linker CD3 16D6 Fc
6298 GA916-D-16D5-02 sf W(1)

FIG. 27

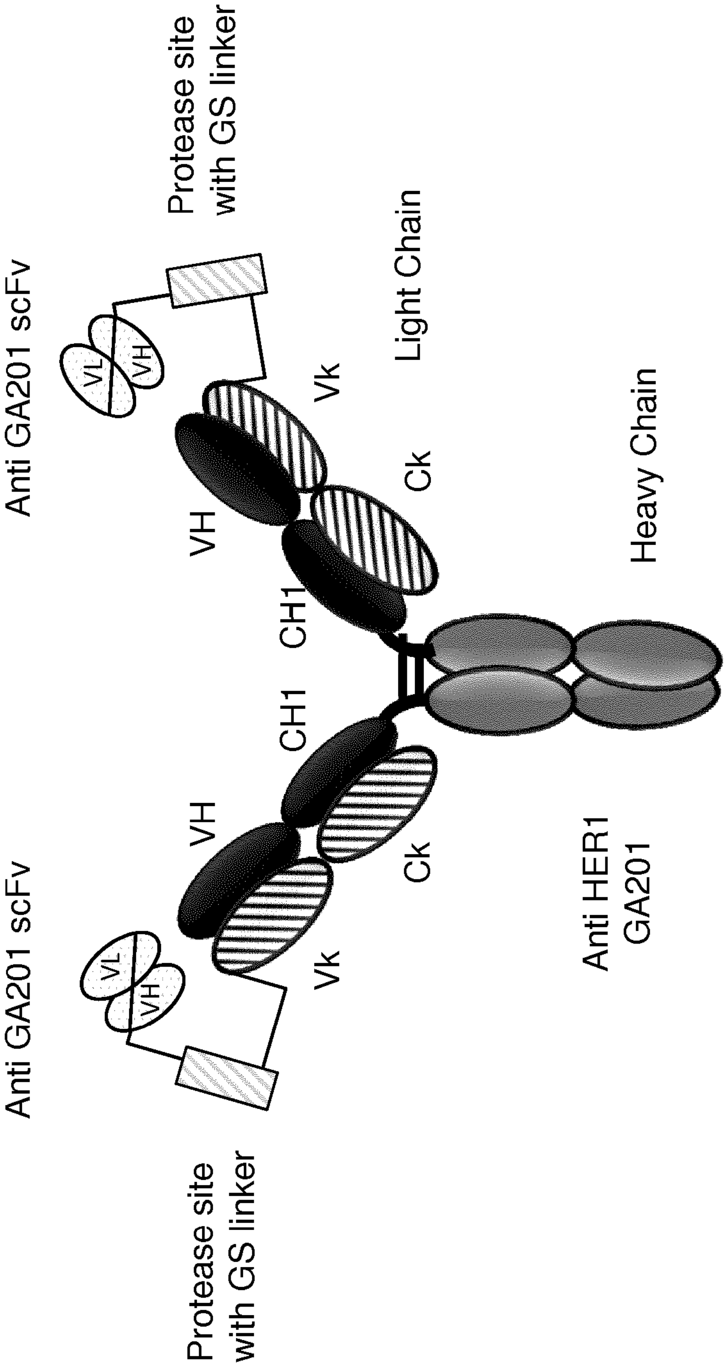
Protease site
with GS linker
Anti GA201 scFv
VL
VH
Light Chain
Vk
Ck
VH
CH1
Heavy Chain
CH1
VH
Ck
Vk
Anti HER1
GA201
Anti GA201 scFv
Protease site
with GS linker

FIG. 28

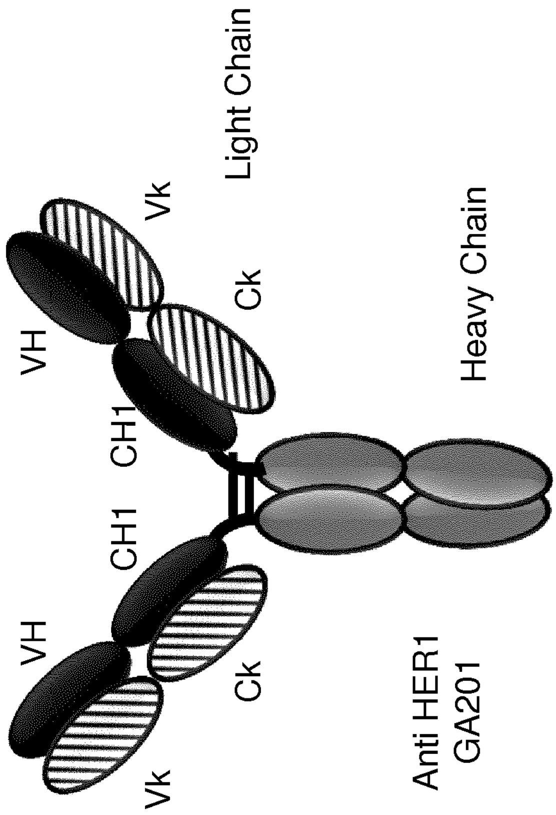
Light Chain
Heavy Chain
Vk
Ck
VH
CH1
CH1
VH
Ck
Vk
Anti HER1
GA201

FIG. 29

FIG. 30A

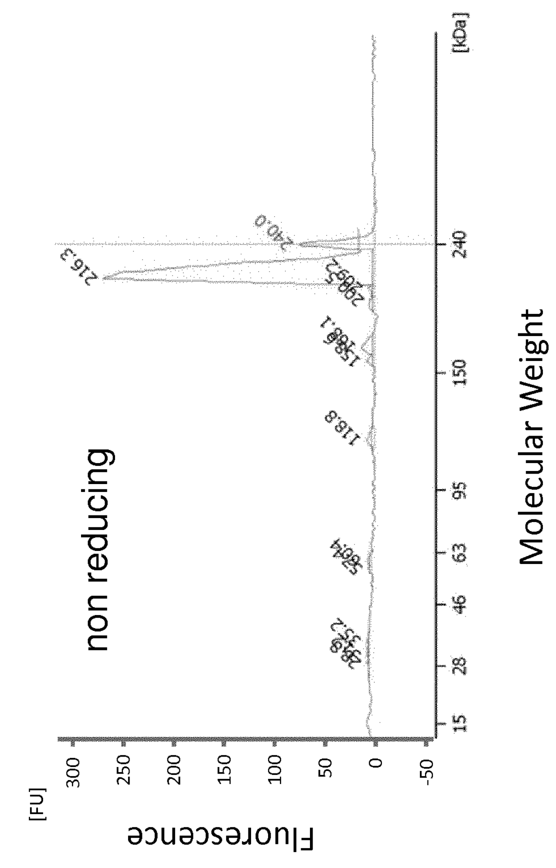


FIG. 30B

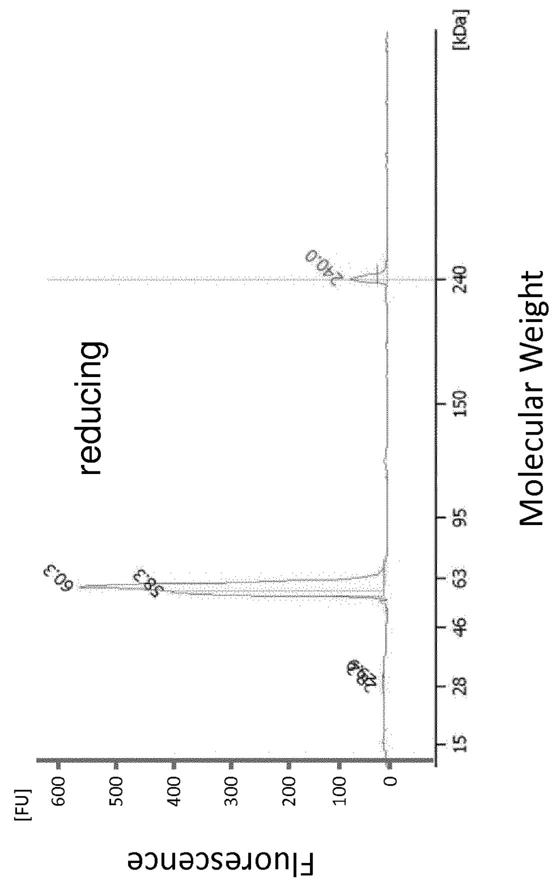

FIG. 31

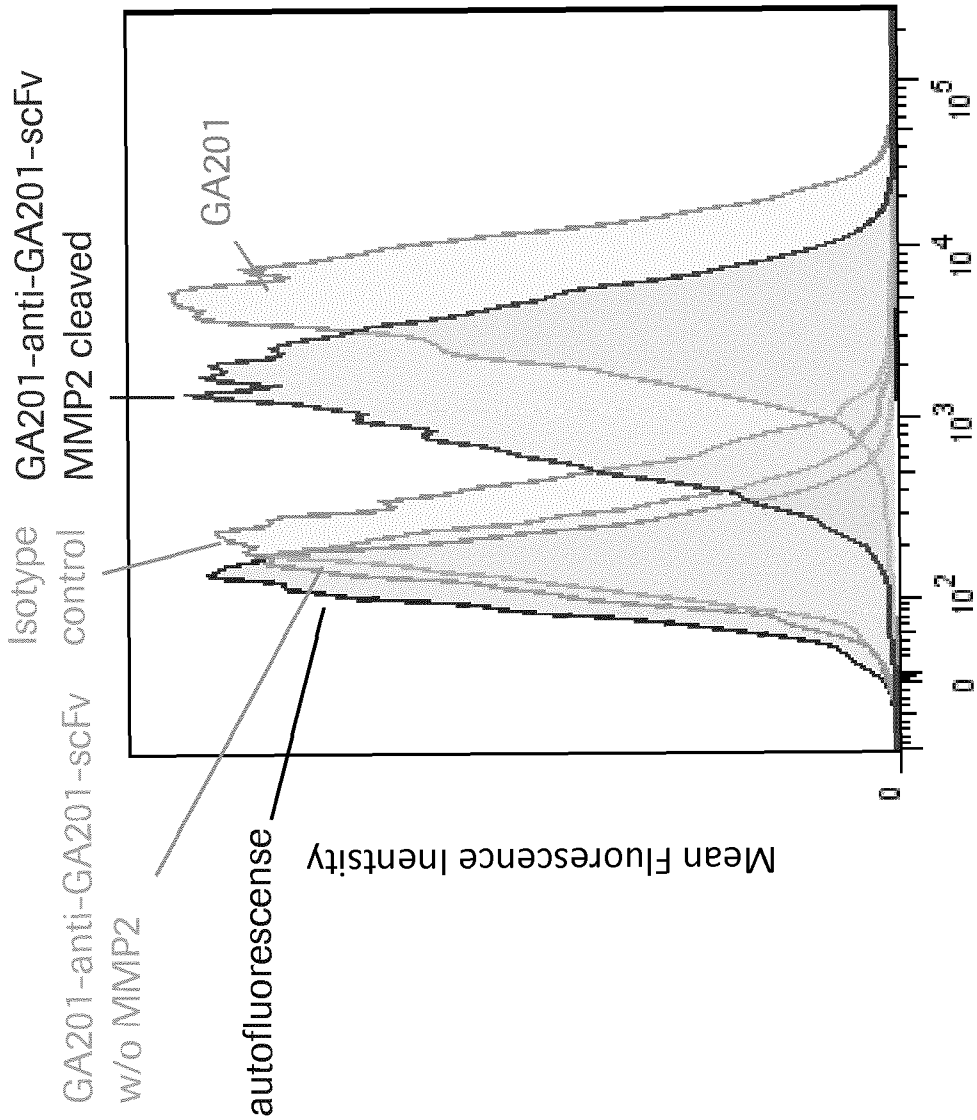
GA201-anti-GA201-scFv
MMP2 cleaved
GA201
Isotype
control
GA201-anti-GA201-scFv
w/o MMP2
autofluorescense
Mean Fluorescence Inentsity
FITC-A
0
$10^2$
$10^3$
$10^4$
$10^5$

FIG. 32

| Molecule | Isotype | Temp. | $k_a$ (1/Ms) | $k_d$ (1/s) | $K_D$ (nM) |
|---|---|---|---|---|---|
| GA201 | IgG1, kappa | 37 °C | 1.68E+05 | 1.20E-04 | 1 |
| GA201-anti-GA201-scFv MMP2 cleaved | IgG1, kappa | 37°C | 1.03E+05 | 1.63E-04 | 2 |
| GA201-anti-GA201-scFv without MMP2 | IgG1, kappa | 37°C | - | - | - |

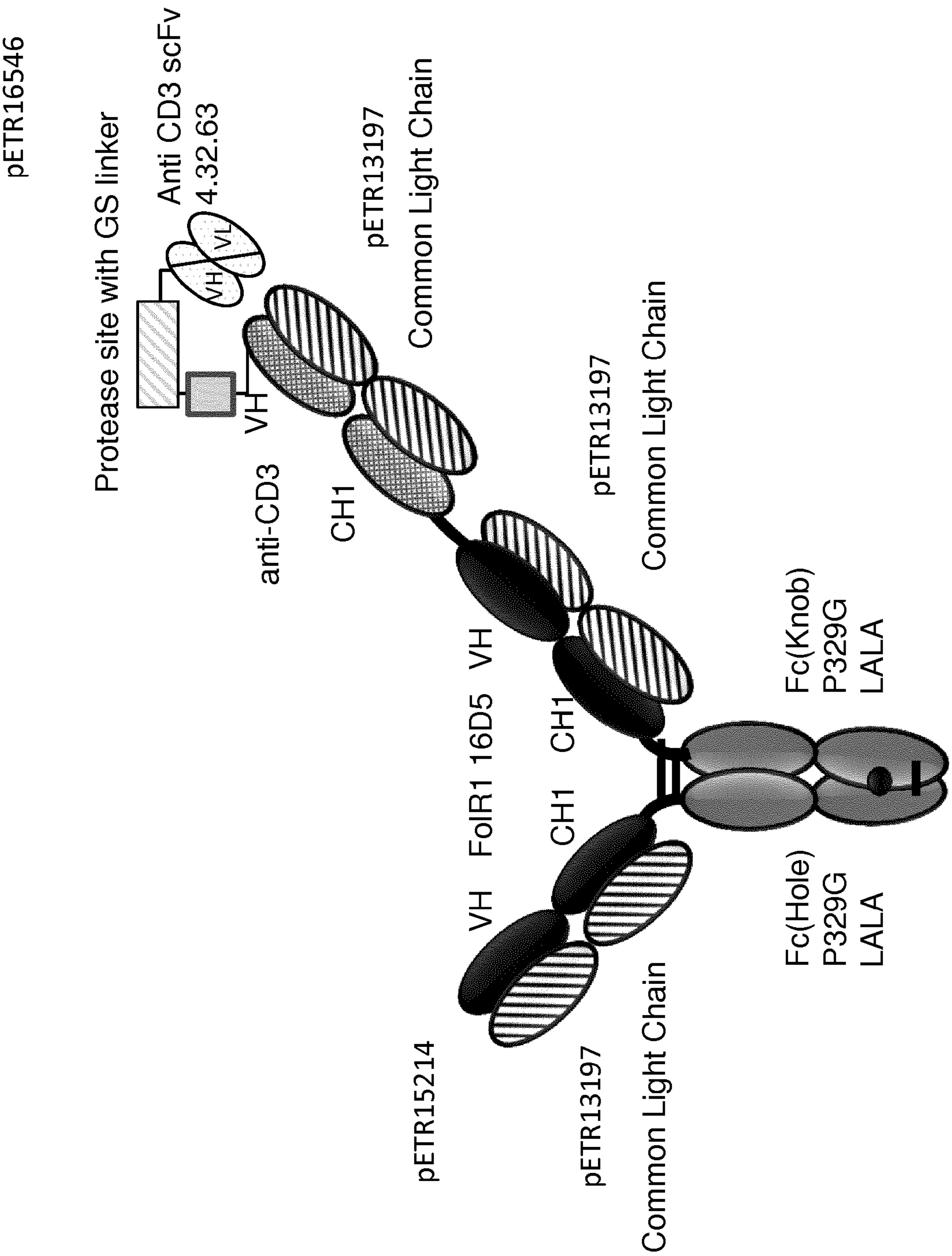
pETR16546
Protease site with GS linker
Anti CD3 scFv
4.32.63
VH
VL
pETR13197
Common Light Chain
VH
anti-CD3
CH1
pETR13197
Common Light Chain
VH
FolR1 16D5
CH1
CH1
VH
Fc(Knob)
P329G
LALA
Fc(Hole)
P329G
LALA
pETR15214
pETR13197
Common Light Chain

8364

FIG. 33A

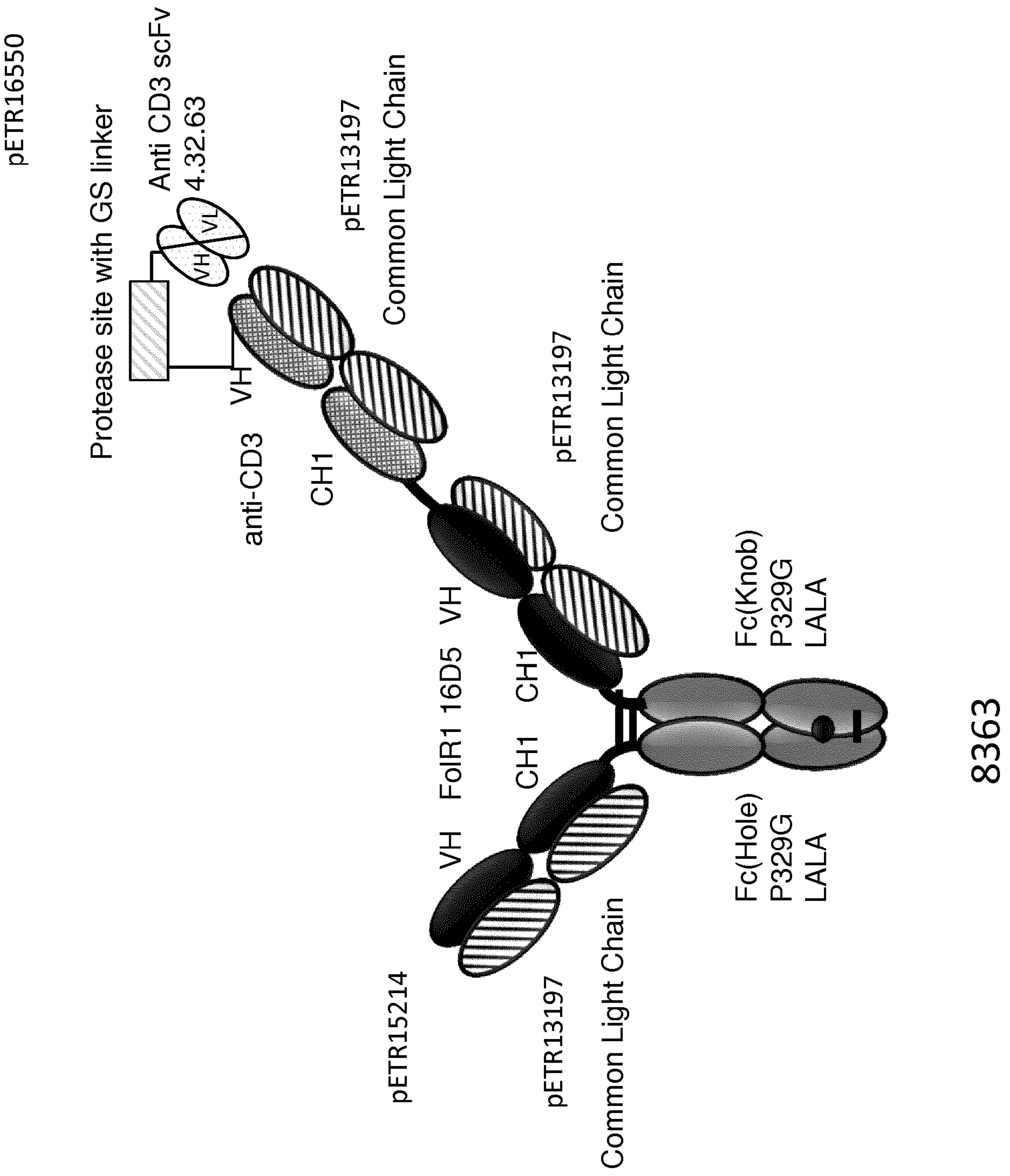
pETR16550
Protease site with GS linker
Anti CD3 scFv
4.32.63
VH
VL
pETR13197
Common Light Chain
anti-CD3
VH
CH1
pETR13197
Common Light Chain
FolR1 16D5 VH
CH1
CH1
VH
Fc(Knob)
P329G
LALA
Fc(Hole)
P329G
LALA
8363
pETR15214
pETR13197
Common Light Chain

FIG. 33B

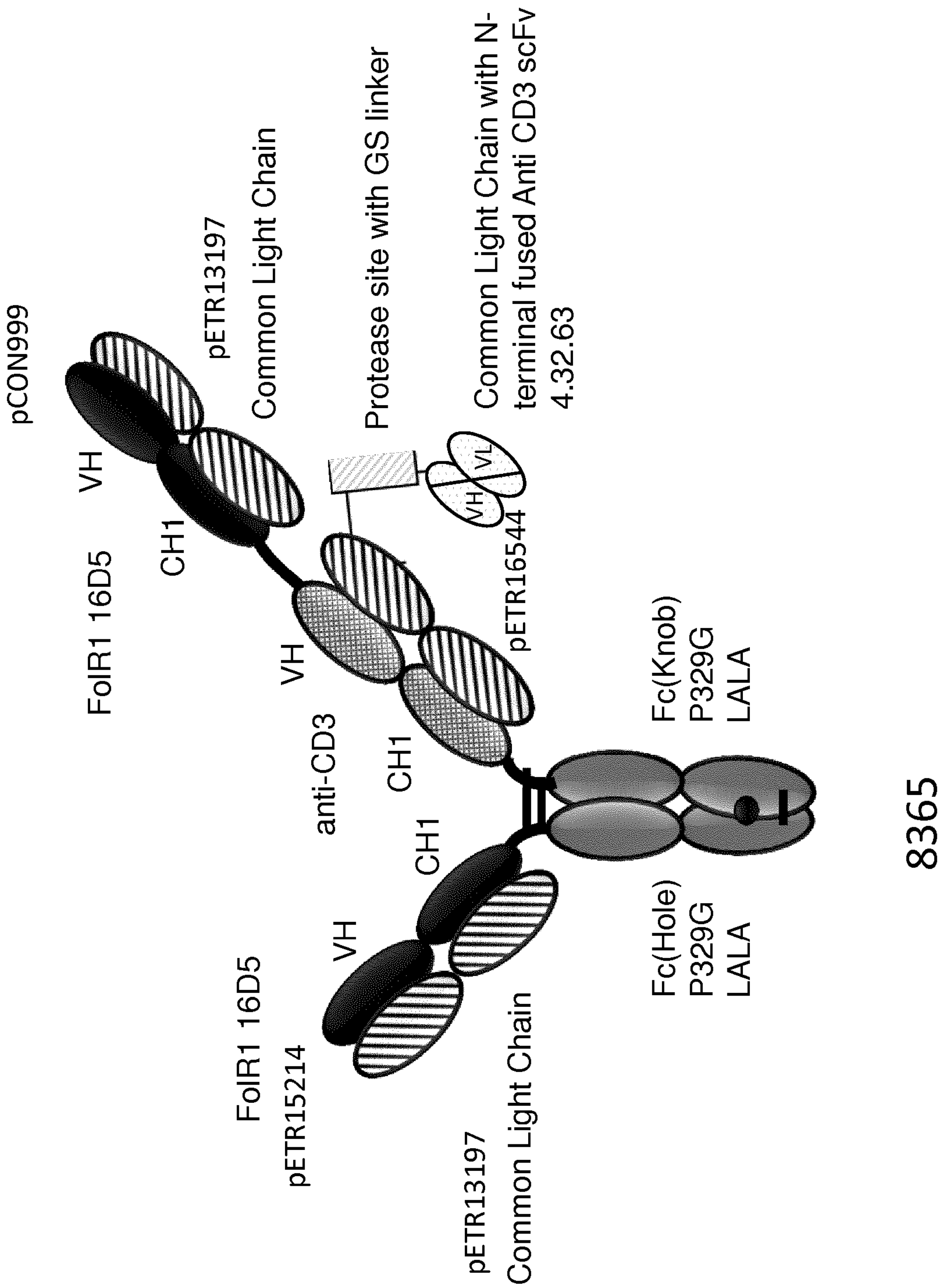
pCON999
pETR13197
Common Light Chain
Protease site with GS linker
Common Light Chain with N-terminal fused Anti CD3 scFv 4.32.63
VH
VL
VH
CH1
FolR1 16D5
VH
anti-CD3
CH1
pETR16544
Fc(Knob)
P329G
LALA
CH1
VH
FolR1 16D5
pETR15214
pETR13197
Common Light Chain
Fc(Hole)
P329G
LALA
8365

FIG. 33C

FIG. 33D

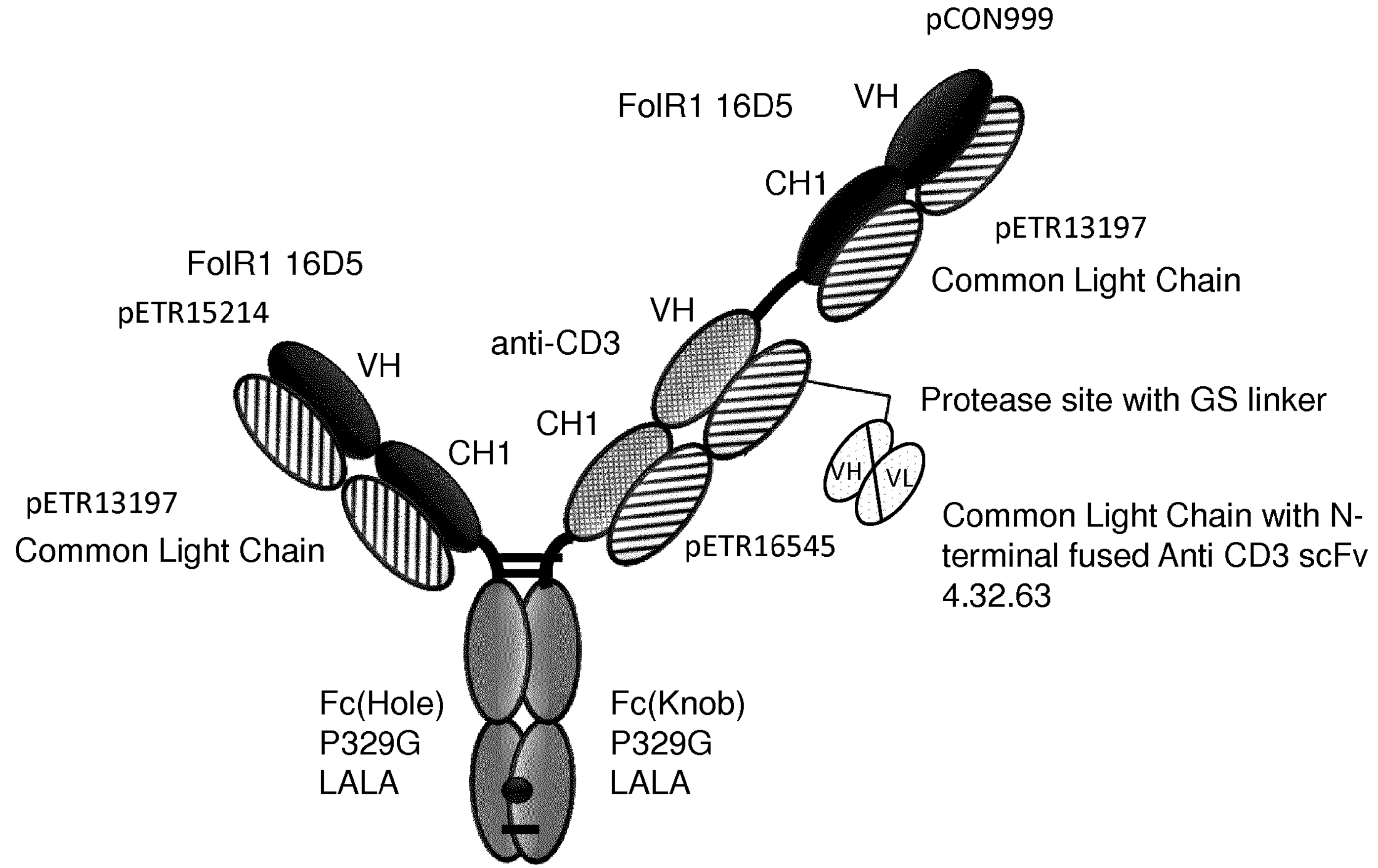

pCON999
FolR1 16D5
VH
CH1
pETR13197
Common Light Chain
FolR1 16D5
pETR15214
VH
CH1
pETR13197
Common Light Chain
anti-CD3
VH
CH1
pETR16545
Protease site with GS linker
VH
VL
Common Light Chain with N-terminal fused Anti CD3 scFv 4.32.63
Fc(Hole)
P329G
LALA
Fc(Knob)
P329G
LALA


8366

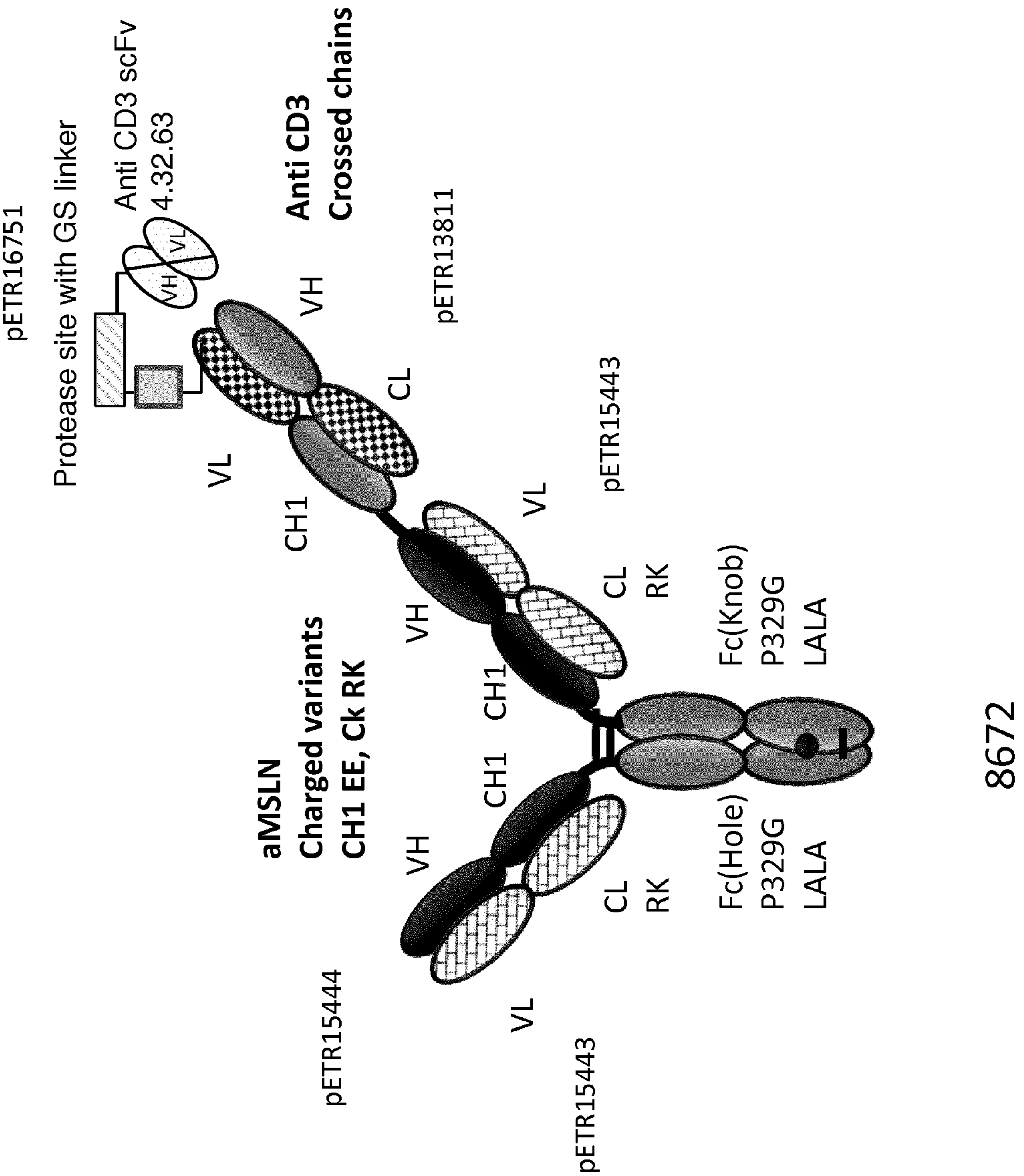
pETR16751
Protease site with GS linker
Anti CD3 scFv
4.32.63
VH
VL
Anti CD3
Crossed chains
pETR13811
VH
VL
CL
CH1
pETR15443
VL
VH
CL
RK
CH1
aMSLN
Charged variants
CH1 EE, Ck RK
Fc(Knob)
P329G
LALA
CH1
VH
CL
RK
Fc(Hole)
P329G
LALA
VL
pETR15444
pETR15443
8672

FIG. 33E

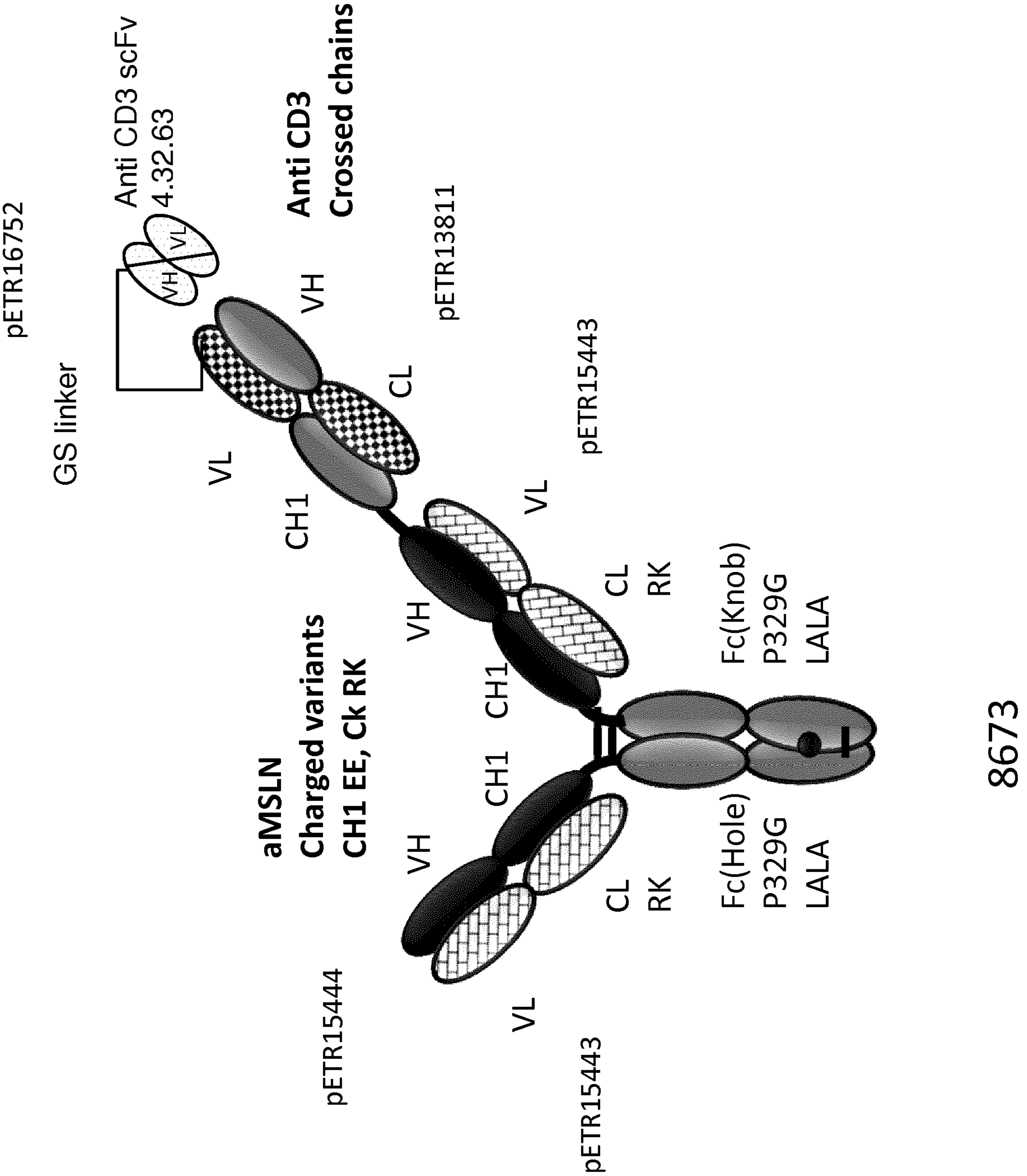
pETR16752
GS linker
Anti CD3 scFv
4.32.63
VH
VL
Anti CD3
Crossed chains
pETR13811
VH
CL
VL
CH1
pETR15443
VL
CL
RK
VH
CH1
Fc(Knob)
P329G
LALA
aMSLN
Charged variants
CH1 EE, Ck RK
CH1
VH
CL
RK
Fc(Hole)
P329G
LALA
VL
pETR15444
pETR15443
8673

FIG. 33F

FIG. 33G

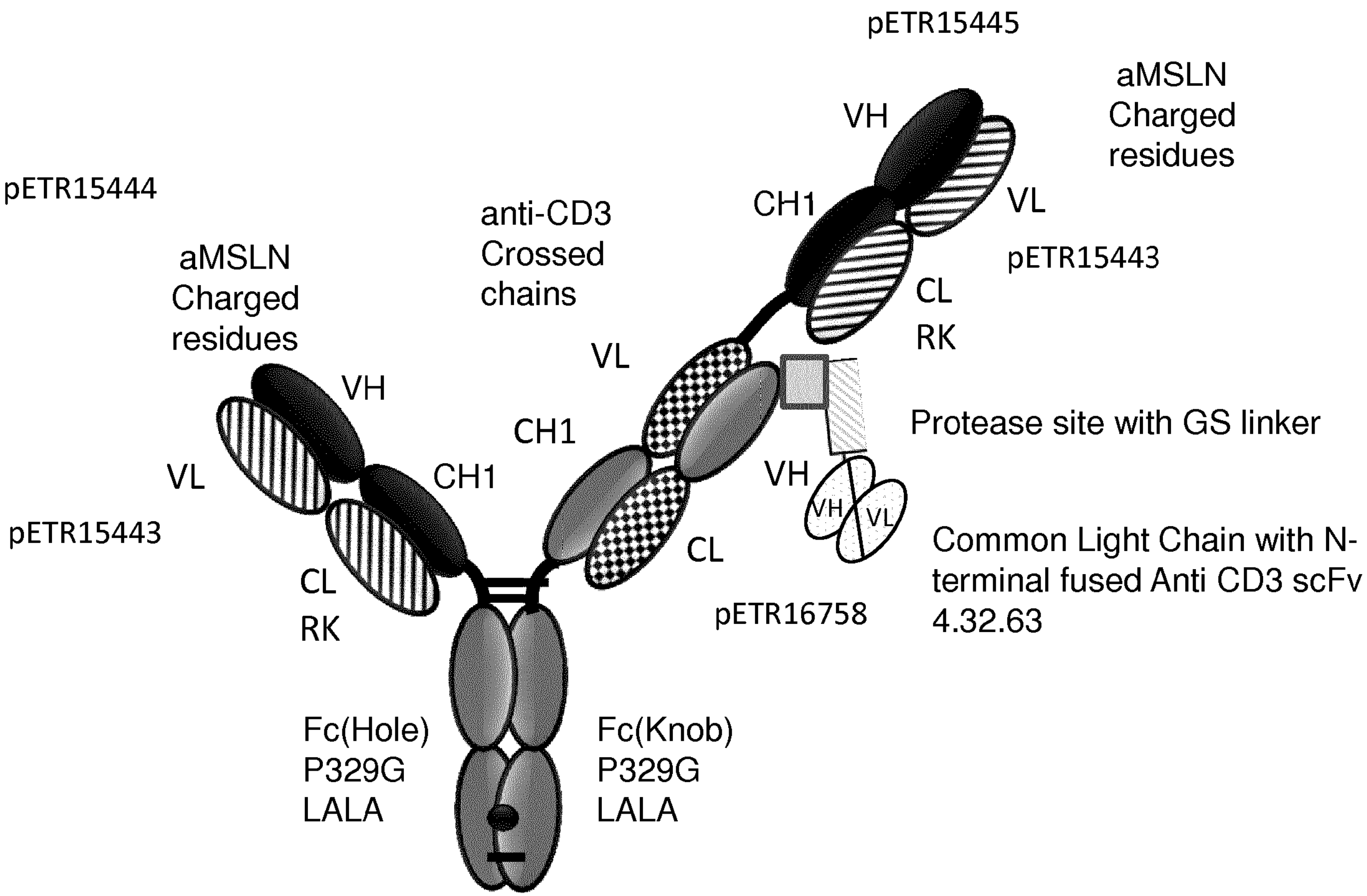
pETR15445
aMSLN
Charged
residues
VH
VL
CH1
pETR15443
CL
RK
pETR15444
anti-CD3
Crossed
chains
aMSLN
Charged
residues
VL
VH
CH1
Protease site with GS linker
VL
CH1
VH
VH
VL
pETR15443
Common Light Chain with N-
terminal fused Anti CD3 scFv
4.32.63
CL
CL
RK
pETR16758
Fc(Hole)
P329G
LALA
Fc(Knob)
P329G
LALA

8674

FIG. 33H

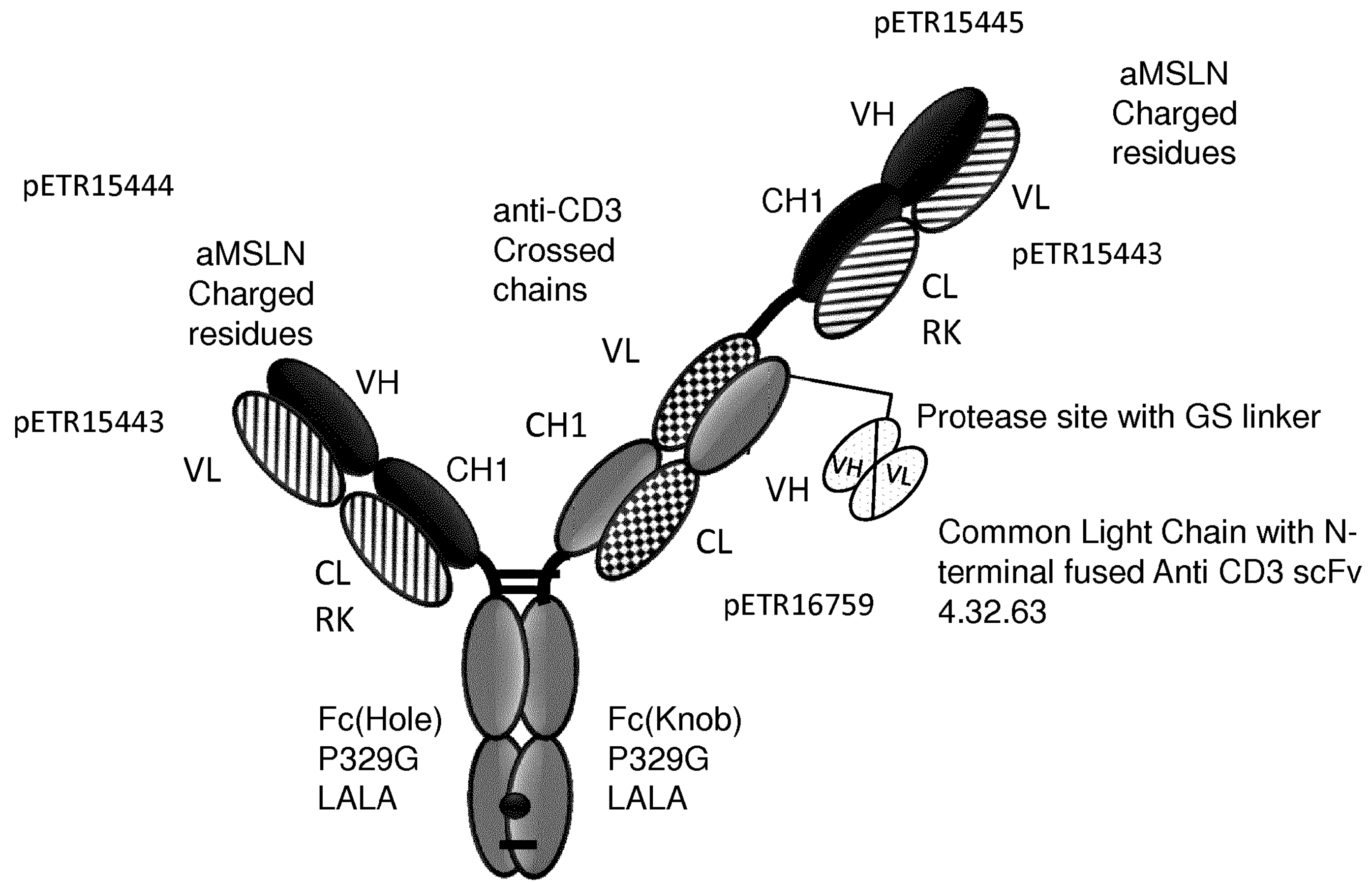
pETR15445
aMSLN
Charged
residues
VH
VL
CH1
pETR15443
CL
RK
pETR15444
aMSLN
Charged
residues
anti-CD3
Crossed
chains
VL
VH
pETR15443
VL
CH1
CH1
Protease site with GS linker
VH
VL
VH
CL
RK
CL
Common Light Chain with N-terminal fused Anti CD3 scFv 4.32.63
pETR16759
Fc(Hole)
P329G
LALA
Fc(Knob)
P329G
LALA

8675

FIG. 33I

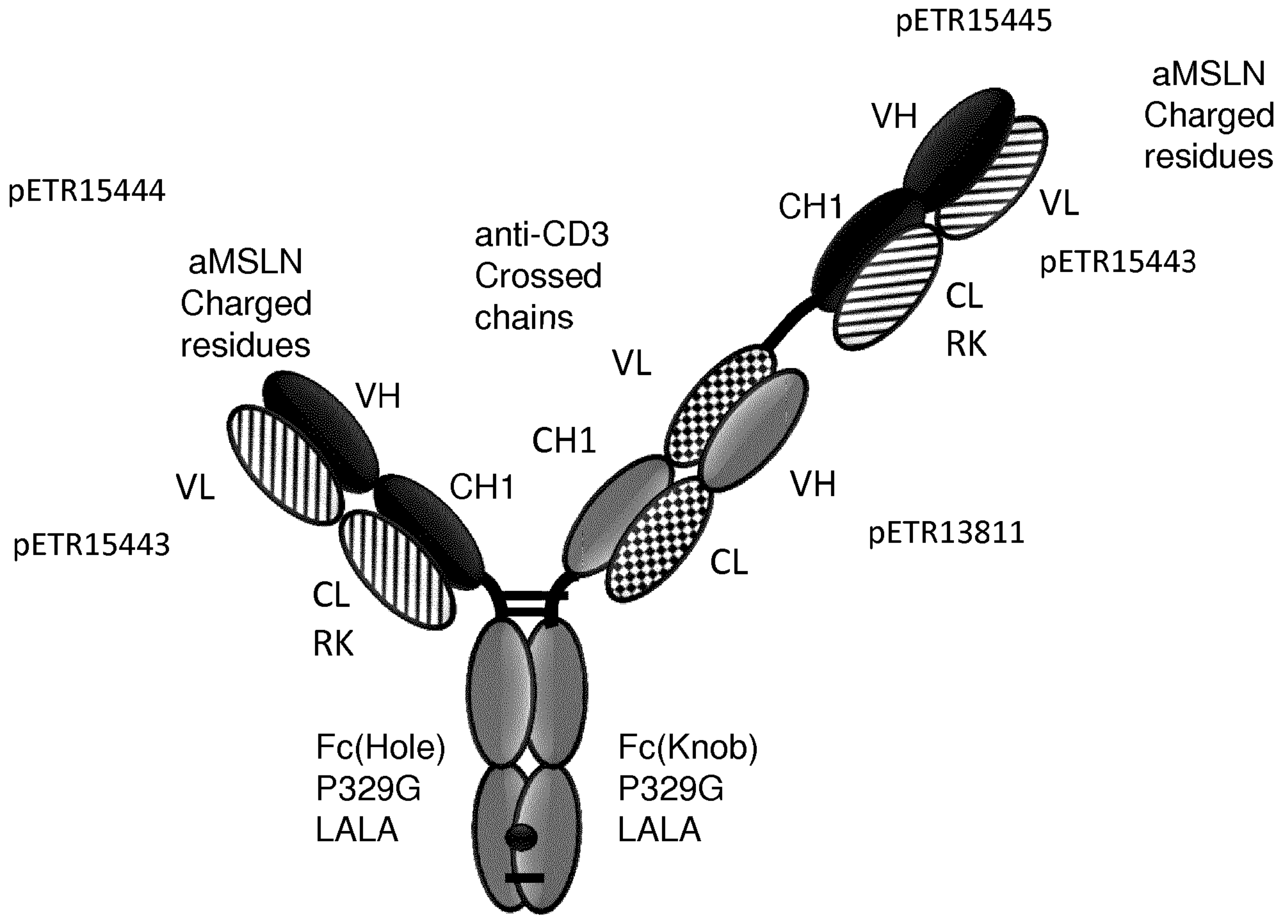
pETR15445
aMSLN
Charged
residues
VH
VL
CH1
pETR15443
CL
RK
pETR15444
anti-CD3
Crossed
chains
aMSLN
Charged
residues
VL
VH
CH1
VL
CH1
VH
pETR13811
pETR15443
CL
CL
RK
Fc(Hole)
P329G
LALA
Fc(Knob)
P329G
LALA

8505

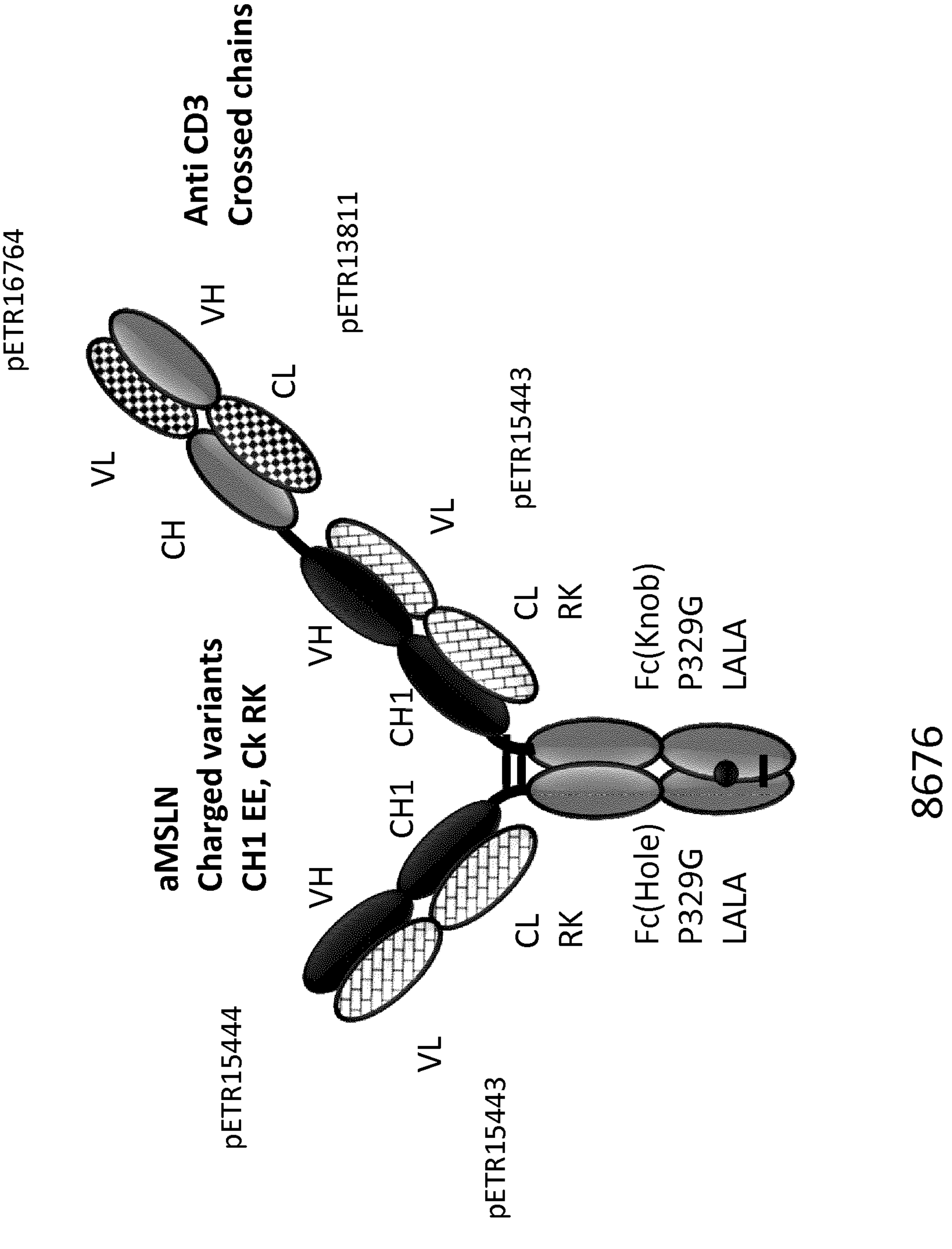
pETR16764
Anti CD3
Crossed chains
pETR13811
VL
VH
CL
CH
pETR15443
VL
VH
CL
RK
CH1
aMSLN
Charged variants
CH1 EE, Ck RK
Fc(Knob)
P329G
LALA
CH1
VH
CL
RK
Fc(Hole)
P329G
LALA
8676
pETR15444
VL
pETR15443

FIG. 33J

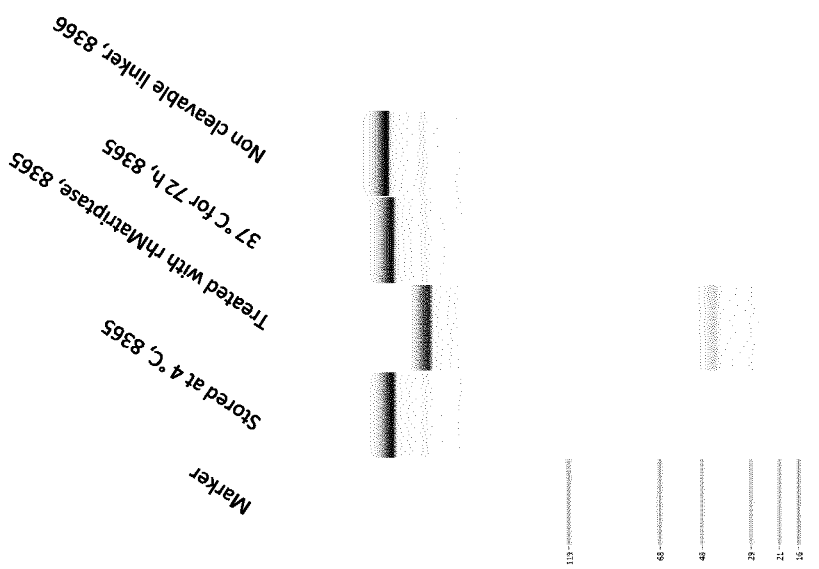
Non cleavable linker, 8366
37 °C for 72 h, 8365
Treated with rhMatriptase, 8365
Stored at 4 °C, 8365
Marker
119
68
48
29
21
16

FIG. 34

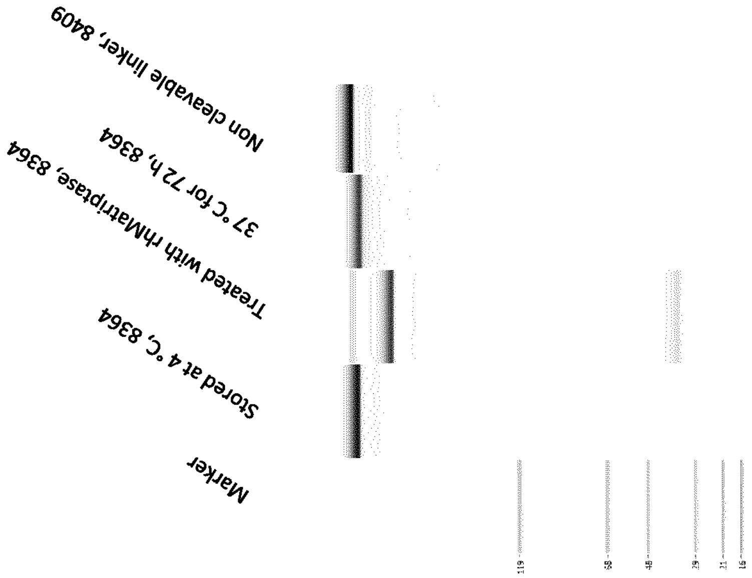
Non cleavable linker, 8409
37 °C for 72 h, 8364
Treated with rhMatriptase, 8364
Stored at 4 °C, 8364
Marker

FIG. 35A

FIG. 35B

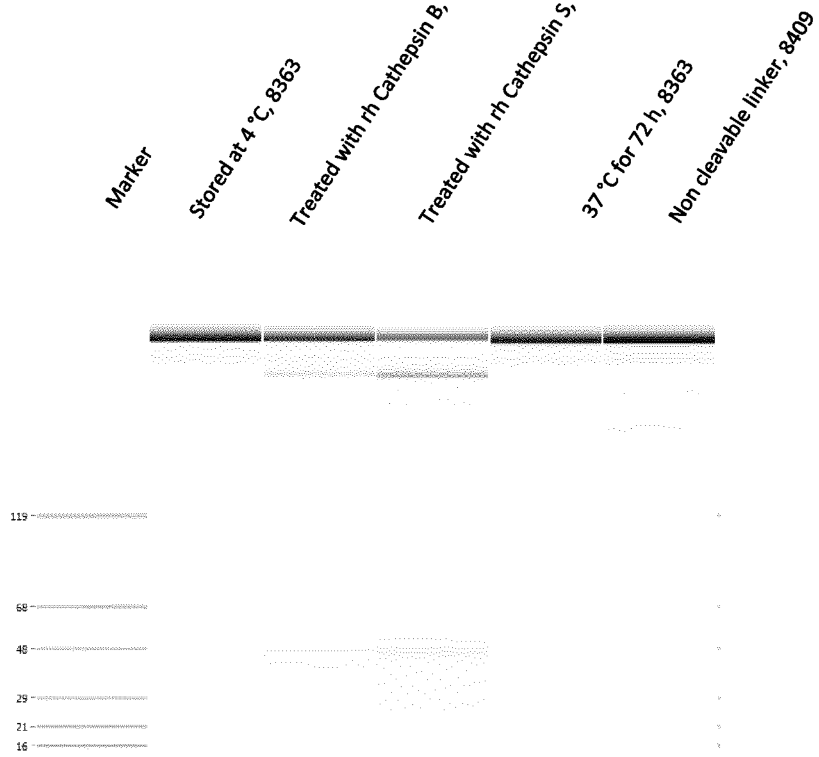
Marker
Stored at 4 °C, 8363
Treated with rh Cathepsin B, 8363
Treated with rh Cathepsin S, 8363
37 °C for 72 h, 8363
Non cleavable linker, 8409
119
68
48
29
21
16

FIG. 36A

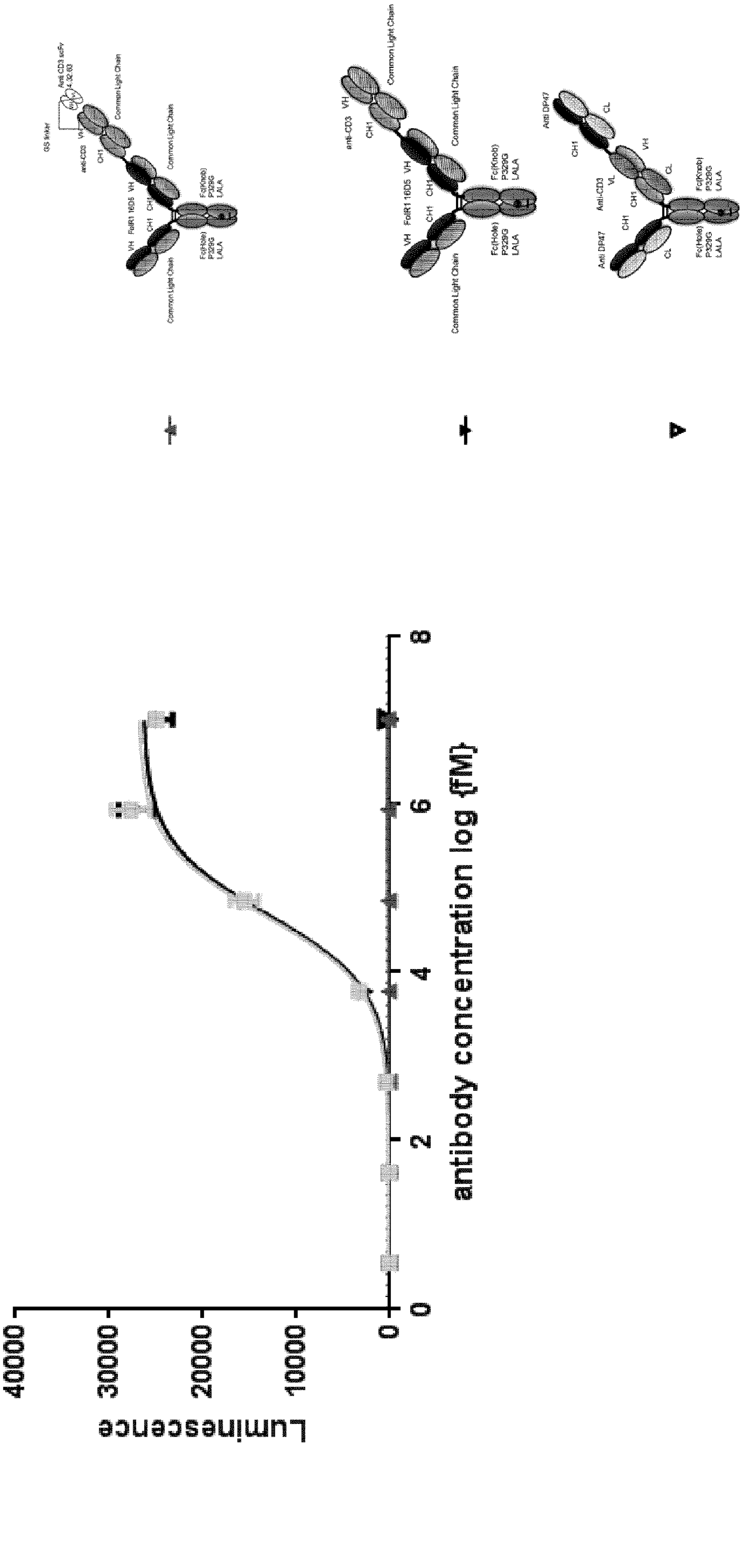
Luminescence
40000
30000
20000
10000
0
0
2
4
6
8
antibody concentration log (fM)
8364
treated
8409
6298
7235
Protease site with GS linker
Anti CD3 scFv
anti-CD3
VH
CH1
Common Light Chain
VH
FolR1 16D5
Fc(Knob)
P329G
LALA
Fc(Hole)
GS linker
Anti DP47
Anti-CD3
VL
CL

FIG. 36B

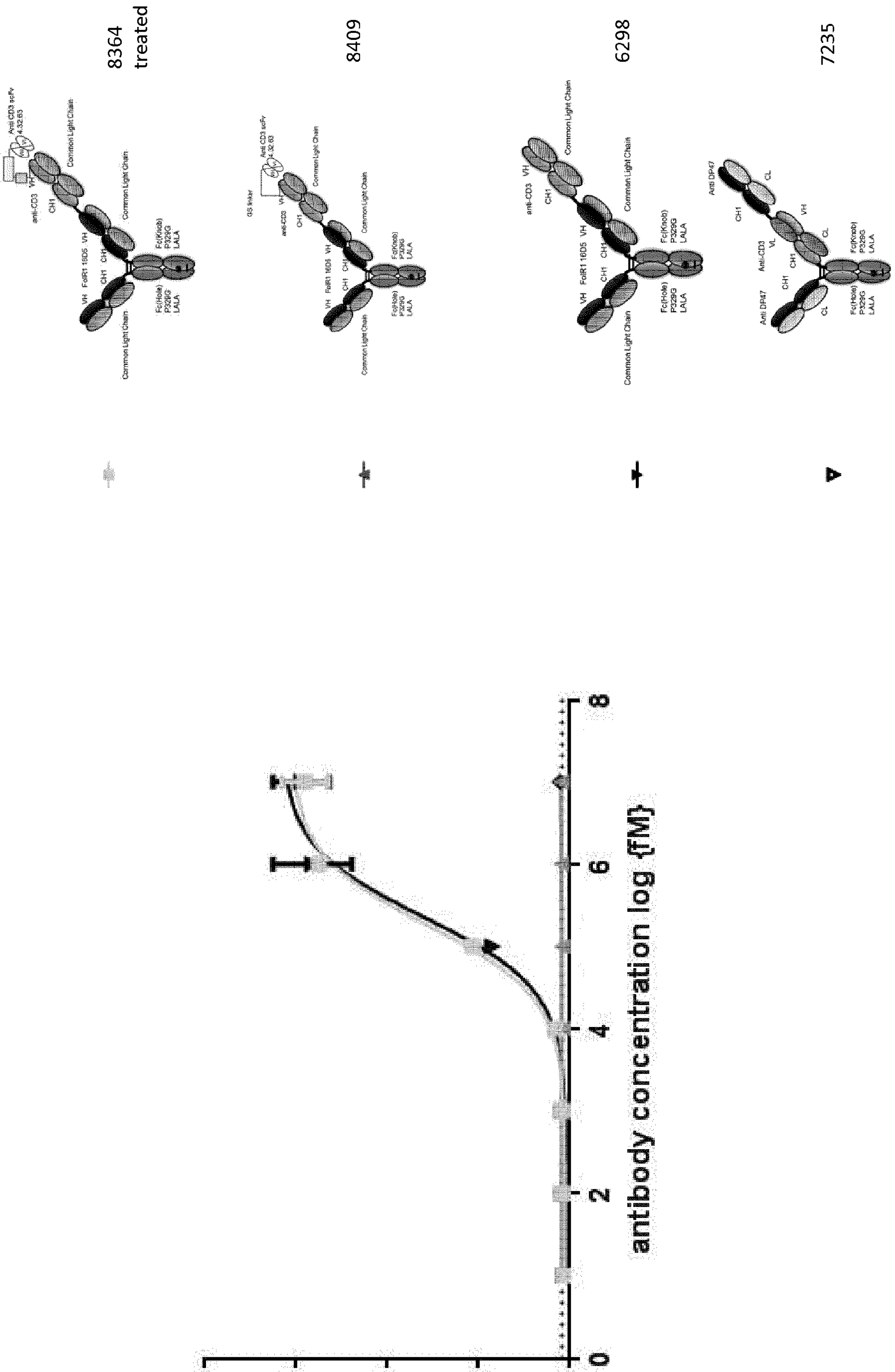

Luminescence
4000
3000
2000
1000
0
0
2
4
6
8
antibody concentration log {fM}
8364 treated
8409
6298
7235
Protease site with GS linker
Anti CD3 scFv
anti-CD3
VH
CH1
Common Light Chain
FolR1 16D5
Fc(Hole)
P329G
LALA
Fc(Knob)
P329G
LALA
GS linker
Anti DP47
CL
VL
Anti-CD3

FIG. 37A

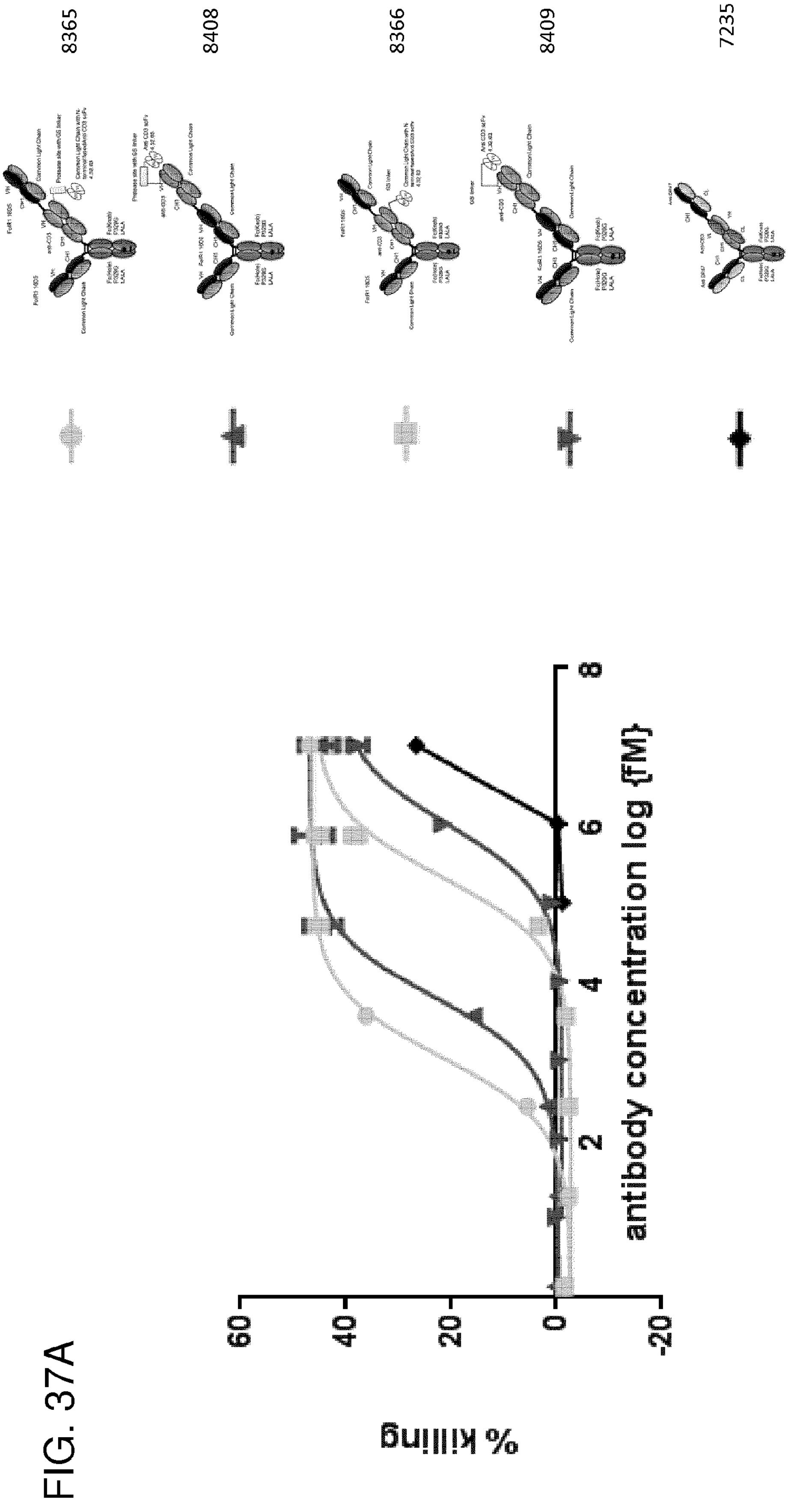
% killing
60
40
20
0
-20
antibody concentration log (fM)
2
4
6
8
8365
8408
8366
8409
7235

FIG. 37B

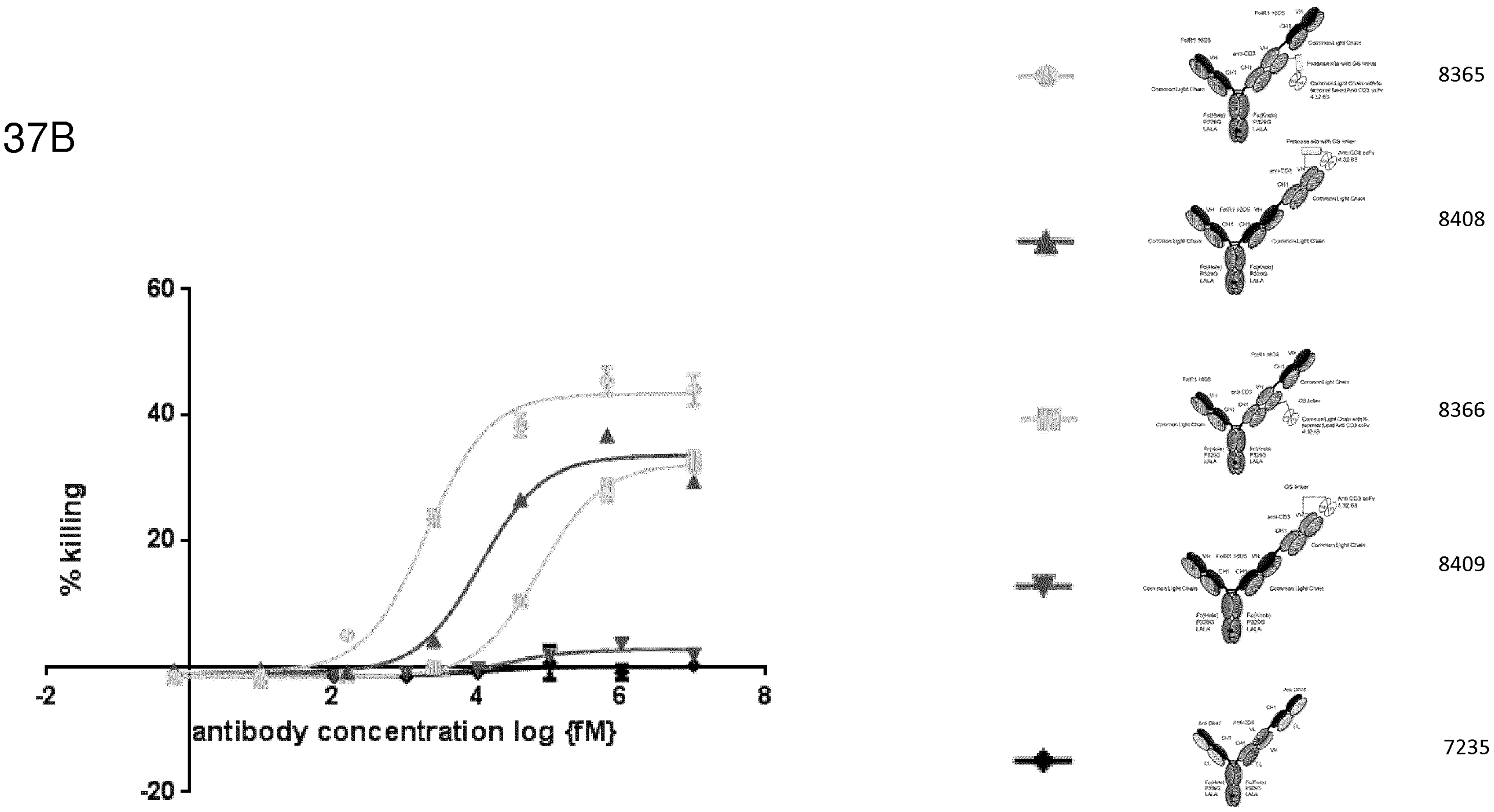
60
40
20
-20
% killing
-2
2
4
6
8
antibody concentration log {fM}
8365
8408
8366
8409
7235

FIG. 37C

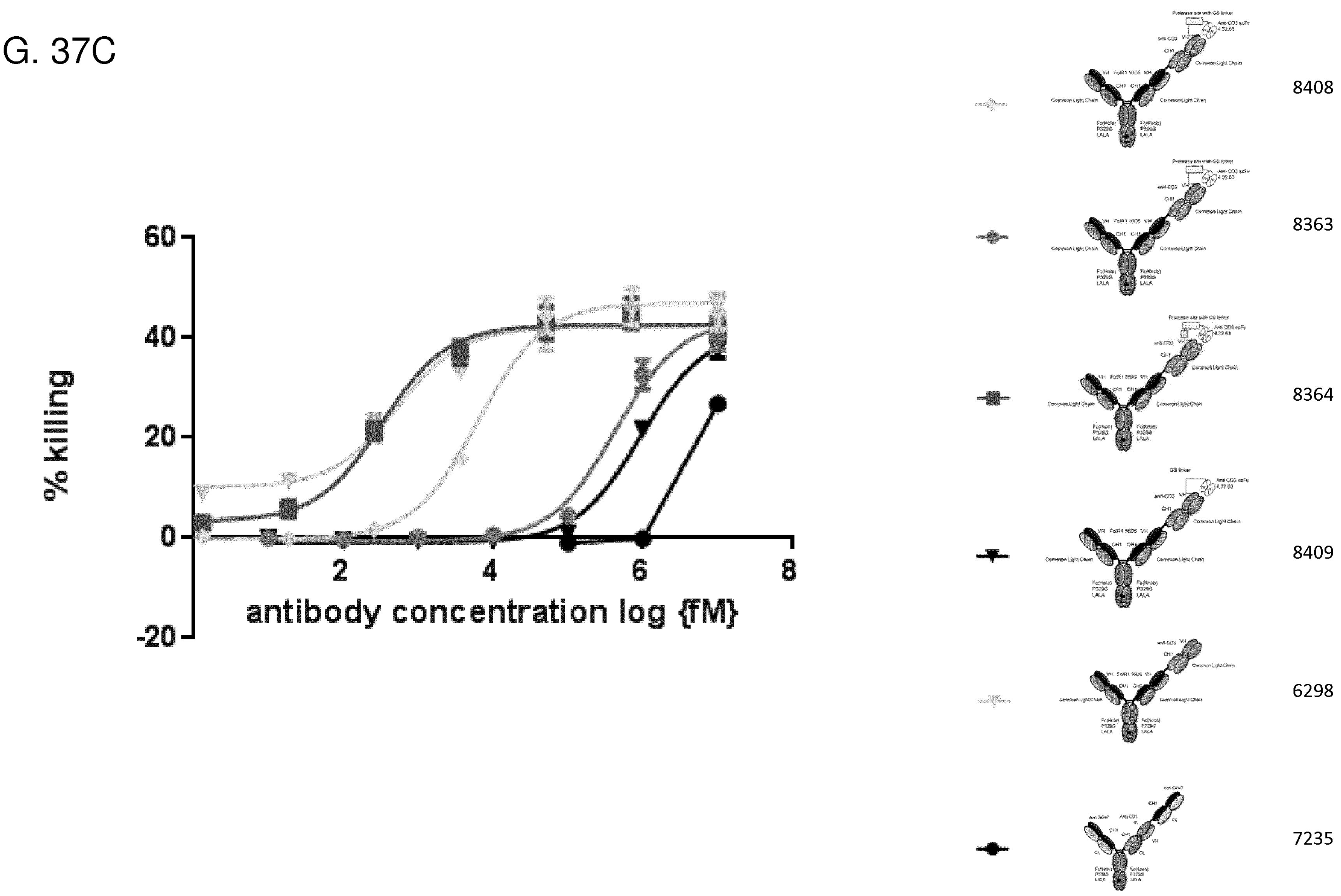
% killing
60
40
20
0
-20
2
4
6
8
antibody concentration log {fM}
8408
8363
8364
8409
6298
7235

FIG. 37D

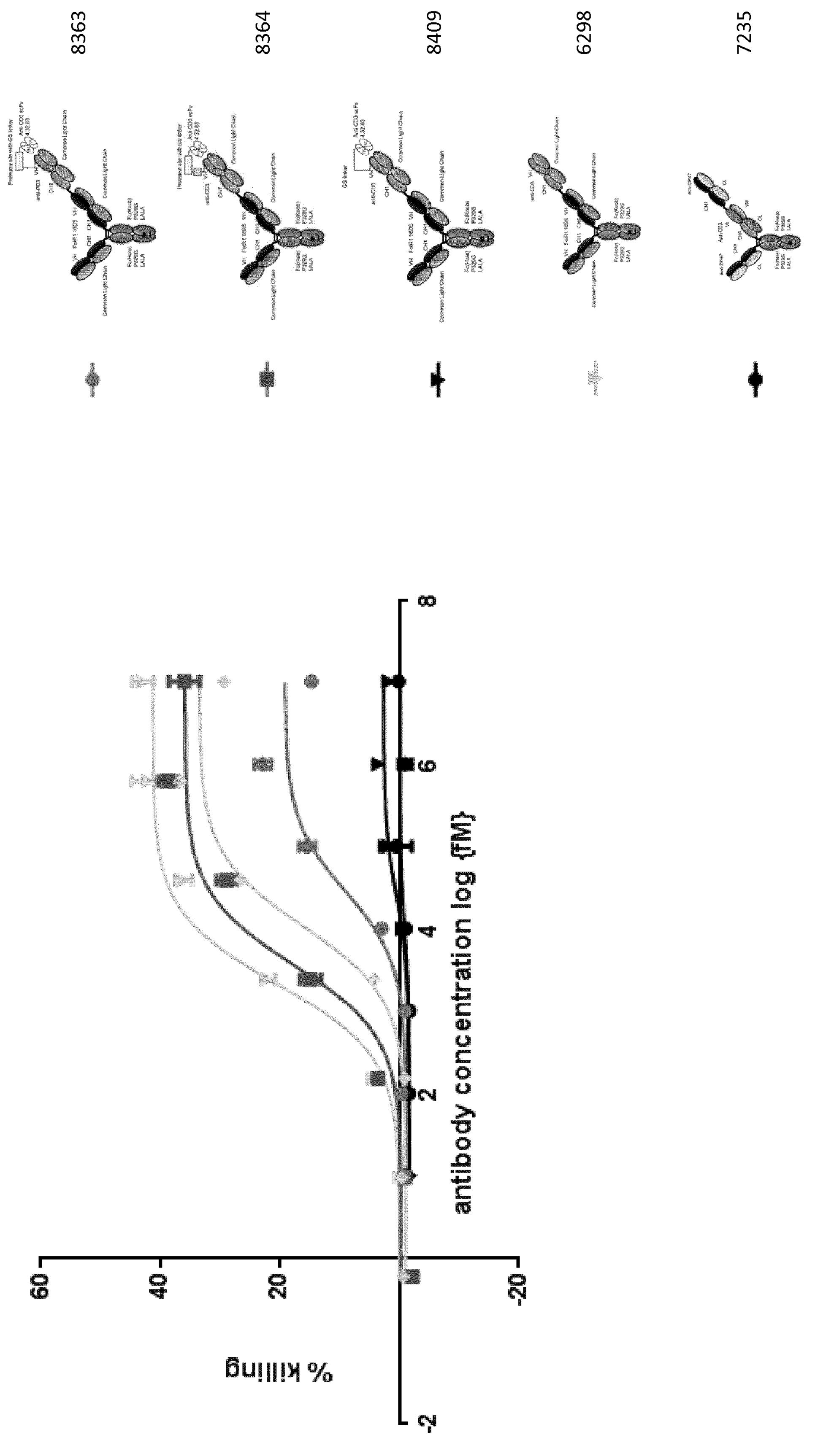
8408
8363
8364
8409
6298
7235
% killing
60
40
20
-20
-2
2
4
6
8
antibody concentration log (fM)

FIG. 38A

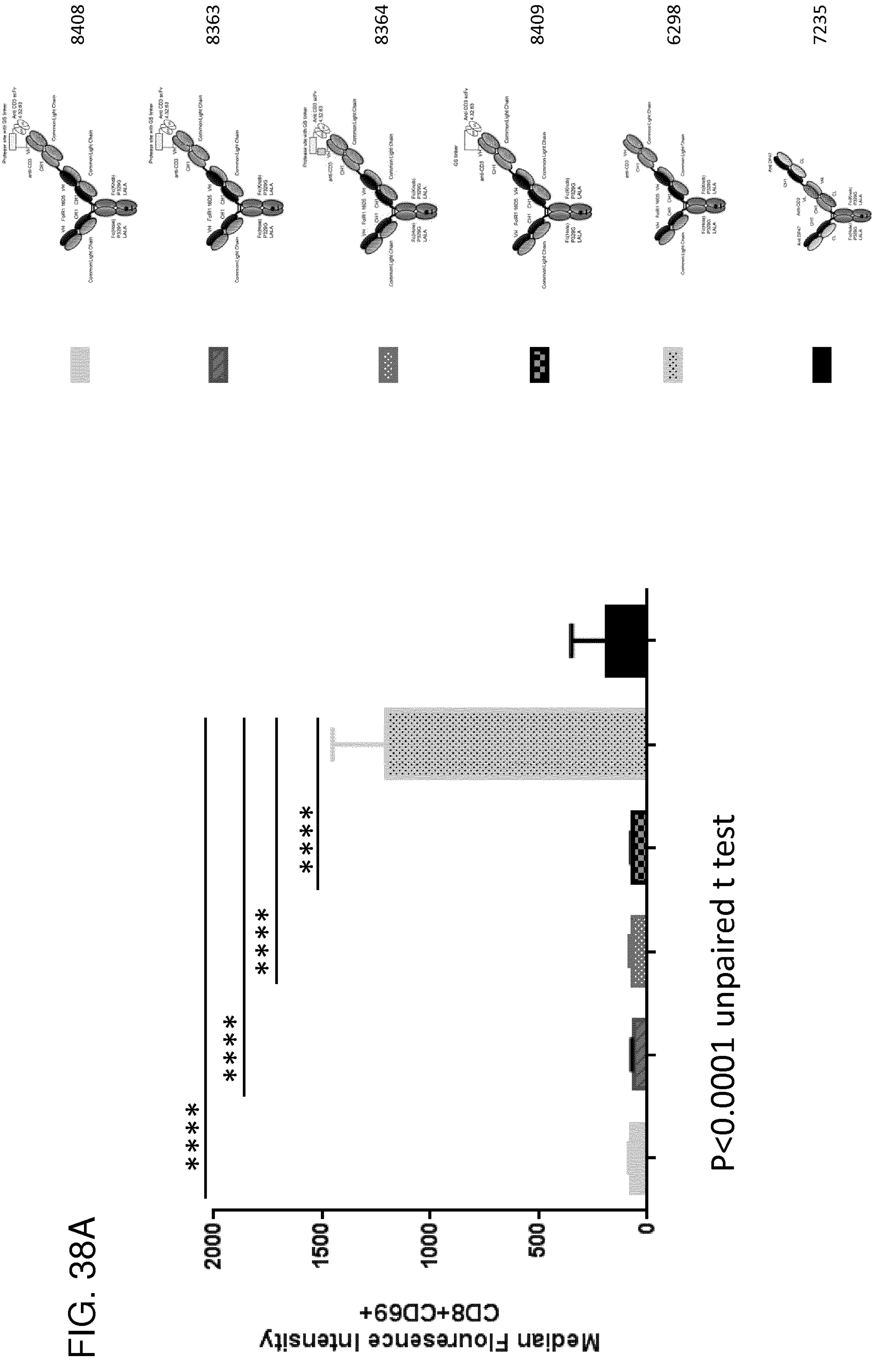
Median Flouresence Intensity
CD8+CD69+
2000
1500
1000
500
0
****
****
****
****
P<0.0001 unpaired t test
8408
8363
8364
8409
6298
7235

FIG. 38B

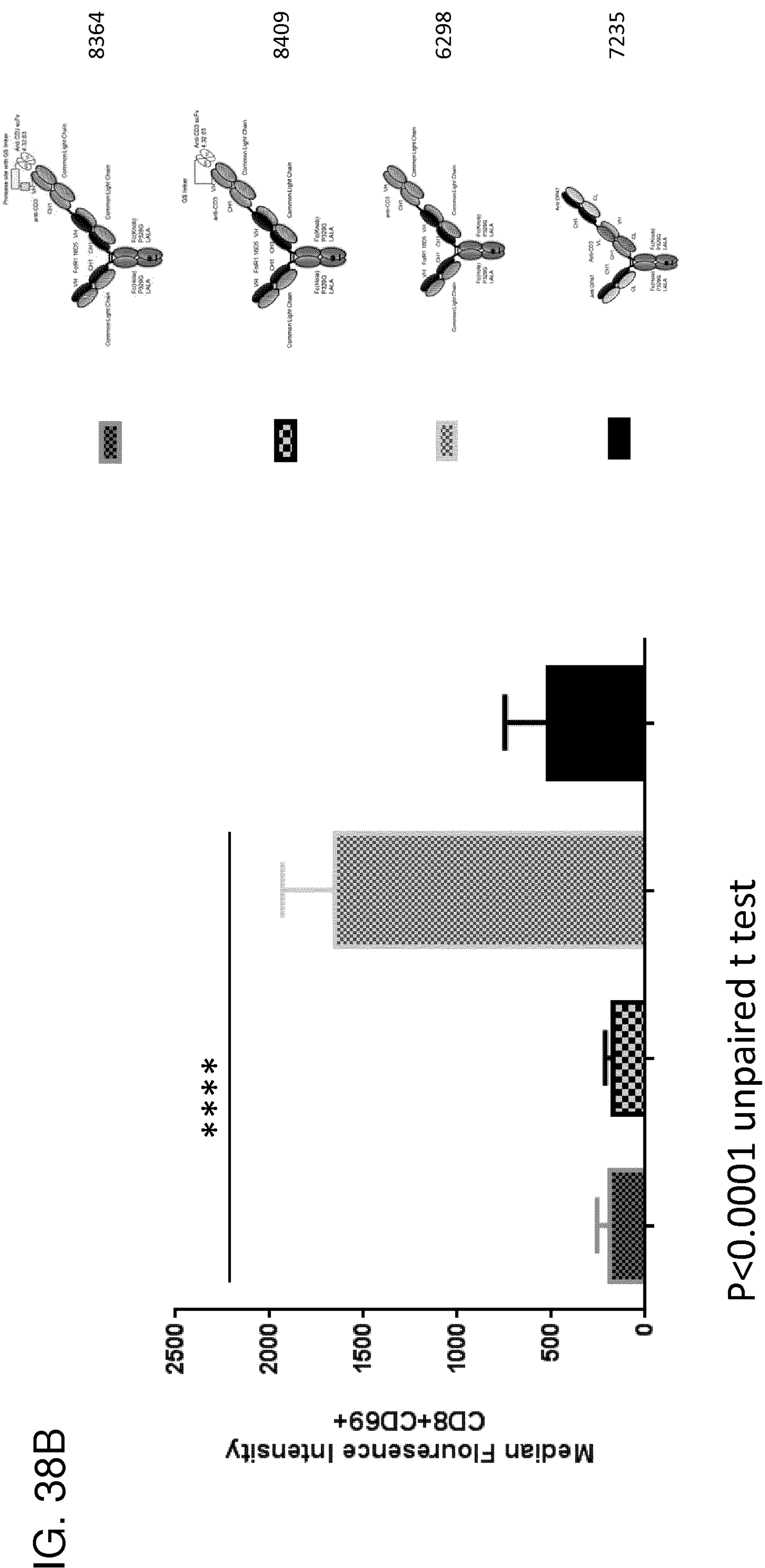
8364
8409
6298
7235
Median Flouresence Intensity
CD8+CD69+
2500
2000
1500
1000
500
0
****
P<0.0001 unpaired t test

FIG. 39A

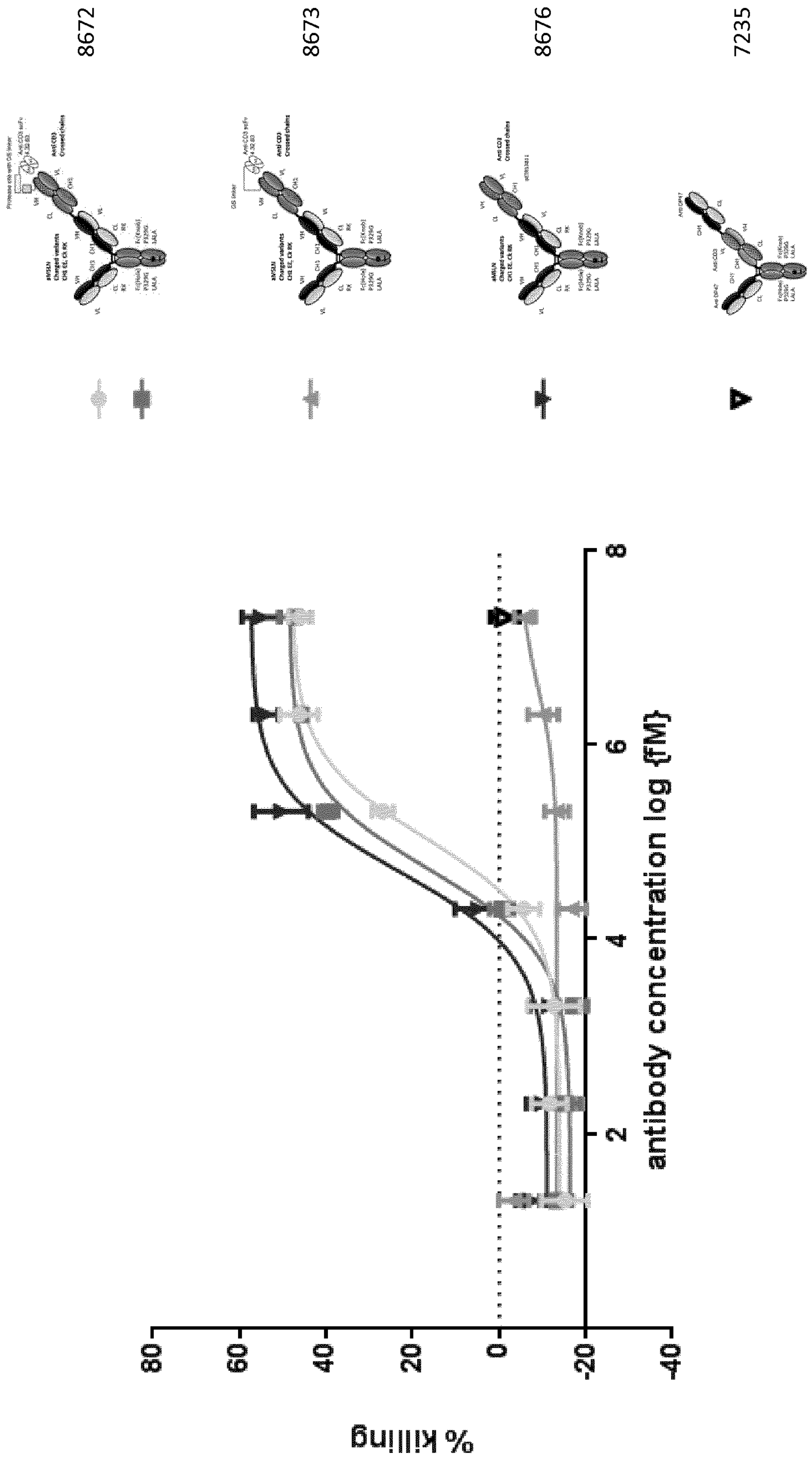

% killing
80
60
40
20
0
-20
-40
2
4
6
8
antibody concentration log {fM}
8672
8673
8676
7235

FIG. 39B

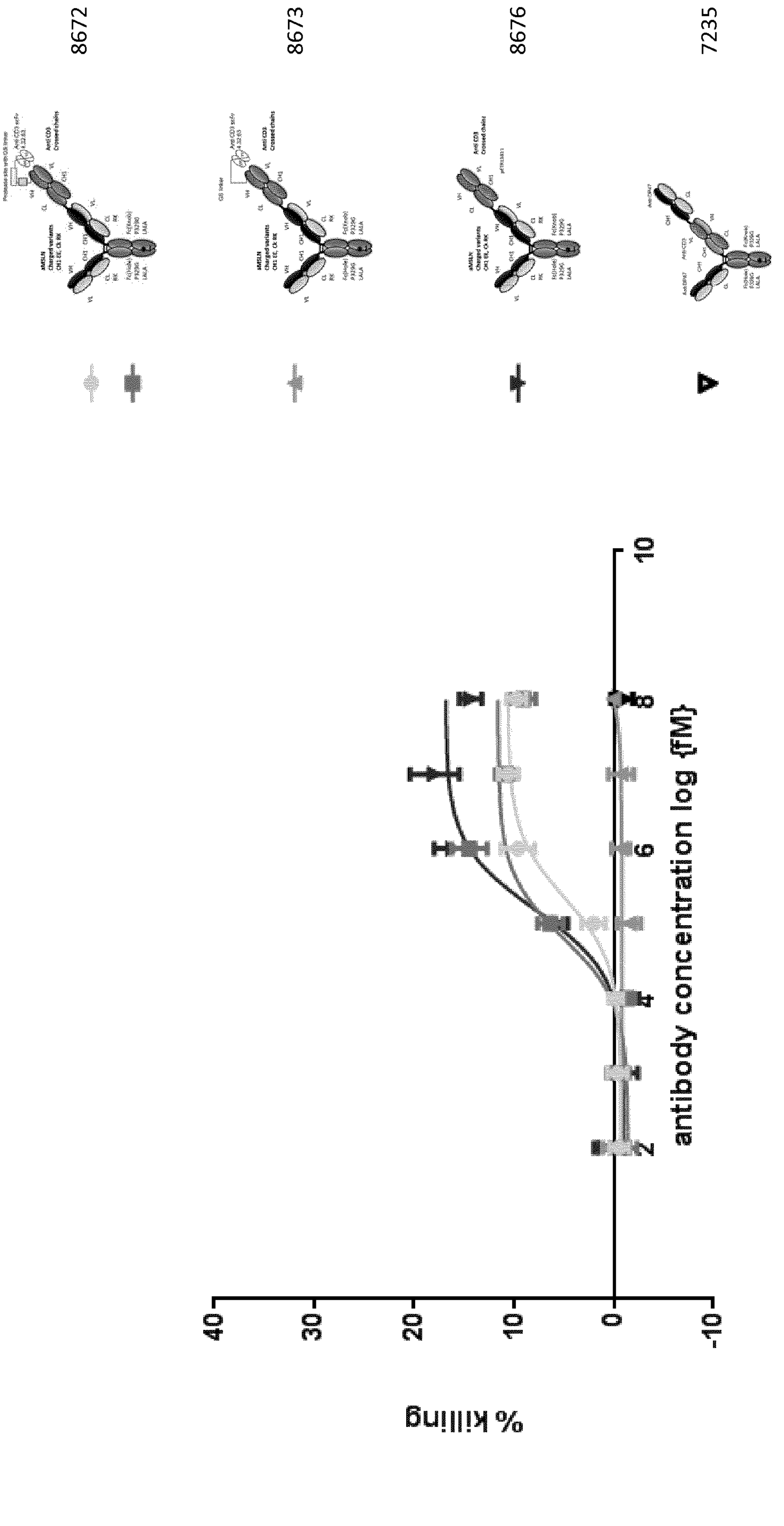
% killing
40
30
20
10
0
-10
2
4
6
8
10
antibody concentration log {fM}
8672
8673
8676
7235

FIG. 40

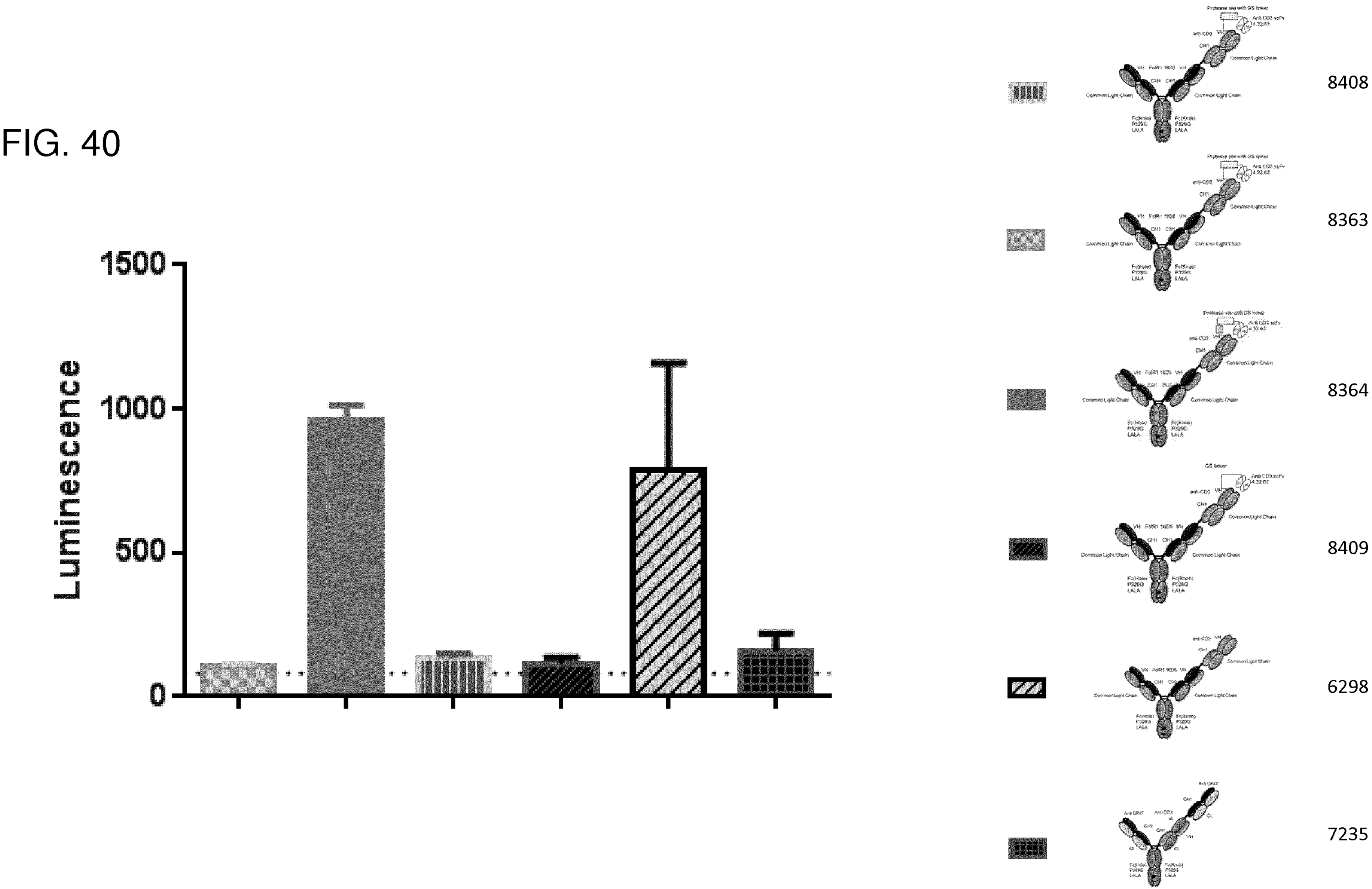

FIG. 41A

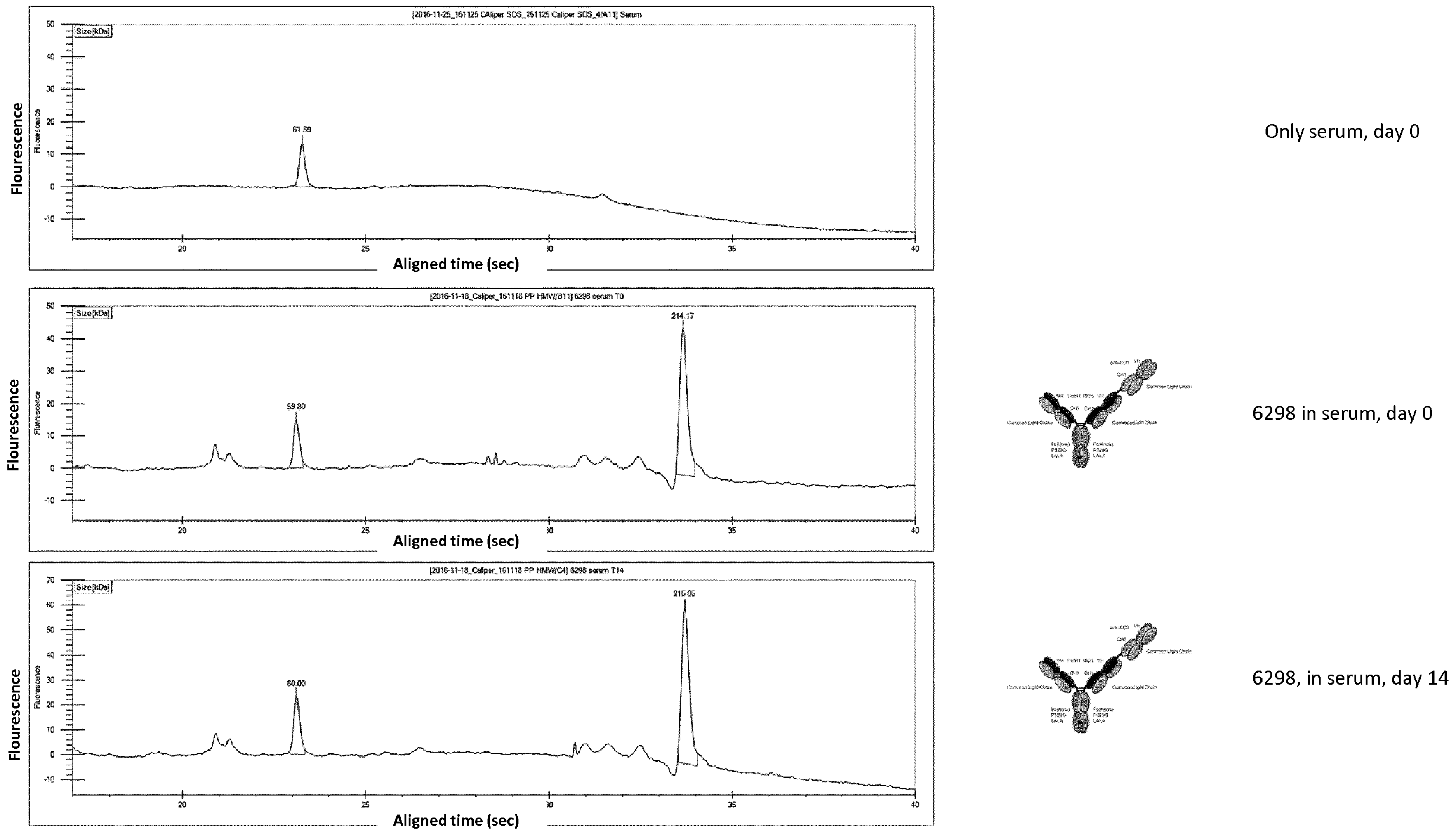
[2016-11-25_161125 CAliper SDS_161125 Caliper SDS_4/A11] Serum
61.59
Flourescence
Aligned time (sec)
Only serum, day 0
[2016-11-18_Caliper_161118 PP HMW/B11] 6298 serum T0
59.80
214.17
Flourescence
Aligned time (sec)
6298 in serum, day 0
[2016-11-18_Caliper_161118 PP HMW/C4] 6298 serum T14
60.00
215.05
Flourescence
Aligned time (sec)
6298, in serum, day 14

FIG. 41B

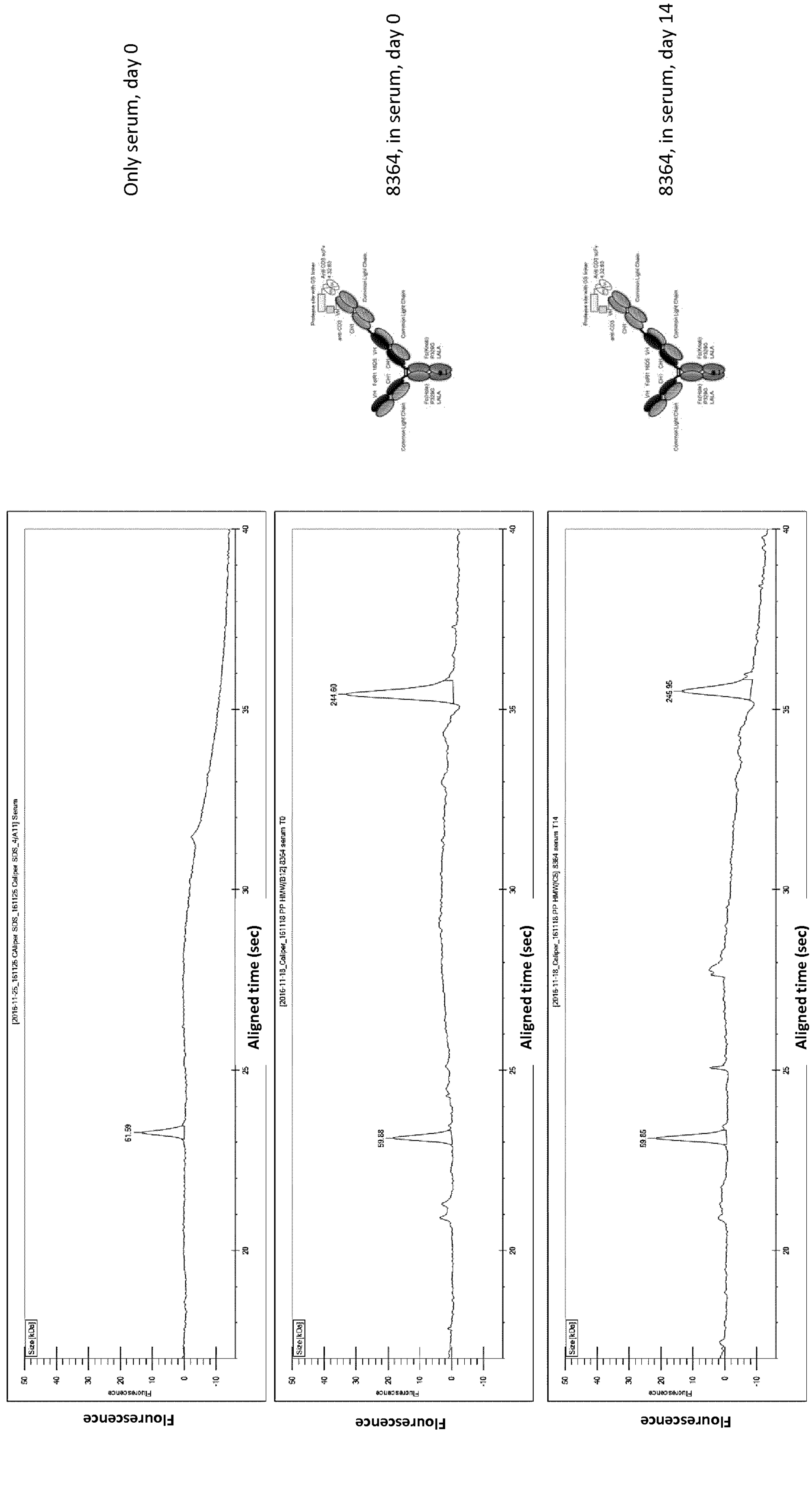
Only serum, day 0
8364, in serum, day 0
8364, in serum, day 14
Flourescence
Aligned time (sec)
61.59
59.88
244.60
59.85
245.95

FIG. 41C

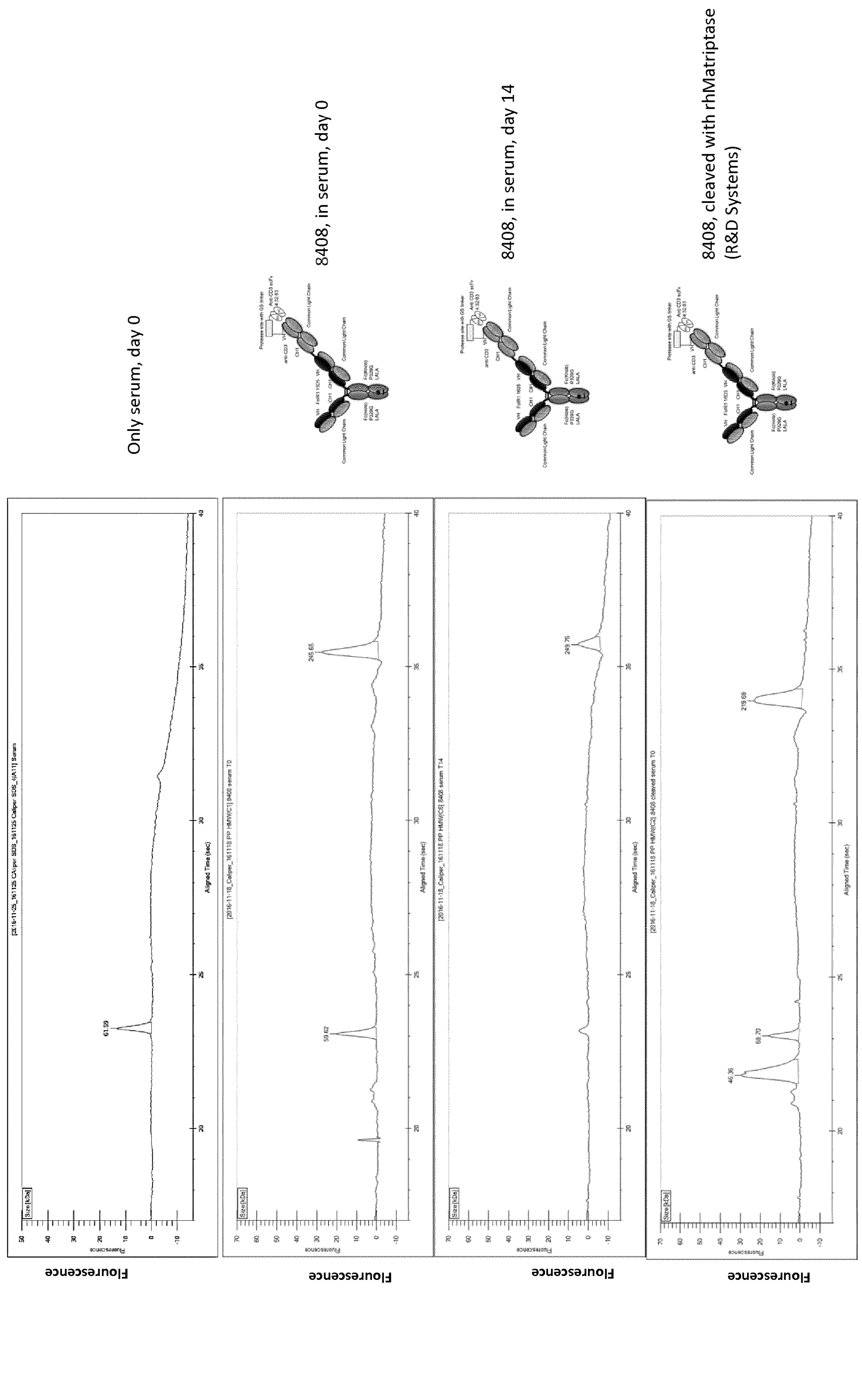
Only serum, day 0
8408, in serum, day 0
8408, in serum, day 14
8408, cleaved with rhMatriptase (R&D Systems)
61.59
59.62
245.65
249.75
46.36
59.70
219.69
Flourescence
Flourescence
Flourescence
Flourescence
Aligned time (sec)

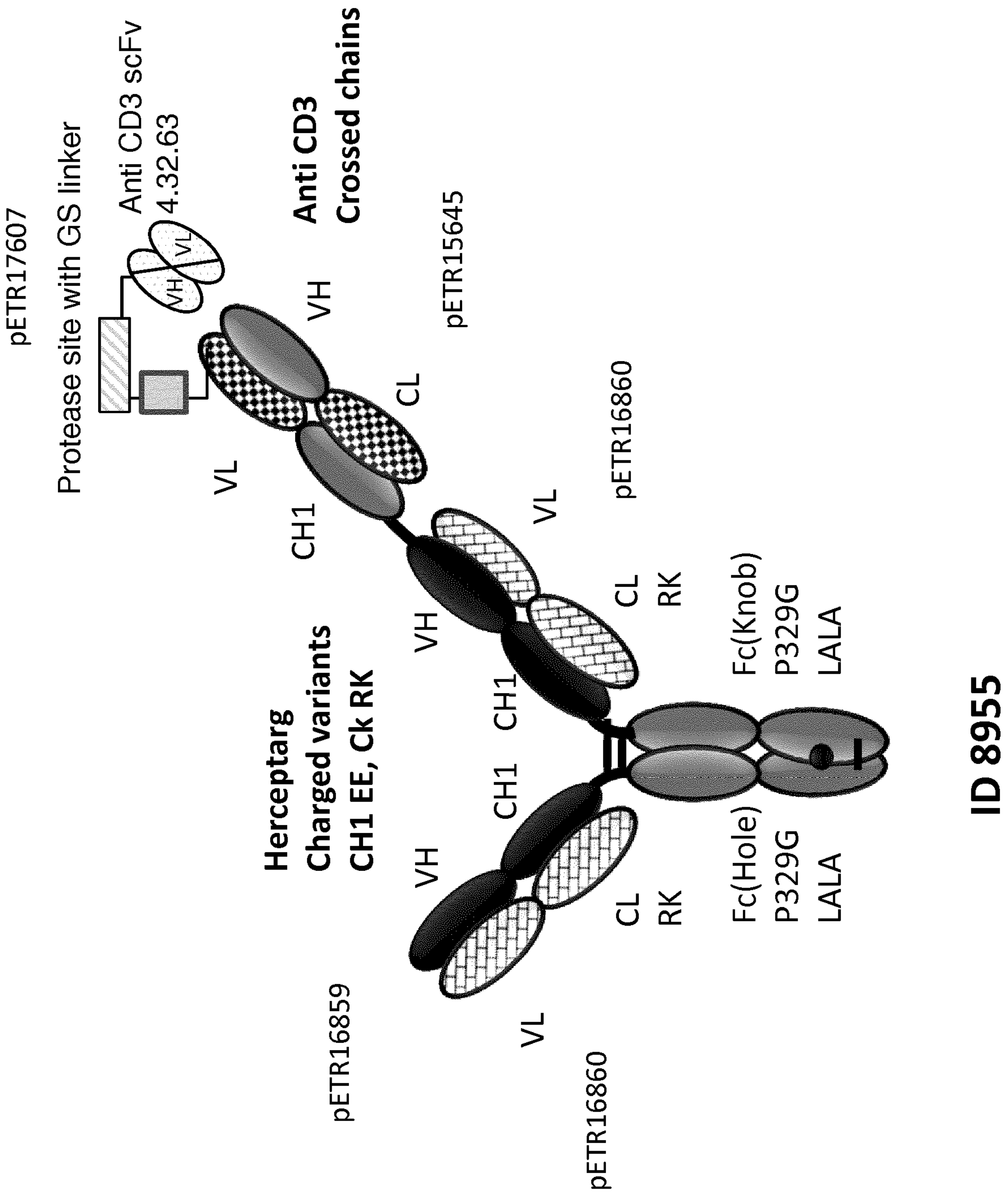
pETR17607
Protease site with GS linker
Anti CD3 scFv
4.32.63
VH
VL
Anti CD3
Crossed chains
pETR15645
VH
CL
VL
CH1
pETR16860
VL
VH
CL
RK
CH1
Fc(Knob)
P329G
LALA
Herceptarg
Charged variants
CH1 EE, Ck RK
CH1
VH
CL
RK
Fc(Hole)
P329G
LALA
VL
pETR16859
pETR16860
ID 8955

FIG. 42A

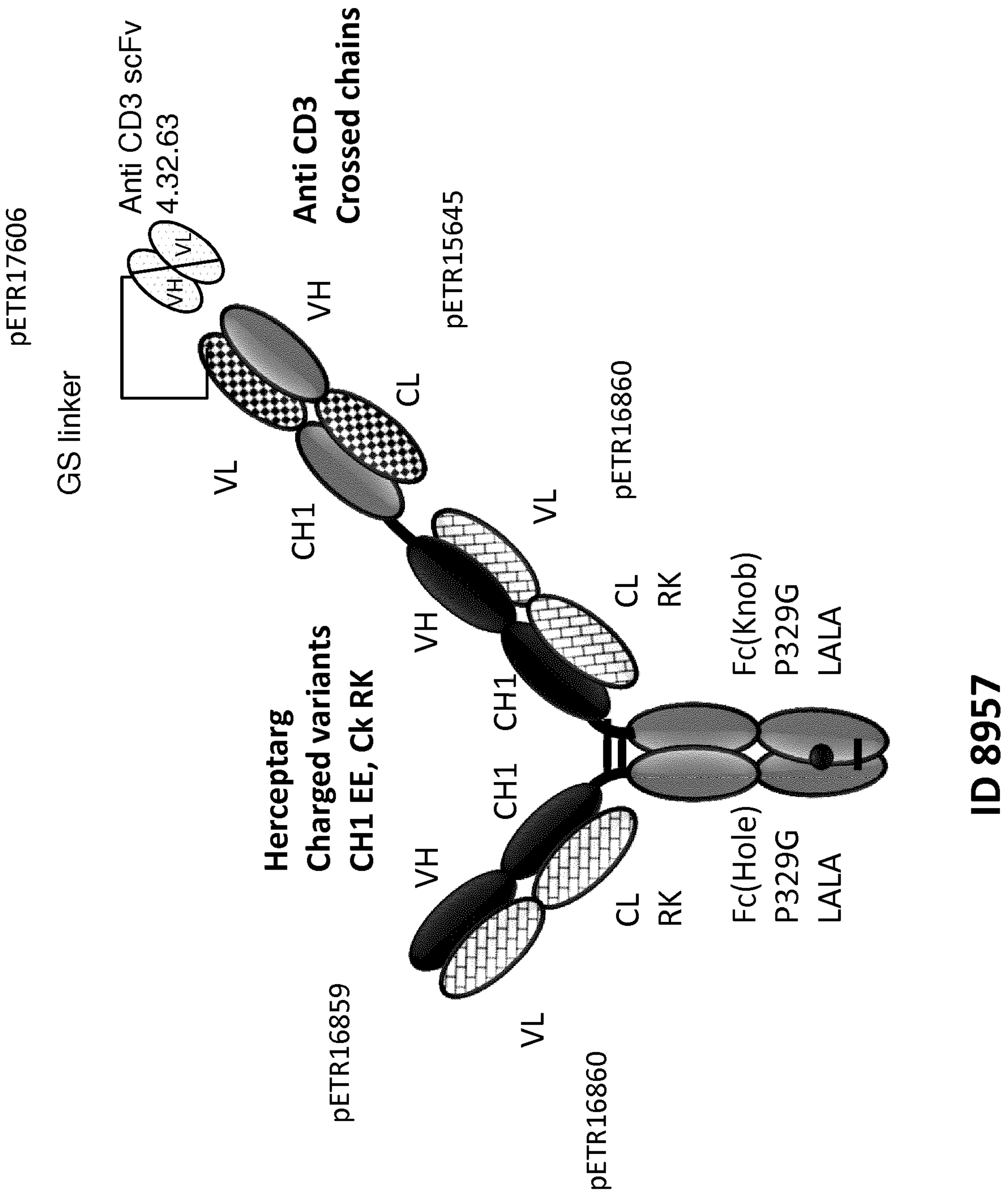
pETR17606
GS linker
Anti CD3 scFv
4.32.63
VH
VL
Anti CD3
Crossed chains
pETR15645
VH
CL
VL
CH1
pETR16860
VL
VH
CL
RK
Herceptarg
Charged variants
CH1 EE, Ck RK
CH1
CH1
Fc(Knob)
P329G
LALA
VH
CL
RK
Fc(Hole)
P329G
LALA
VL
pETR16859
pETR16860
ID 8957

FIG. 42B

FIG. 42C

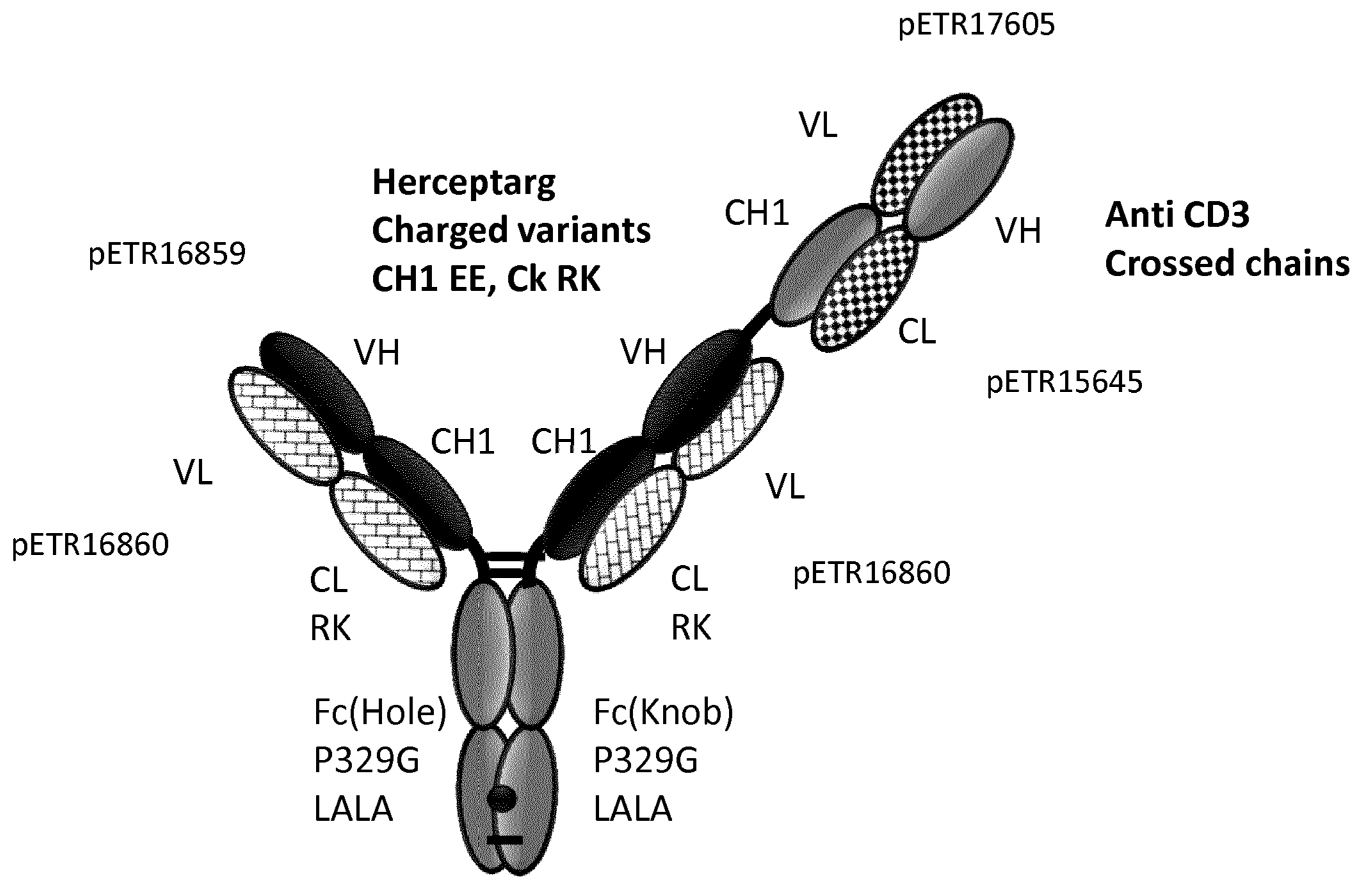


ID 8959

FIG. 42D

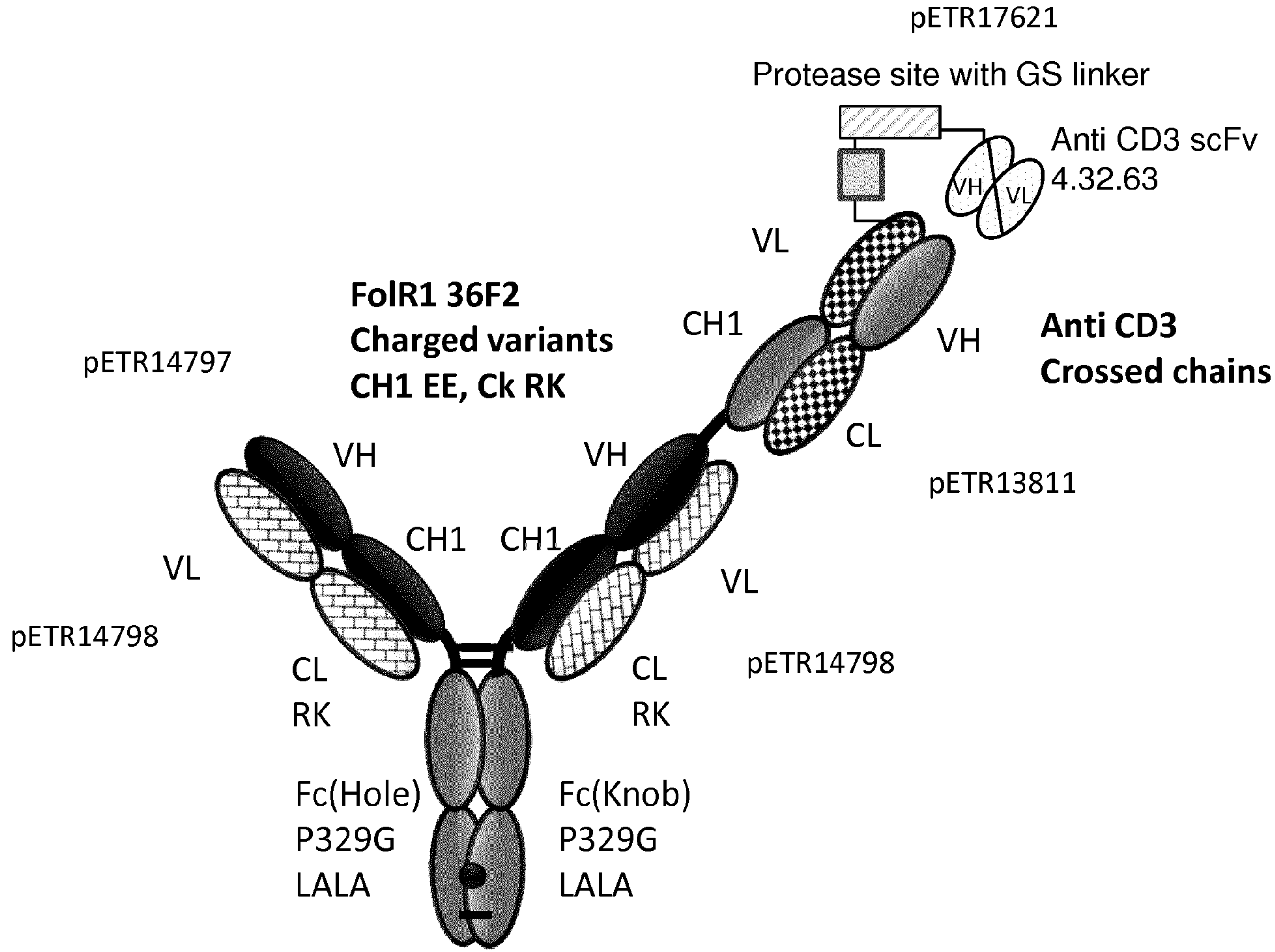


ID 8997

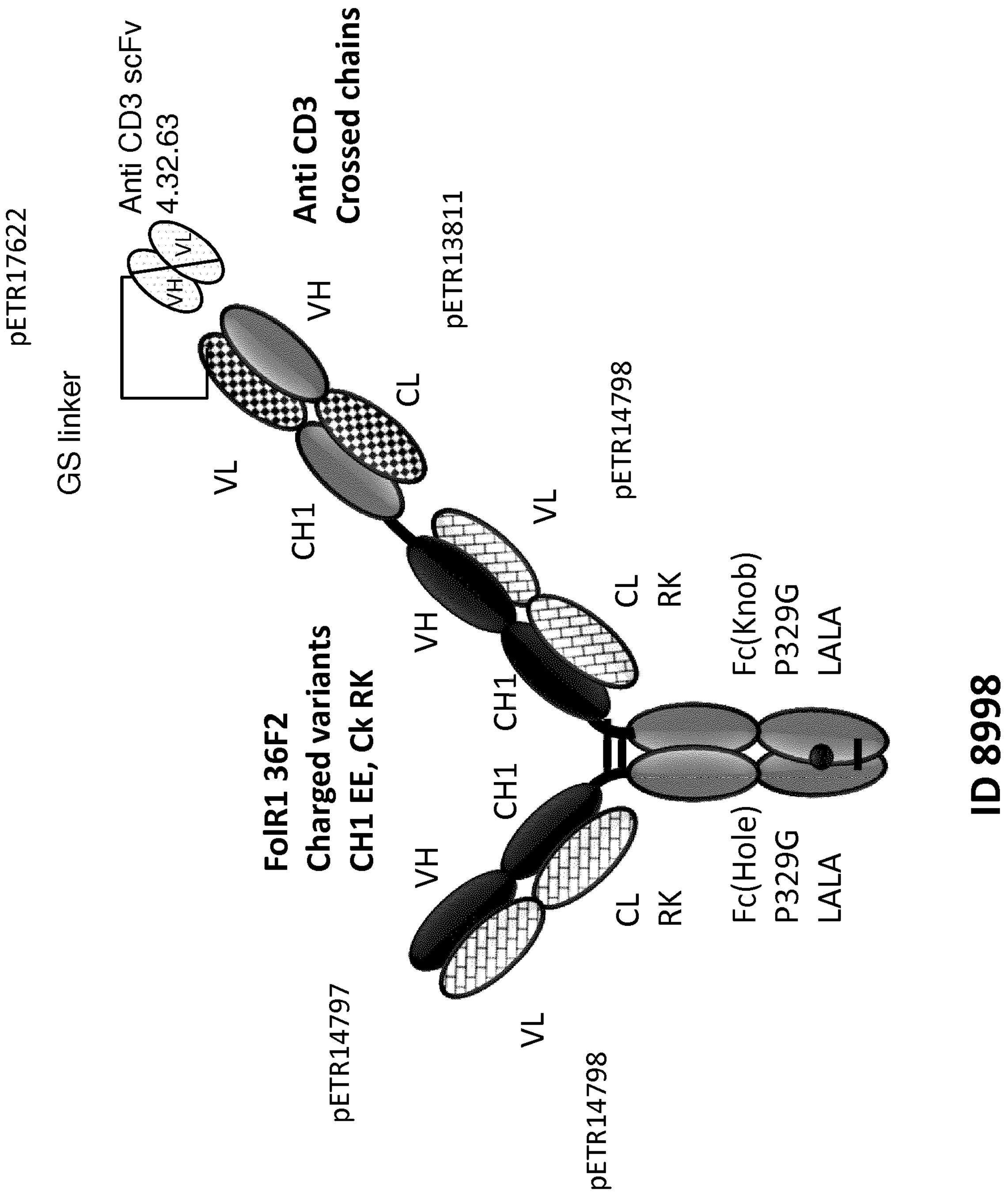
pETR17622
GS linker
VH
VL
Anti CD3 scFv
4.32.63
Anti CD3
Crossed chains
VH
pETR13811
CL
VL
CH1
pETR14798
VL
VH
CL
RK
CH1
Fc(Knob)
P329G
LALA
FolR1 36F2
Charged variants
CH1 EE, Ck RK
VH
CH1
CL
RK
Fc(Hole)
P329G
LALA
VL
pETR14797
pETR14798
ID 8998

FIG. 42E

FIG. 42F

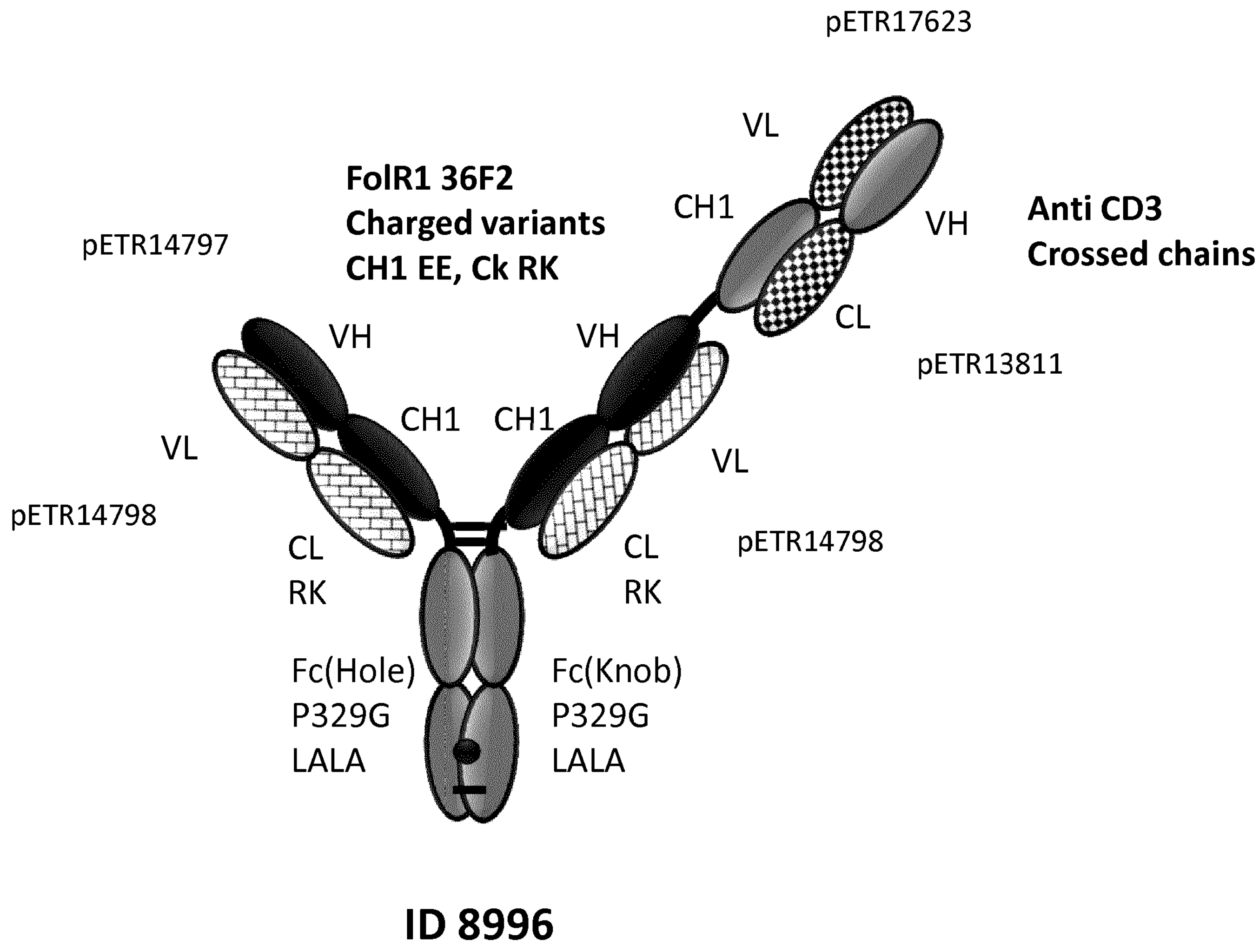
pETR17623
VL
FolR1 36F2
Charged variants
CH1 EE, Ck RK
CH1
Anti CD3
Crossed chains
VH
pETR14797
VH
VH
CL
pETR13811
CH1
CH1
VL
VL
pETR14798
CL
RK
pETR14798
CL
RK
Fc(Hole)
P329G
LALA
Fc(Knob)
P329G
LALA
ID 8996

FIG. 43

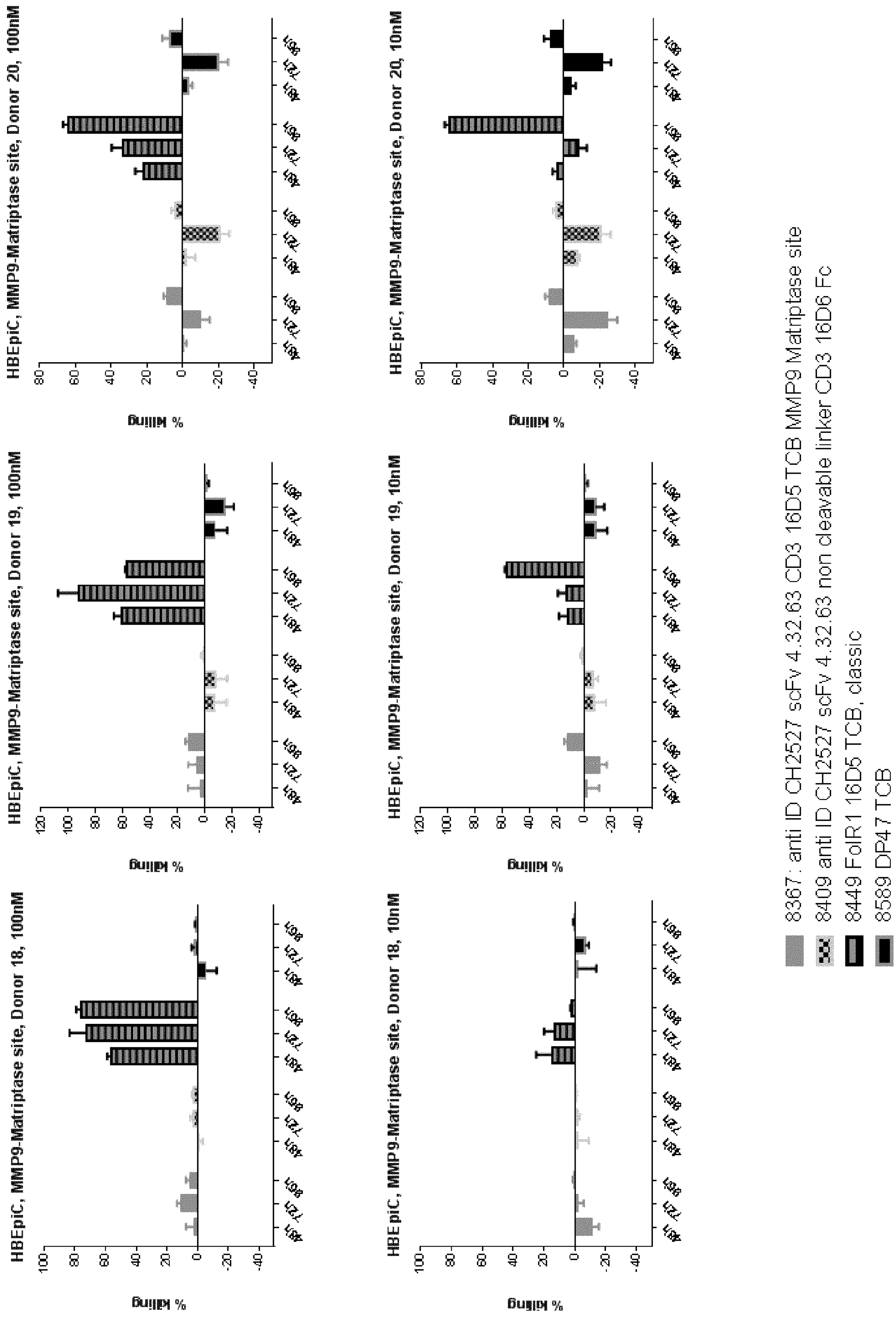
HBEpiC, MMP9-Matriptase site, Donor 18, 100nM
HBEpiC, MMP9-Matriptase site, Donor 19, 100nM
HBEpiC, MMP9-Matriptase site, Donor 20, 100nM
HBEpiC, MMP9-Matriptase site, Donor 18, 10nM
HBEpiC, MMP9-Matriptase site, Donor 19, 10nM
HBEpiC, MMP9-Matriptase site, Donor 20, 10nM
% killing
48h
72h
96h
8367: anti ID CH2527 scFv 4.32.63 CD3 16D5 TCB MMP9 Matriptase site
8409 anti ID CH2527 scFv 4.32.63 non cleavable linker CD3 16D6 Fc
8449 FolR1 16D5 TCB, classic
8589 DP47 TCB

FIG. 44

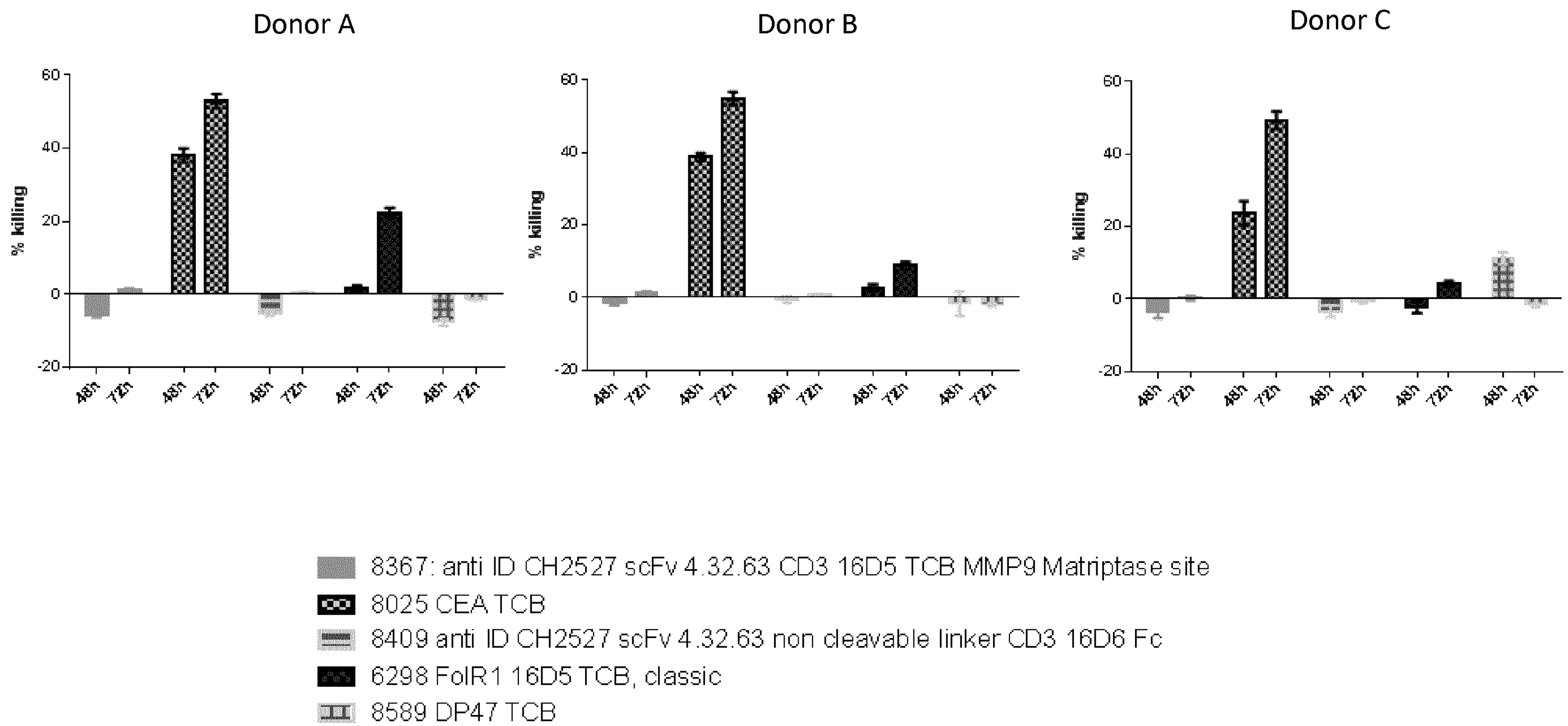
Donor A
Donor B
Donor C
% killing
60
40
20
0
-20
48h
72h
8367: anti ID CH2527 scFv 4.32.63 CD3 16D5 TCB MMP9 Matriptase site
8025 CEA TCB
8409 anti ID CH2527 scFv 4.32.63 non cleavable linker CD3 16D6 Fc
6298 FolR1 16D5 TCB, classic
8589 DP47 TCB

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016014974 A
- WO 2006082515 A
- WO 9316185 A
- US 5571894 A
- US 5587458 A
- US 5869046 A
- EP 404097 A
- WO 199301161 A
- US 6248516 B1
- WO 2012130831 A
- US 5731168 A
- US 7695936 B
- WO 2009089004 A
- US 6737056 B
- US 7332581 B
- US 5500362 A
- US 5821337 A
- WO 2006029879 A
- WO 2005100402 A
- WO 2013128194 A
- US 5959177 A
- US 6040498 A
- US 6420548 B
- US 7125978 B
- US 6417429 B
- US 4186567 A
- US 5969108 A, McCafferty
- US 5565332 A, Winter
- US 7527791 B
- US 6982321 B
- US 7087409 B
- US 20040132066 A
- US 6054297 A


### Non-patent literature cited in the description


- **NAGORSEN ; BÄUERLE.** *Exp Cell Res,* 2011, vol. 317, 1255-1260
- **HOLLIGER et al.** *Prot Eng,* 1996, vol. 9, 299-305
- **KIPRIYANOV et al.** *J Mol Biol,* 1999, vol. 293, 41-66
- **MOORE et al.** *Blood,* 2011, vol. 117, 4542-51
- **SEIMETZ et al.** *Cancer Treat Rev,* 2010, vol. 36, 458-467
- **CARRENO et al.** *Hum Immunol,* 1991, vol. 33 (4), 249-58
- **LILJEBLAD et al.** *Glyco J,* 2000, vol. 17, 323-329
- **HEELEY.** *Endocr Res,* 2002, vol. 28, 217-229
- **HUDSON et al.** *Nat Med,* 2003, vol. 9, 129-134
- **PLÜCKTHUN.** The Pharmacology of Monoclonal Antibodies. Springer-Verlag, 1994, vol. 113, 269-315
- **HOLLINGER et al.** *Proc Natl Acad Sci USA,* 1993, vol. 90, 6444-6448
- **KINDT et al.** Kuby Immunology. W.H. Freeman and Co, 2007, 91
- **KABAT et al.** Sequences of Proteins of Immunological Interest. U.S. Dept. of Health and Human Services, 1983
- **CHOTHIA et al.** *J Mol Biol,* 1987, vol. 196, 901-917
- **KABAT et al.** Sequence of Proteins of Immunological Interest. U.S. Dept. of Health and Human Services, 1983
- **KABAT et al.** Sequences of Proteins of Immunological Interest. Public Health Service, National Institutes of Health, 1991
- **STUBENRAUCH et al.** *Drug Metabolism and Disposition,* 2010, vol. 38, 84-91
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **HELLSTROM et al.** *Proc Natl Acad Sci USA,* 1986, vol. 83, 7059-7063
- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **GAZZANO-SANTORO et al.** *J Immunol Methods,* 1996, vol. 202, 163
- **CRAGG et al.** *Blood,* 2003, vol. 101, 1045-1052
- **CRAGG ; GLENNIE.** *Blood,* 2004, vol. 103, 2738-2743

- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates and Wiley Interscience, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** *Annals N.Y. Acad Sci,* 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front Biosci,* 2008, vol. 13, 1619-1633
- **RIECHMANN et al.** *Nature,* 1988, vol. 332, 323-329
- **QUEEN et al.** *Proc Natl Acad Sci USA,* 1989, vol. 86, 10029-10033
- **JONES et al.** *Nature,* 1986, vol. 321, 522-525
- **MORRISON et al.** *Proc Natl Acad Sci,* 1984, vol. 81, 6851-6855
- **MORRISON ; OI.** *Adv Immunol,* 1988, vol. 44, 65-92
- **VERHOEYEN et al.** *Science,* 1988, vol. 239, 1534-1536
- **PADLAN.** *Molec Immun,* 1994, vol. 31 (3), 169-217
- **KASHMIRI et al.** *Methods,* 2005, vol. 36, 25-34
- **PADLAN.** *Mol Immunol,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br J Cancer,* 2000, vol. 83, 252-260
- **VAN DIJK,VAN DE WINKEL.** *Curr Opin Pharmacol,* 2001, vol. 5, 368-74
- **LONBERG.** *Curr Opin Immunol,* 2008, vol. 20, 450-459
- Monoclonal Antibody Production Techniques and Applications. Marcel Dekker, Inc, 1987, 51-63
- **LONBERG.** *Nat Biotech,* 2005, vol. 23, 1117-1125
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- **CHEN et al.** *J Mol Biol,* 1999, vol. 293, 865-881
- Remington's Pharmaceutical Sciences. Mack Printing Company, 1990
- Remington's Pharmaceutical Sciences. Mack Printing Company, 1990, 1289-1329
- **FINGL et al.** The Pharmacological Basis of Therapeutics. 1975, 1